(19) Europäisches Patentamt
European Patent Office
Office européen des brevets

(11) EP 4 741 396 A1

(12) EUROPEAN PATENT APPLICATION
published in accordance with Art. 153(4) EPC

(43) Date of publication:
**13.05.2026 Bulletin 2026/20**

(21) Application number: **24839454.6**

(22) Date of filing: **21.06.2024**

(51) International Patent Classification (IPC):
*C07D 491/048* (2006.01) *C07B 61/00* (2006.01)
*C09K 11/06* (2006.01) *H10K 50/12* (2023.01)
*H10K 59/12* (2023.01) *H10K 59/60* (2023.01)
*H10K 59/65* (2023.01) *H10K 59/95* (2026.01)
*H10K 85/60* (2023.01) *H10K 101/30* (2023.01)
*H10K 101/40* (2023.01)

(52) Cooperative Patent Classification (CPC):
**C07B 61/00; C07D 491/048; C09K 11/06; H10K 50/12; H10K 59/12; H10K 59/60; H10K 59/65; H10K 59/95; H10K 85/60;**
H10K 2101/30; H10K 2101/40

(86) International application number:
**PCT/JP2024/022486**

(87) International publication number:
**WO 2025/013558 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.07.2023 JP 2023112257**
**30.05.2024 JP 2024088438**

(71) Applicant: **Canon Kabushiki Kaisha**
**Tokyo, 146-8501 (JP)**

(72) Inventors:
- **YAMADA, Takayoshi**
**Tokyo 146-8501 (JP)**
- **ITO, Yuto**
**Tokyo 146-8501 (JP)**
- **FUJISAWA, Kaori**
**Tokyo 146-8501 (JP)**

(74) Representative: **TBK**
**Bavariaring 4-6**
**80336 München (DE)**

(54) **ORGANIC COMPOUND AND ORGANIC LIGHT-EMITTING ELEMENT**

(57) The present disclosure provides an organic compound represented by general formula (1-1) or (1-2).

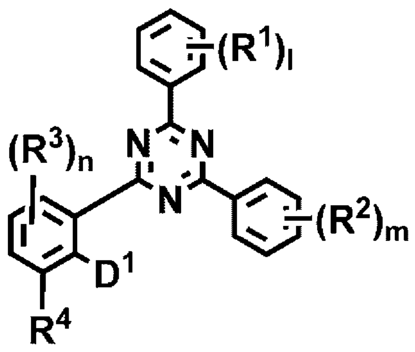
(1-1)

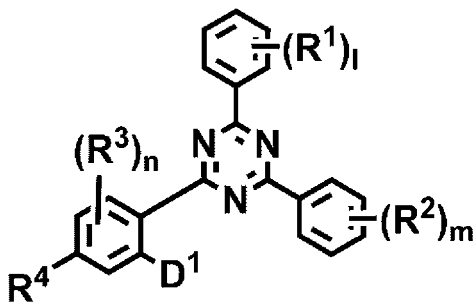
(1-2)

In general formulas (1-1) and (1-2), $R^1$ to $R^3$ are each a deuterium atom or a substituent. $R^4$ is an alkyl group or an alkoxy group. 1 and m are each an integer of 0 to 5, and n is an integer of 0 to 3. $D^1$ is represented by general formula (2).

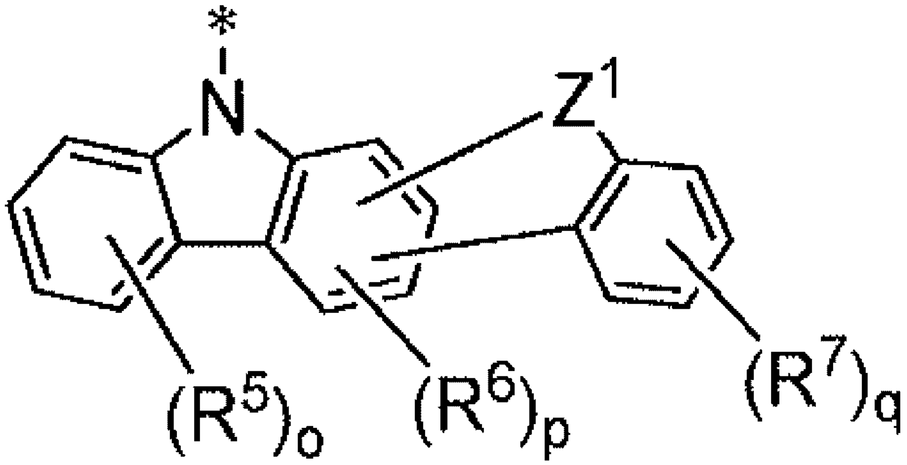

(2)

In general formula (2), $R^5$ to $R^7$ are each a deuterium atom or a substituent. o and q are each an integer of 0 to 4, and p is an integer of 0 to 2. $Z^1$ is an oxygen atom, a sulfur atom, a selenium atom, $NR^8$, or $CR^9R^{10}$. $R^8$ to $R^{10}$ are each a hydrogen atom, a deuterium atom, or a substituent. * represents a position of bonding to general formula (1-1) or (1-2).

## Description

Technical Field

The present invention relates to an organic compound and an organic light-emitting element including the organic compound.

Background Art

An organic light-emitting element (hereinafter also referred to as an "organic electroluminescent element" or an "organic EL element") is an electronic element including a pair of electrodes and an organic compound layer disposed between the electrodes. By injecting electrons and holes into the organic compound layer through the pair of electrodes, excitons of a luminescent organic compound in the organic compound layer are generated, and the organic light-emitting element emits light when the excitons return to their ground state.

Compounds suitable for organic light-emitting elements have been actively created to date. PTLs 1 to 3 respectively disclose compound Ref-1 to compound Ref-3 as donor-acceptor type organic compounds having a donor moiety and an acceptor moiety.

[Chem. 1]

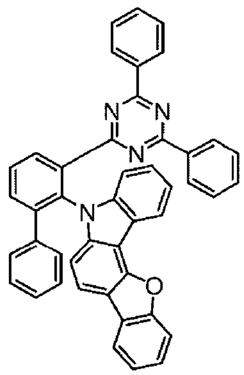

Compound Ref-1

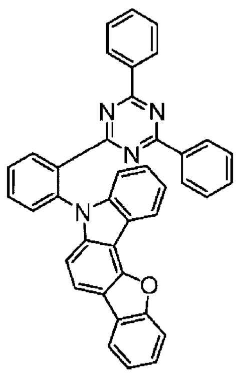

Compound Ref-2

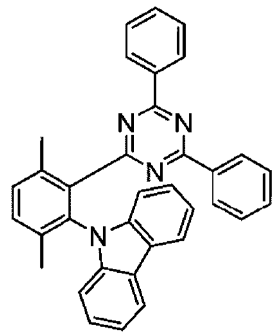

Compound Ref-3

Citation List

Patent Literature

PTL 1: Japanese Patent Laid-Open No. 2022-019638
PTL 2: International Publication No. 2014/092083
PTL 3: International Publication No. 2016/159479

Summary of Invention

Technical Problem

However, the above compounds each have a large $\Delta E_{ST}$, which is the difference between a lowest excited singlet energy (S1) and a lowest excited triplet energy (T1), and thus have room for improvement.

The present invention has been made in view of the above problem, and an object thereof is to provide an organic compound having a smaller $\Delta E_{ST}$.

Solution to Problem

An organic compound according to the present invention is represented by general formula (1-1) or (1-2).

[Chem. 2]

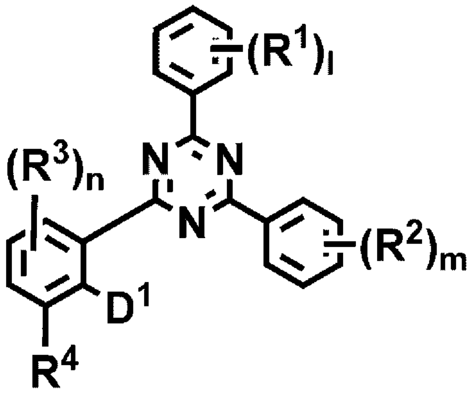

(1-1)

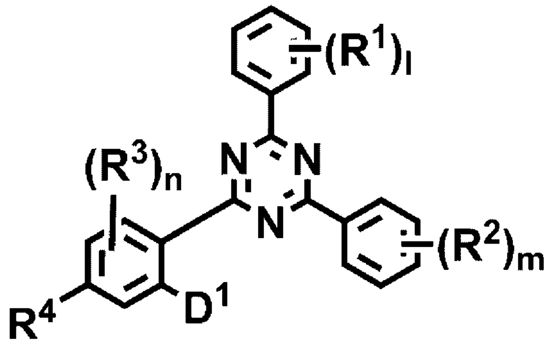

(1-2)

In general formulas (1-1) and (1-2), $R^1$ to $R^3$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. $R^4$ is a substituted or unsubstituted alkyl group or a substituted or unsubstituted alkoxy group. l and m are each an integer of 0 to 5, and n is an integer of 0 to 3. A plurality of $R^1$'s may be the same as or different from each other, a plurality of $R^2$'s may be the same as or different from each other, and a plurality of $R^3$'s may be the same as or different from each other. $D^1$ is represented by general formula (2).

[Chem. 3]

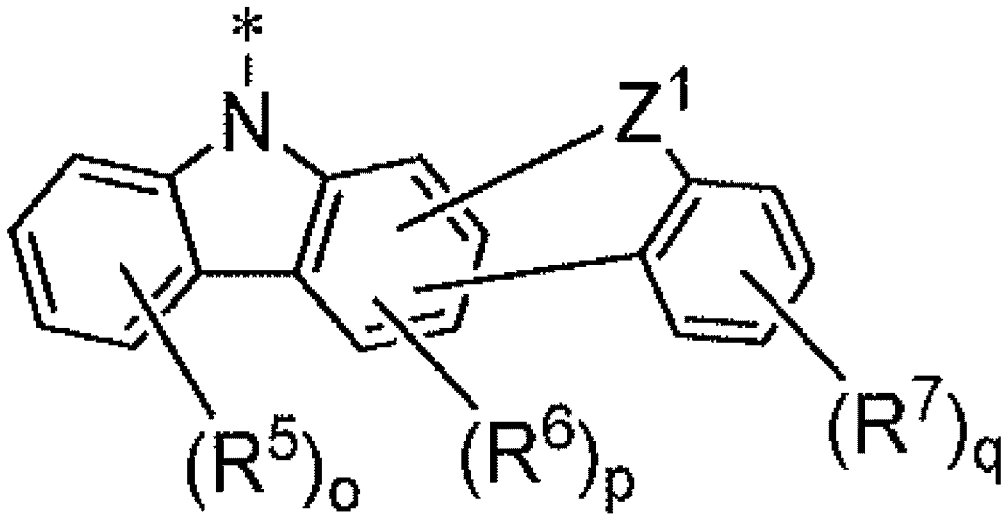

(2)

In general formula (2), $R^5$ to $R^7$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. o and q are each an integer of 0 to 4, and p is an integer of 0 to 2. A plurality of $R^5$'s may be the same as or different from each other, a plurality of $R^6$'s may be the same as or different from each other, and a plurality of $R^7$'s may be the same as or different from each other. $Z^1$ is an oxygen atom, a sulfur atom, a

selenium atom, $NR^8$, or $CR^9R^{10}$. $R^8$ to $R^{10}$ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. * represents a position of bonding to general formula (1-1) or (1-2).

Advantageous Effects of Invention

According to the present invention, an organic compound having a smaller $\Delta E_{ST}$ can be provided.

Brief Description of Drawings

[Fig. 1A] Fig. 1A is a schematic sectional view showing an example of a pixel of a display apparatus according to an embodiment of the present invention.
[Fig. 1B] Fig. 1B is a schematic sectional view of an example of a display apparatus including an organic light-emitting element according to an embodiment of the present invention.
[Fig. 2] Fig. 2 is a schematic view showing an example of a display apparatus according to an embodiment of the present invention.
[Fig. 3A] Fig. 3A is a schematic view showing an example of an image pickup apparatus according to an embodiment of the present invention.
[Fig. 3B] Fig. 3B is a schematic view showing an example of an electronic apparatus according to an embodiment of the present invention.
[Fig. 4A] Fig. 4A is a schematic view showing an example of a display apparatus according to an embodiment of the present invention.
[Fig. 4B] Fig. 4B is a schematic view showing an example of a foldable display apparatus.
[Fig. 5A] Fig. 5A is a schematic view showing an example of a lighting apparatus according to an embodiment of the present invention.
[Fig. 5B] Fig. 5B is a schematic view showing an example of an automobile including a vehicle lighting fixture according to an embodiment of the present invention.
[Fig. 6A] Fig. 6A is a schematic view showing an example of a wearable device according to an embodiment of the present invention.
[Fig. 6B] Fig. 6B is a schematic view of an example of a wearable device according to an embodiment of the present invention, the wearable device including an image pickup apparatus.
[Fig. 7A] Fig. 7A is a schematic view showing an example of an image-forming apparatus according to an embodiment of the present invention.
[Fig. 7B] Fig. 7B is a schematic view showing an example of an exposure light source of an image-forming apparatus according to an embodiment of the present invention.
[Fig. 7C] Fig. 7C is a schematic view showing an example of an exposure light source of an image-forming apparatus according to an embodiment of the present invention.
[Fig. 8] Fig. 8 compares the HOMO orbital distribution and LUMO orbital distribution of organic compounds according to the present invention and comparative compounds.
[Fig. 9] Fig. 9 compares the HOMO orbital distribution and LUMO orbital distribution of organic compounds according to the present invention.

Description of Embodiments

In this specification, examples of halogen atoms include a fluorine atom, a chlorine atom, a bromine atom, and an iodine atom, but are not limited thereto.
Alkyl groups may have 1 to 40 carbon atoms, 1 to 20 carbon atoms, or 1 to 10 carbon atoms. Specific examples include a methyl group, an ethyl group, a normal propyl group, an isopropyl group, a normal butyl group, a tertiary butyl group, a secondary butyl group, an octyl group, a cyclohexyl group, a tertiary pentyl group, a 3-methylpentan-3-yl group, a 1-adamantyl group, and a 2-adamantyl group, but are not limited thereto.
Aryl groups may have 6 to 20 carbon atoms or 6 to 12 carbon atoms. Specific examples include a phenyl group, a naphthyl group, an indenyl group, a biphenyl group, a terphenyl group, a fluorenyl group, a phenanthryl group, a triphenylenyl group, a pyrenyl group, an anthranil group, a perylenyl group, a chrysenyl group, and a fluoranthenyl

group, but are not limited thereto.

Heterocyclic groups may have 3 to 24 carbon atoms, 3 to 18 carbon atoms, or 3 to 12 carbon atoms. Specific examples include a pyridyl group, a pyrimidyl group, a pyrazyl group, a triazyl group, a benzofuranyl group, a benzothiophenyl group, a dibenzofuranyl group, a dibenzothiophenyl group, an oxazolyl group, an oxadiazolyl group, a thiazolyl group, a thiadiazolyl group, a carbazolyl group, an acridinyl group, and a phenanthrolyl group, but are not limited thereto.

Amino groups may be substituted amino groups substituted with an alkyl group or an aryl group, or substituted amino group substituted with an alkyl group having 1 to 4 carbon atoms or an aryl group having 6 to 12 carbon atoms. Specific examples include an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, N,N-dimesitylamino group, an N-phenyl-N-(4-tertiary butylphenyl)amino group, an N-phenyl-N-(4-trifluoromethylphenyl)amino group, and an N-piperidyl group, but are not limited thereto.

Alkoxy groups may have 1 to 40 carbon atoms, 1 to 20 carbon atoms, or 1 to 10 carbon atoms. Specific examples include a methoxy group, an ethoxy group, a propoxy group, a 2-ethyl-octyloxy group, and a benzyloxy group, but are not limited thereto.

Specific examples of aryloxy groups include a phenoxy group, but are not limited thereto.

Specific examples of heteroaryloxy groups include a thienyloxy group, but are not limited thereto.

Specific examples of silyl groups include a trimethylsilyl group and a triphenylsilyl group, but are not limited thereto.

Examples of substituents that the above alkyl groups, alkoxy groups, amino groups, aryloxy groups, silyl groups, aryl groups, and heterocyclic groups may further have include halogen atoms such as fluorine, chlorine, bromine, and iodine, alkyl groups such as a methyl group, an ethyl group, a normal propyl group, an isopropyl group, a normal butyl group, and a tertiary butyl group, alkoxy groups such as a methoxy group, an ethoxy group, and a propoxy group, amino groups such as a dimethylamino group, a diethylamino group, a dibenzylamino group, a diphenylamino group, and a ditolylamino group, aryloxy groups such as a phenoxy group, aryl groups such as a phenyl group and a biphenyl group, heterocyclic groups such as a pyridyl group and a pyrrolyl group, and a cyano group, but are not limited thereto.

(1) Organic Compound

First, an organic compound according to the present invention will be described.

The organic compound according to the present invention is a compound represented by general formula (1-1) or (1-2).

[Chem. 4]

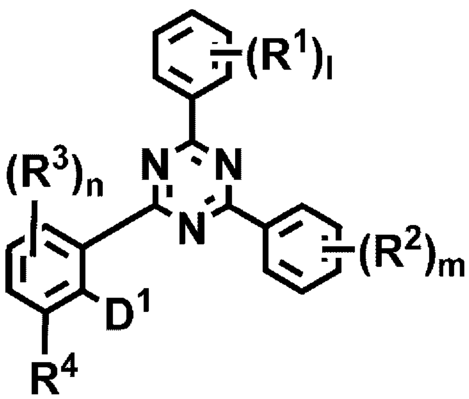

(1-1)

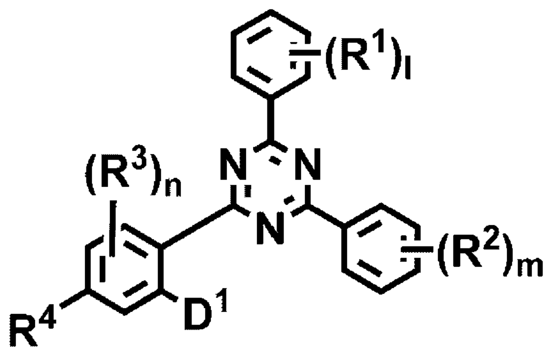

(1-2)

In general formulas (1-1) and (1-2), $R^1$ to $R^3$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a

substituted or unsubstituted silyl group, and a cyano group. $R^4$ is a substituted or unsubstituted alkyl group or a substituted or unsubstituted alkoxy group. 1 and m are each an integer of 0 to 5, and n is an integer of 0 to 3. A plurality of $R^1$'s may be the same as or different from each other, a plurality of $R^2$'s may be the same as or different from each other, and a plurality of $R^3$'s may be the same as or different from each other. $D^1$ is represented by general formula (2).

In general formulas (1-1) and (1-2), l, m, and n may be 0, and l, m, and n may each be an integer of 1 or greater or an integer of 0 to 2. When l, m, and n are each an integer of 1 or greater, $R^1$ to $R^3$ may each be an alkyl group having 1 to 4 carbon atoms or an aryl group having 6 to 12 carbon atoms, and may each be a methyl group, a tert-butyl group, a phenyl group, or a biphenyl group.

[Chem. 5]

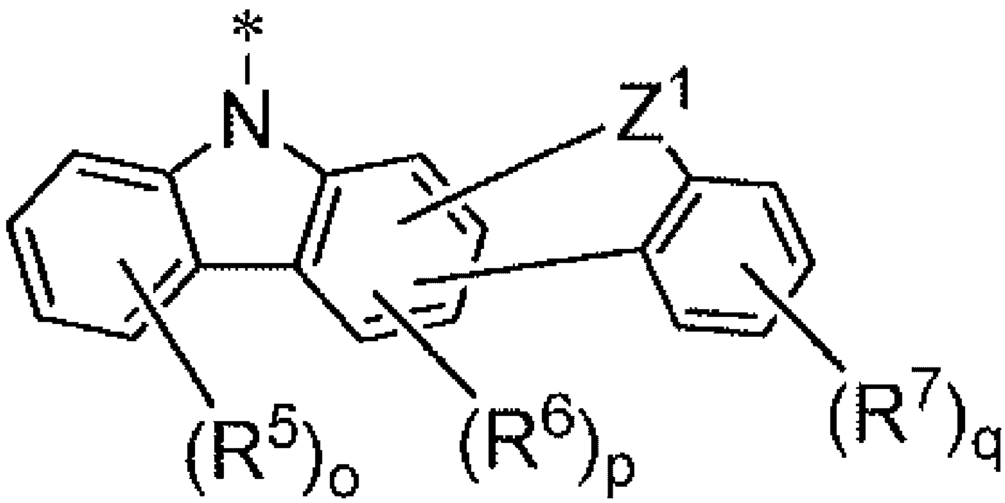

(2)

In general formula (2), $R^5$ to $R^7$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. o and q are each an integer of 0 to 4, and p is an integer of 0 to 2. A plurality of $R^5$'s may be the same as or different from each other, a plurality of $R^6$'s may be the same as or different from each other, and a plurality of $R^7$'s may be the same as or different from each other. $Z^1$ is an oxygen atom, a sulfur atom, a selenium atom, $NR^8$, or $CR^9R^{10}$. $R^8$ to $R^{10}$ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. * represents a position of bonding to general formula (1-1) or (1-2).

In general formula (2), o, p, and q may be 0, and o may be 1. When o is 1, $R^5$ may be an alkyl group having 1 to 7 carbon atoms, an aryl group having 6 to 10 carbon atoms, or a heterocyclic group having 6 to 12 carbon atoms, may be an alkyl group having 1 to 4 carbon atoms, an aryl group having 6 to 10 carbon atoms, or a heterocyclic group having 6 to 12 carbon atoms, and may be a methyl group, a tert-butyl group, a phenyl group, or a carbazolyl group.

From the viewpoint of durability, $Z^1$ is preferably an oxygen atom, a sulfur atom, a selenium atom, or $NR^8$. From the viewpoint of ease of synthesis, $Z^1$ is more preferably an oxygen atom, a sulfur atom, or $NR^8$. In particular, when the organic compound according to the present invention is used as an assist material of a blue light-emitting material, $Z^1$ is preferably an oxygen atom or a sulfur atom, more preferably an oxygen atom from the viewpoint of ease of synthesis. When the organic compound according to the present invention is used as an assist material of a green light-emitting material, $Z^1$ is preferably $NR^8$.

Specifically, general formula (2) may be general formula (2-1).

[Chem. 6]

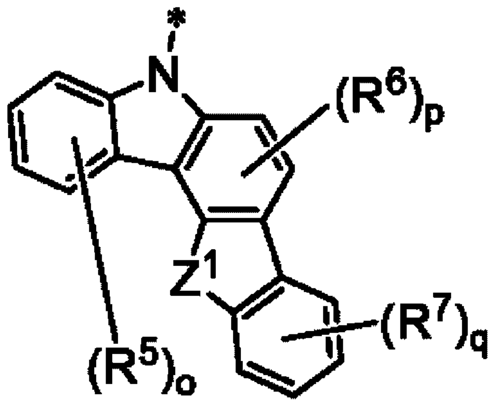

(2 − 1)

General formula (1-1) is preferably general formula (3), more preferably general formula (3) where $Z^1$ is an oxygen atom, particularly preferably general formula (5). General formula (1-2) is preferably general formula (4), more preferably general formula (4) where $Z^1$ is an oxygen atom, particularly preferably general formula (6).

[Chem. 7]

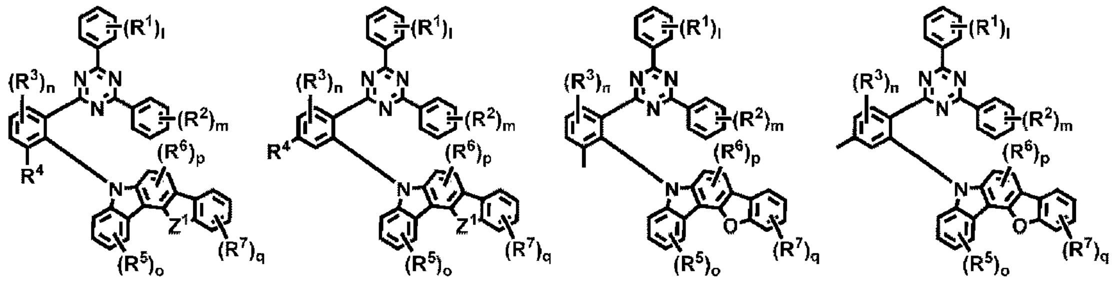

(3) (4) (5) (6)

Hereinafter, features of the organic compound according to the present invention will be described.

The organic compound according to the present invention is an organic compound in which a donor is a fused carbazole skeleton and which has, in a phenyl group to which the fused carbazole skeleton is bonded, an alkyl group or an alkoxy group at an ortho-position with respect to the fused carbazole skeleton. Thus, the organic compound according to the present invention exhibits a smaller $\Delta E_{ST}$.

In general, in order to obtain a compound having a small $\Delta E_{ST}$, the overlap between a HOMO (Highest Occupied Molecular Orbital) orbital distribution and a LUMO (Lowest Unoccupied Molecular Orbital) orbital distribution needs to be small.

Fig. 8 shows a comparison of the HOMO orbital distribution and LUMO orbital distribution of compound 1 and compound 5, which are organic compounds according to the present invention, and compound Ref-1 and compound Ref-2, which are comparative examples.

The HOMO orbital distribution and LUMO orbital distribution above were visualized using molecular orbital calculations. The molecular orbital calculations were performed using Gaussian 16 (Gaussian 16, Revision C.01, M. J. Frisch, et al, Gaussian, Inc., Wallingford CT, 2019.), which is molecular orbital calculation software produced by Gaussian, Inc., USA.

As shown in Fig. 8, compound Ref-1 and compound Ref-2 have, in a phenyl group to which a fused carbazole skeleton is bonded, a HOMO orbital distribution also on the carbon atom located at the para position with respect to the fused carbazole skeleton (circled by a black dotted line in Fig. 8). In contrast, compound 1 and compound 5 do not have, in a phenyl group to which a fused carbazole skeleton is bonded, a HOMO orbital distribution on the carbon atom located at the

para position with respect to the fused carbazole skeleton (circled by a black dotted line in Fig. 8). Therefore, compound 1 and compound 5 have a smaller overlap between the HOMO orbital distribution and the LUMO orbital distribution than compounds Ref-1 and Ref-2, and thus the organic compound according to the present invention exhibits a smaller $\Delta E_{ST}$. Similarly, when $R^4$ is an alkoxy group, the same effect as that of compound 1 can be obtained because $R^4$ has an alkyl skeleton.

Furthermore, the organic compound according to the present invention, because of exhibiting a small $\Delta E_{ST}$, tends to exhibit a high reverse intersystem crossing rate ($k_{RISC}$) of excitons from T1 to S1. A high $k_{RISC}$ leads to a short time period during which excitons stay in T1, so that degradation of a light-emitting layer due to excitons staying in T1 can be reduced. Thus, an organic light-emitting element obtained using the organic compound according to the present invention in the light-emitting layer is expected to have high durability. In addition, by using the organic compound according to the present invention in the light-emitting layer, quenching from T1 can be reduced. Thus, the organic light-emitting element obtained using the organic compound according to the present invention in the light-emitting layer is expected to also have high luminous efficiency.

In the organic compound according to the present invention, a donor is a fused carbazole skeleton. In contrast, compound Ref-3 has a diphenylcarbazole skeleton in which two benzene rings are bonded to a carbazole skeleton serving as a donor. In general, compared with a skeleton in which substituents are bonded to a carbazole skeleton, such as a diphenylcarbazole skeleton, a fused carbazole skeleton in which a substituent is fused is a skeleton having high bonding stability. Therefore, the organic compound according to the present invention has higher stability than organic compounds having a diphenylcarbazole skeleton.

In the organic compound according to the present invention, $R^4$ is preferably an alkyl group, preferably an alkyl group having 1 to 7 carbon atoms, more preferably a methyl group, a n-butyl group, an iso-butyl group, a tert-butyl group, a n-hexyl group, or a n-heptyl group, still more preferably a methyl group, a n-butyl group, or a tert-butyl group, particularly preferably a methyl group.

Fig. 9 shows the HOMO orbital distribution and LUMO orbital distribution of compound 1, compound 2, compound 3, and compound 4, which are organic compounds according to the present invention. Compound 1 is a compound in which $R^4$ is a methyl group (1 carbon atom), compound 2 is a compound in which $R^4$ is a n-heptyl group (7 carbon atoms), compound 3 is a compound in which $R^4$ is a n-octyl group (8 carbon atoms), and compound 4 is a compound in which $R^4$ is a n-undecyl group (11 carbon atoms).

As shown in Fig. 9, compound 1 and compound 2 do not have, in a phenyl group to which a fused carbazole skeleton is bonded, a HOMO orbital distribution on the carbon atom located at the para position with respect to the fused carbazole skeleton (circled by a black dotted line in Fig. 9). In contrast, compound 3 and compound 4 slightly have, in a phenyl group to which a fused carbazole skeleton is bonded, a HOMO orbital distribution on the carbon atom located at the para position with respect to the fused carbazole skeleton (circled by a black dotted line in Fig. 9). Thus, compound 1 and compound 2 have a smaller overlap between the HOMO orbital distribution and the LUMO orbital distribution than compound 3 and compound 4. Therefore, among the organic compounds according to the present invention, $R^4$ is preferably an alkyl group having 1 to 7 carbon atoms, more preferably a methyl group, a n-butyl group, an iso-butyl group, a tert-butyl group, a n-hexyl group, or a n-heptyl group, still more preferably a methyl group, a n-butyl group, or a tert-butyl group, particularly preferably a methyl group.

Among the organic compounds according to the present invention, the organic compound represented by general formula (1-1) is preferred because it is a compound that exhibits a particularly small $\Delta E_{ST}$ value.

Among the organic compounds according to the present invention, the organic compound represented by general formula (1-2) is preferred because it is an organic compound having particularly high stability. This is because, in the organic compound represented by general formula (1-2), the anion spin density on a carbon atom bonded to a hydrogen atom is small.

During operation of an organic light-emitting element, an organic compound used in the organic light-emitting element may form an anion radical or a cation radical, which decomposes or reacts with other substances to cause luminance degradation. Thus, the organic compound is preferably less likely to form an anion radical or a cation radical during operation of the organic light-emitting element.

As an index indicating the likelihood of formation of an anion radical or a cation radical, the spin density on an atom is known. A smaller spin density indicates a lower likelihood of forming an anion radical or a cation radical.

Table 1 shows the calculation results of the anion spin density on carbon atoms expressed as C1 to C5 of the following compound. An anion radical or a cation radical is likely to be formed in a phenyl group to which a donor is bonded and at a C-H (carbon-hydrogen) bond which has a relatively low bond energy. Therefore, in Table 1, C-H bonds of a phenyl group to which a donor is bonded are focused, and the anion spin density on the carbon atoms is calculated.

[Chem. 8]

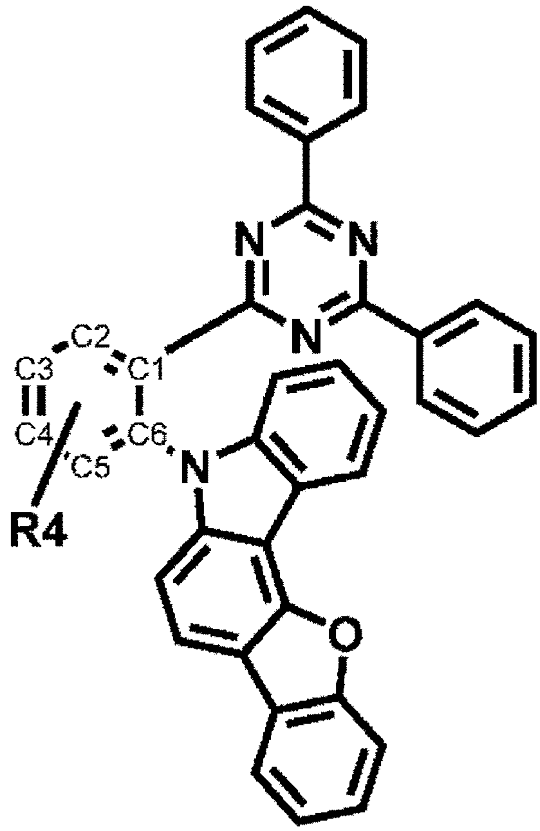

[Table 1]

Table 1

| | Compound 1 | Compound 5 |
|---|---|---|
| Structural formula | 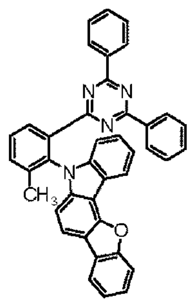 | 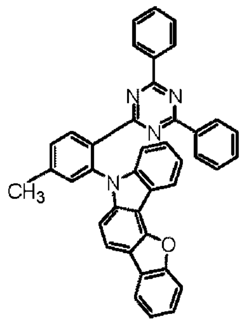 |
| Spin density on C2 | 0.11 | 0.10 |
| Spin density on C3 | 0.05 | 0.05 |
| Spin density on C4 | 0.21 | - |
| Spin density on C5 | - | 0.10 |

It has been found from Table 1 that the spin density values on carbon atoms of C-H bonds in compound 5 are equal to or smaller than the spin density values on carbon atoms of C-H bonds in compound **1.** These results show that among the organic compounds according to the present invention, the organic compound represented by general formula

(1-2) is preferred because it is an organic compound having particularly high stability. Using this organic compound in an organic light-emitting element can provide an organic light-emitting element having a longer lifetime.

Hereinafter, specific examples of the organic compound according to the present invention will be shown below. However, the present invention is not limited thereto.

[Chem. 9]

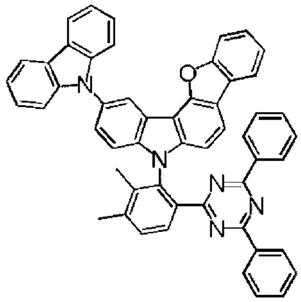
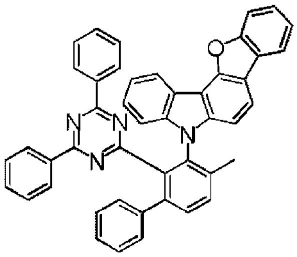
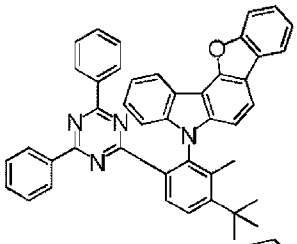
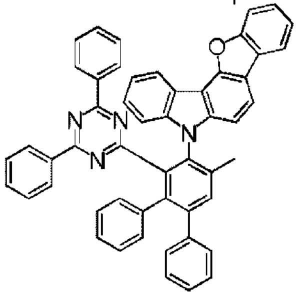
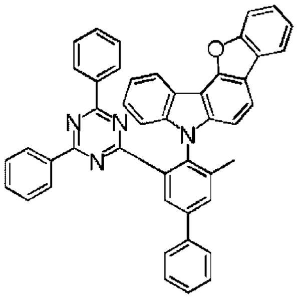
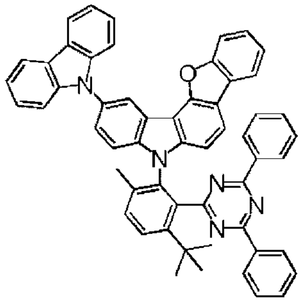
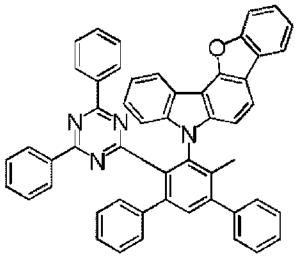
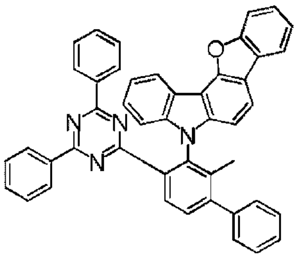
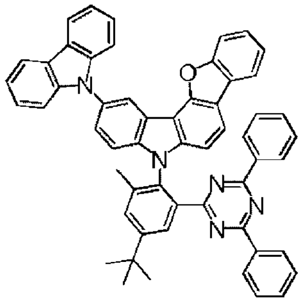
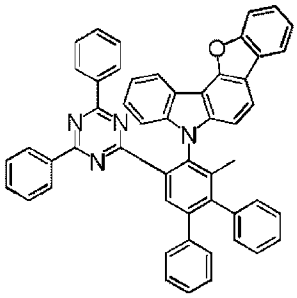
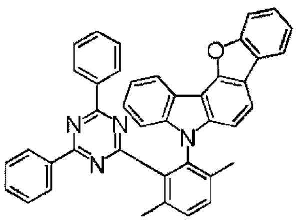
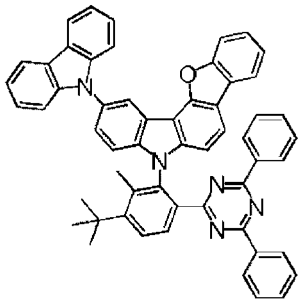
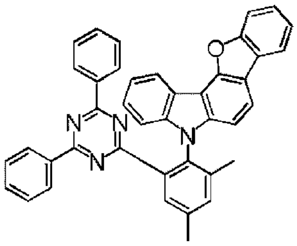
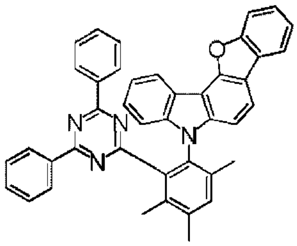
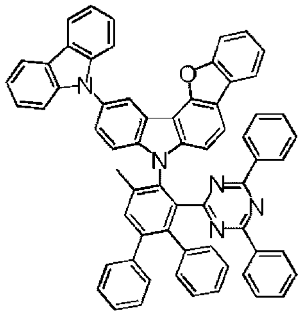
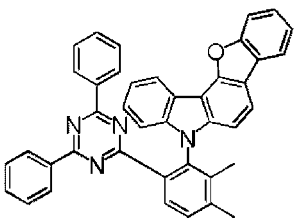
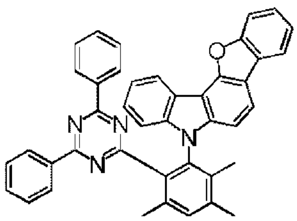

[Chem. 10]

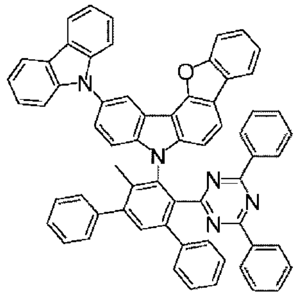
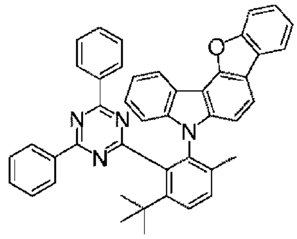
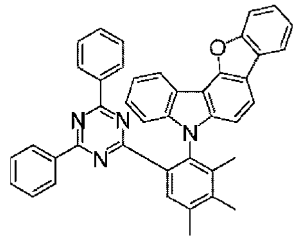
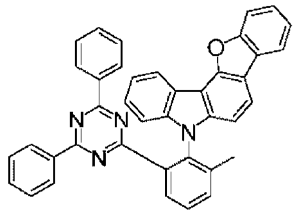
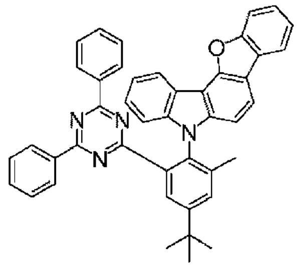
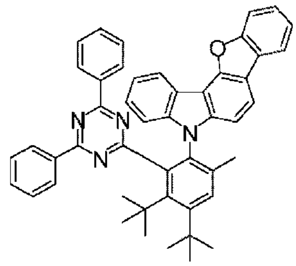
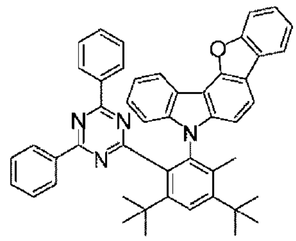
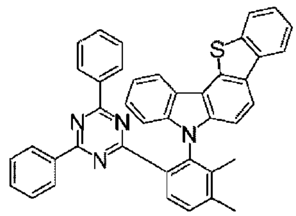
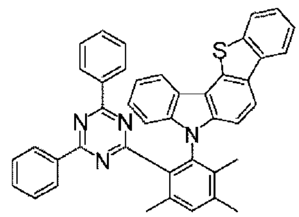
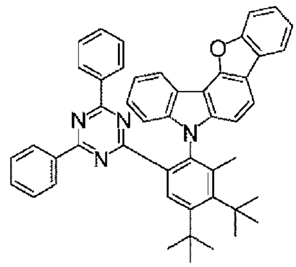
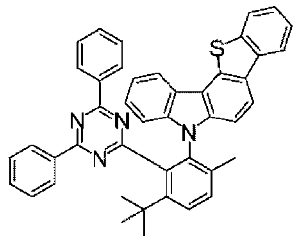
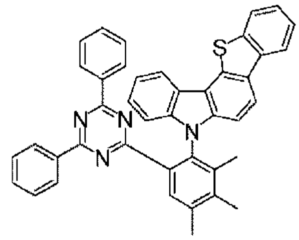
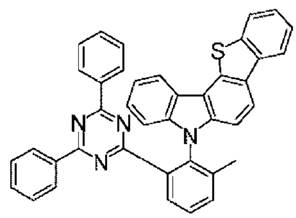
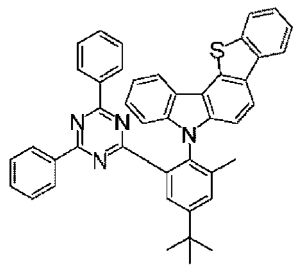
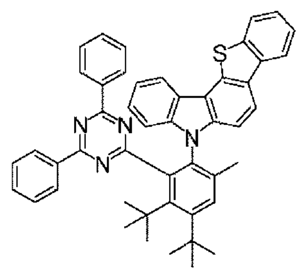
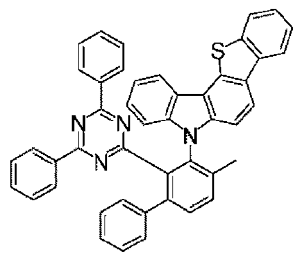
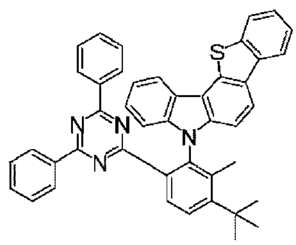
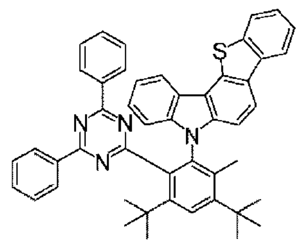

[Chem. 11]

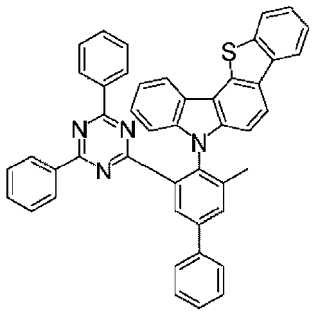
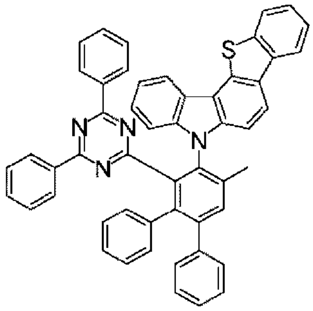
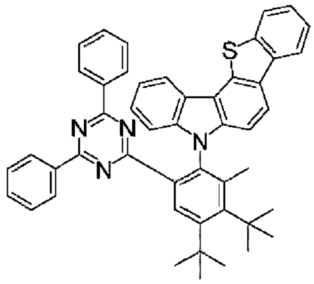
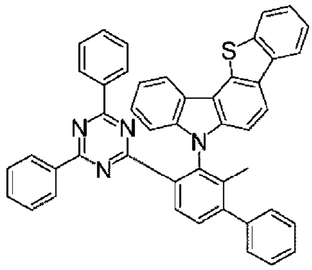
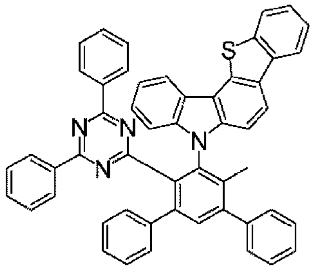
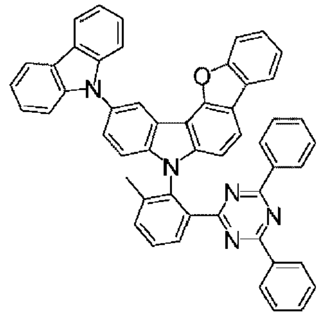
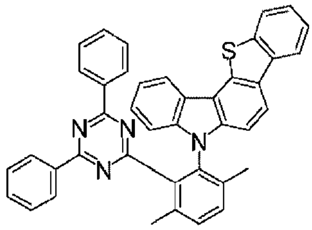
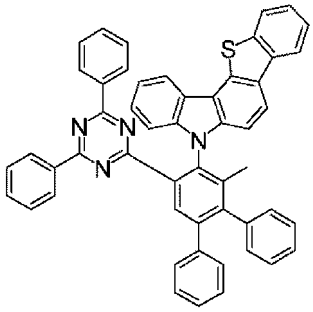
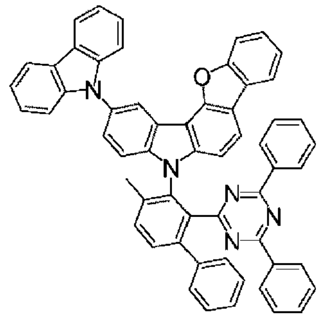
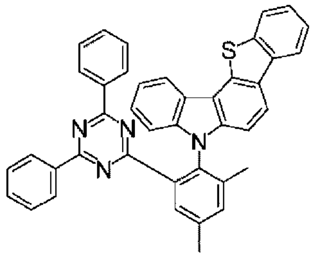
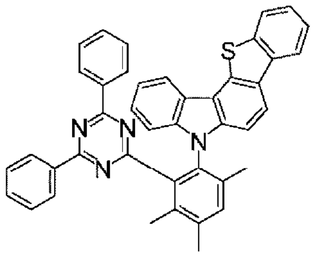
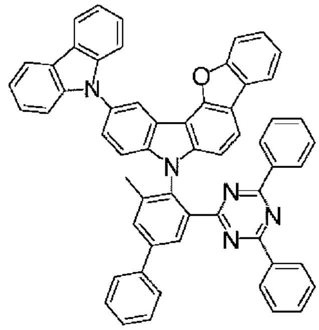
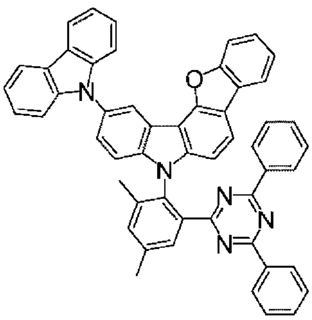
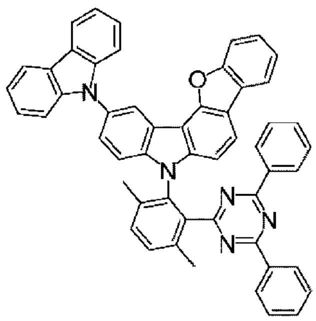
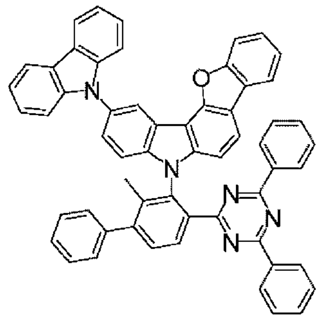

[Chem. 12]

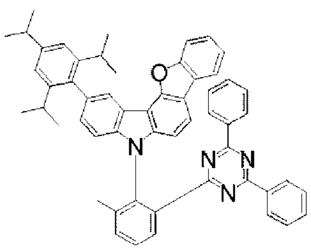
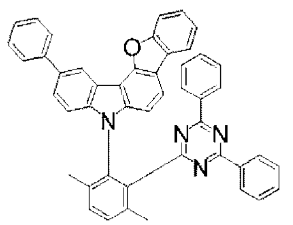
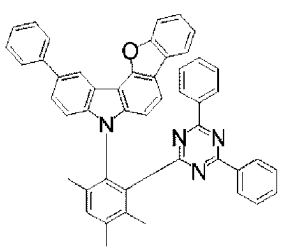
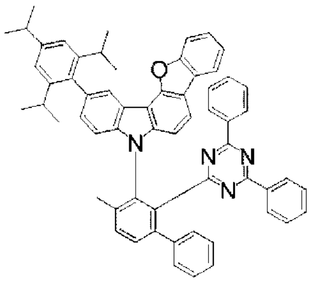
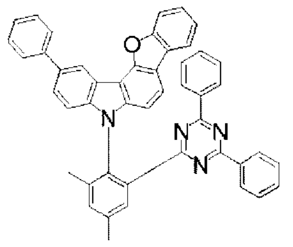
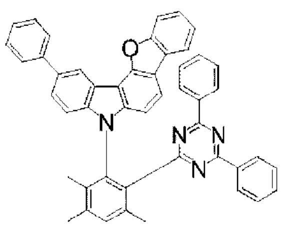
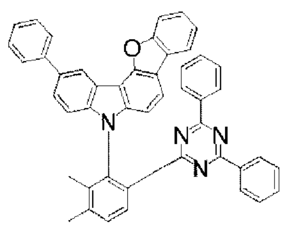
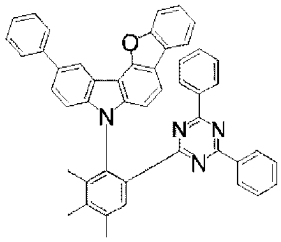
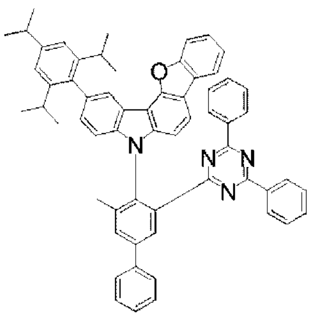
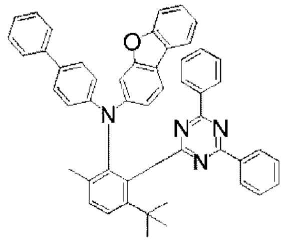
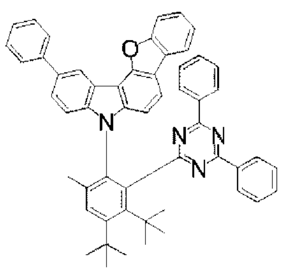
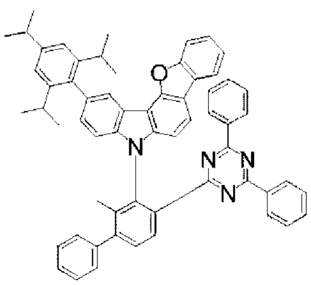
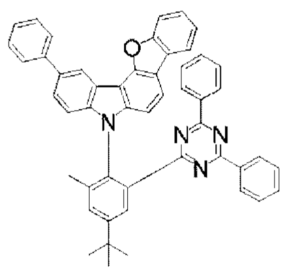
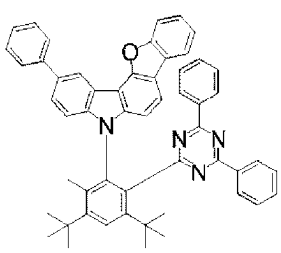
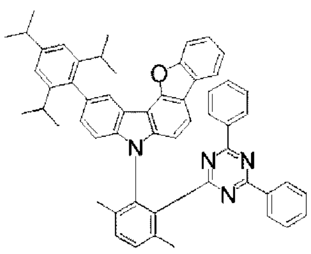
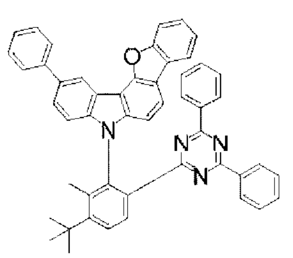
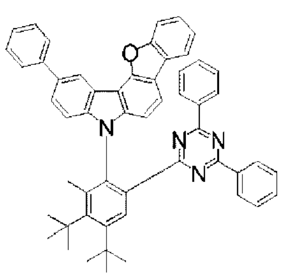
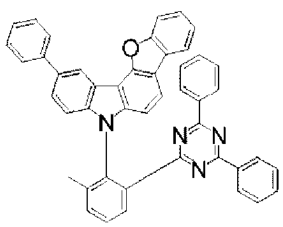

[Chem. 13]

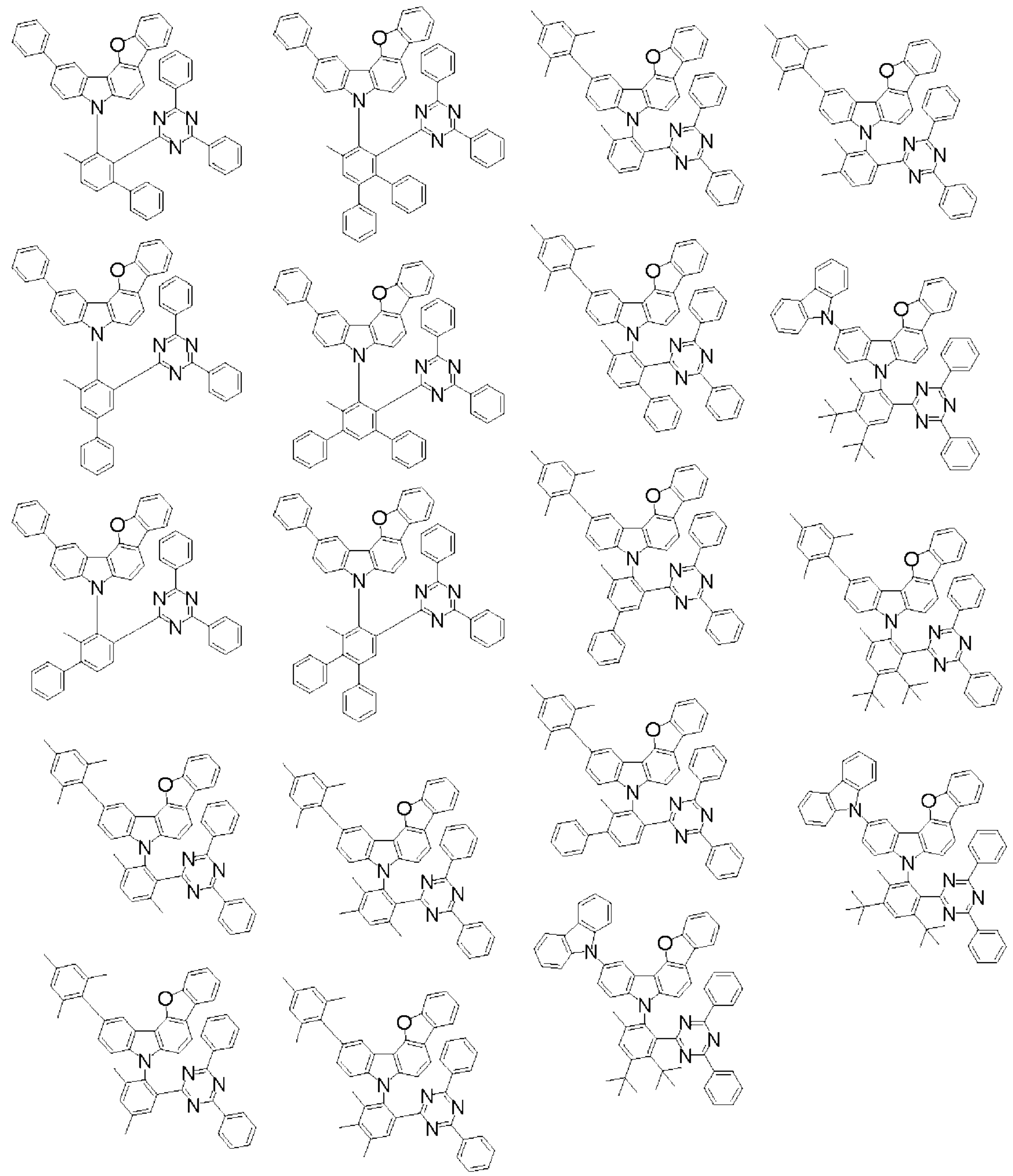

[Chem. 14]

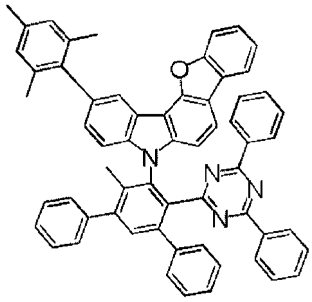
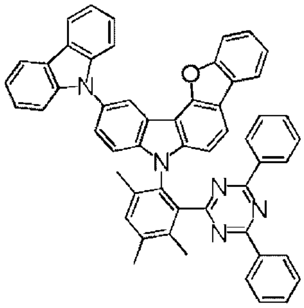
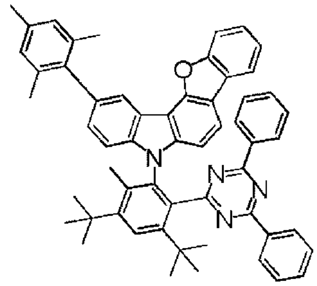
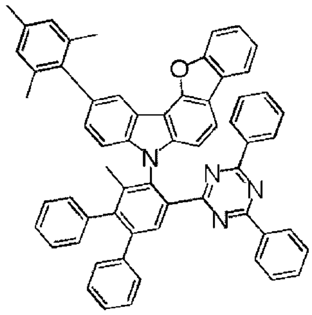
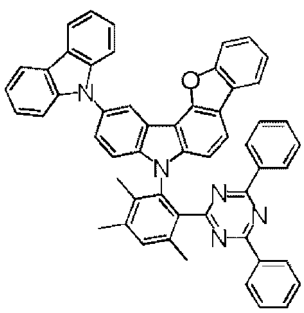
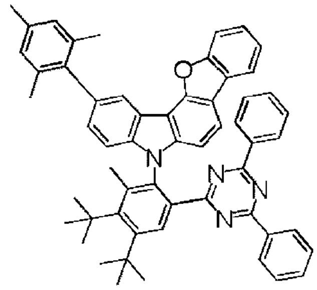
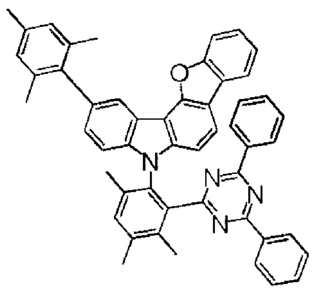
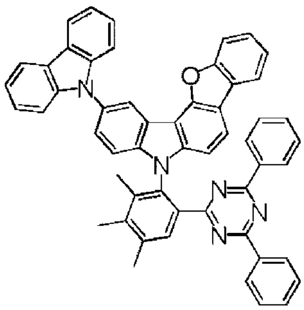
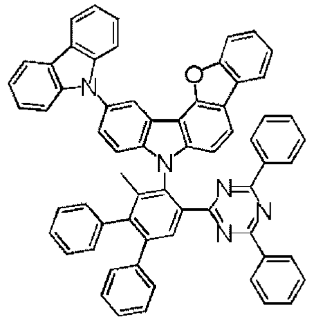
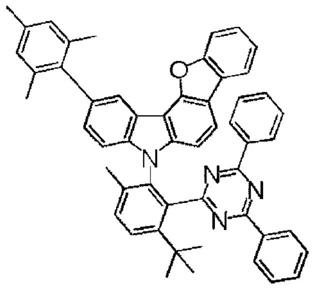
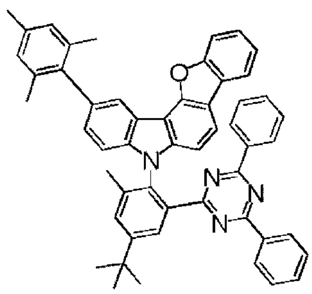
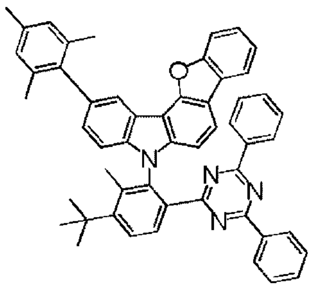
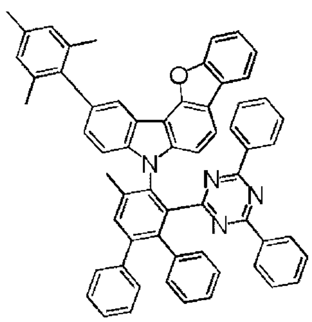

[Chem. 15]

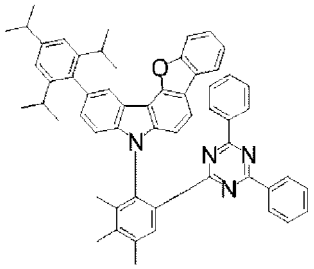
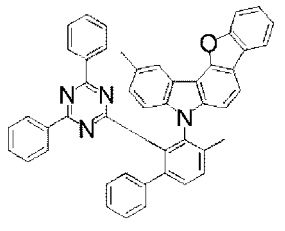
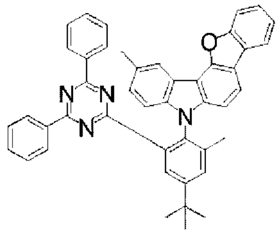
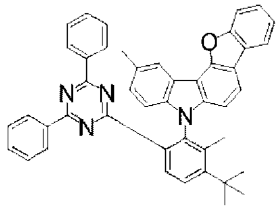
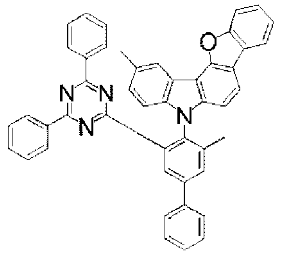
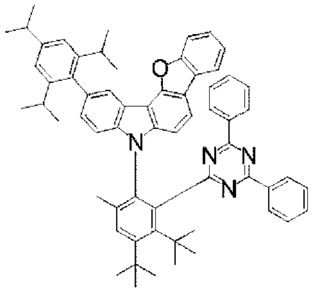
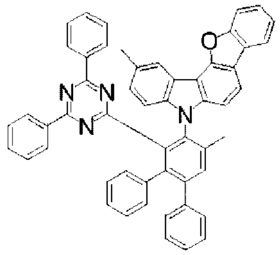
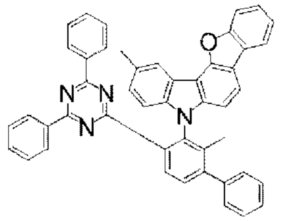
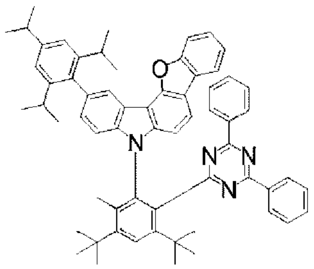
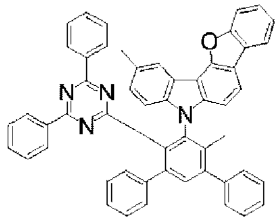
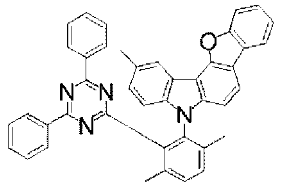
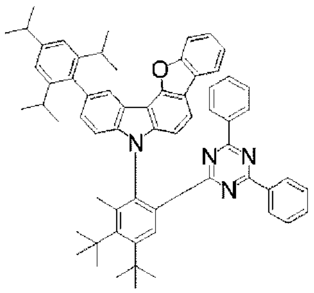
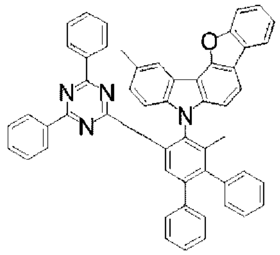
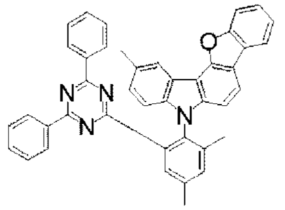
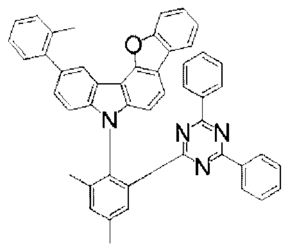
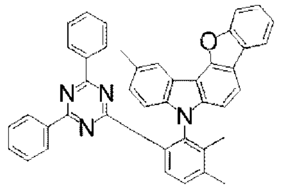
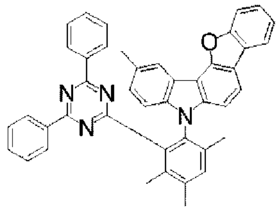
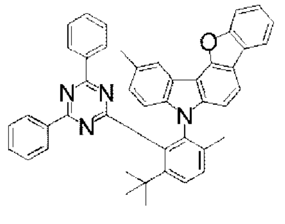
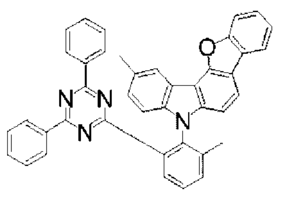
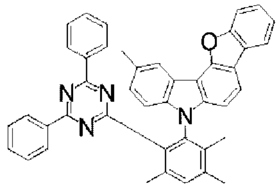

[Chem. 16]

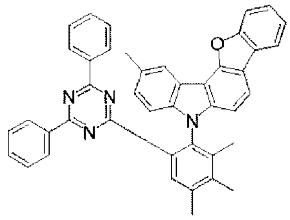
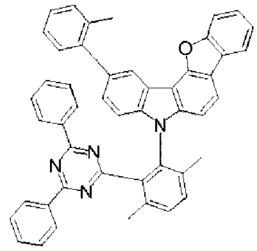
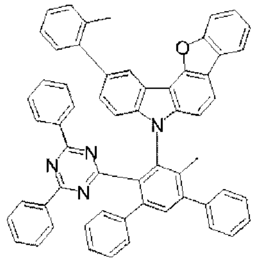
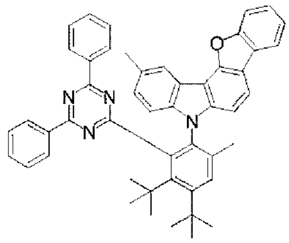
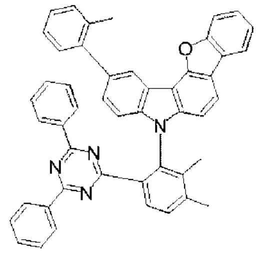
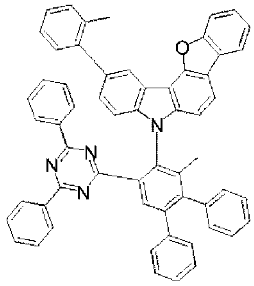
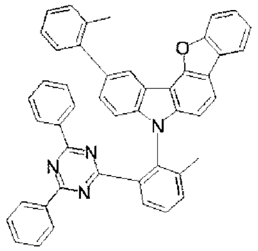
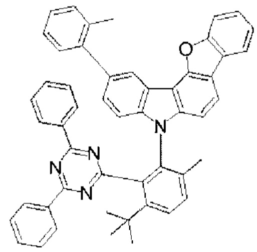
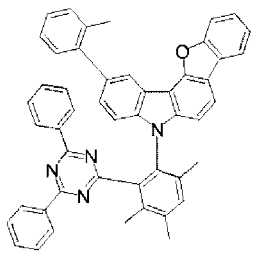
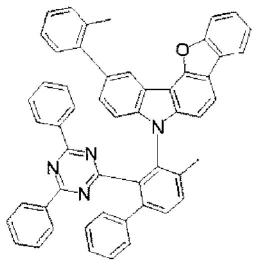
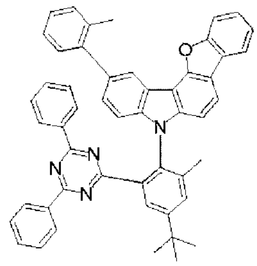
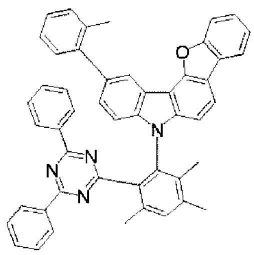
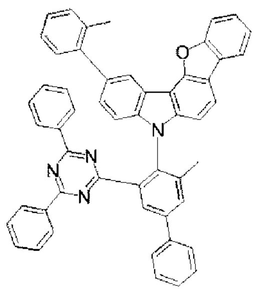
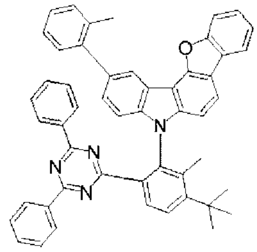
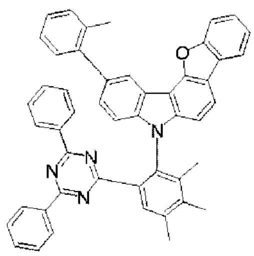
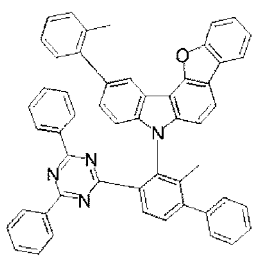
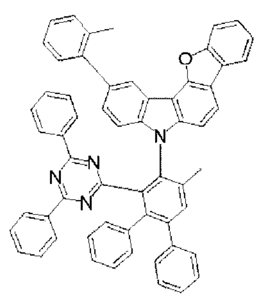
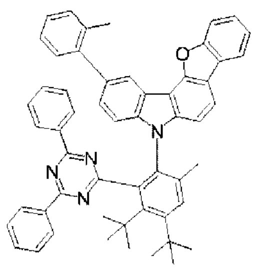

[Chem. 17]

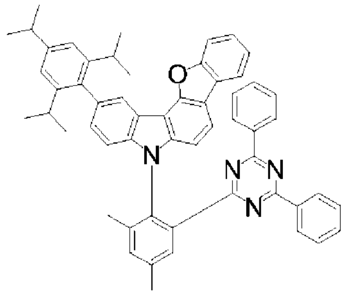
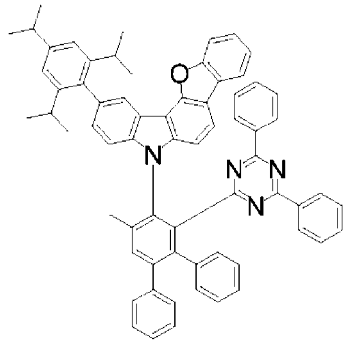
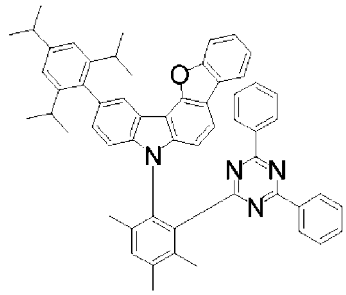
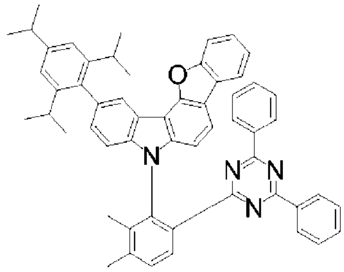
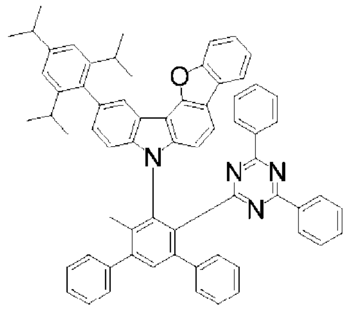
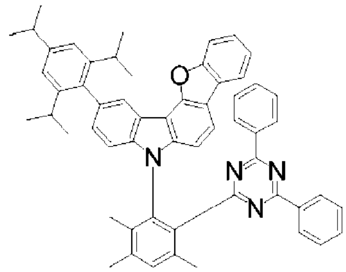
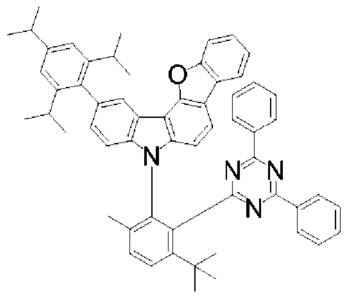
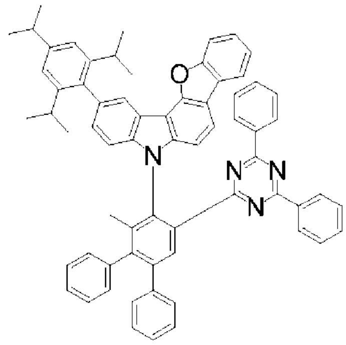
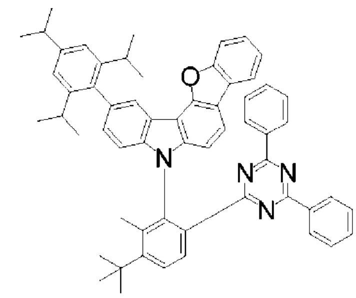
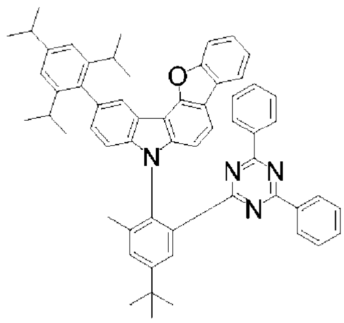
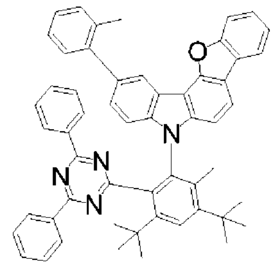
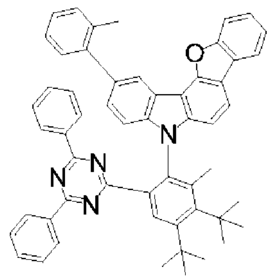

[Chem. 18]

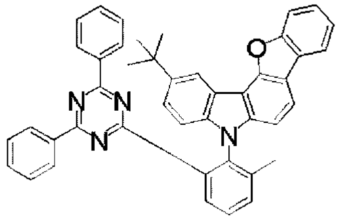
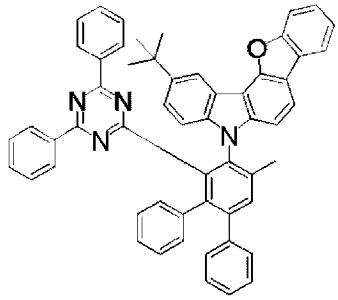
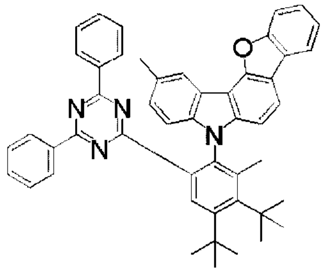
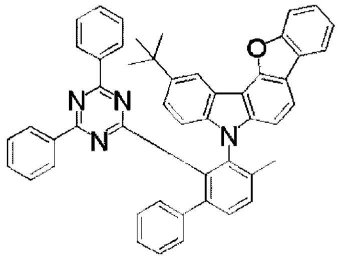
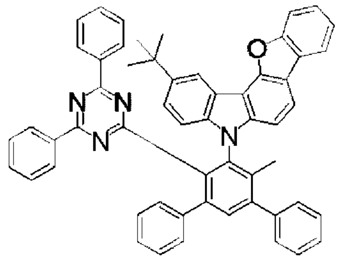
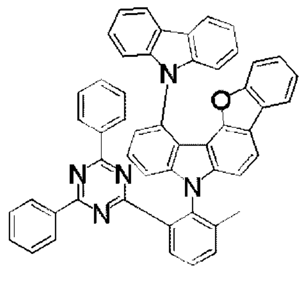
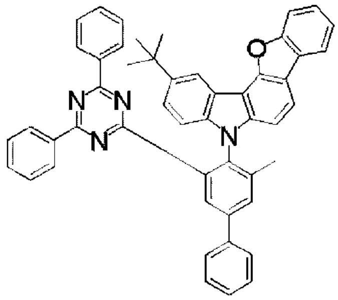
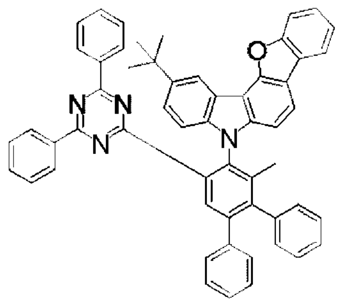
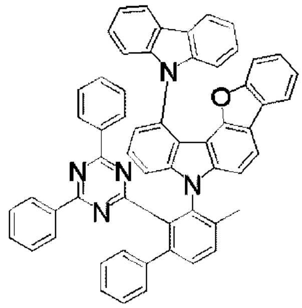
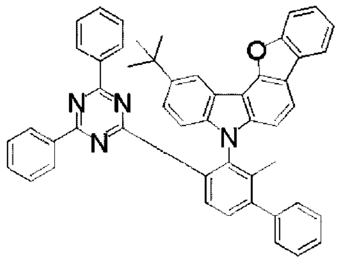
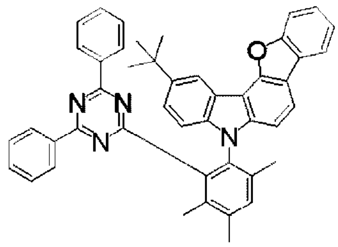
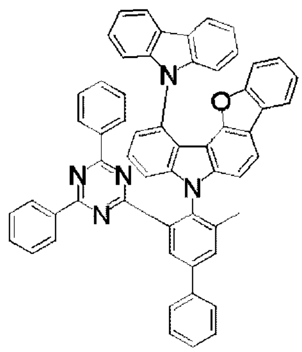
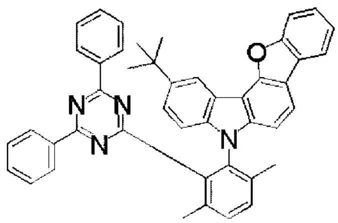
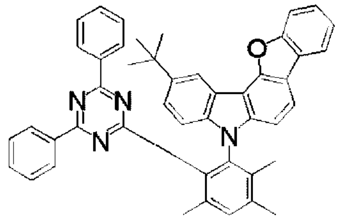
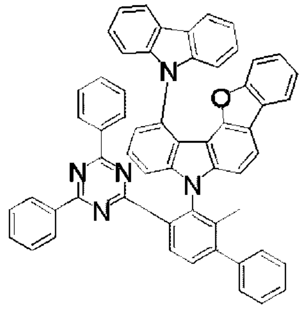
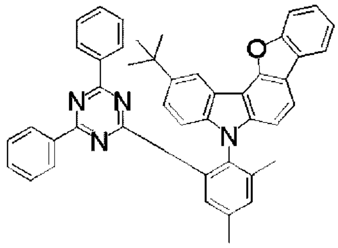
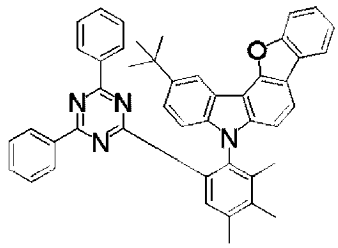

[Chem. 19]

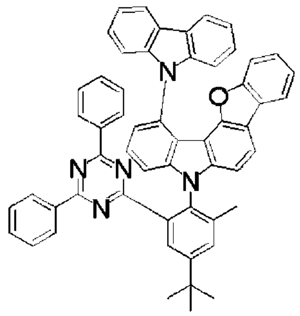
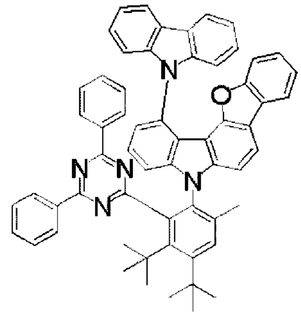
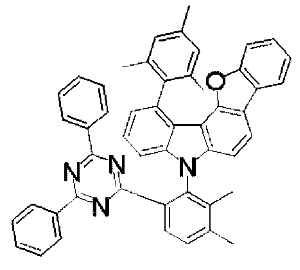
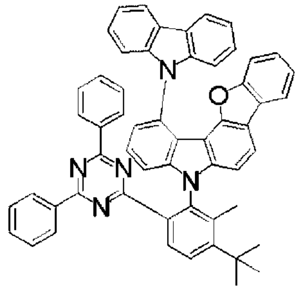
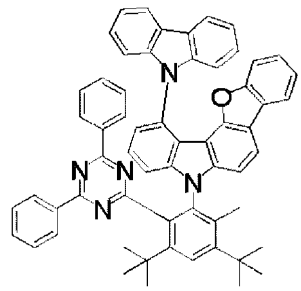
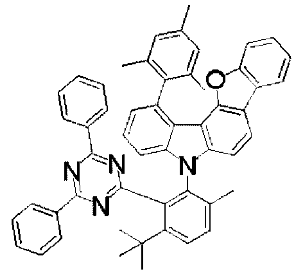
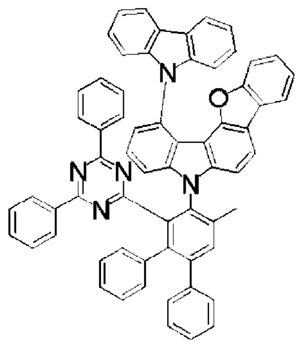
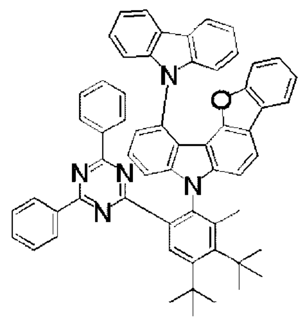
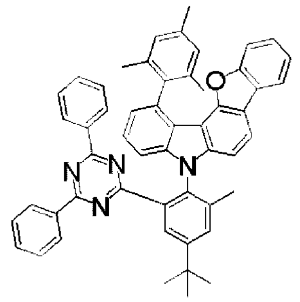
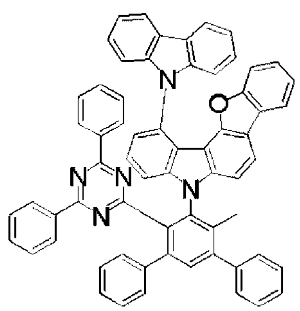
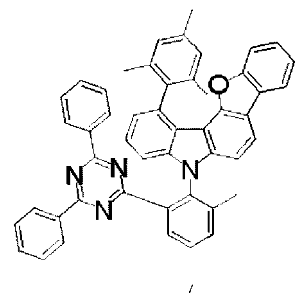
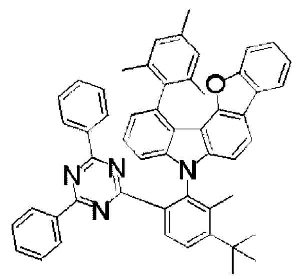
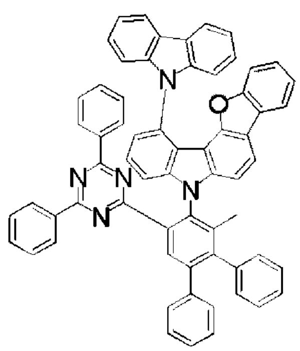
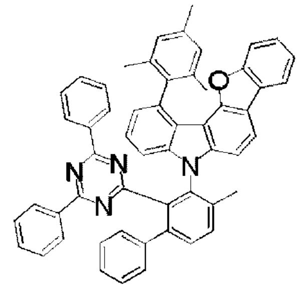
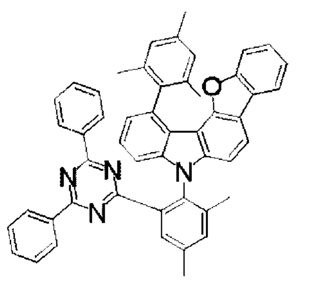

[Chem. 20]

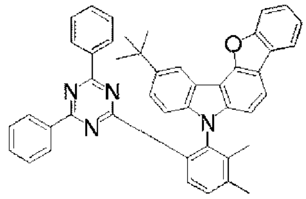
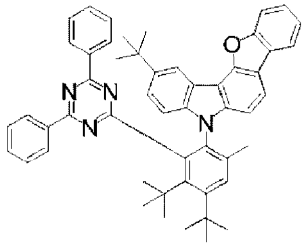
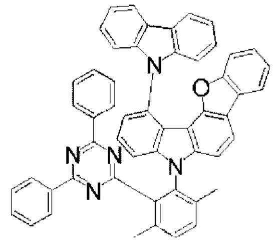
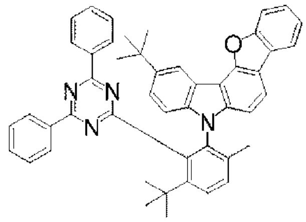
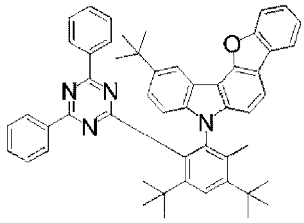
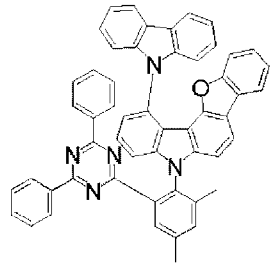
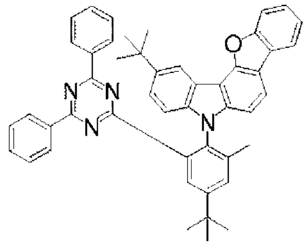
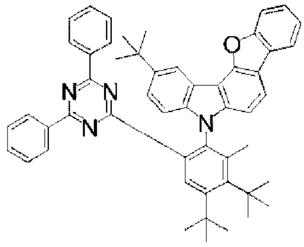
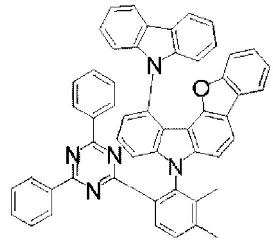
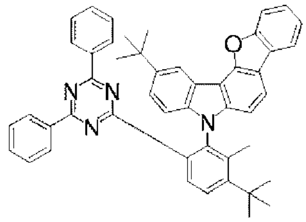
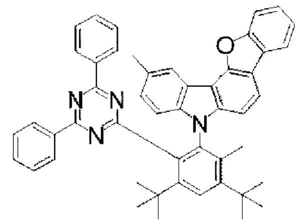
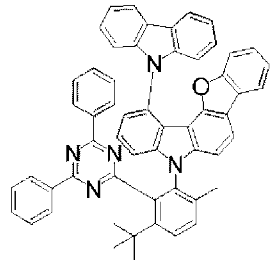
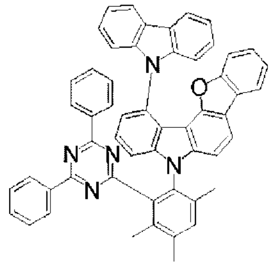
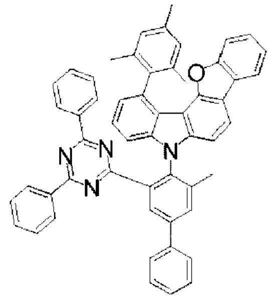
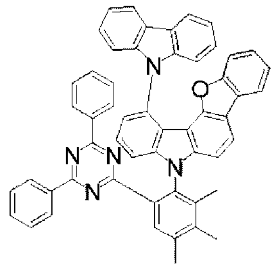
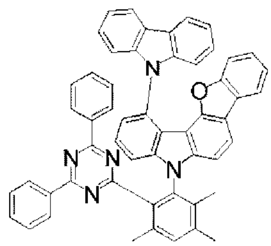
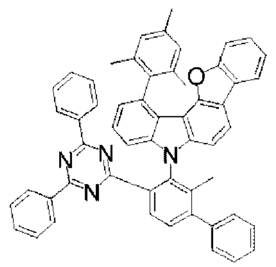
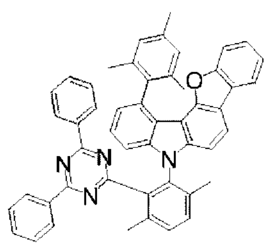

[Chem. 21]

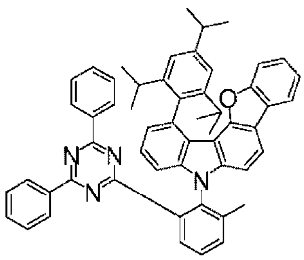
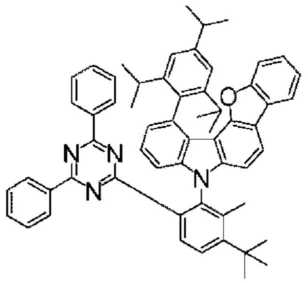
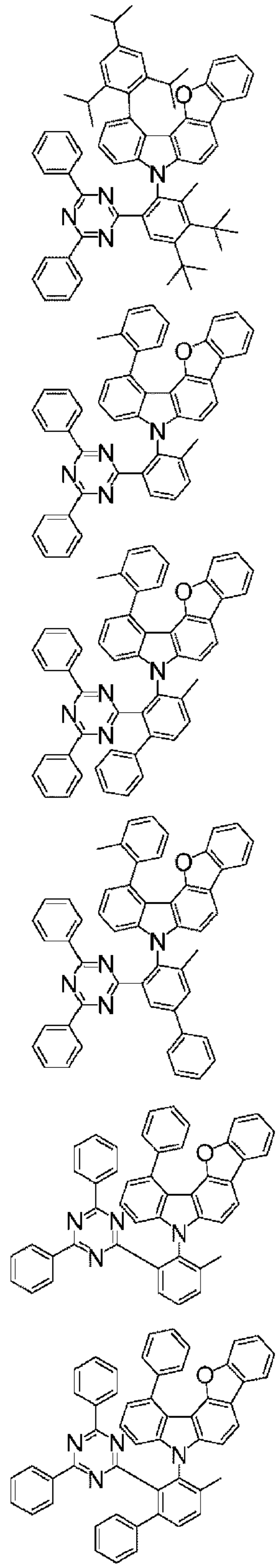
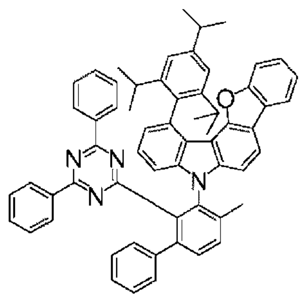
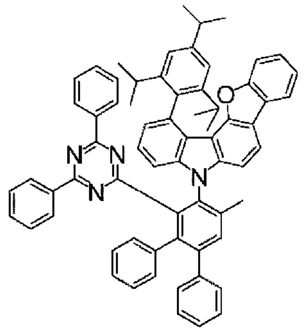
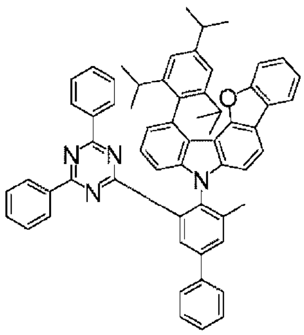
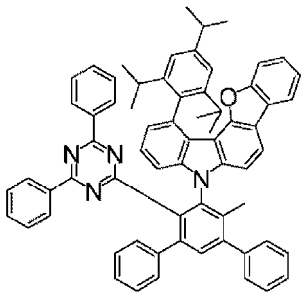
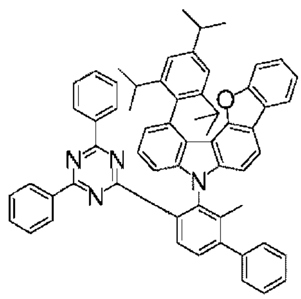
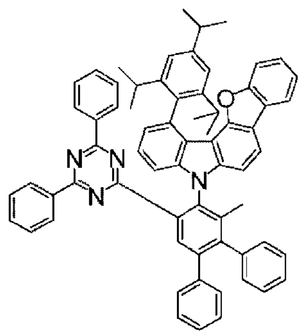
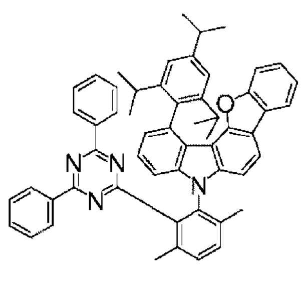
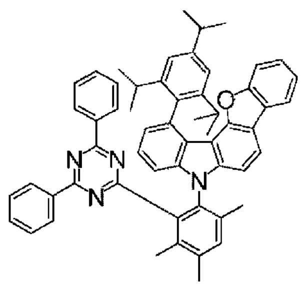

[Chem. 22]

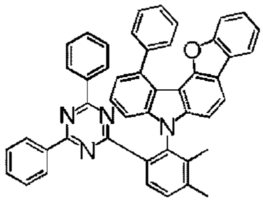
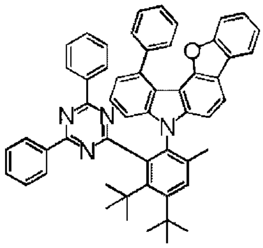
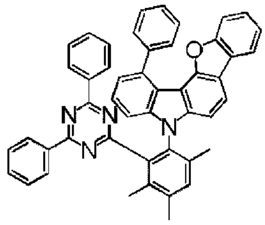
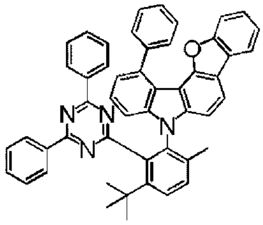
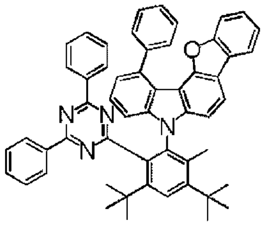
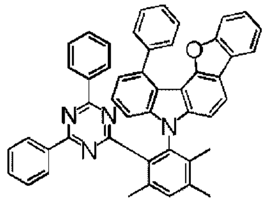
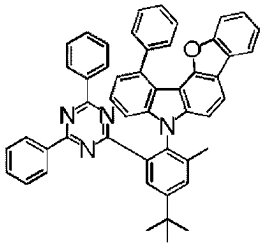
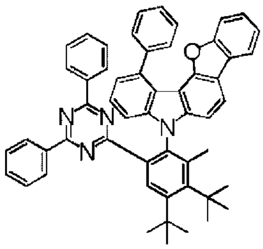
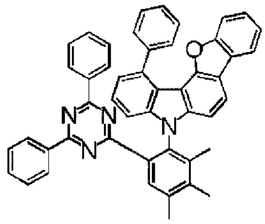
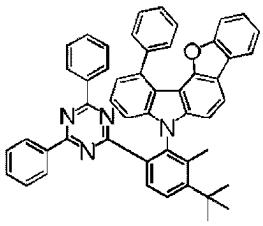
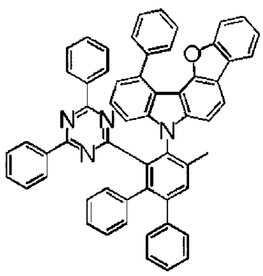
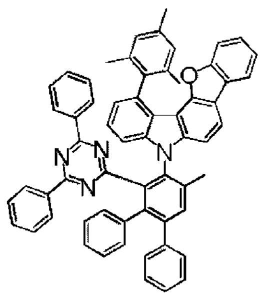
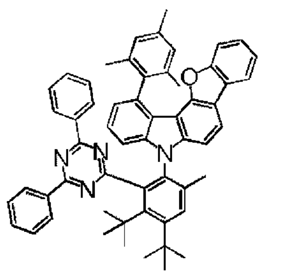
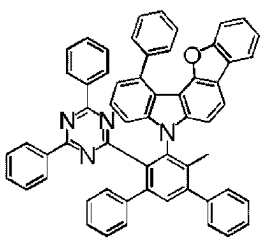
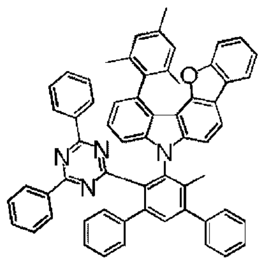
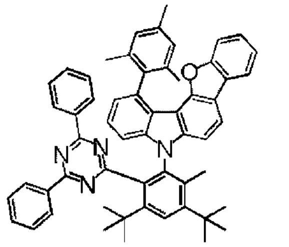
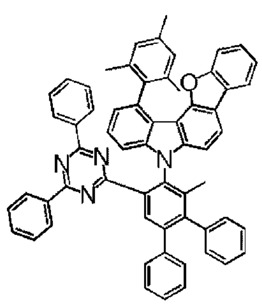
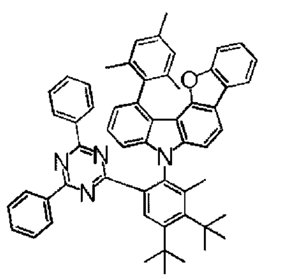

[Chem. 23]

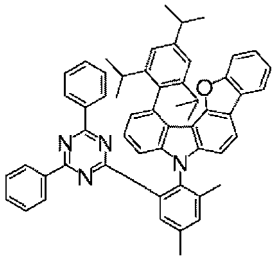
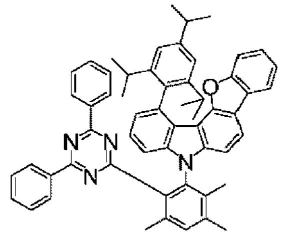
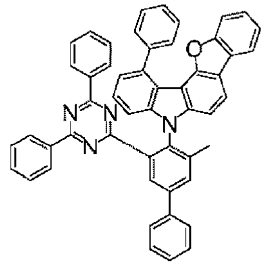
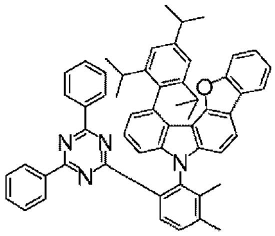
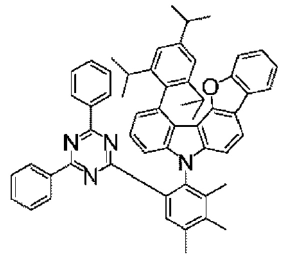
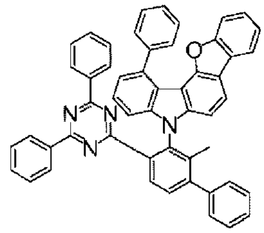
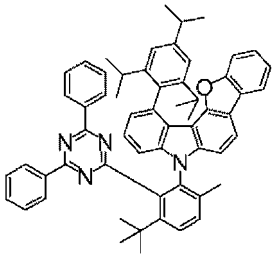
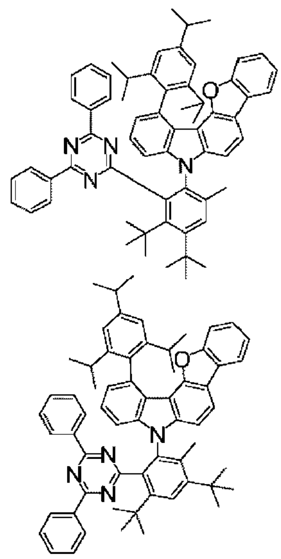
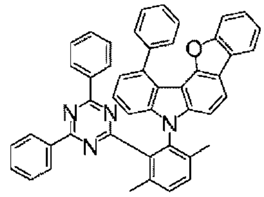
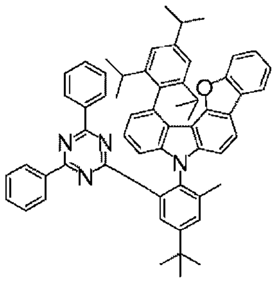
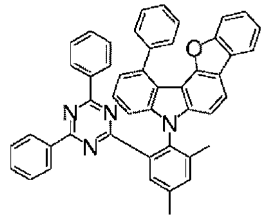
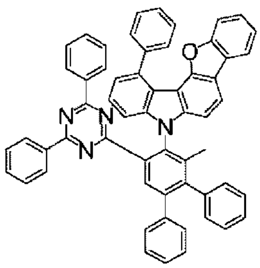
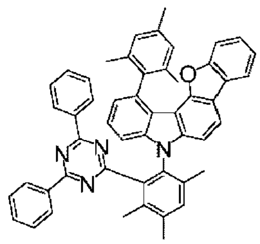
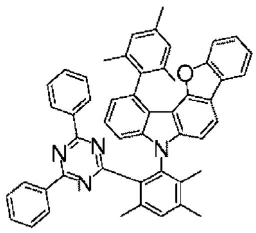
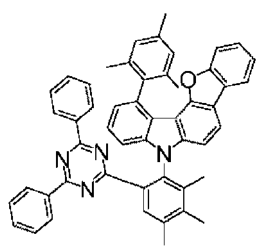

[Chem. 24]

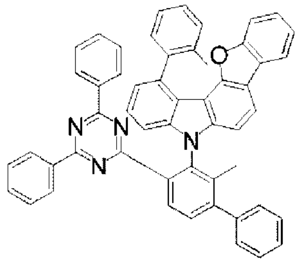

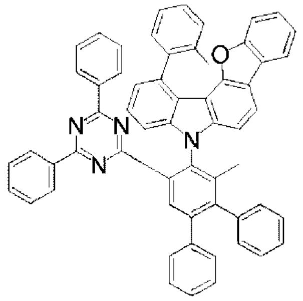

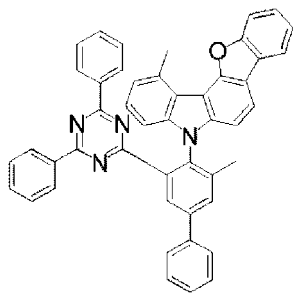

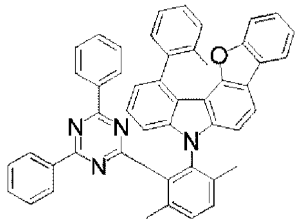

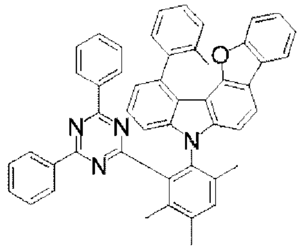

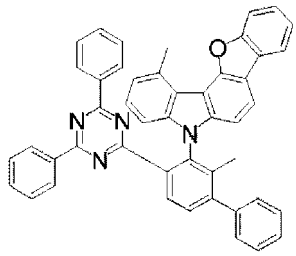

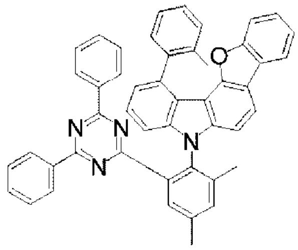

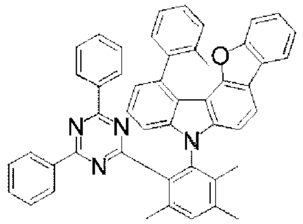

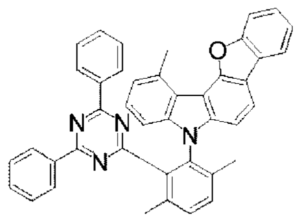

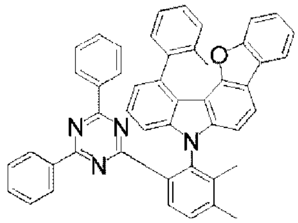

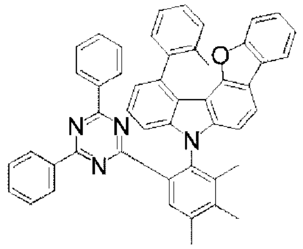

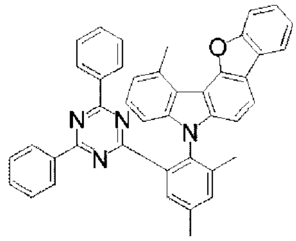

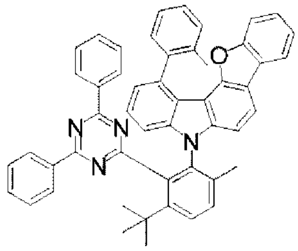

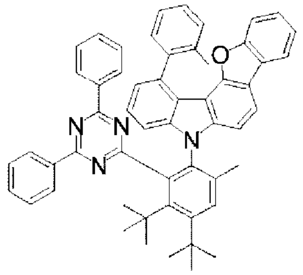

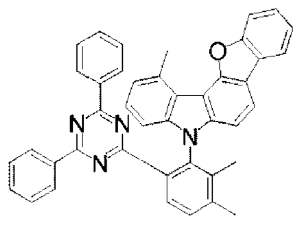

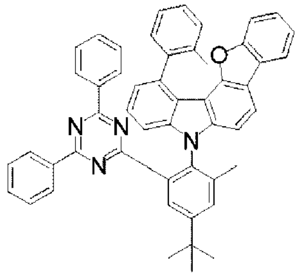

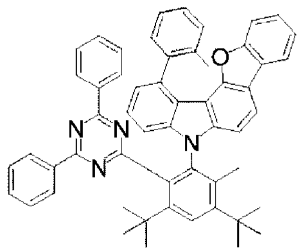

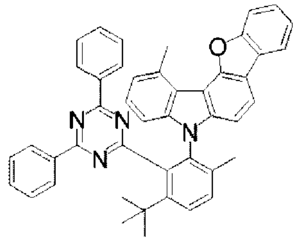

[Chem. 25]

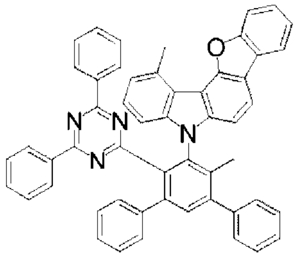
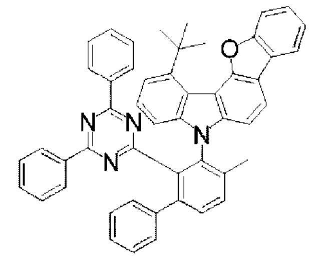
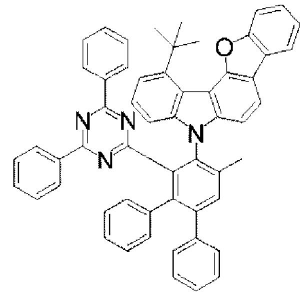
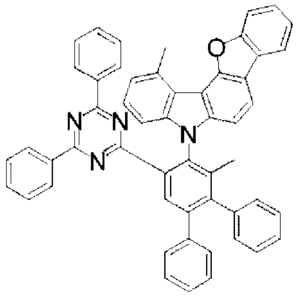
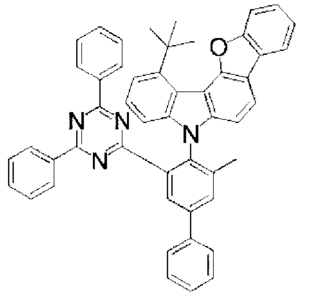
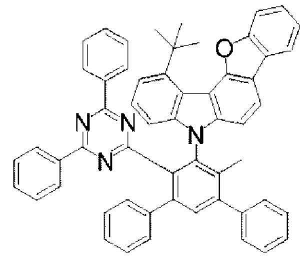
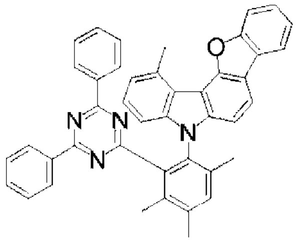
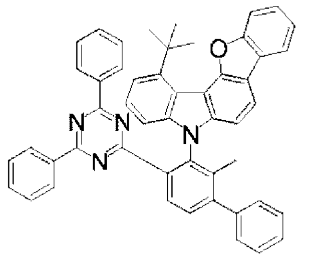
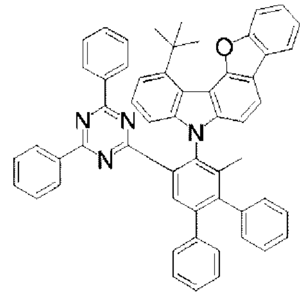
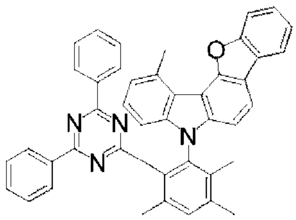
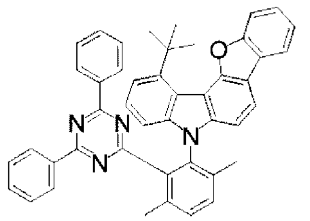
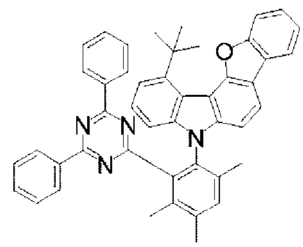
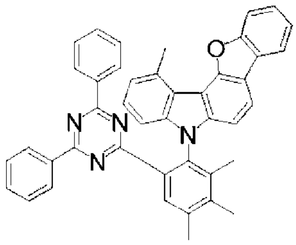
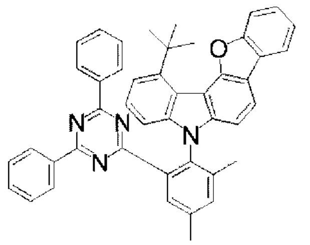
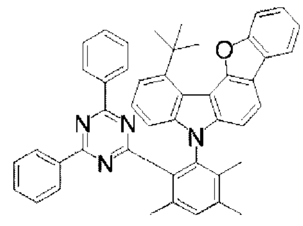
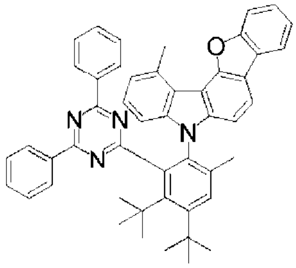
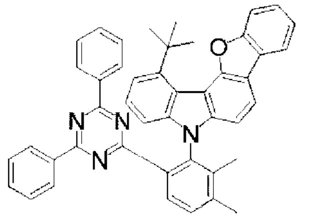
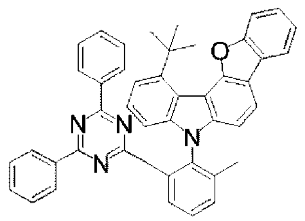

[Chem. 26]

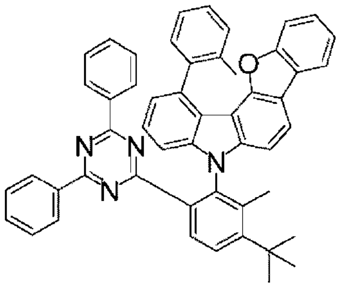
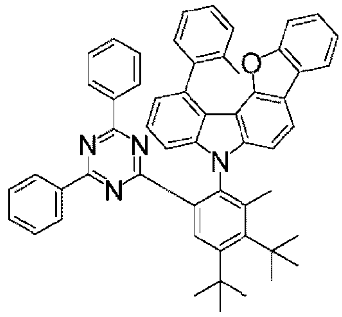
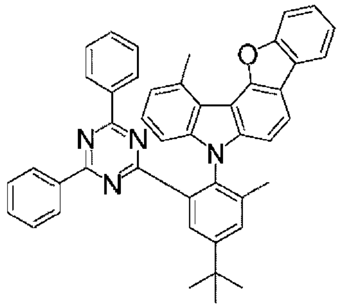
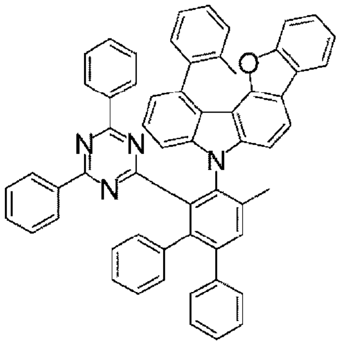
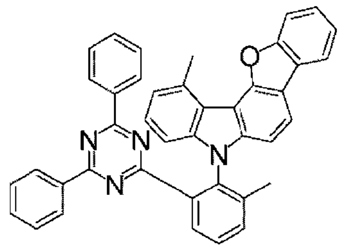
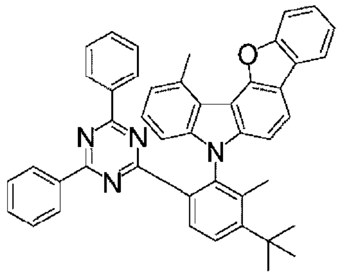
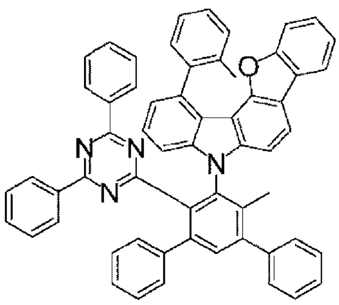
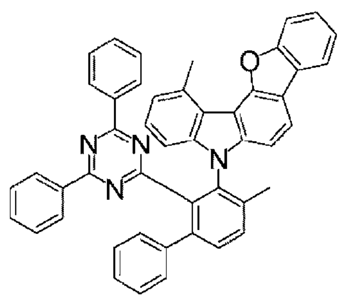
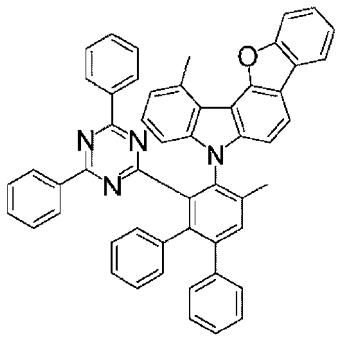
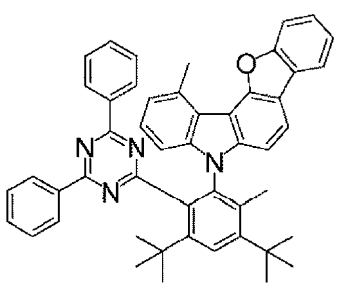
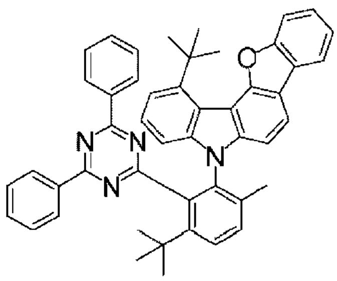
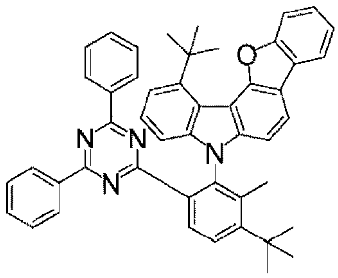
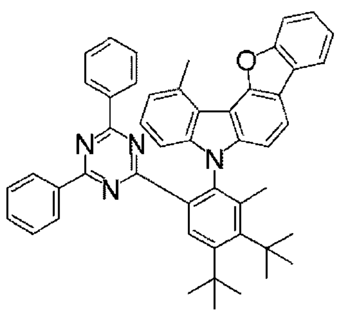
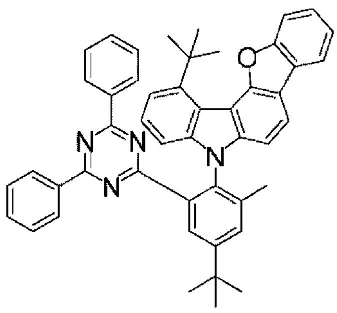

[Chem. 27]

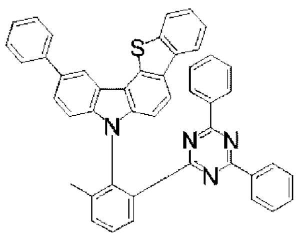
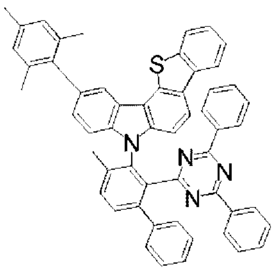
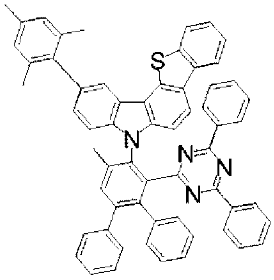

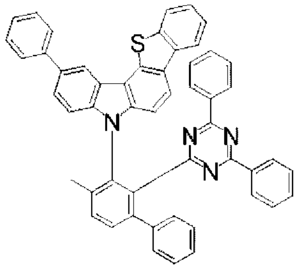
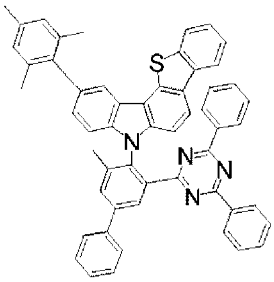
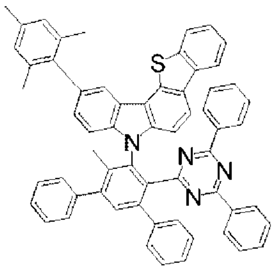

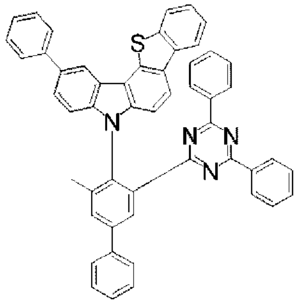
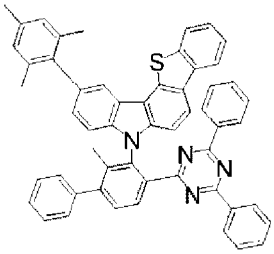
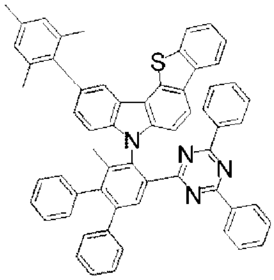

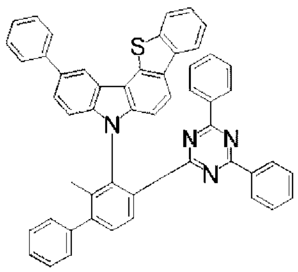
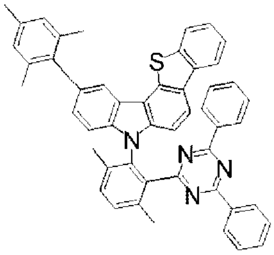
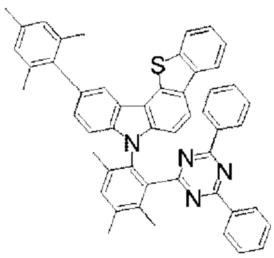

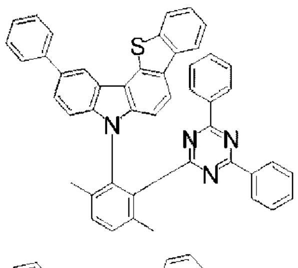
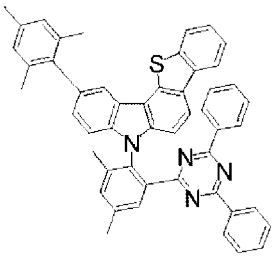
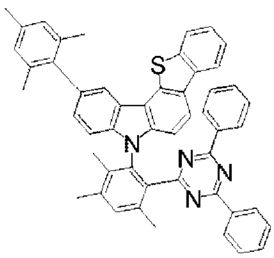

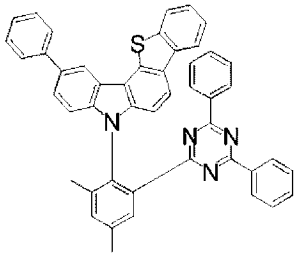
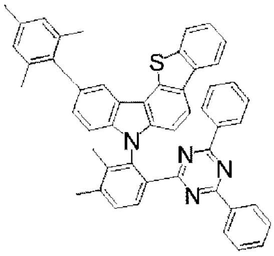
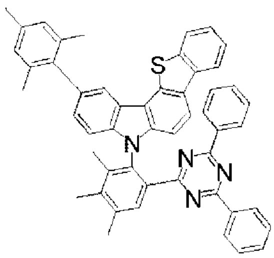

[Chem. 28]

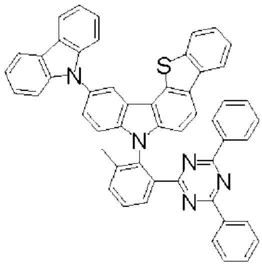
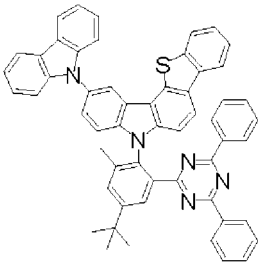
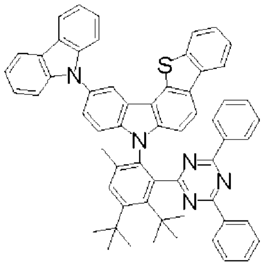
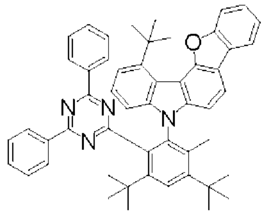
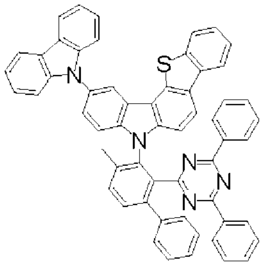
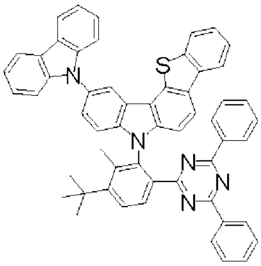
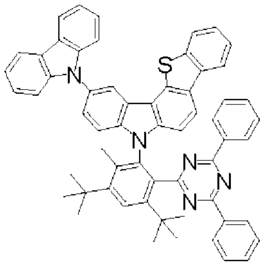
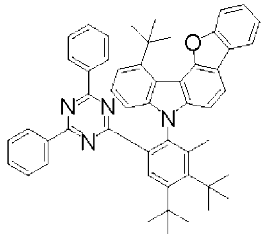
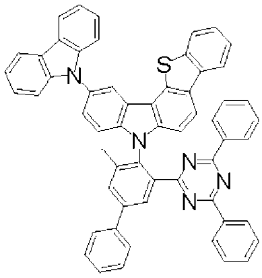
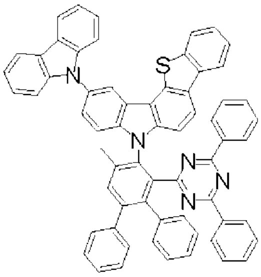
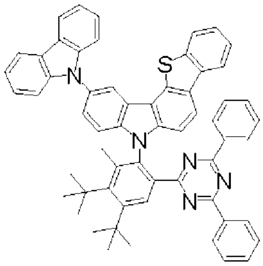
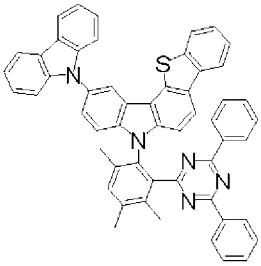
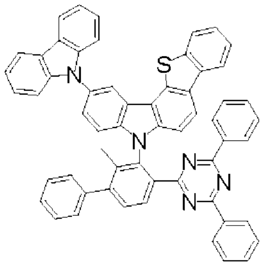
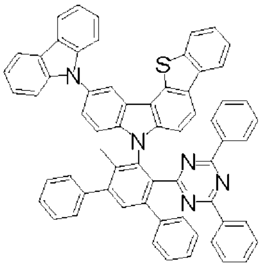
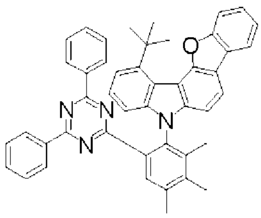
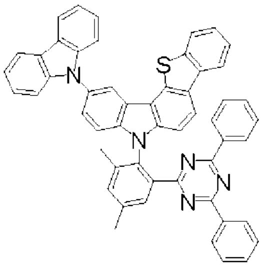
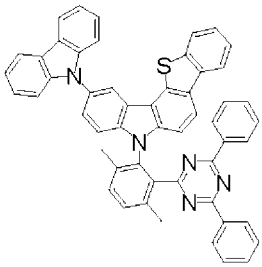
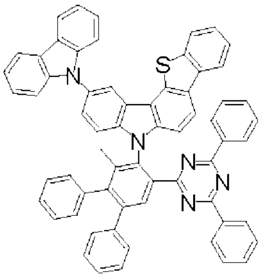
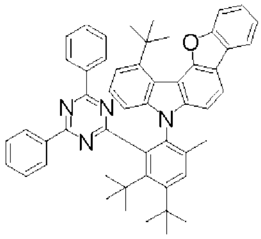

[Chem. 29]

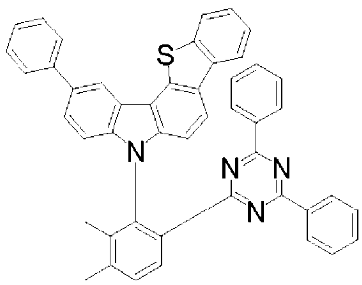
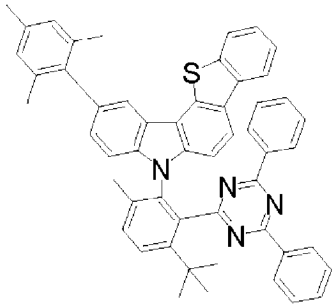
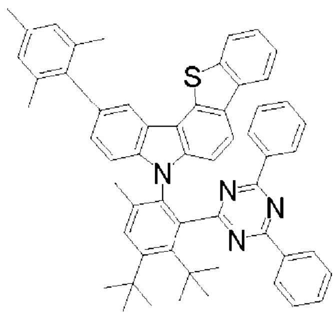
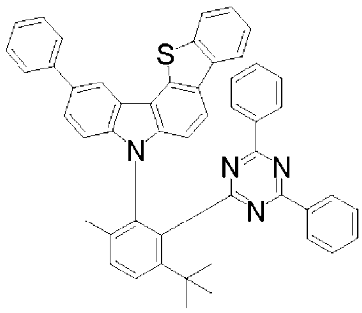
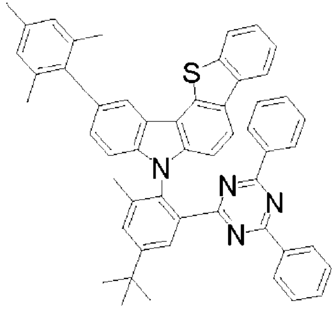
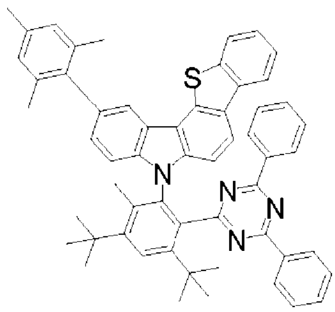
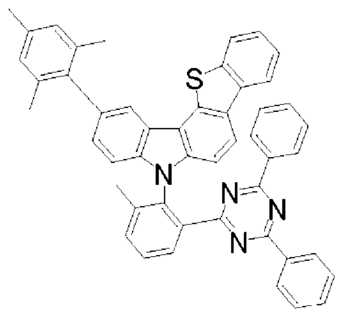
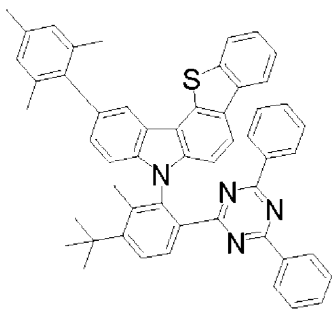
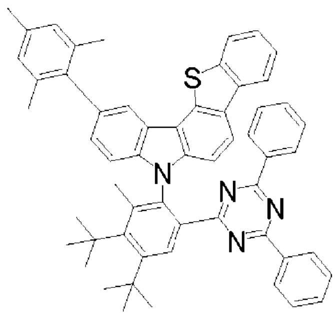
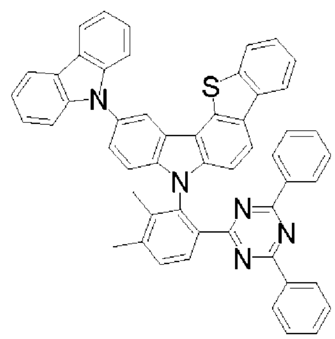
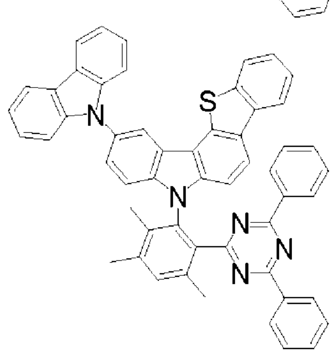
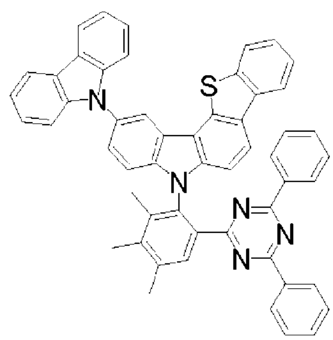
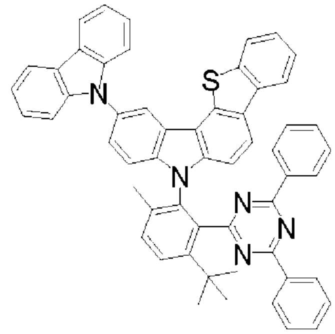

[Chem. 30]

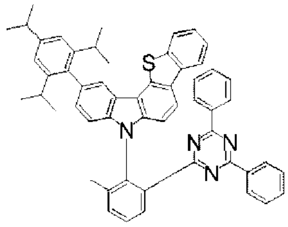
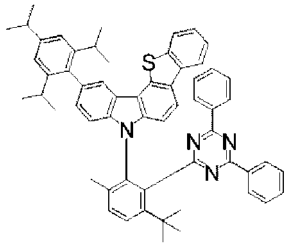
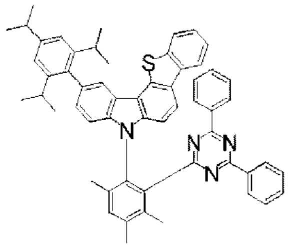
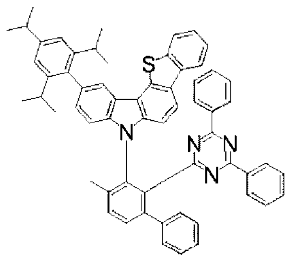
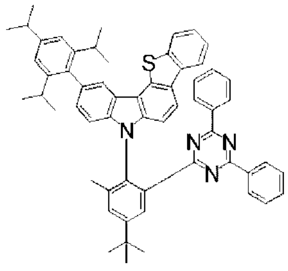
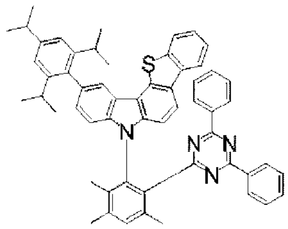
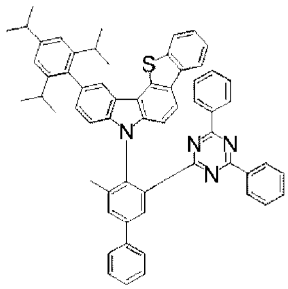
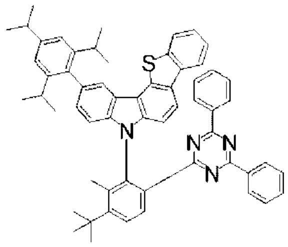
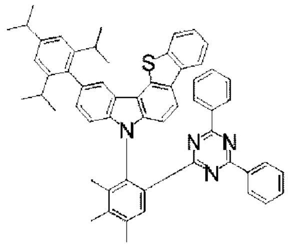
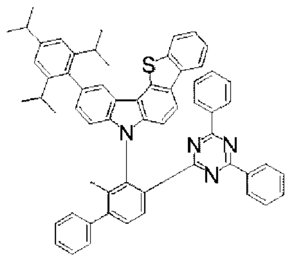
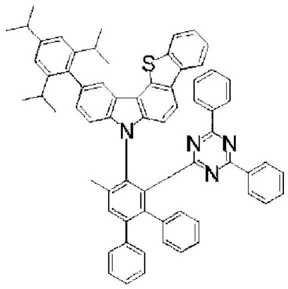
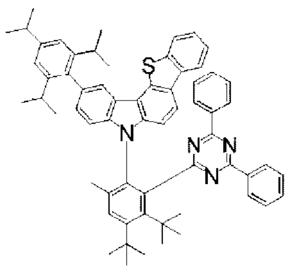
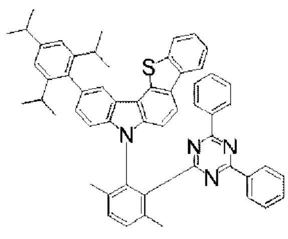
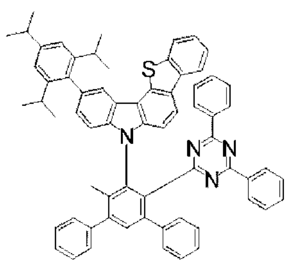
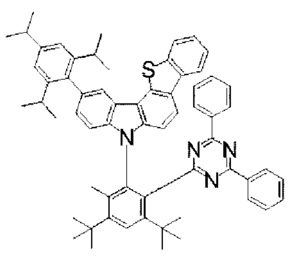
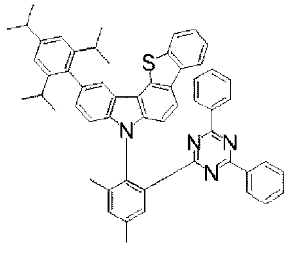
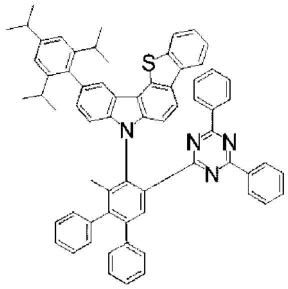
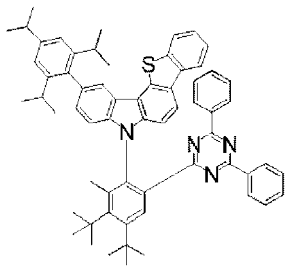

[Chem. 31]

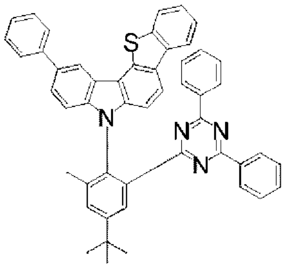
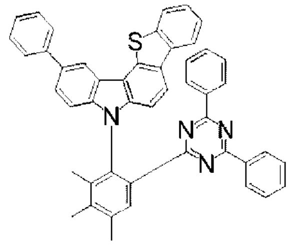
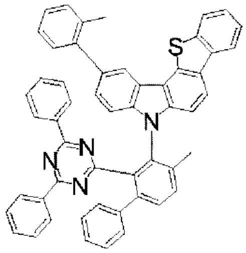
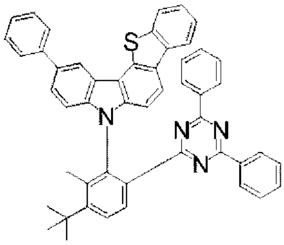
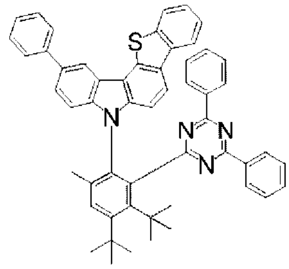
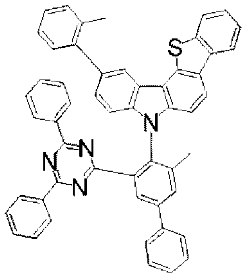
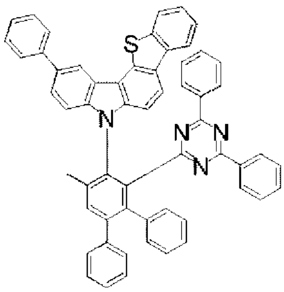
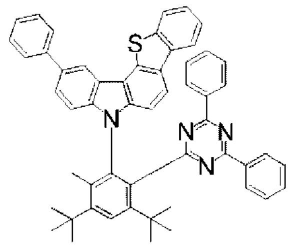
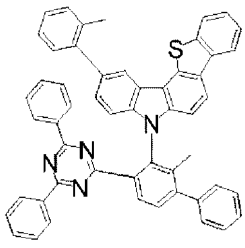
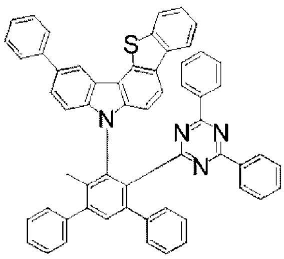
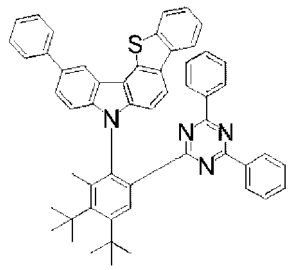
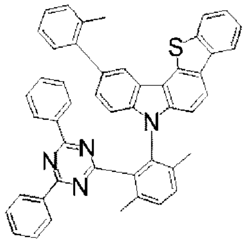
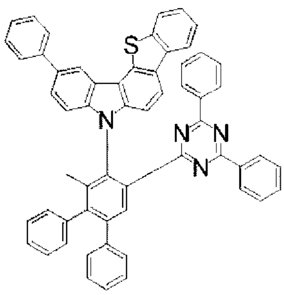
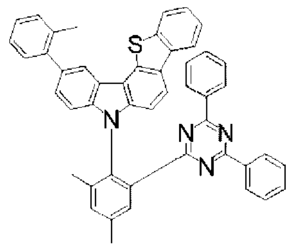
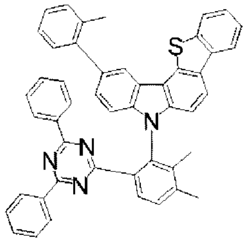
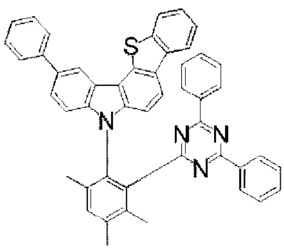
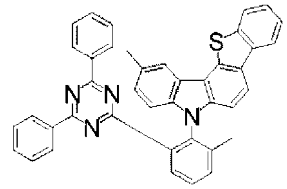
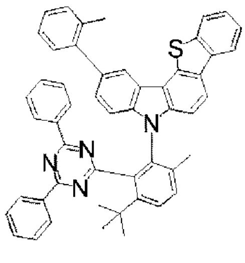

[Chem. 32]

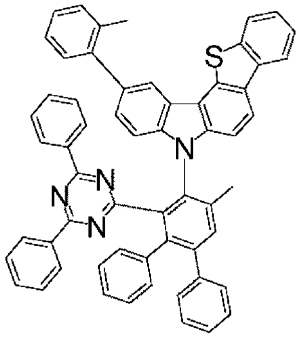
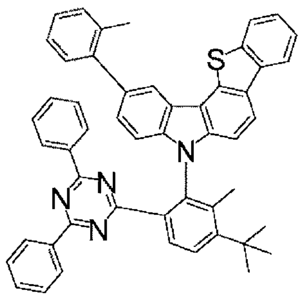
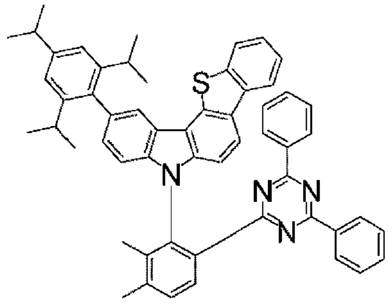
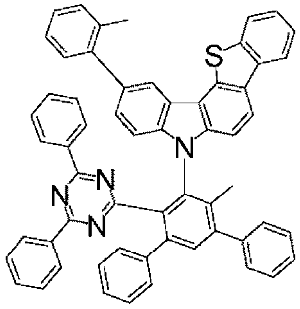
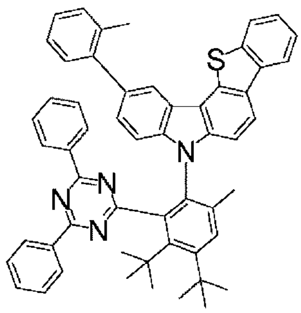
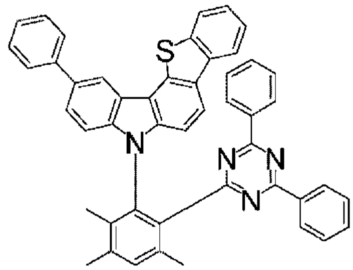
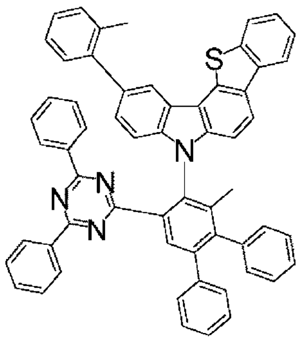
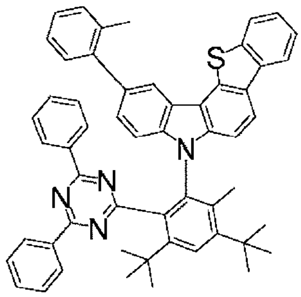
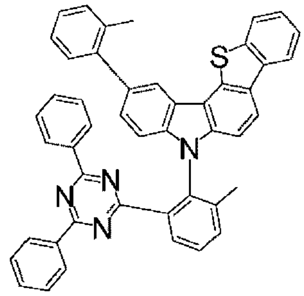
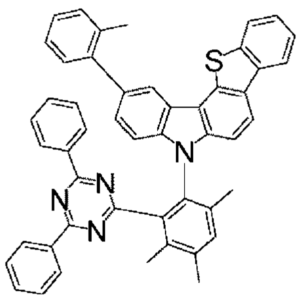
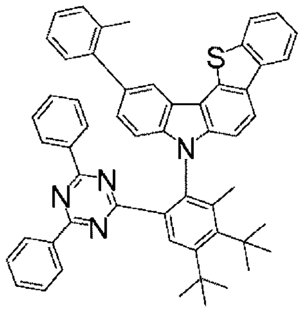
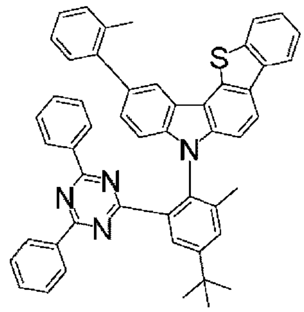
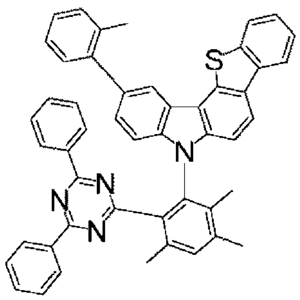
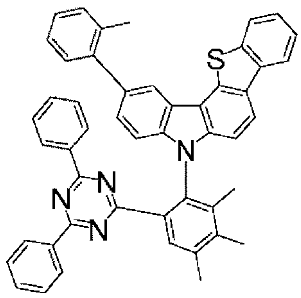

[Chem. 33]

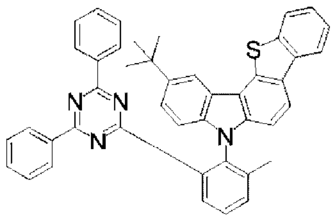
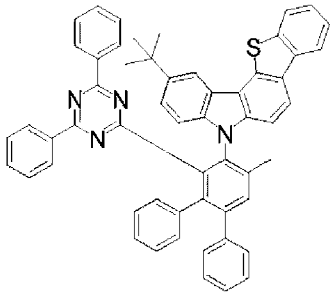
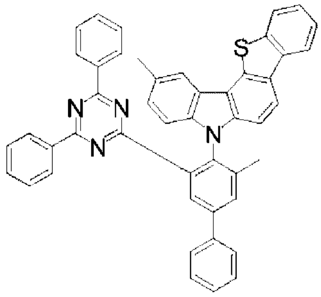
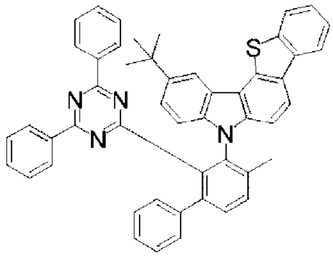
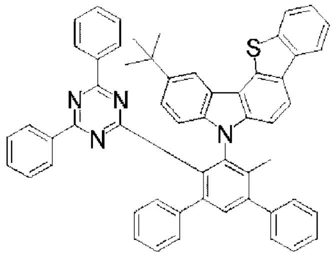
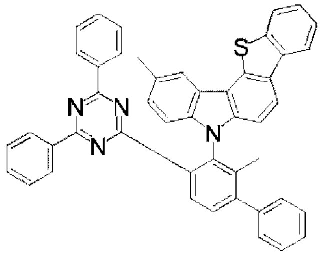
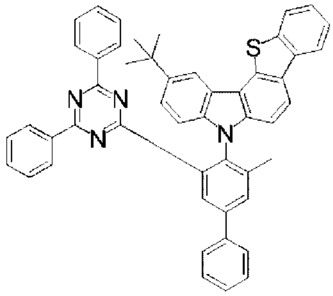
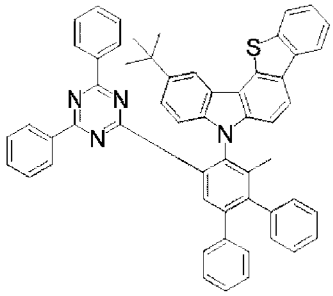
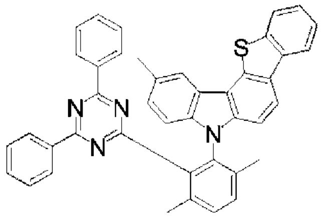
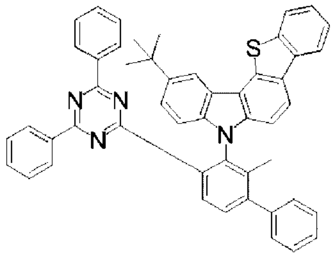
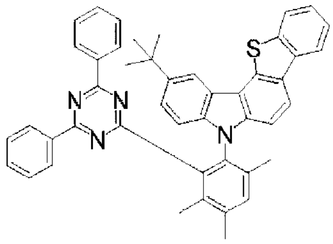
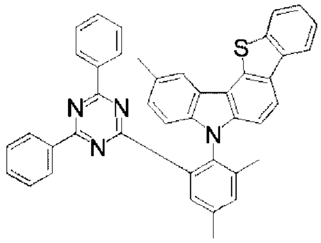
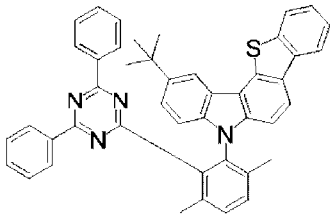
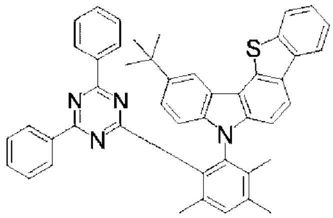
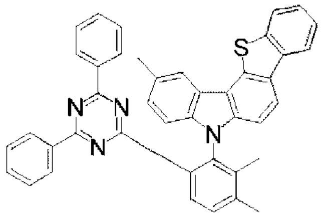
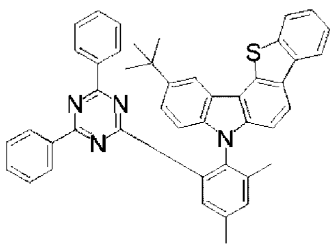
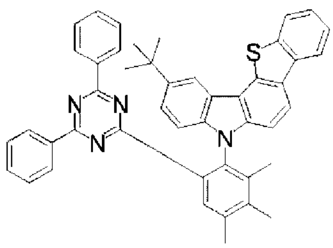
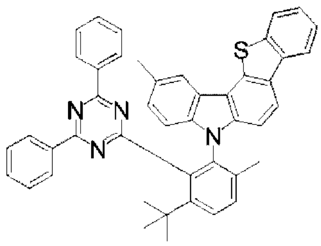

[Chem. 34]

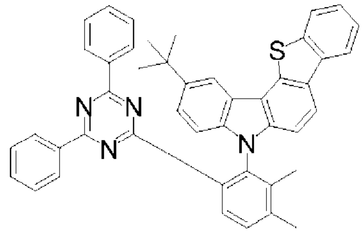
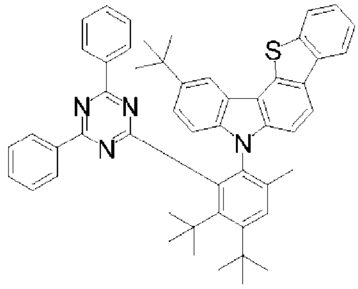
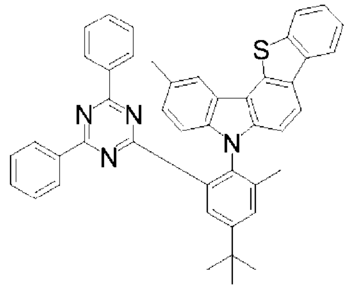
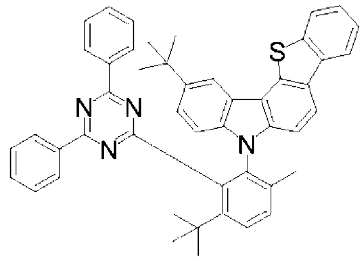
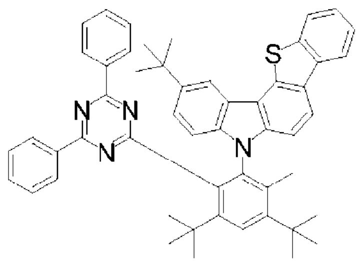
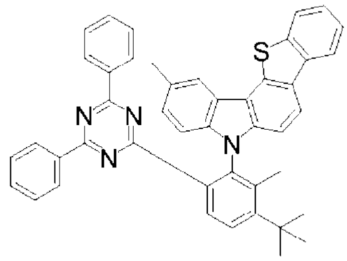
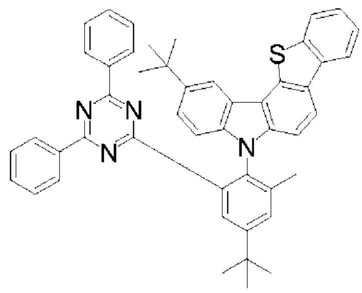
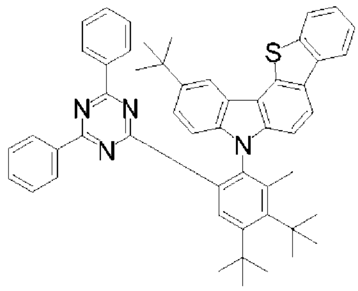
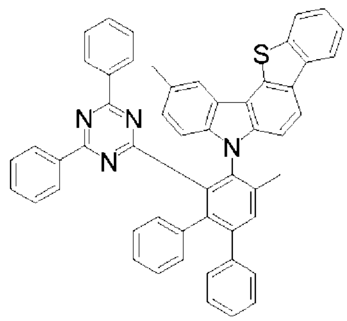
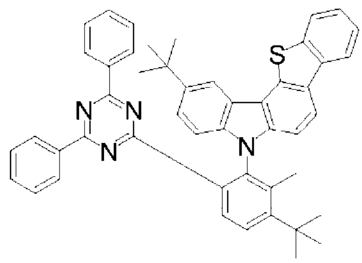
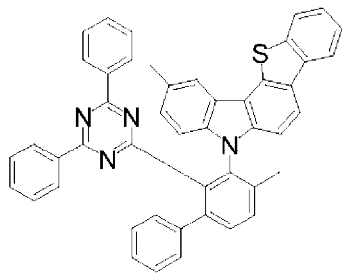
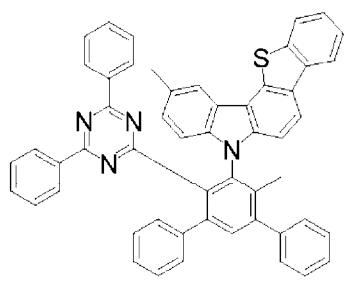
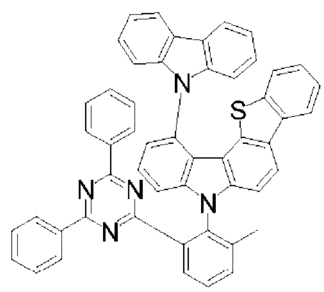
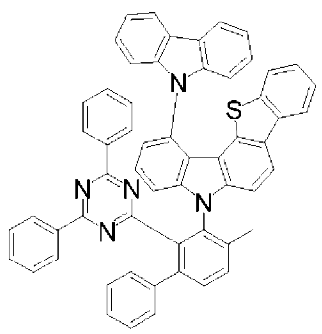
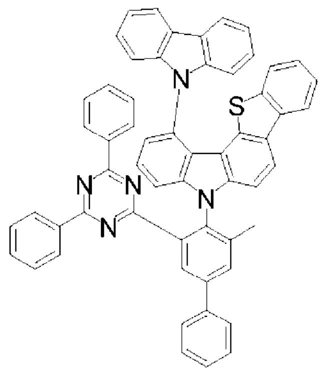

[Chem. 35]

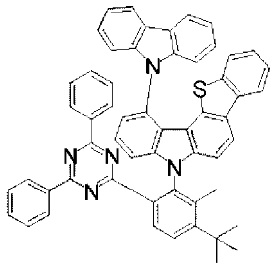
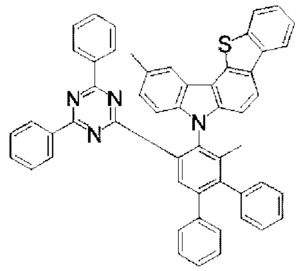
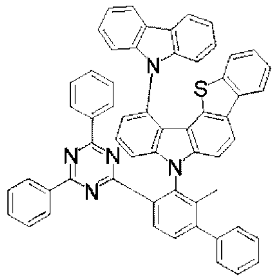
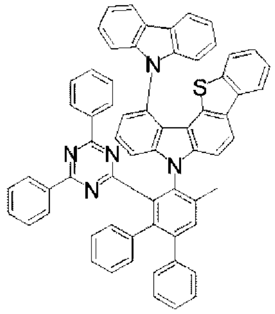
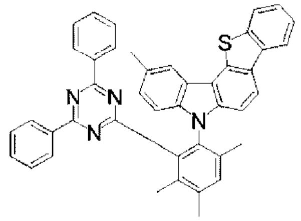
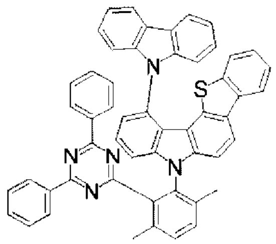
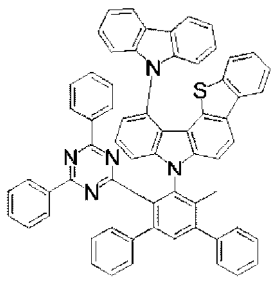
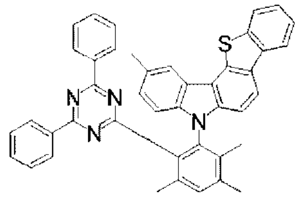
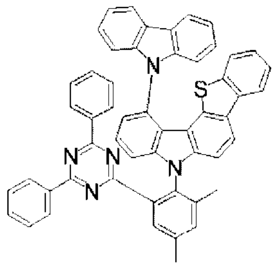
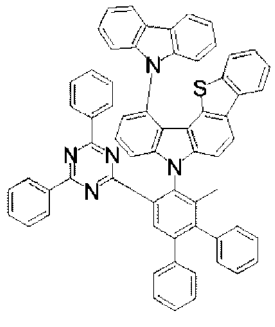
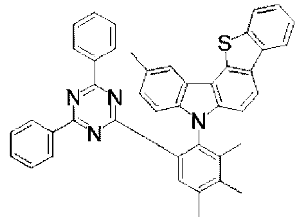
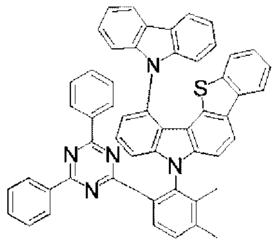
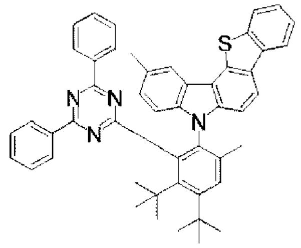
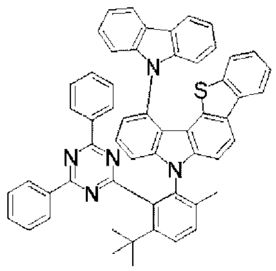
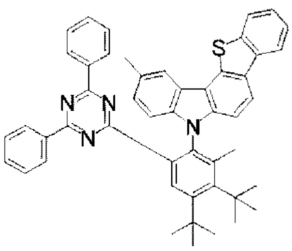
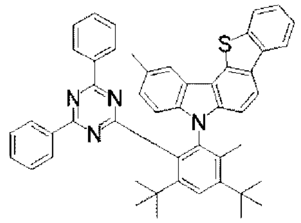
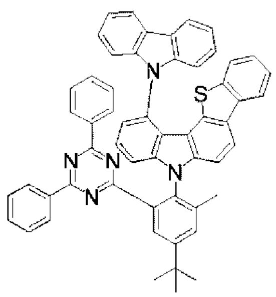

[Chem. 36]

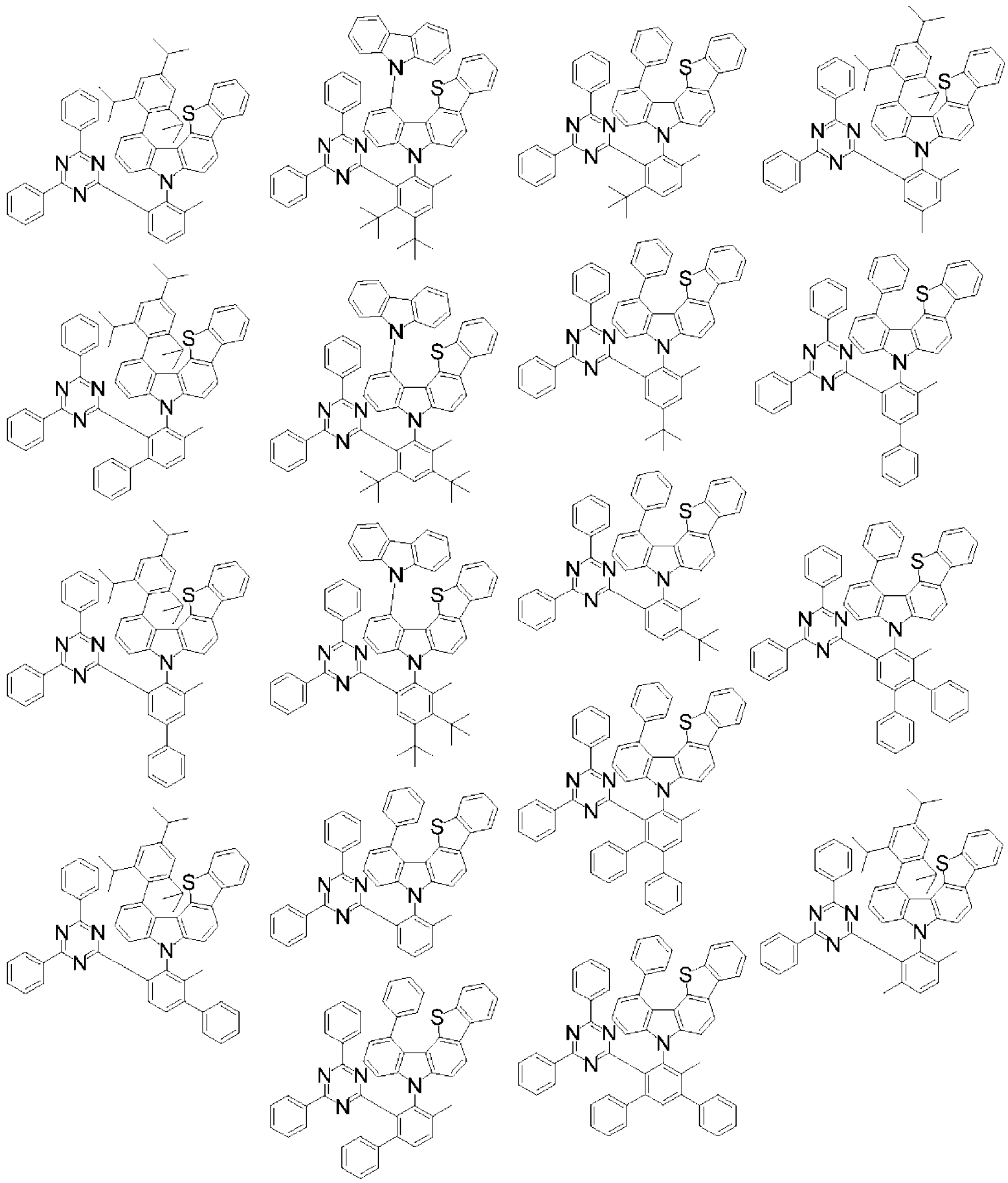

[Chem. 37]

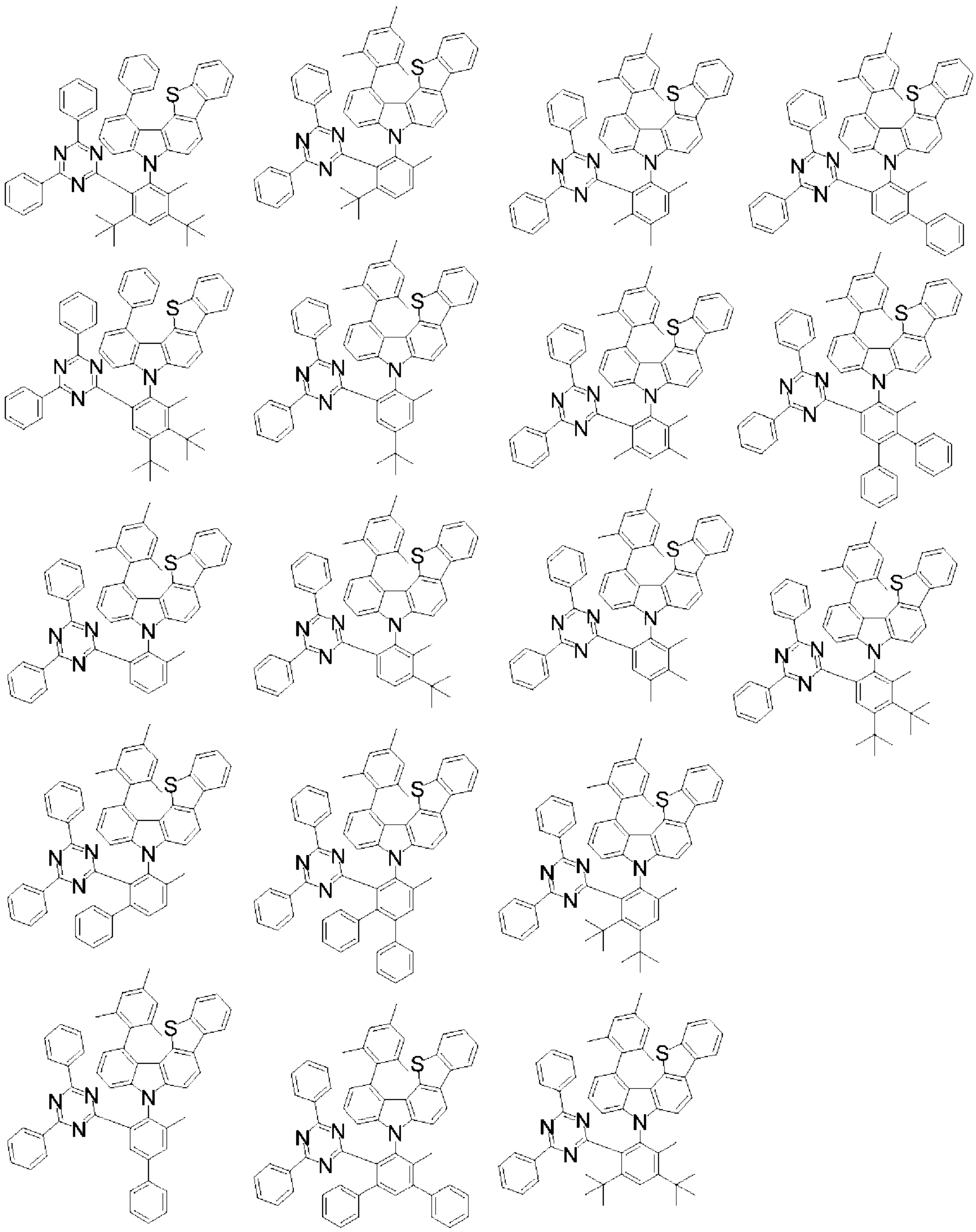

[Chem. 38]

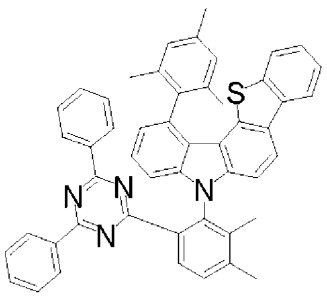
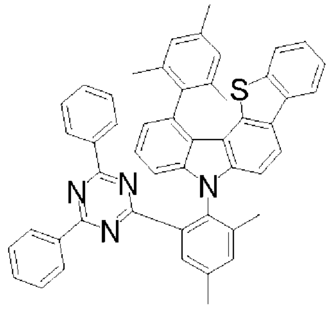
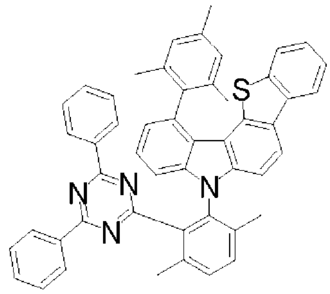
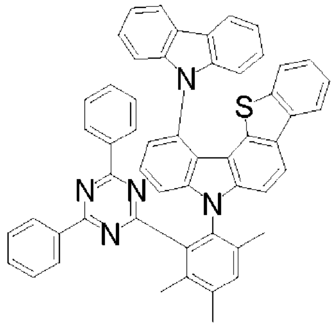
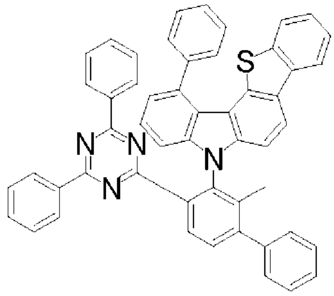
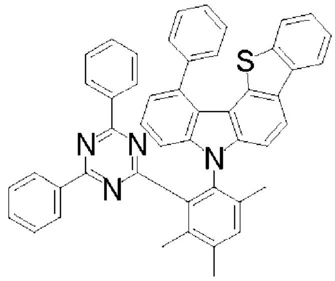
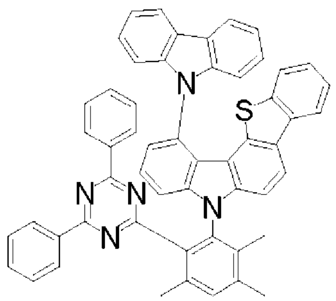
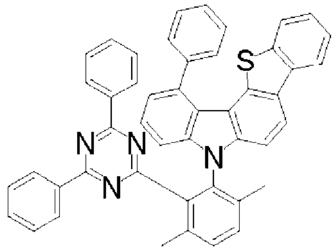
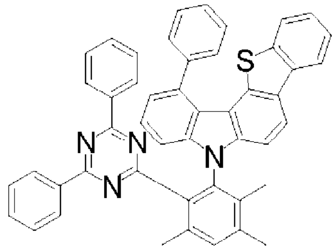
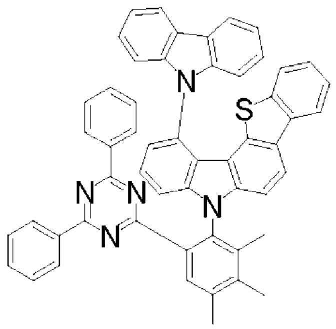
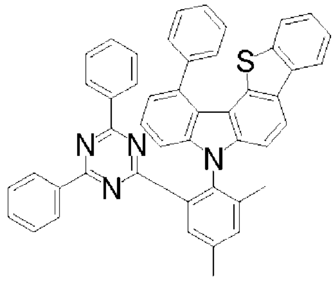
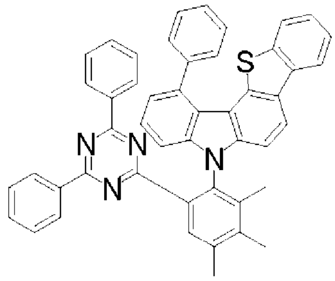
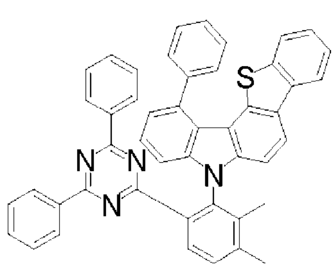
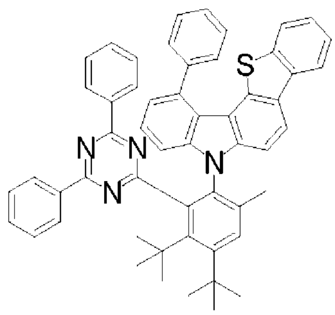

[Chem. 39]

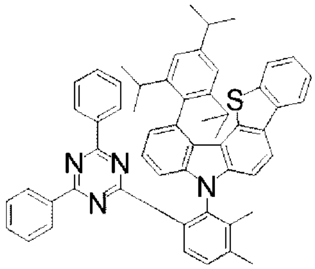
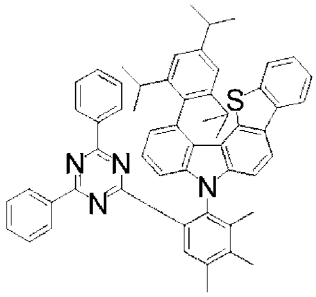
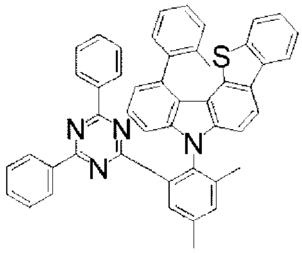
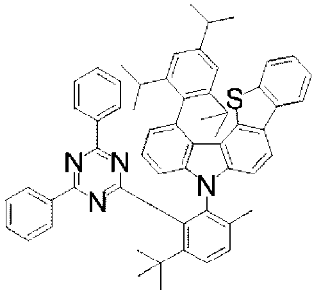
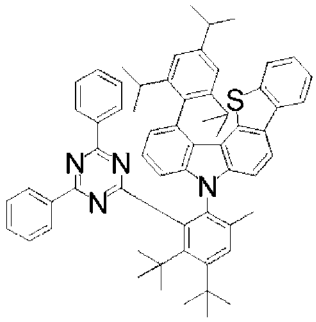
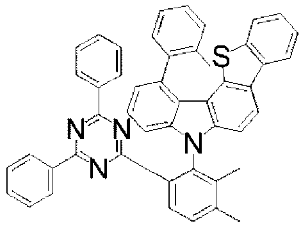
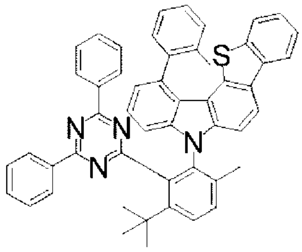
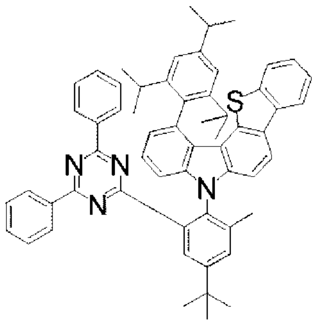
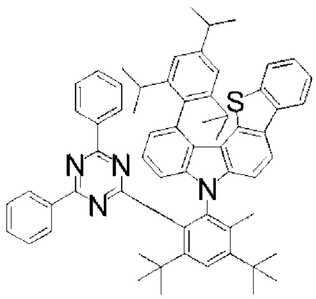
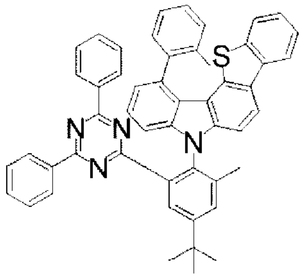
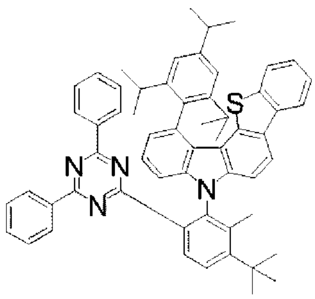
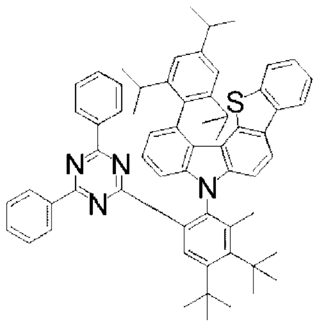
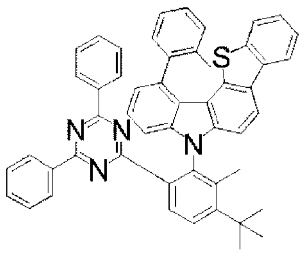
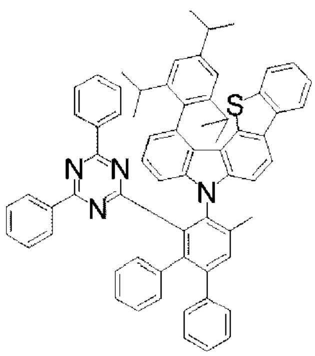
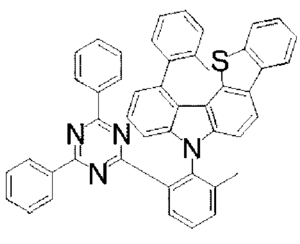
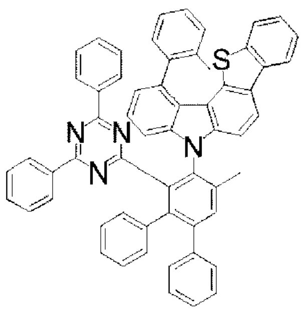

[Chem. 40]

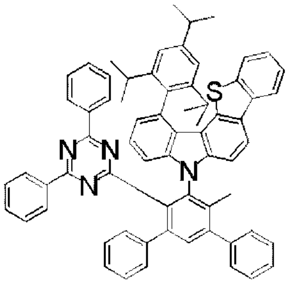
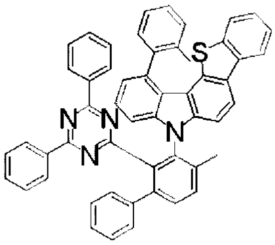
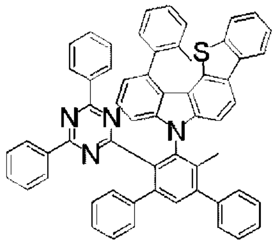
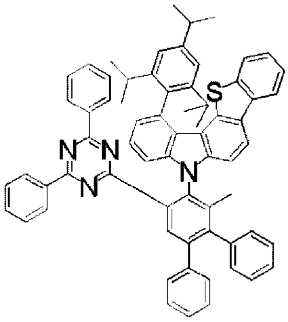
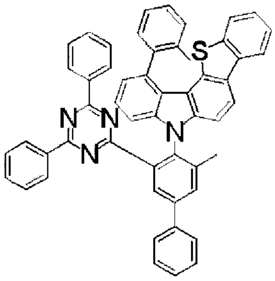
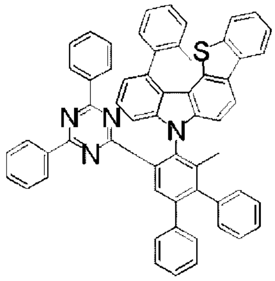
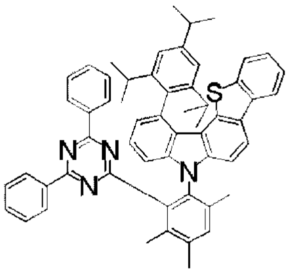
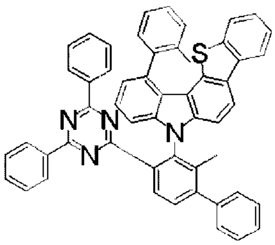
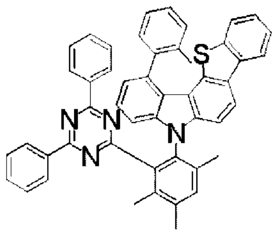
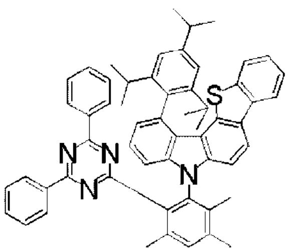
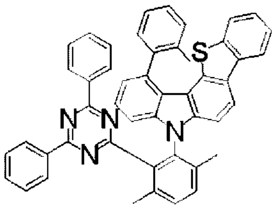
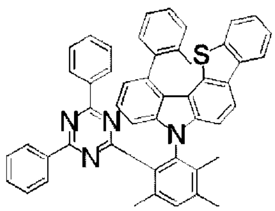
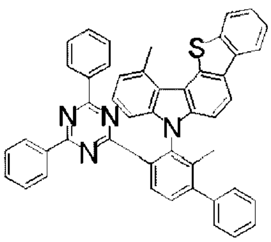
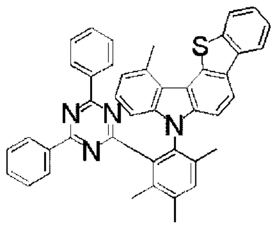
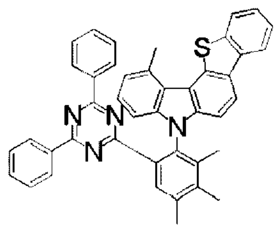
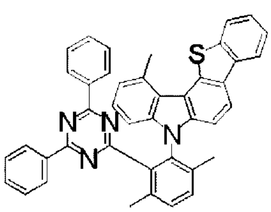
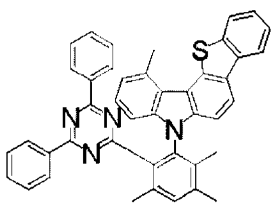
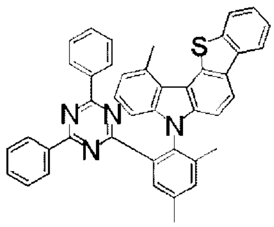

[Chem. 41]

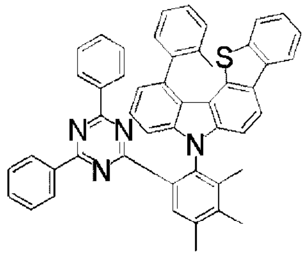
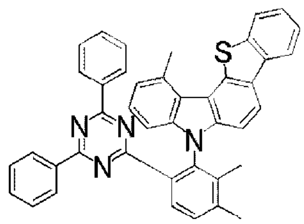
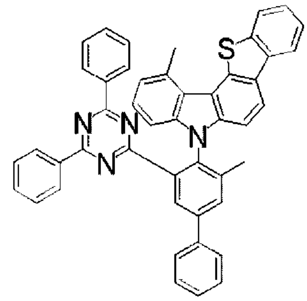
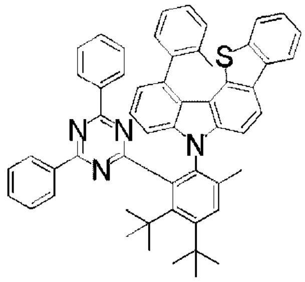
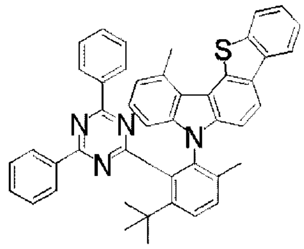
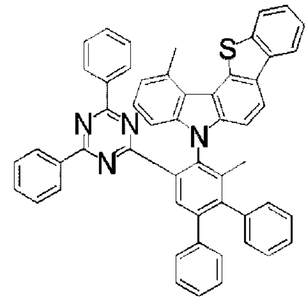
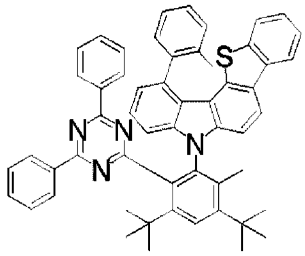
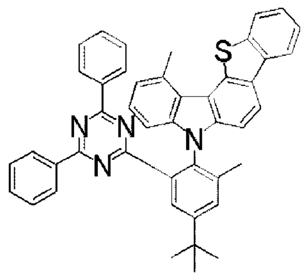
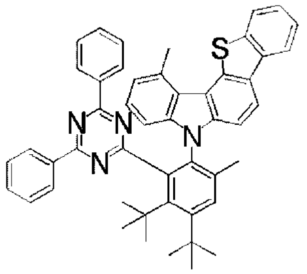
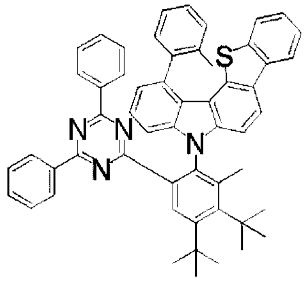
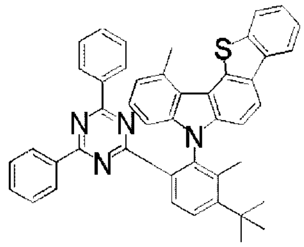
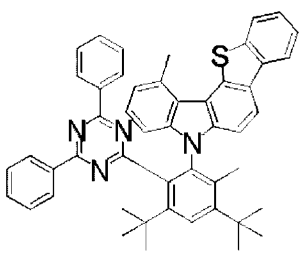
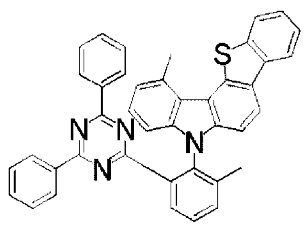
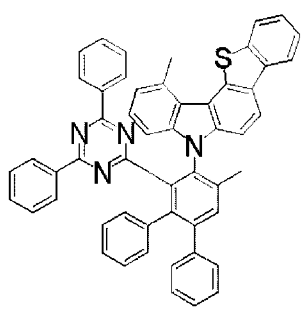
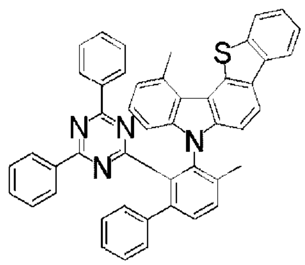
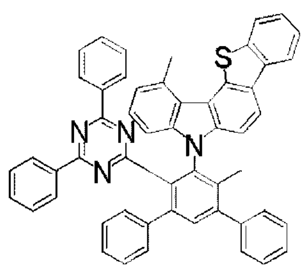

[Chem. 42]

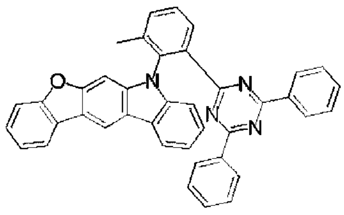
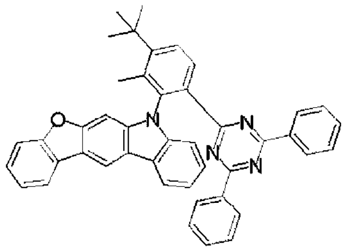
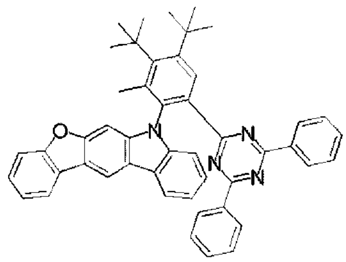
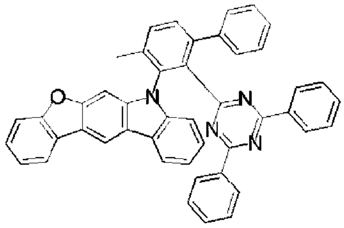
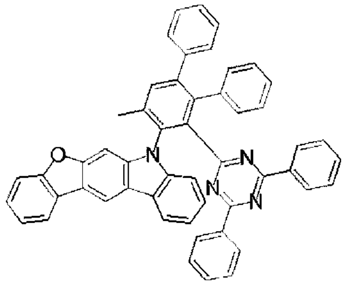
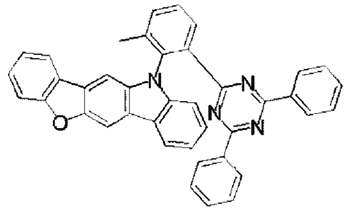
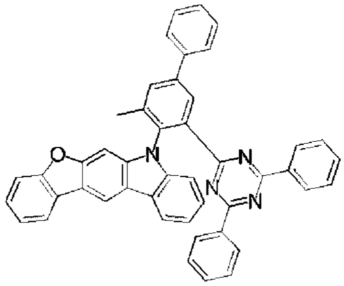
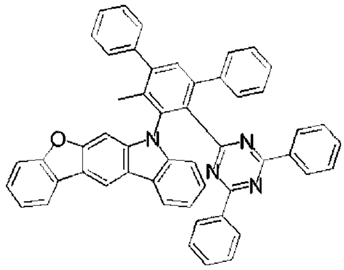
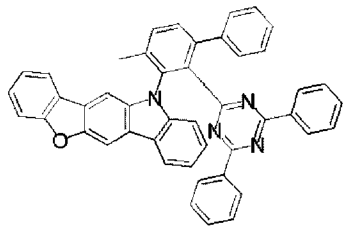
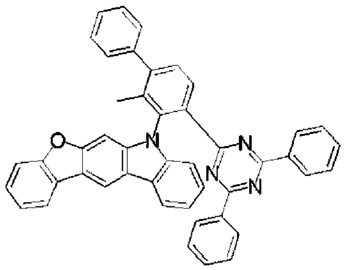
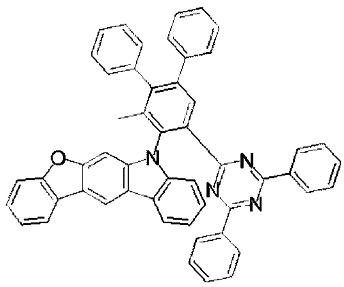
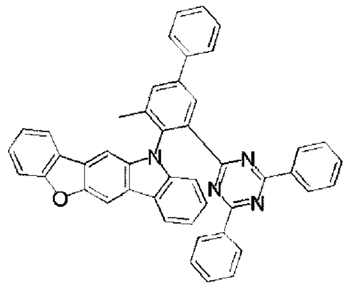
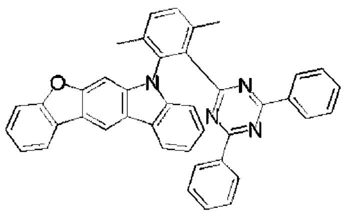
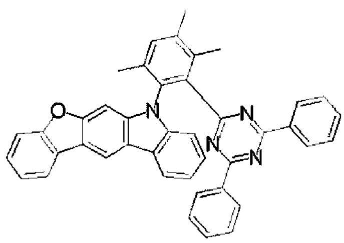
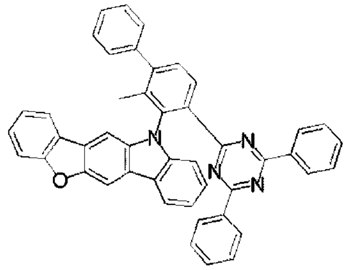
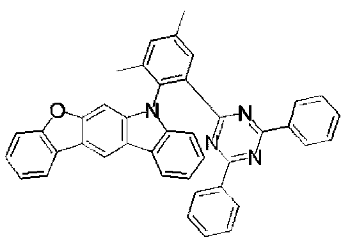
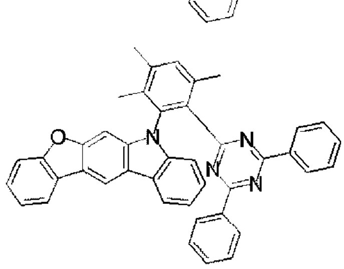
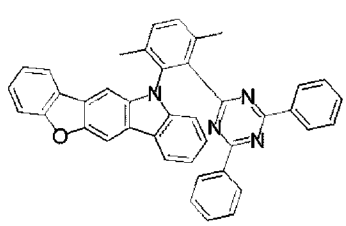

[Chem. 43]

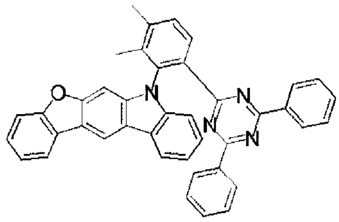
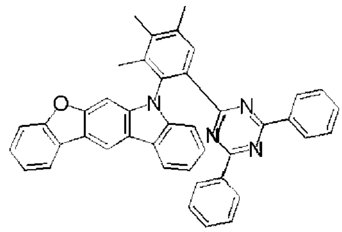
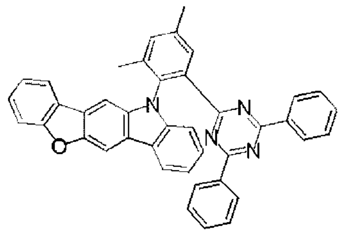
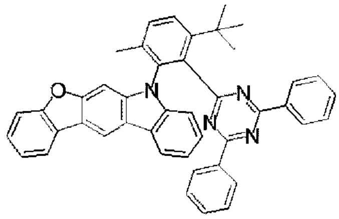
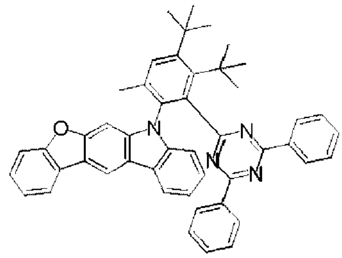
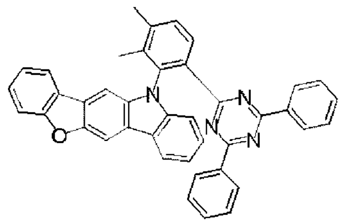
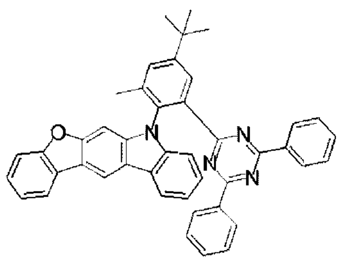
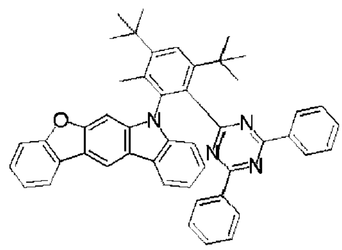
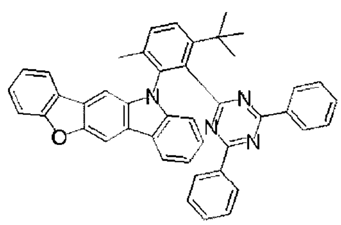
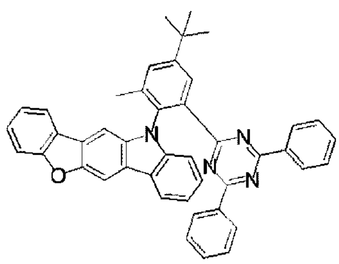
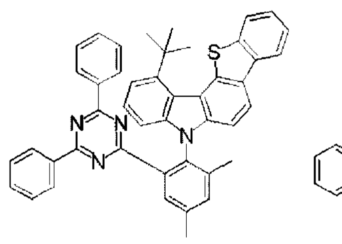
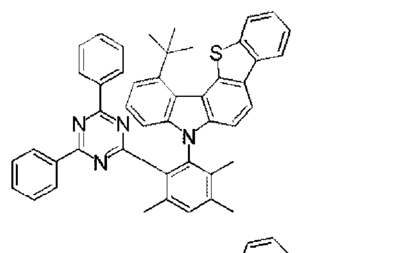
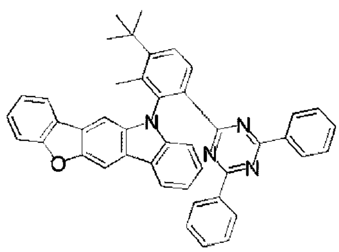
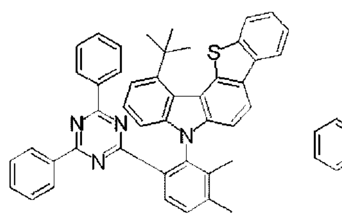
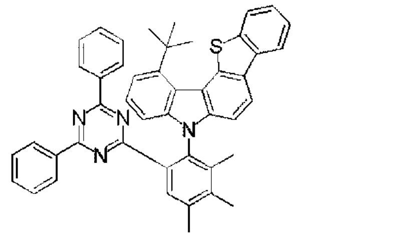
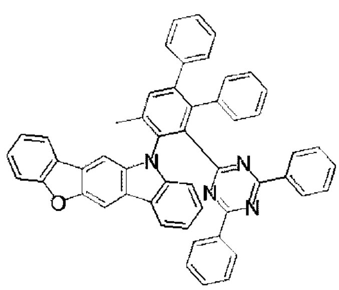
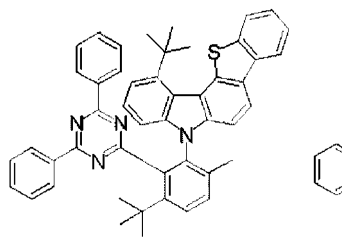
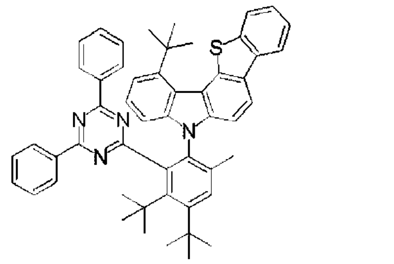

[Chem. 44]

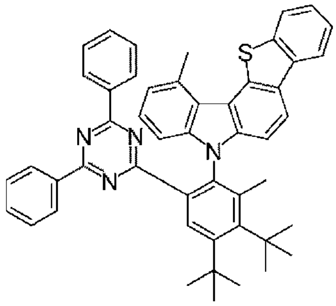
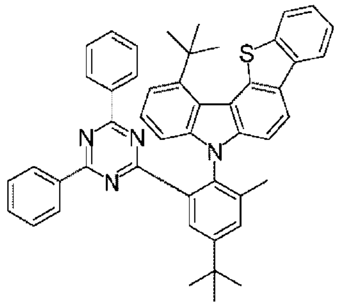
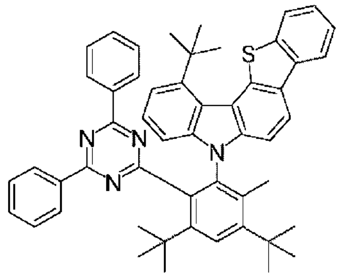
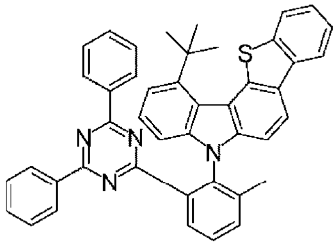
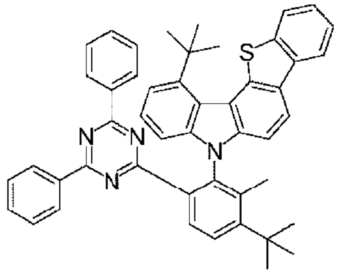
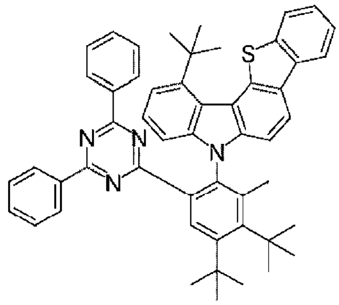
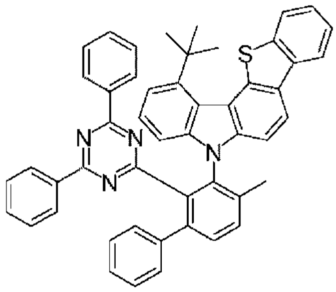
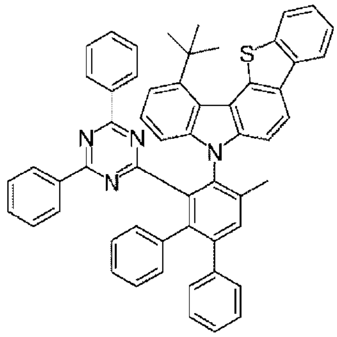
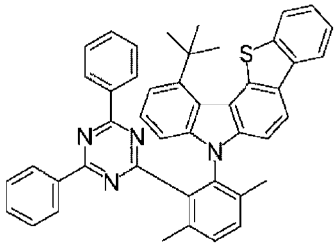
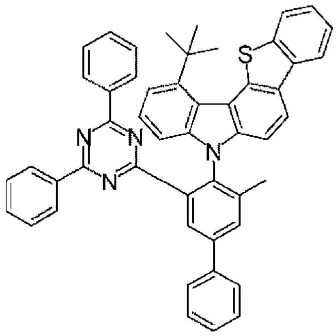
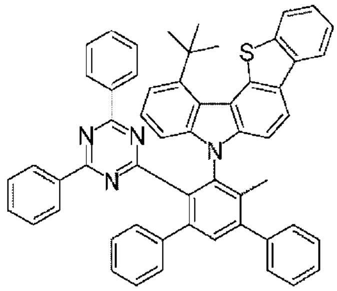
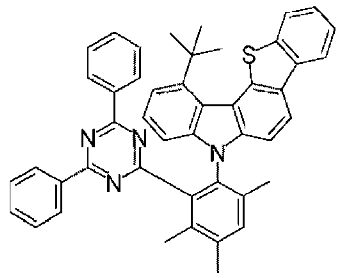
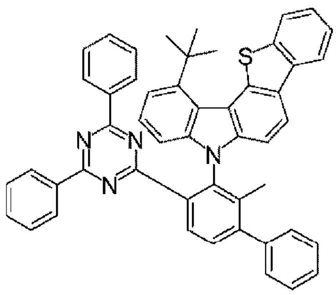
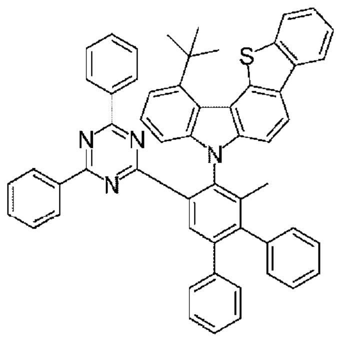

[Chem. 45]

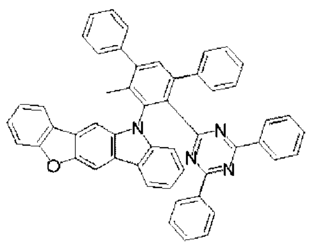
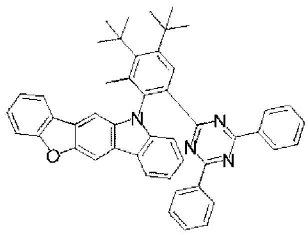
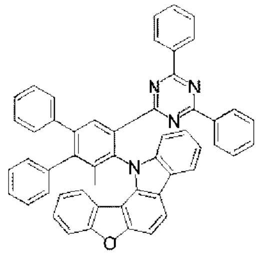
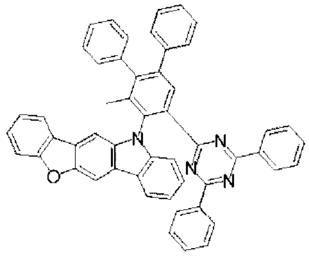
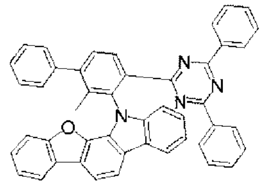
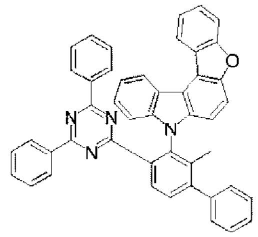
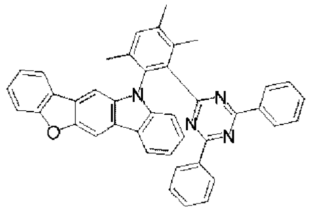
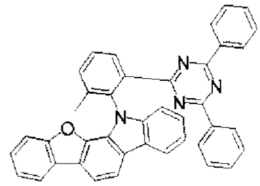
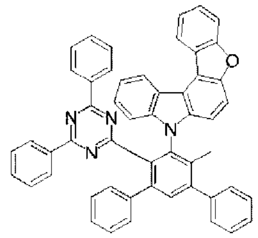
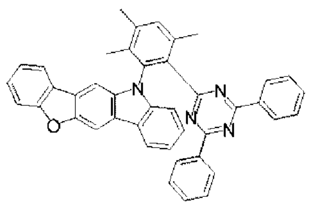
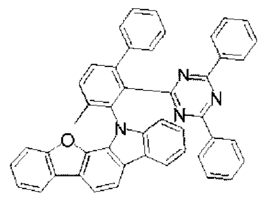
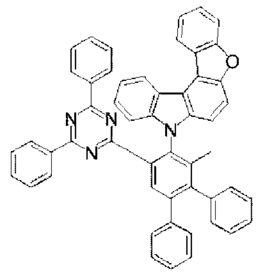
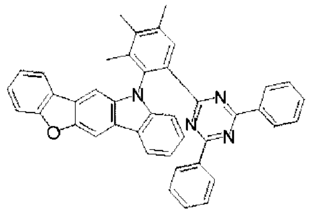
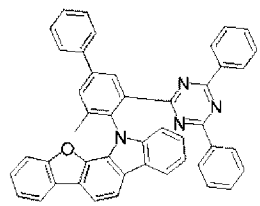
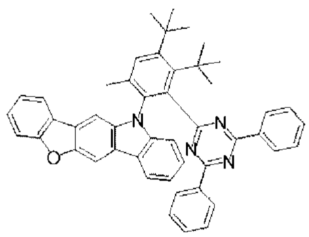
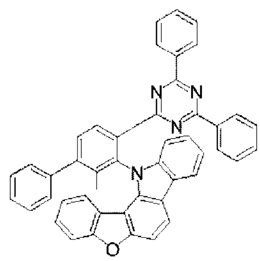
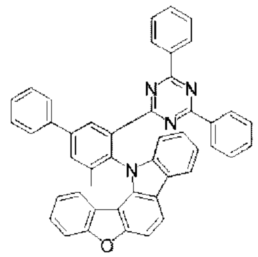
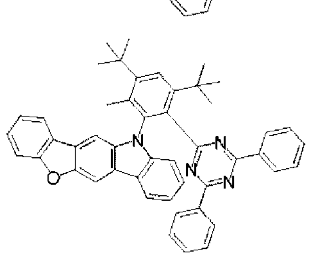
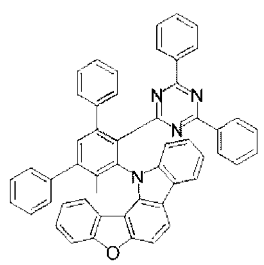
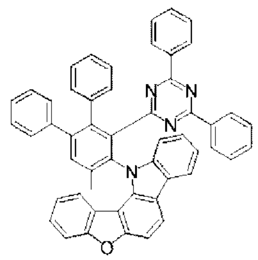

[Chem. 46]

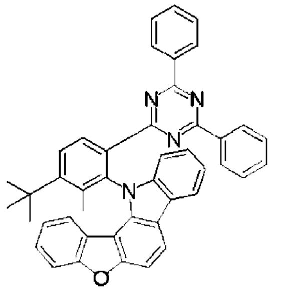

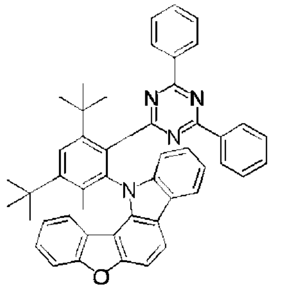

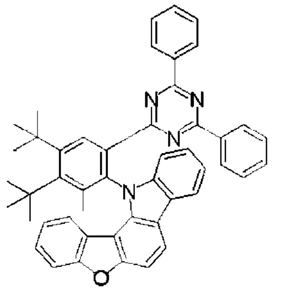

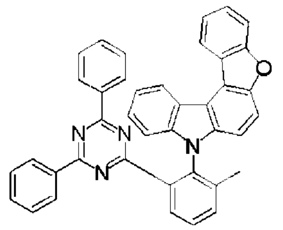

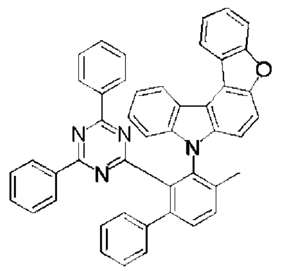

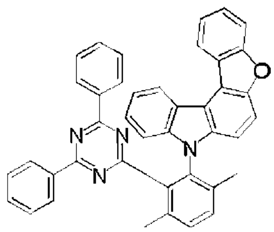

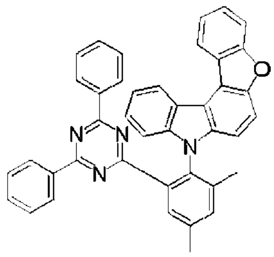

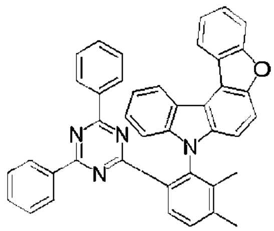

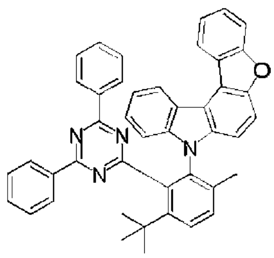

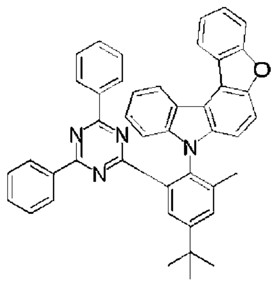

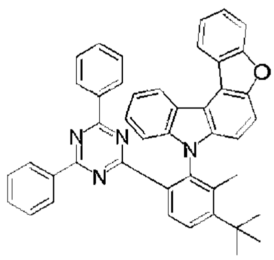

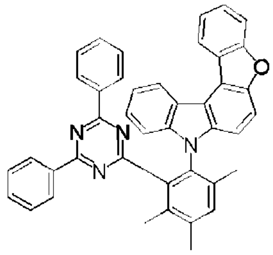

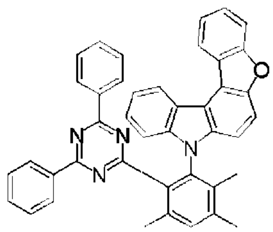

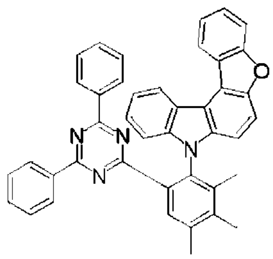

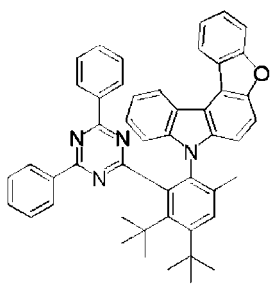

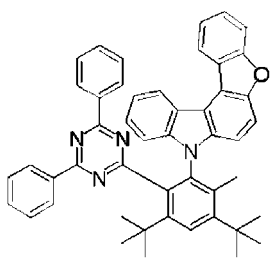

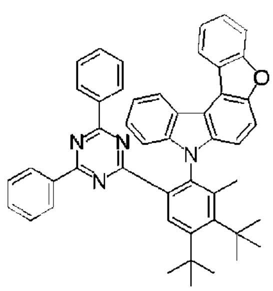

[Chem. 47]

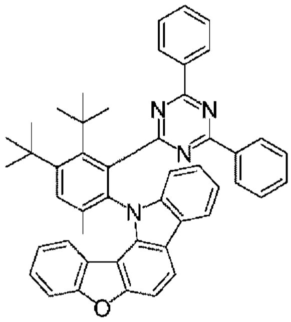
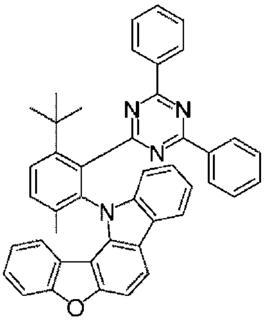
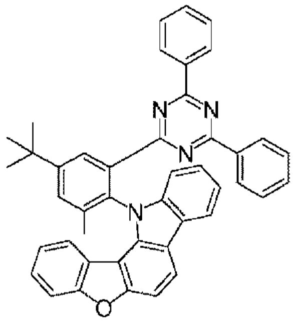
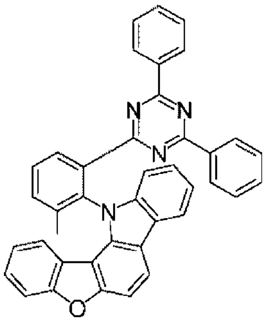
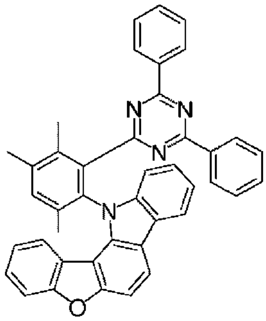
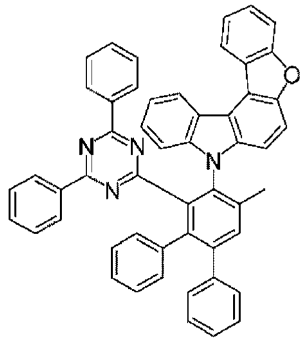
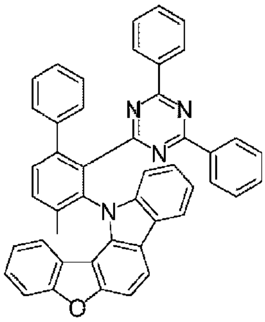
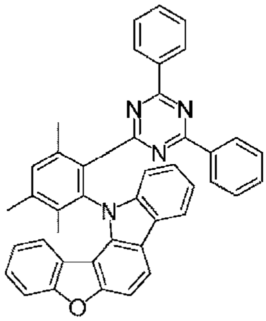
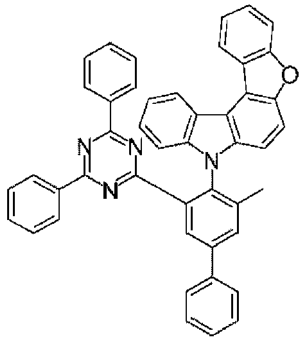
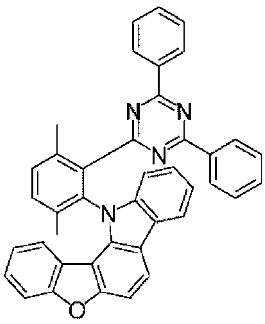
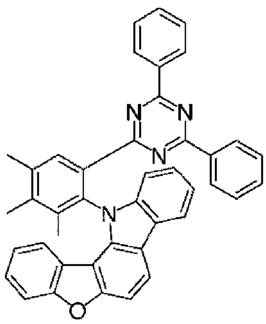
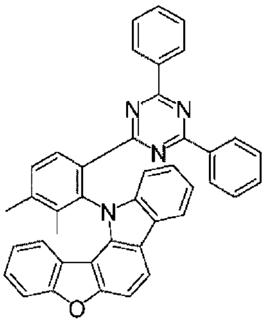
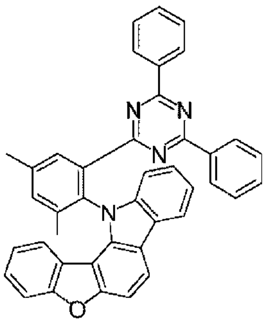

[Chem. 48]

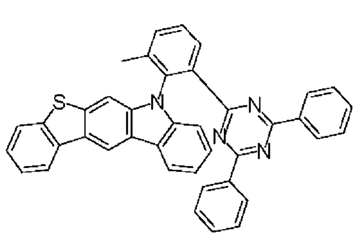

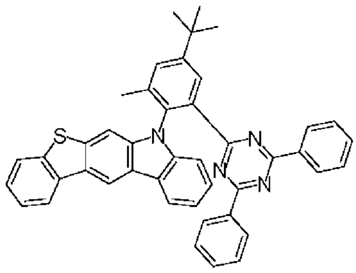

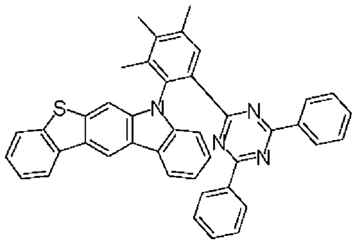

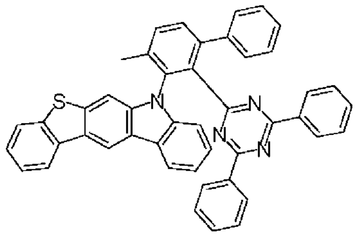

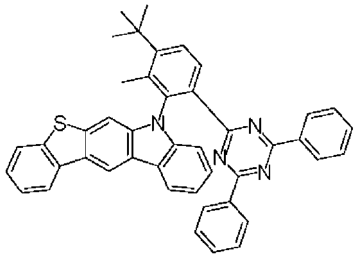

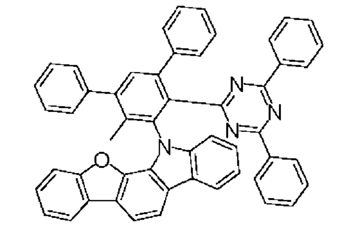

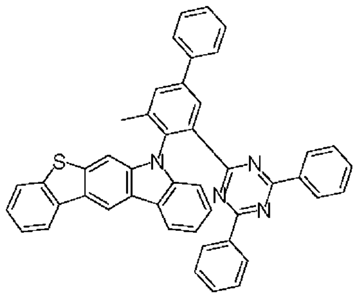

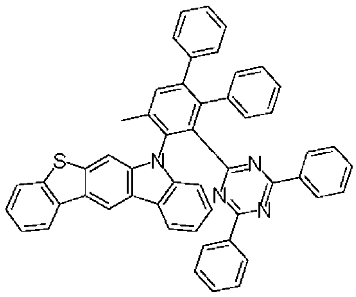

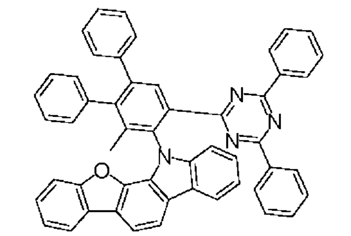

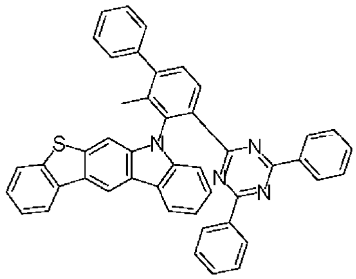

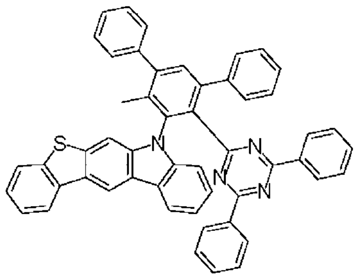

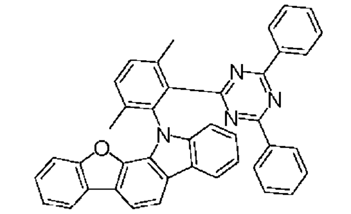

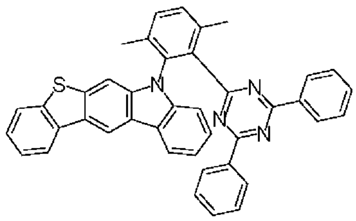

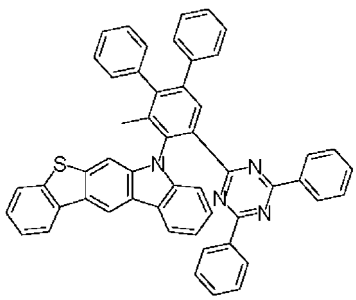

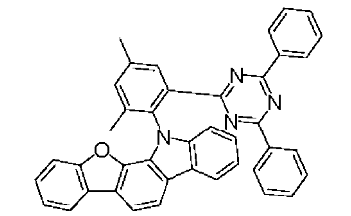

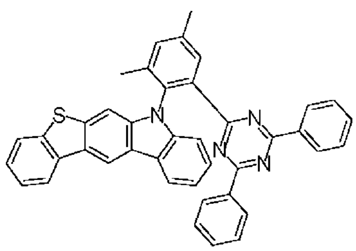

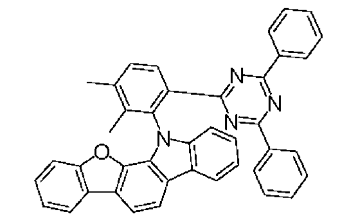

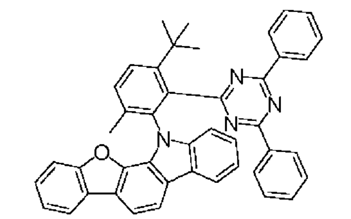

[Chem. 49]

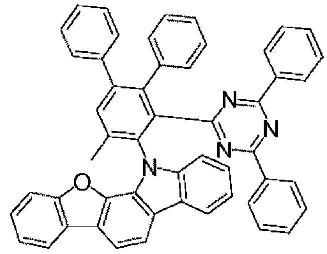
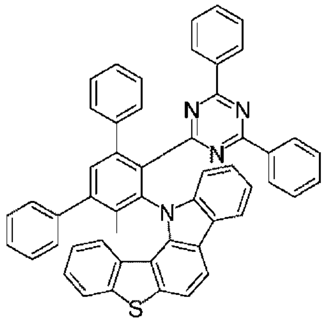
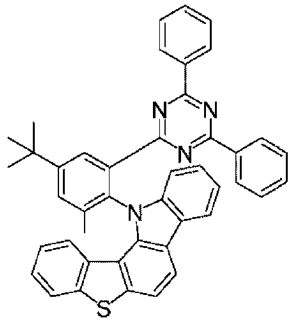
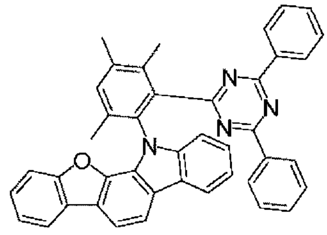
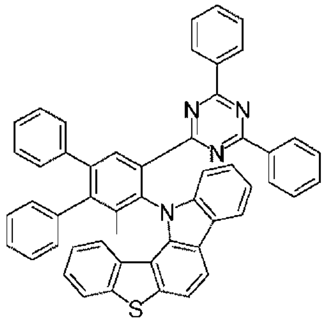
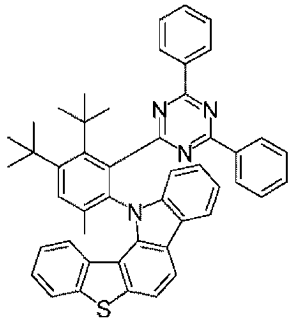
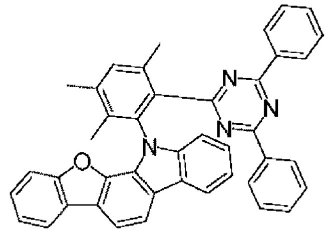
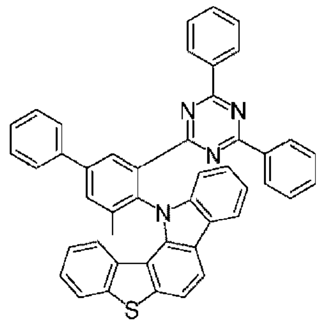
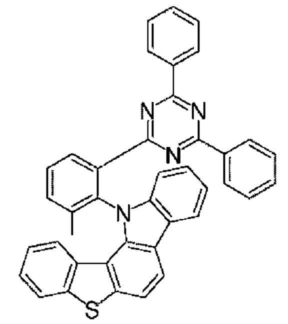
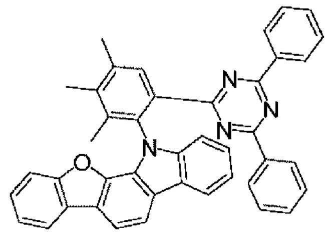
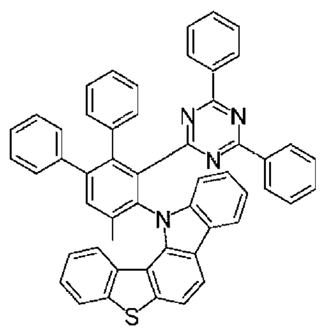
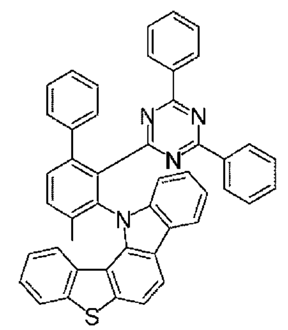
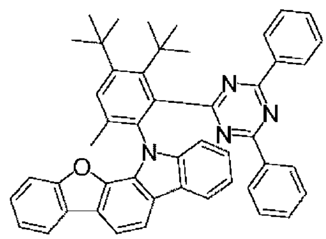
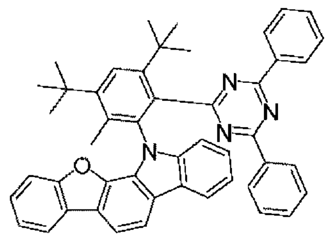
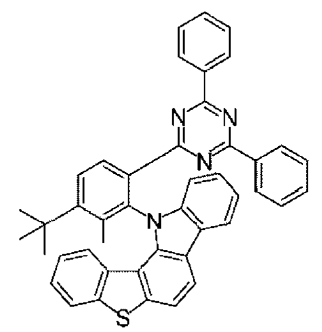
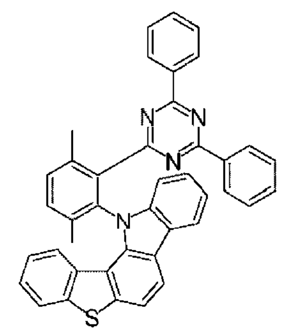

[Chem. 50]

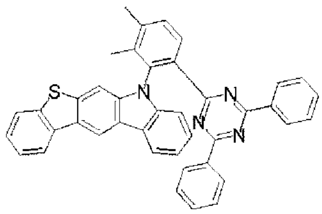
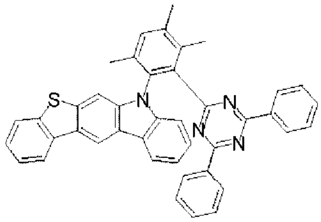
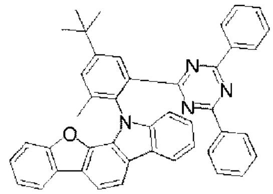
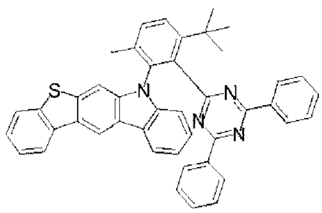
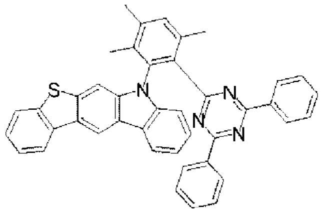
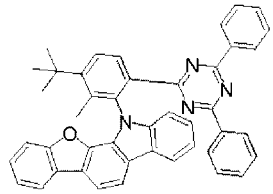
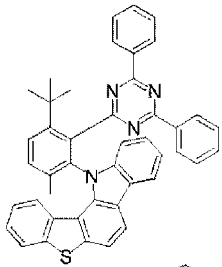
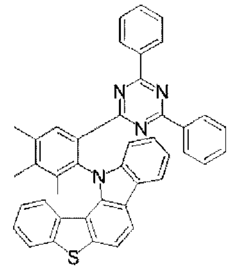
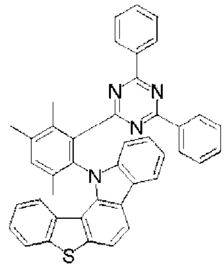
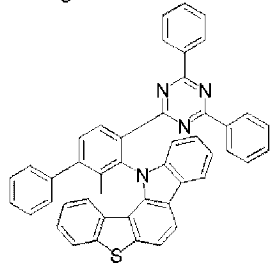
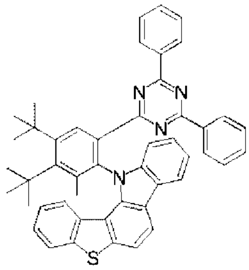
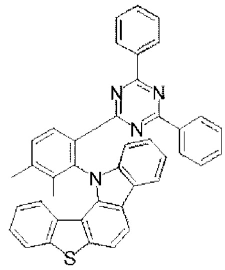
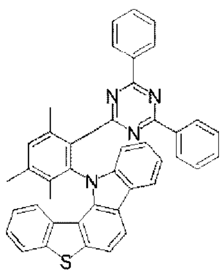
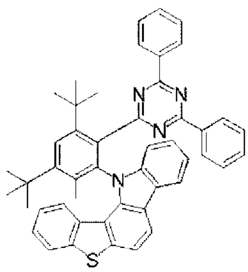
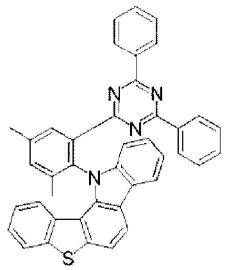
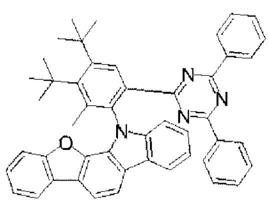

[Chem. 51]

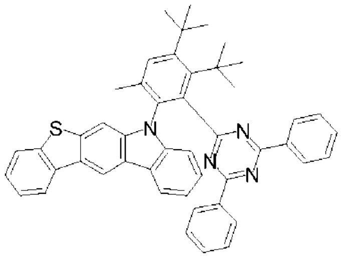
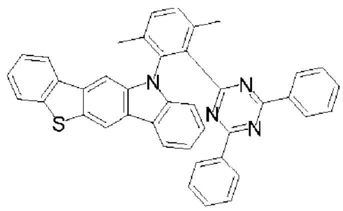
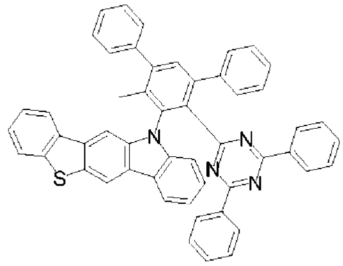
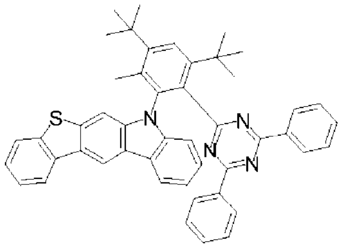
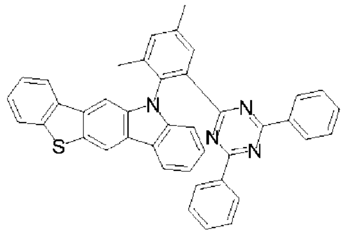
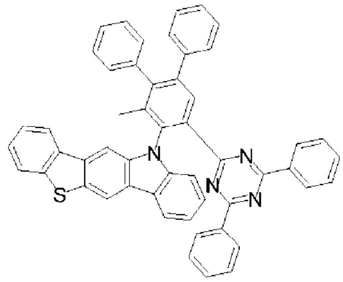
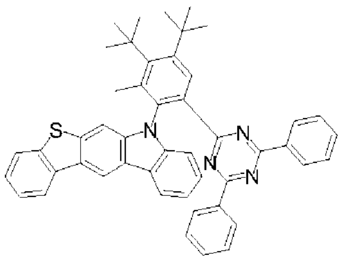
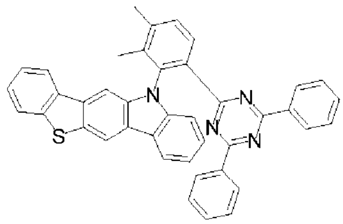
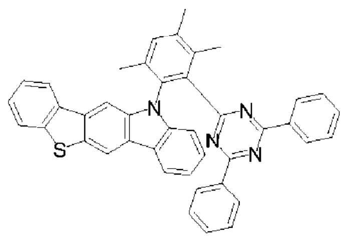
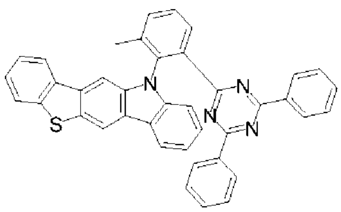
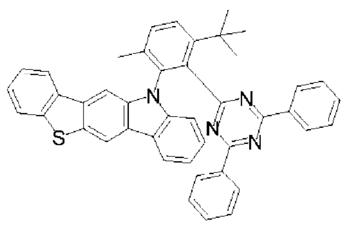
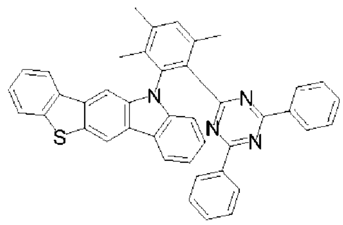
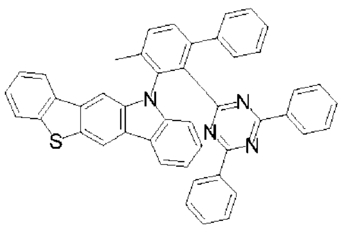
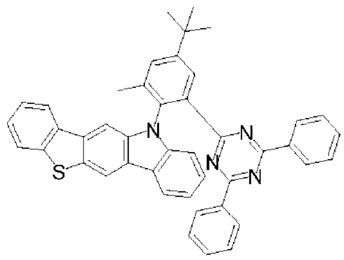
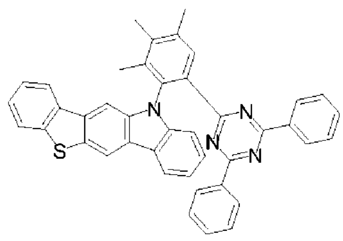
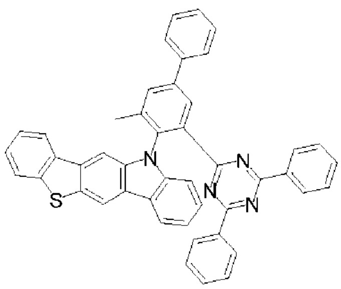
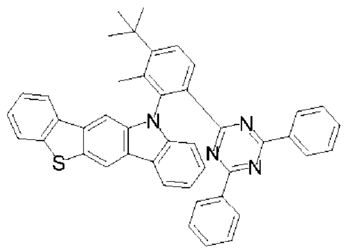
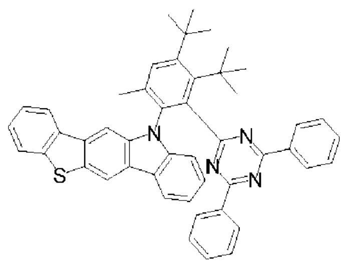

[Chem. 52]

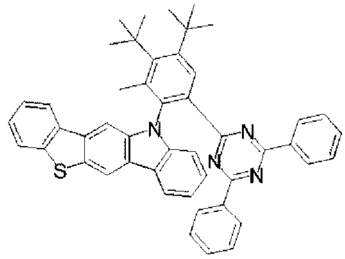
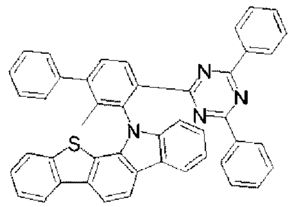
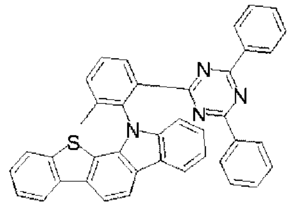
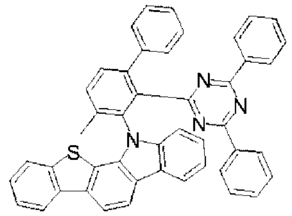
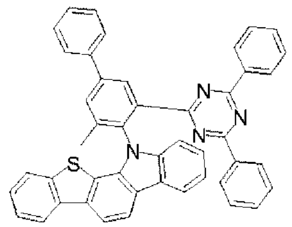
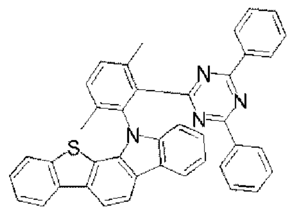
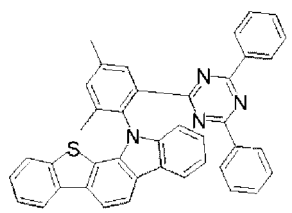
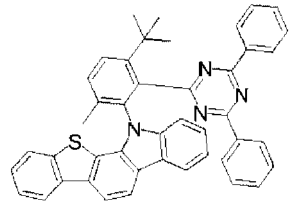
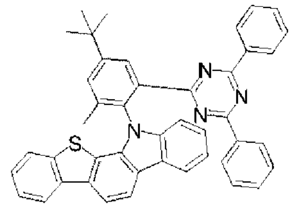
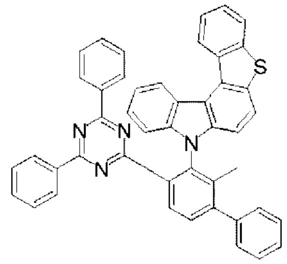
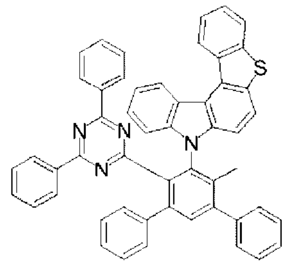
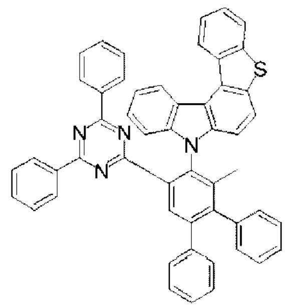
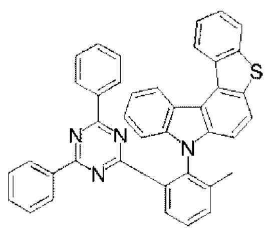
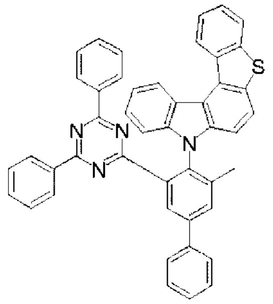
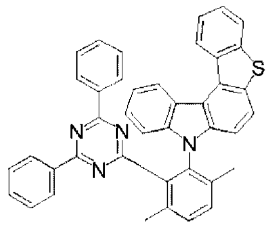
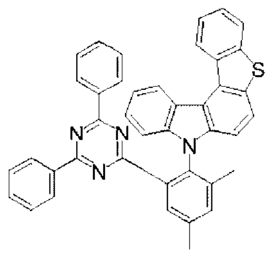
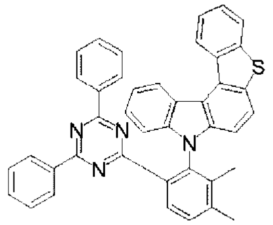
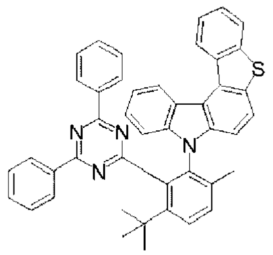
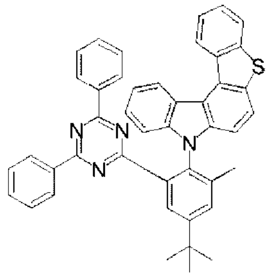

[Chem. 53]

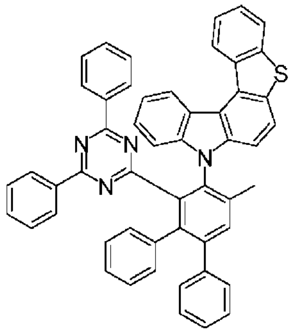
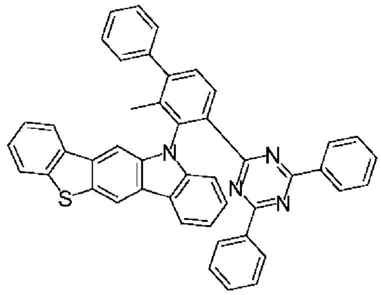
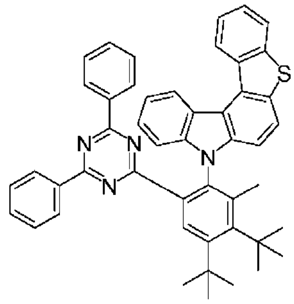
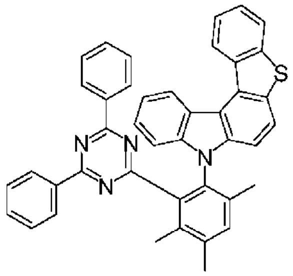
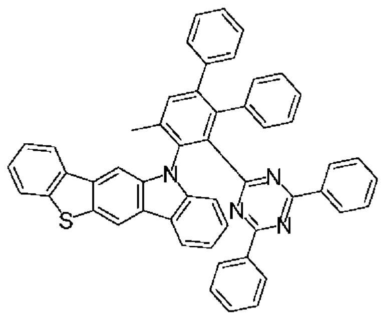
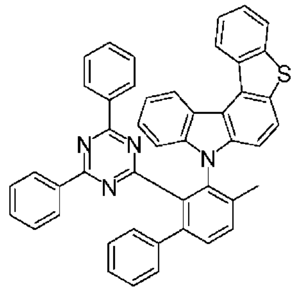
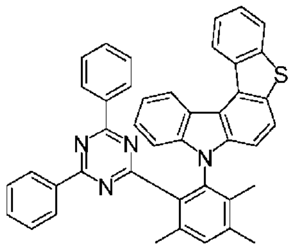
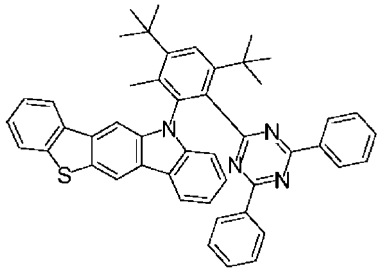
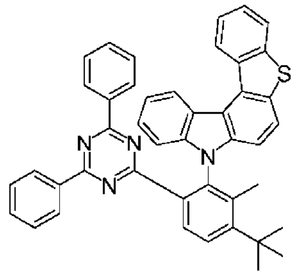
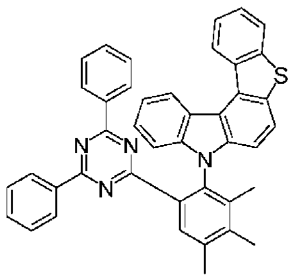
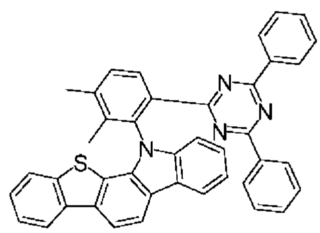
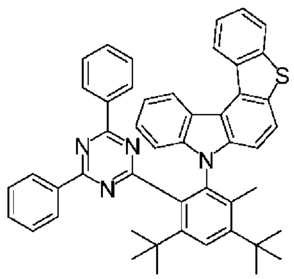

[Chem. 54]

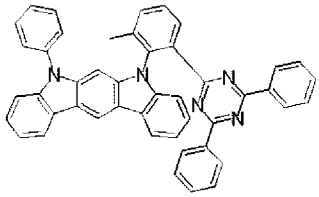
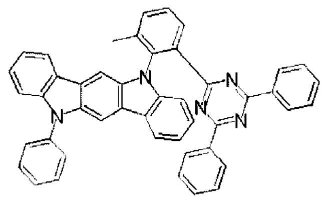
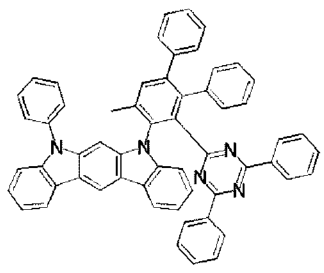
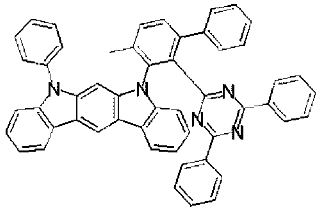
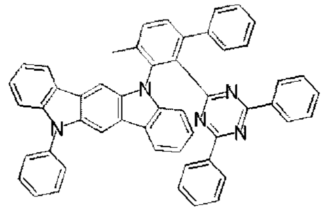
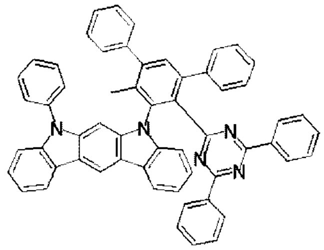
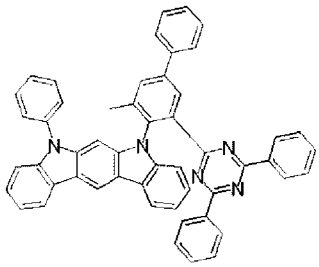
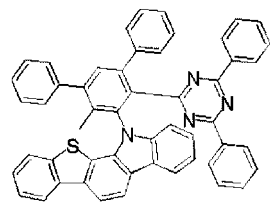
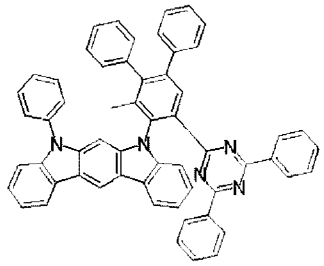
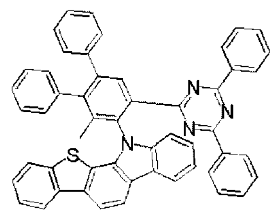
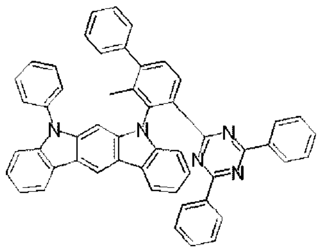
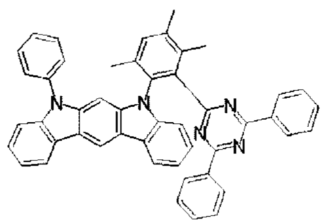
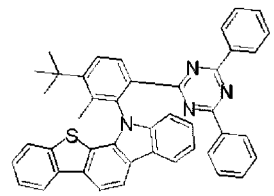
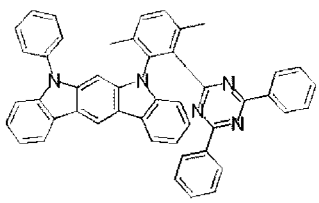
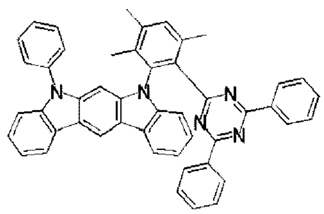
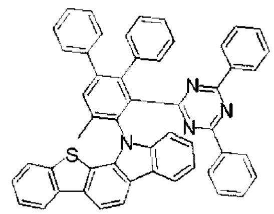
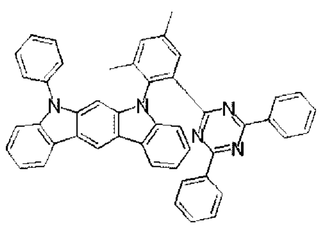
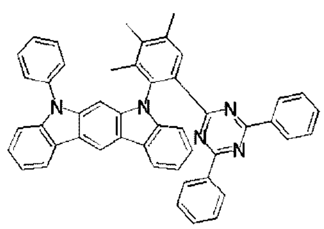
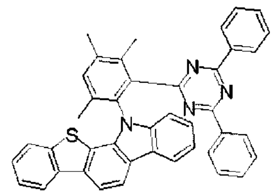
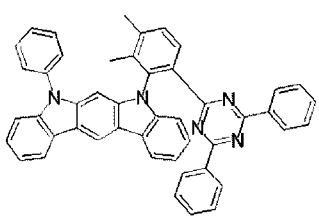

[Chem. 55]

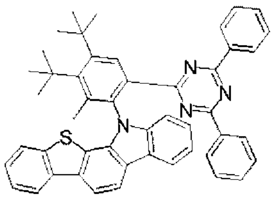
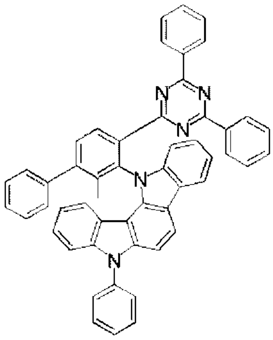
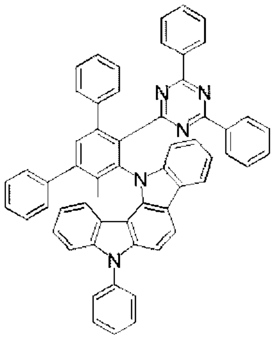
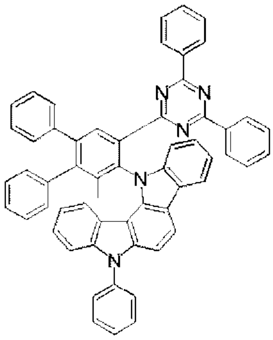
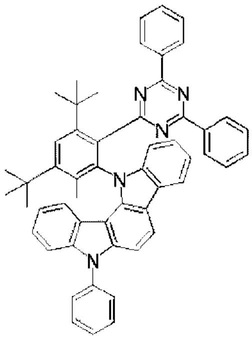
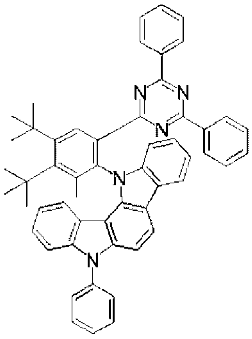
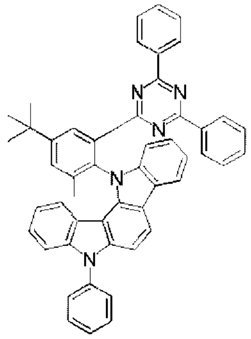
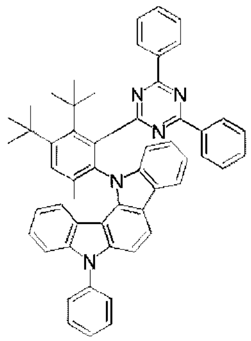
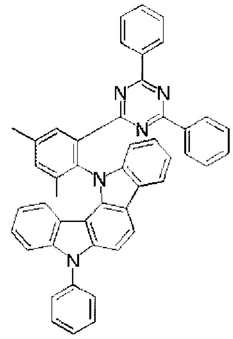
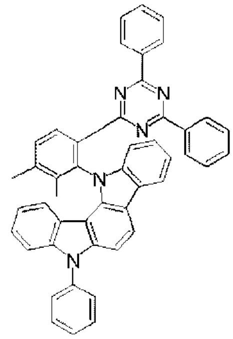
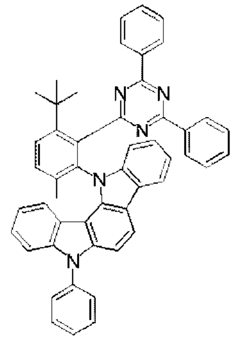
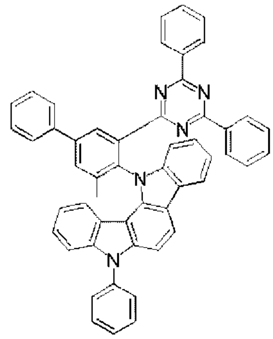
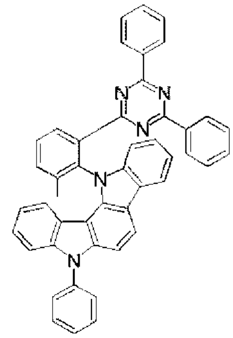
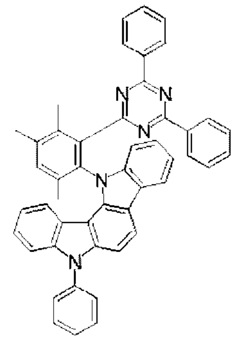

[Chem. 56]

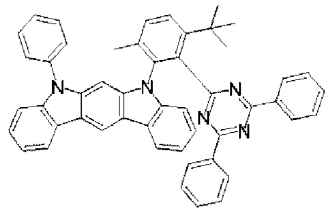
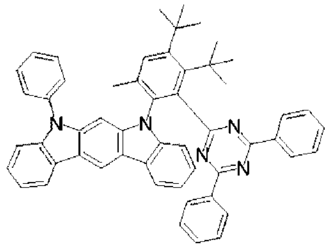
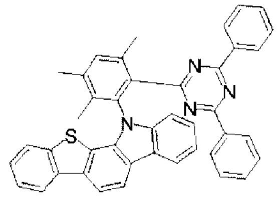
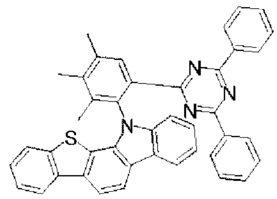
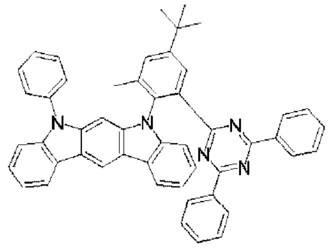
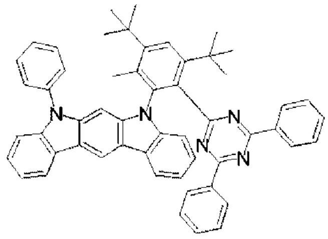
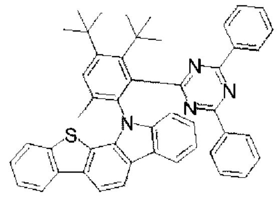
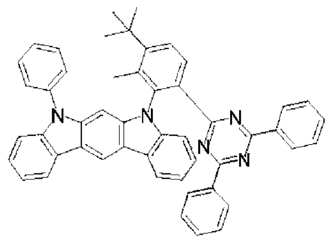
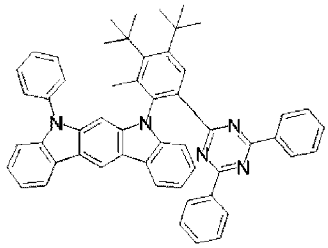
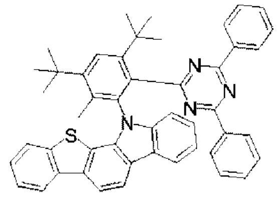
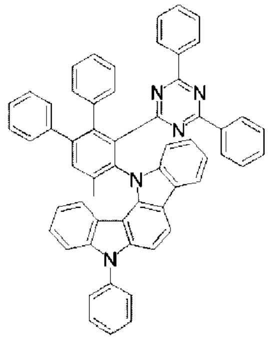
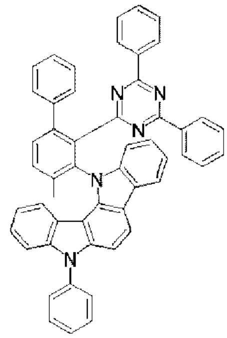
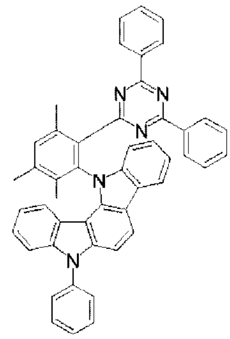
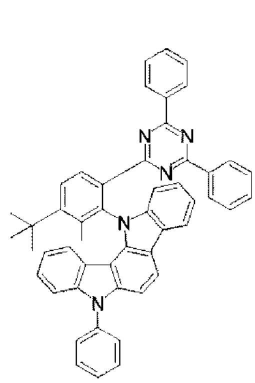
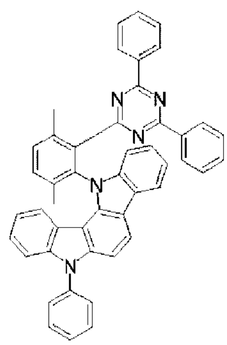
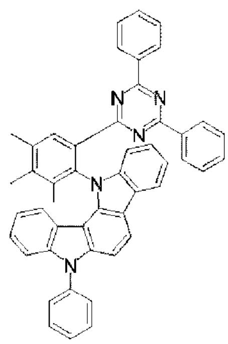

[Chem. 57]

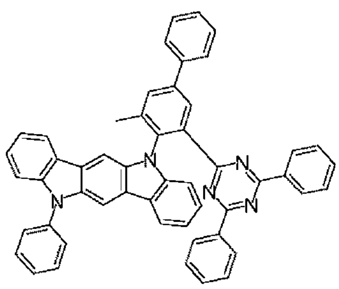
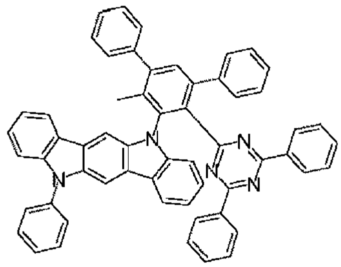
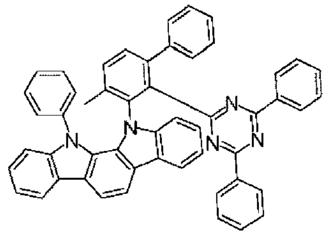
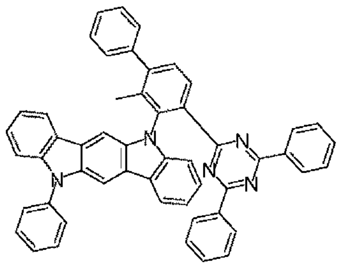
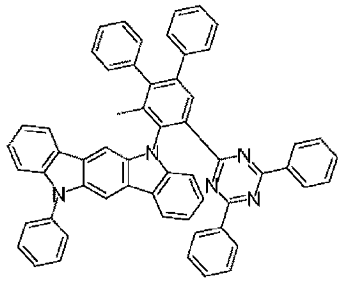
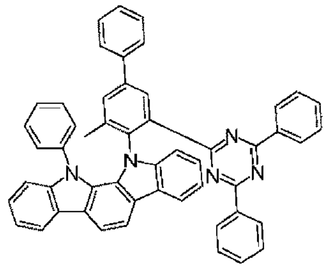
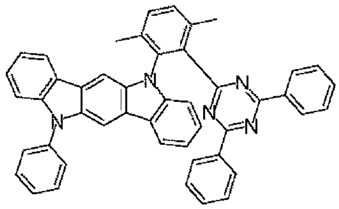
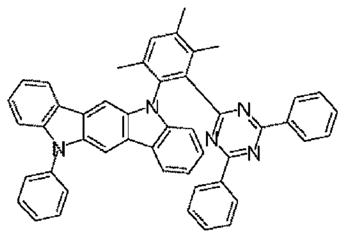
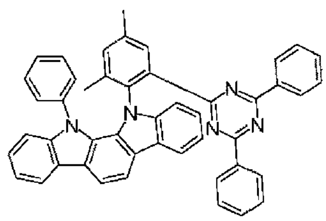
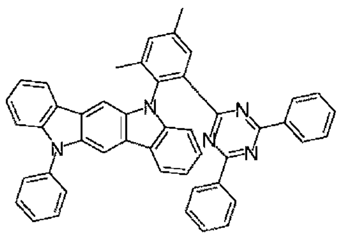
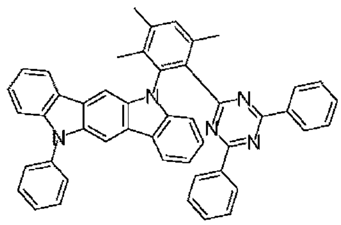
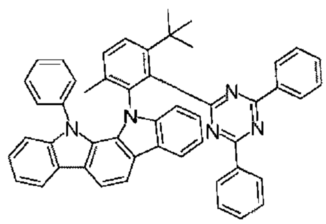
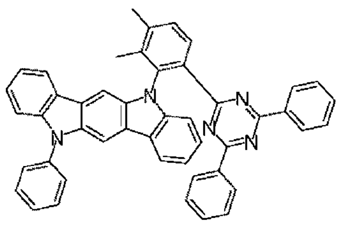
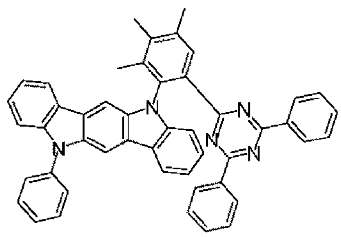
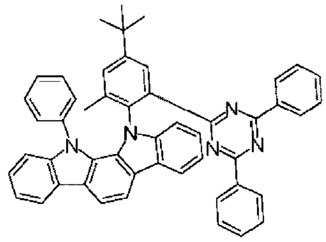
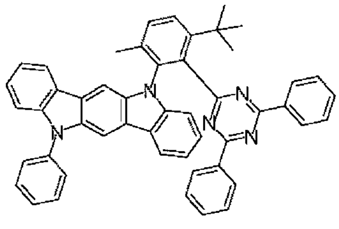
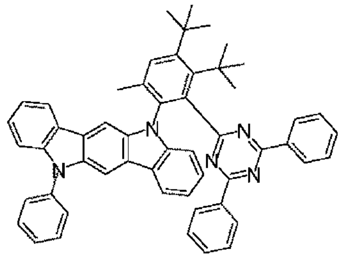
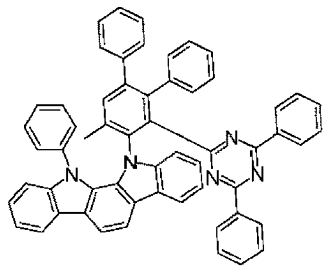

[Chem. 58]

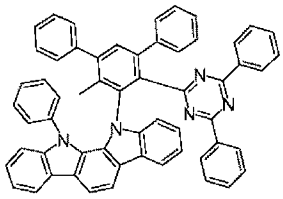

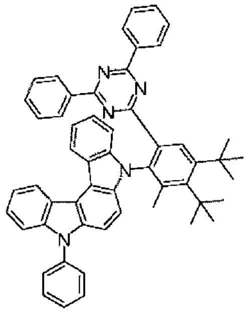

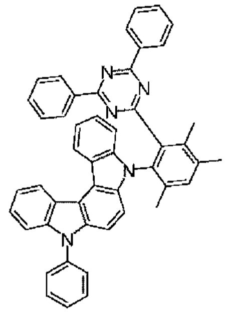

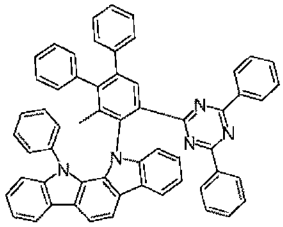

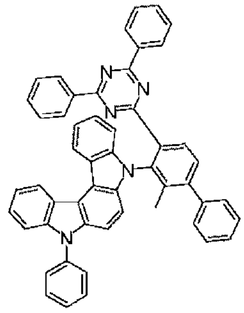

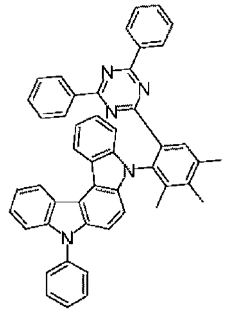

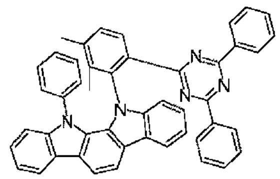

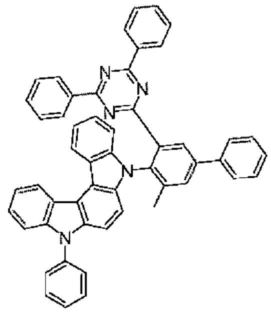

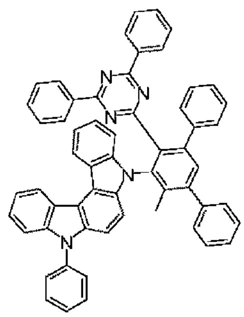

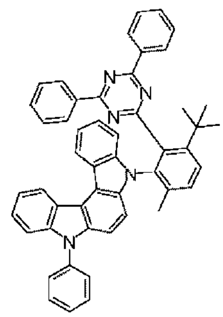

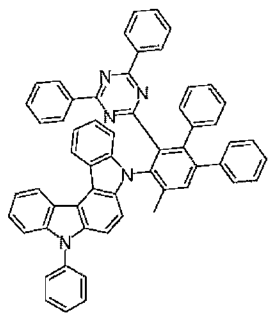

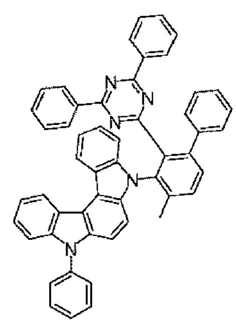

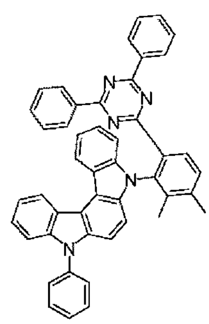

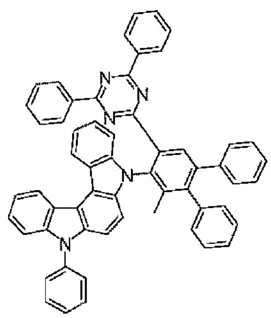

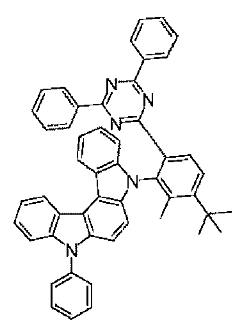

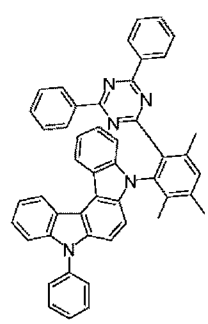

[Chem. 59]

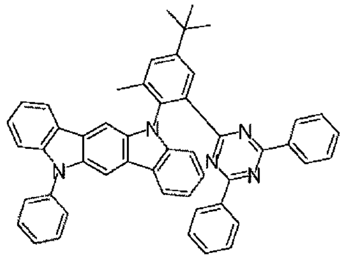
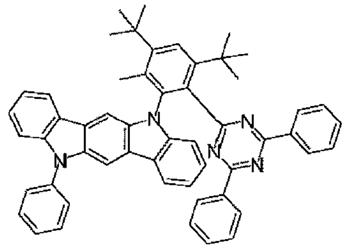
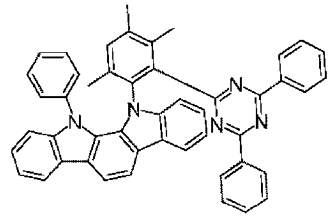
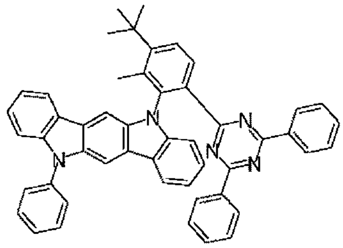
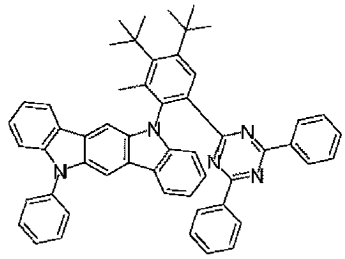
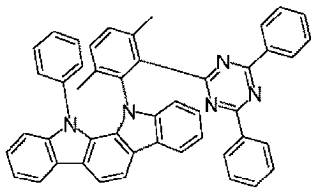
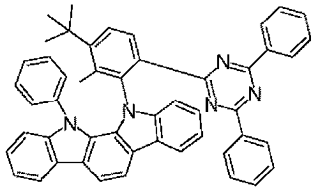
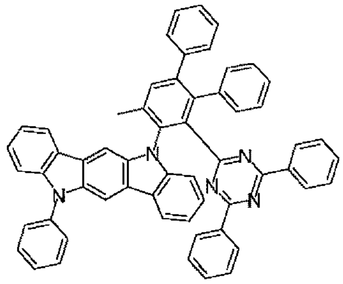
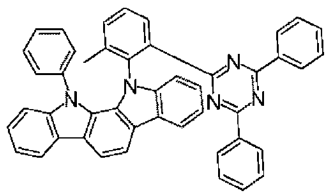
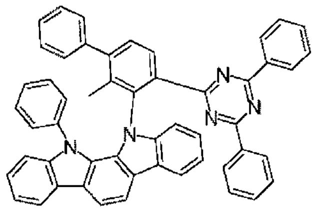
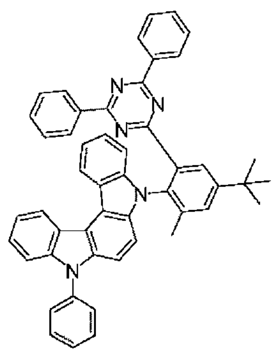
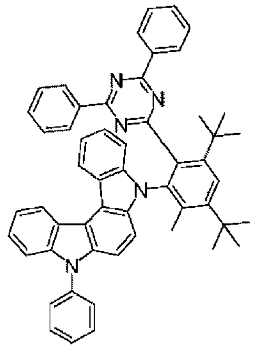
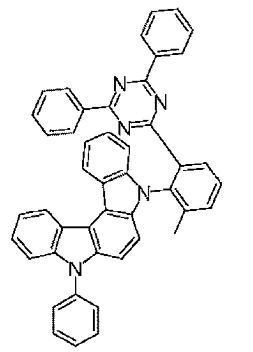
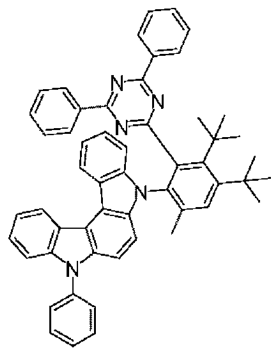
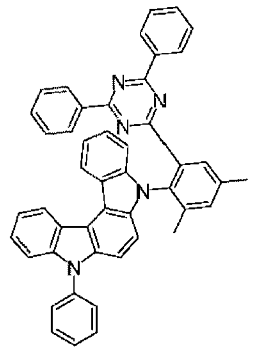
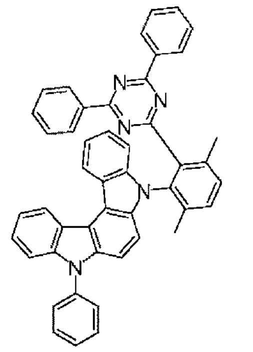

[Chem. 60]

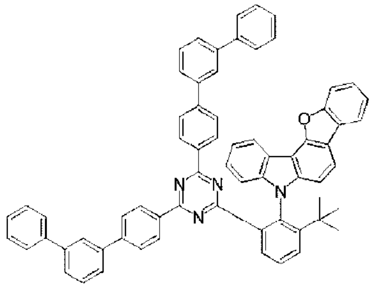
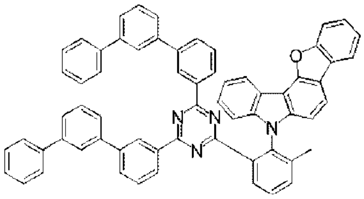
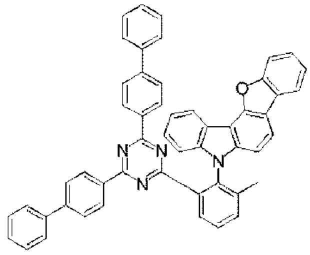
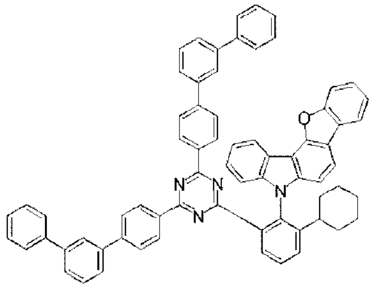
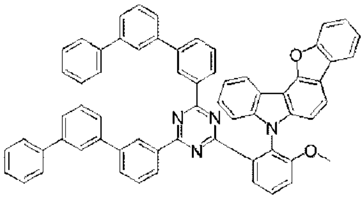
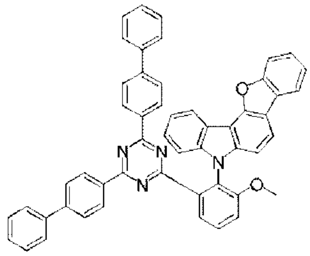
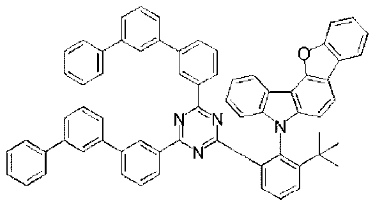
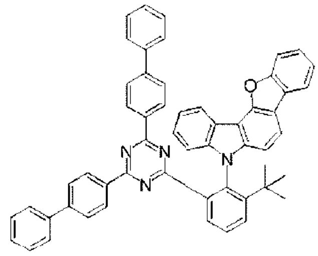
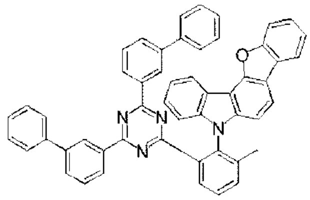
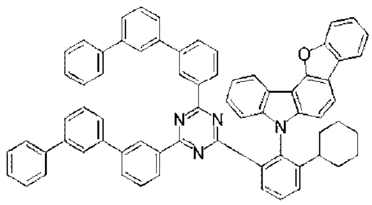
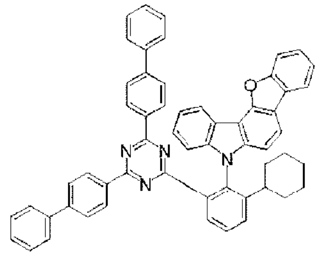
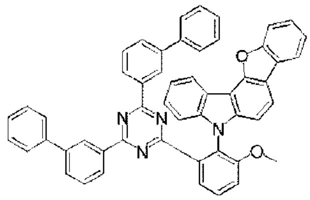
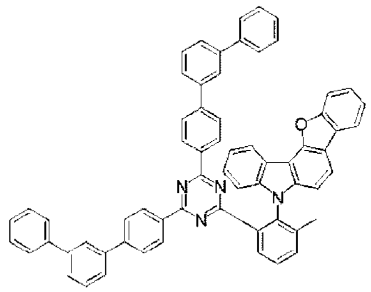
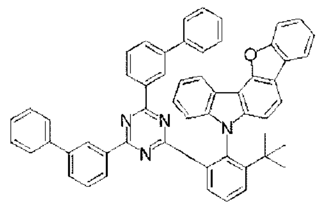
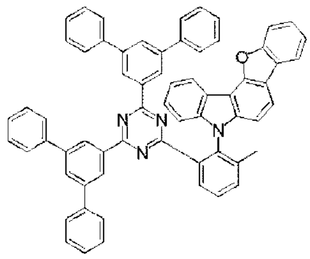
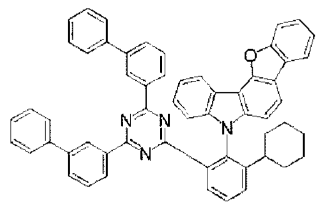
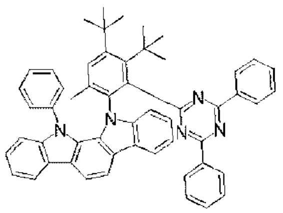

[Chem. 61]

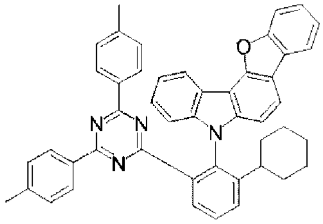
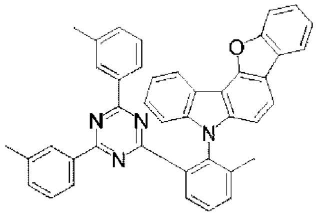
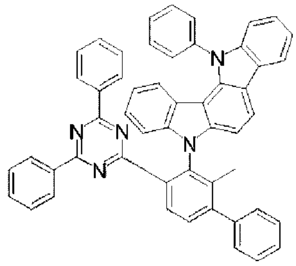
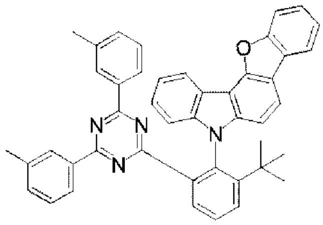
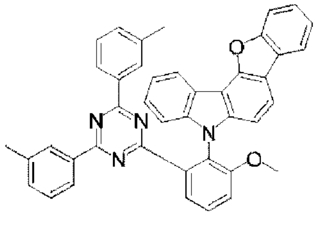
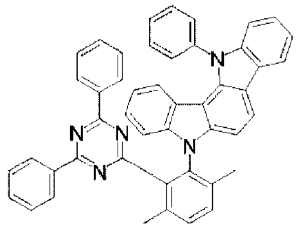
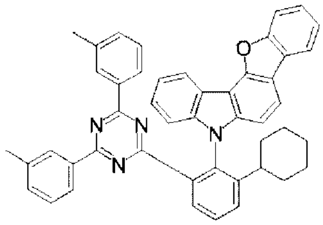
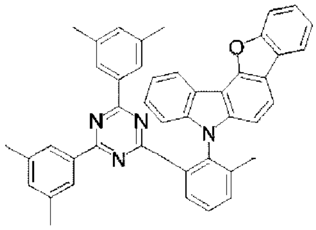
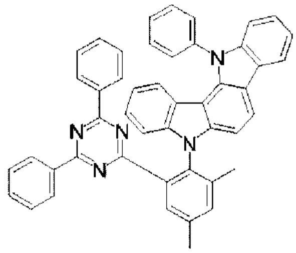
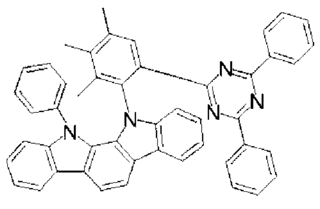
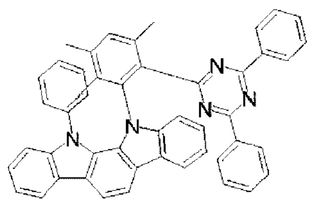
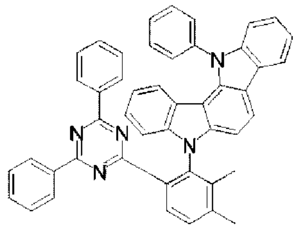
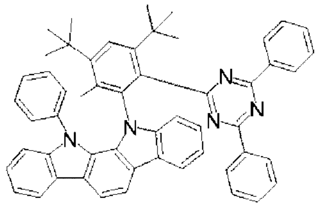
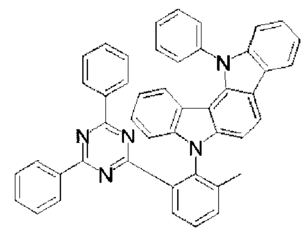
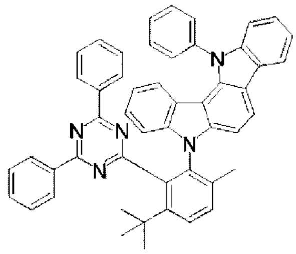
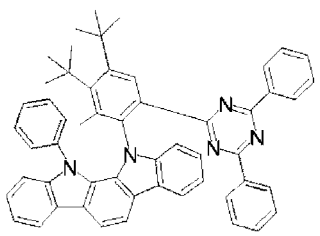
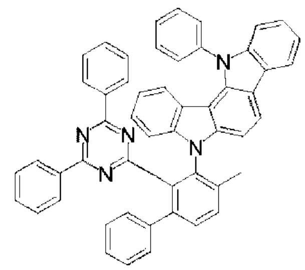
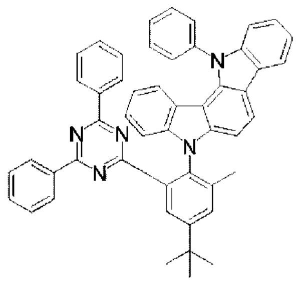
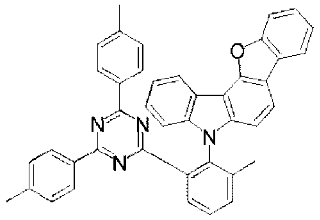

[Chem. 62]

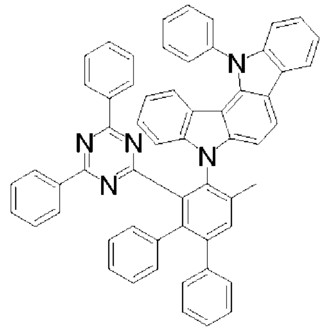
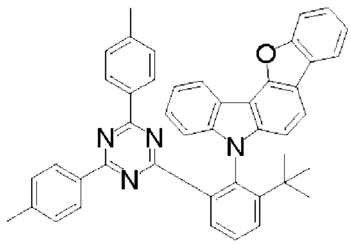
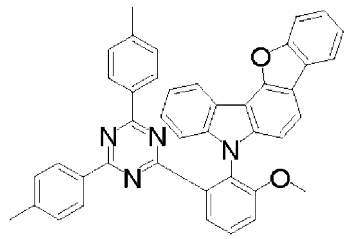
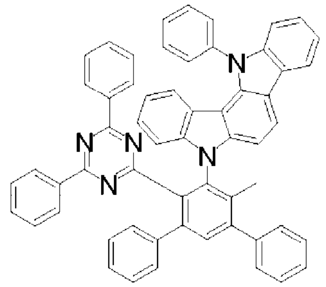
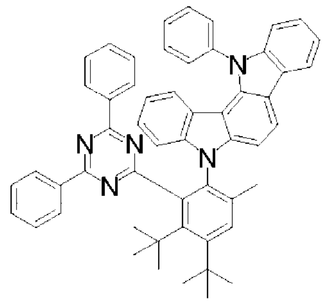
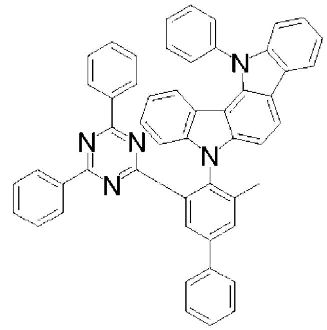
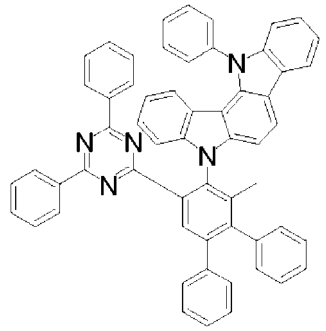
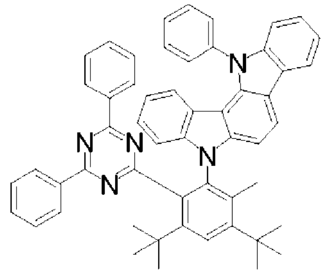
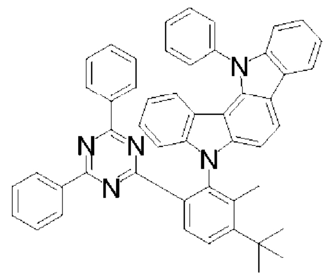
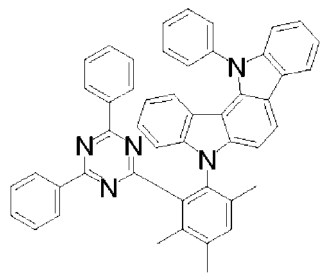
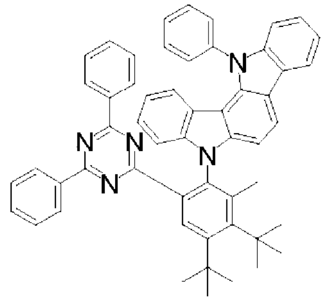
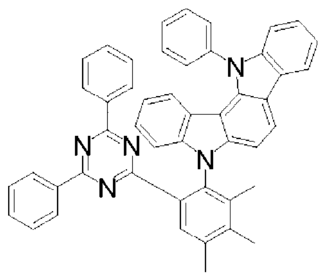
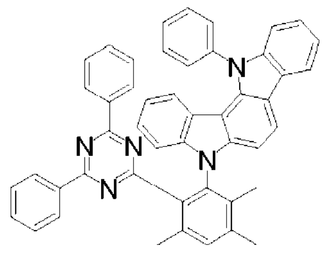
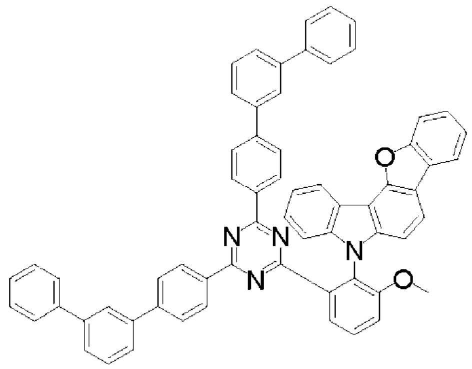

[Chem. 63]

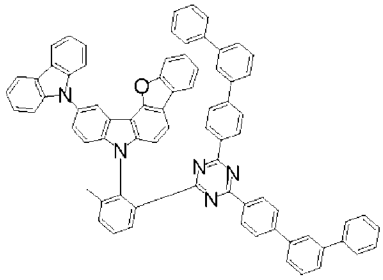
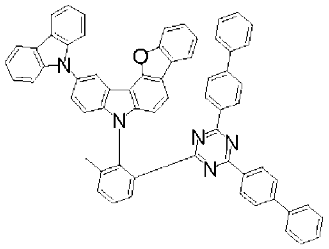
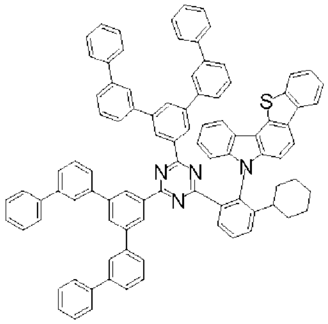
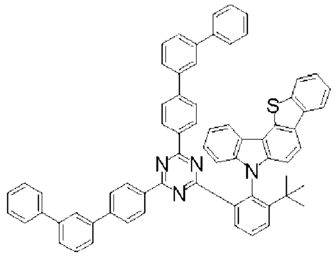
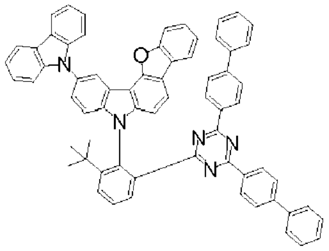
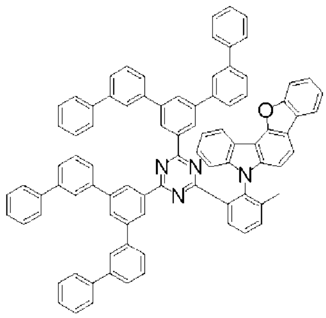
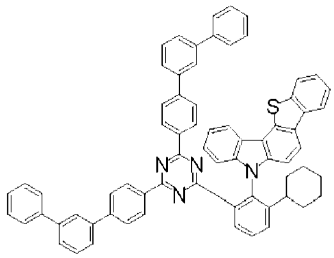
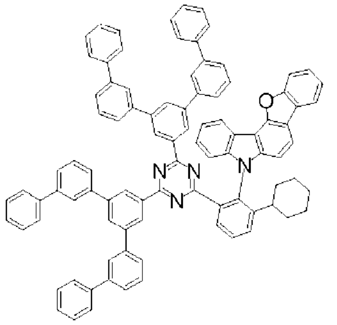
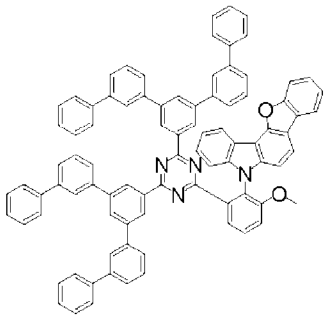
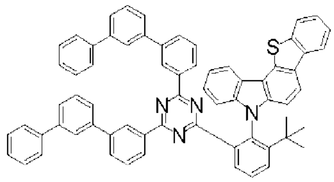
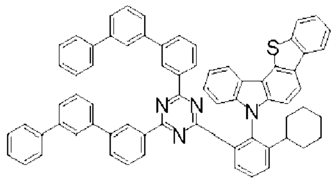
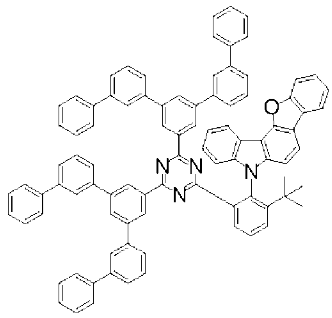
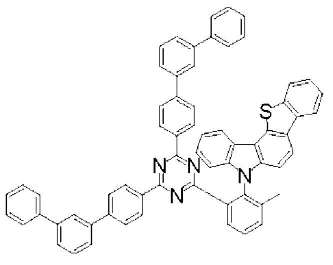
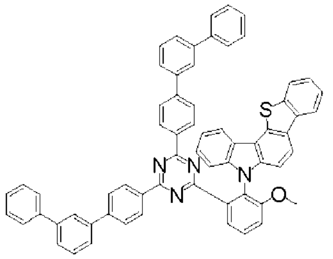
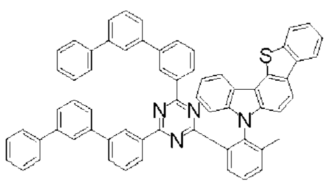
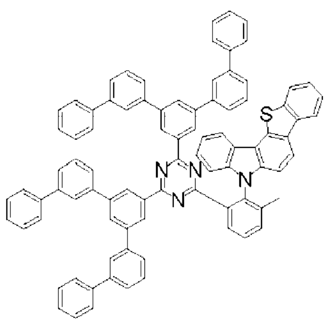
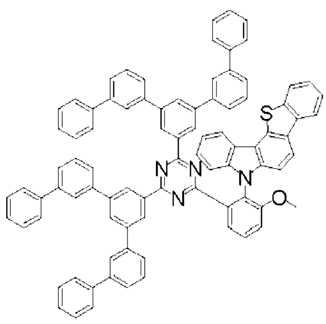

[Chem. 64]

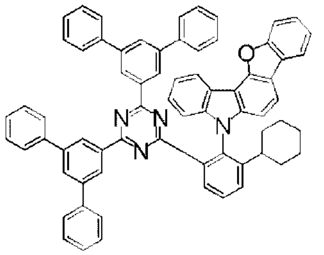

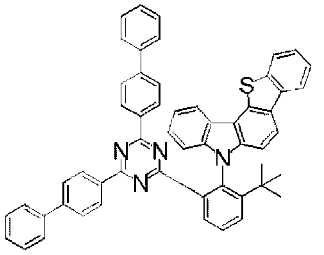

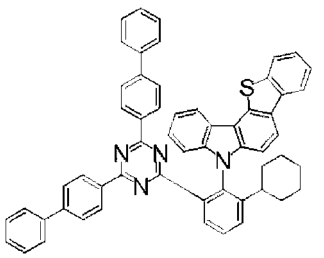

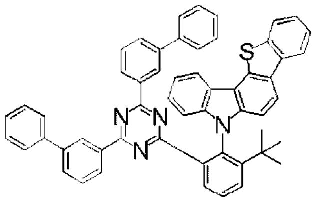

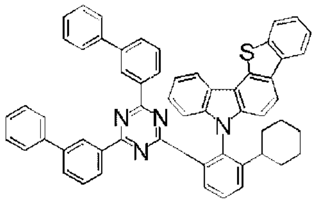

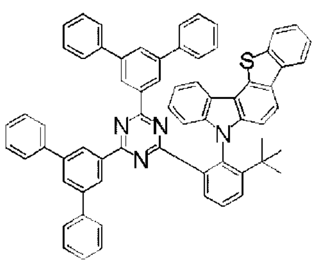

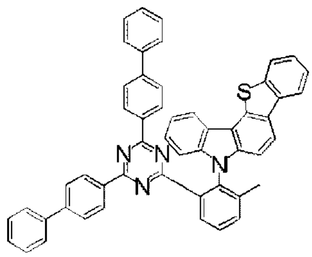

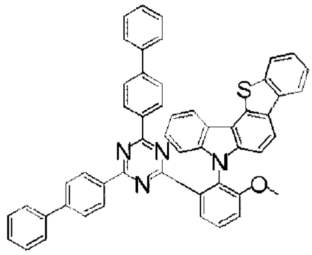

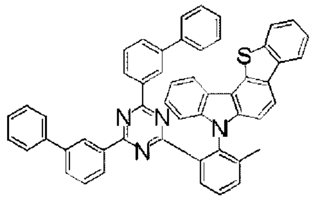

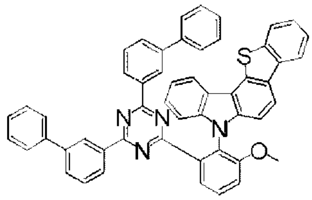

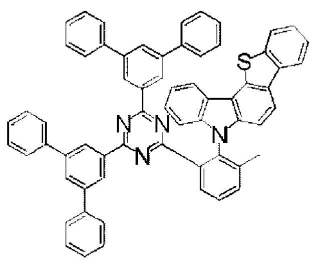

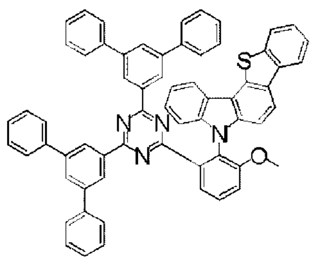

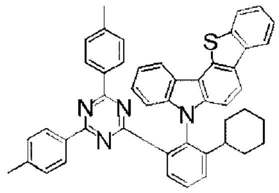

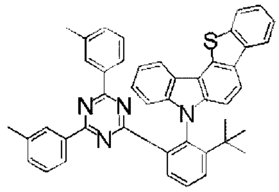

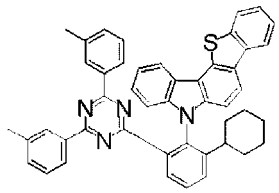

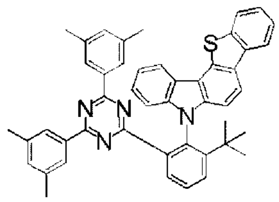

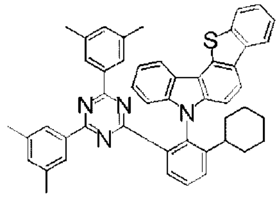

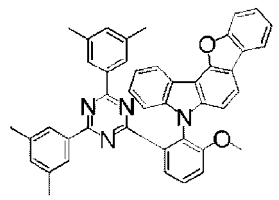

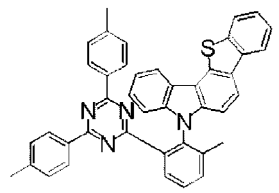

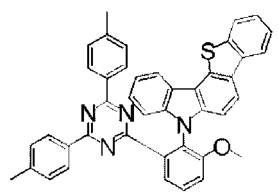

[Chem. 65]

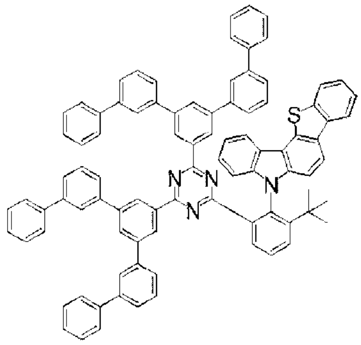
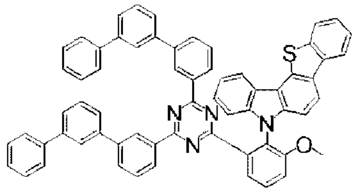
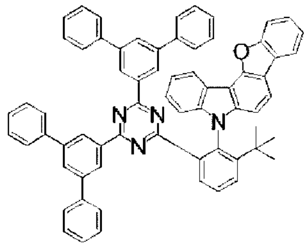
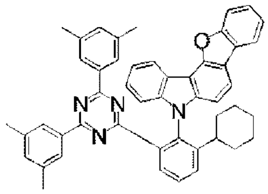
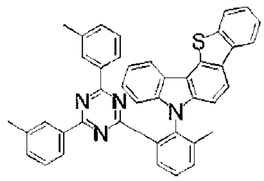
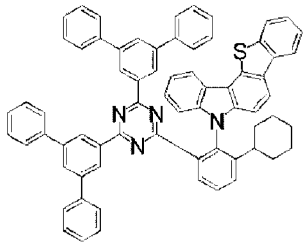
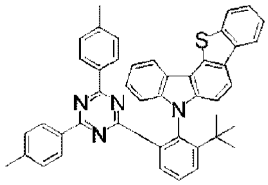
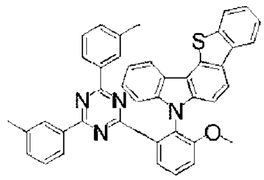
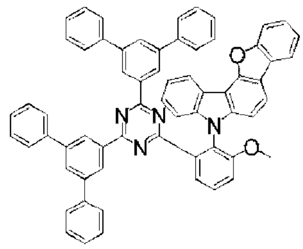
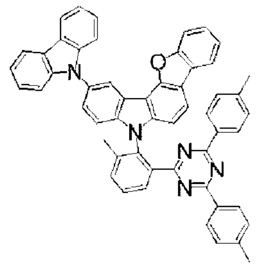
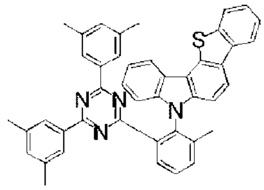
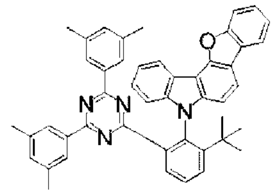
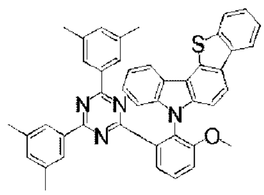

[Chem. 66]

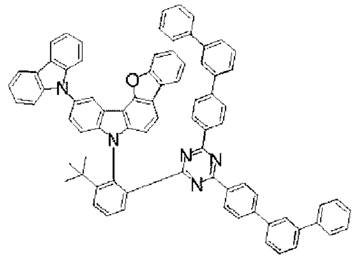
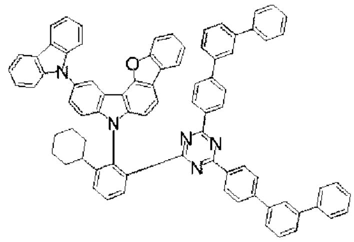
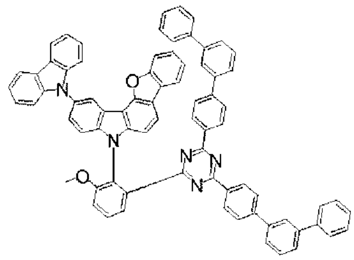
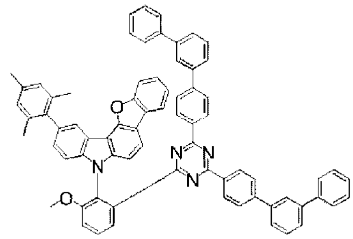
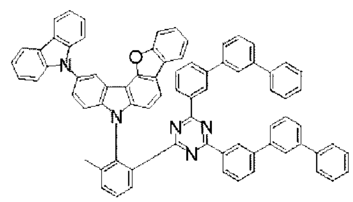
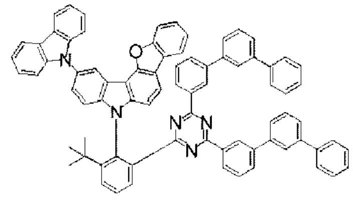
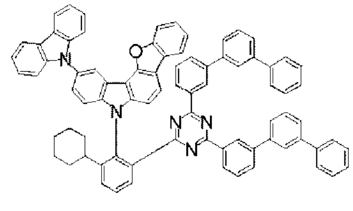
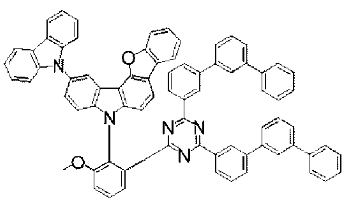
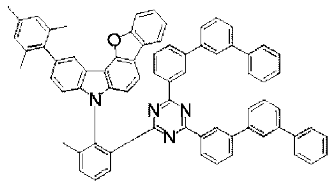
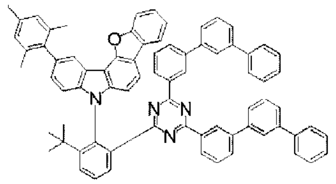
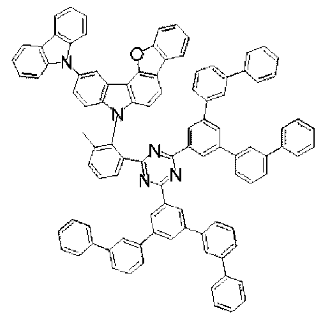
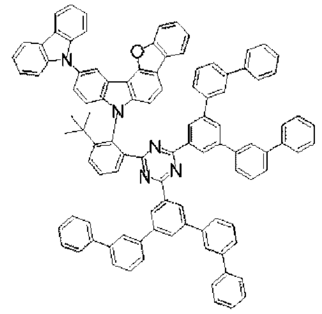
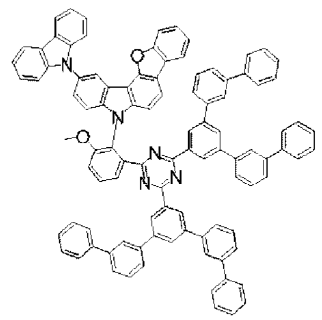
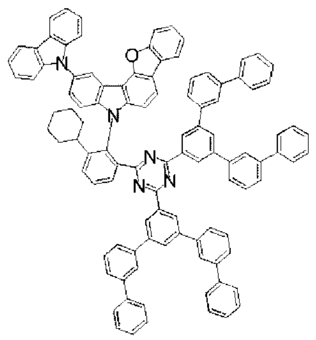
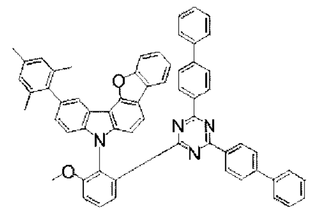
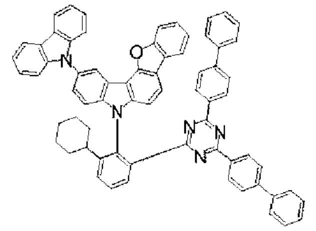
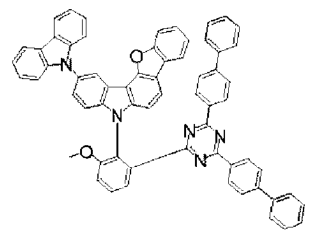
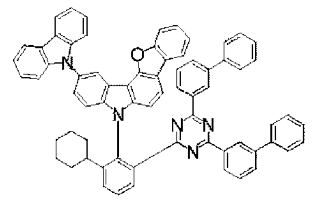
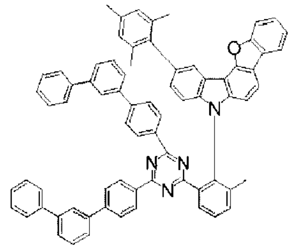

[Chem. 67]

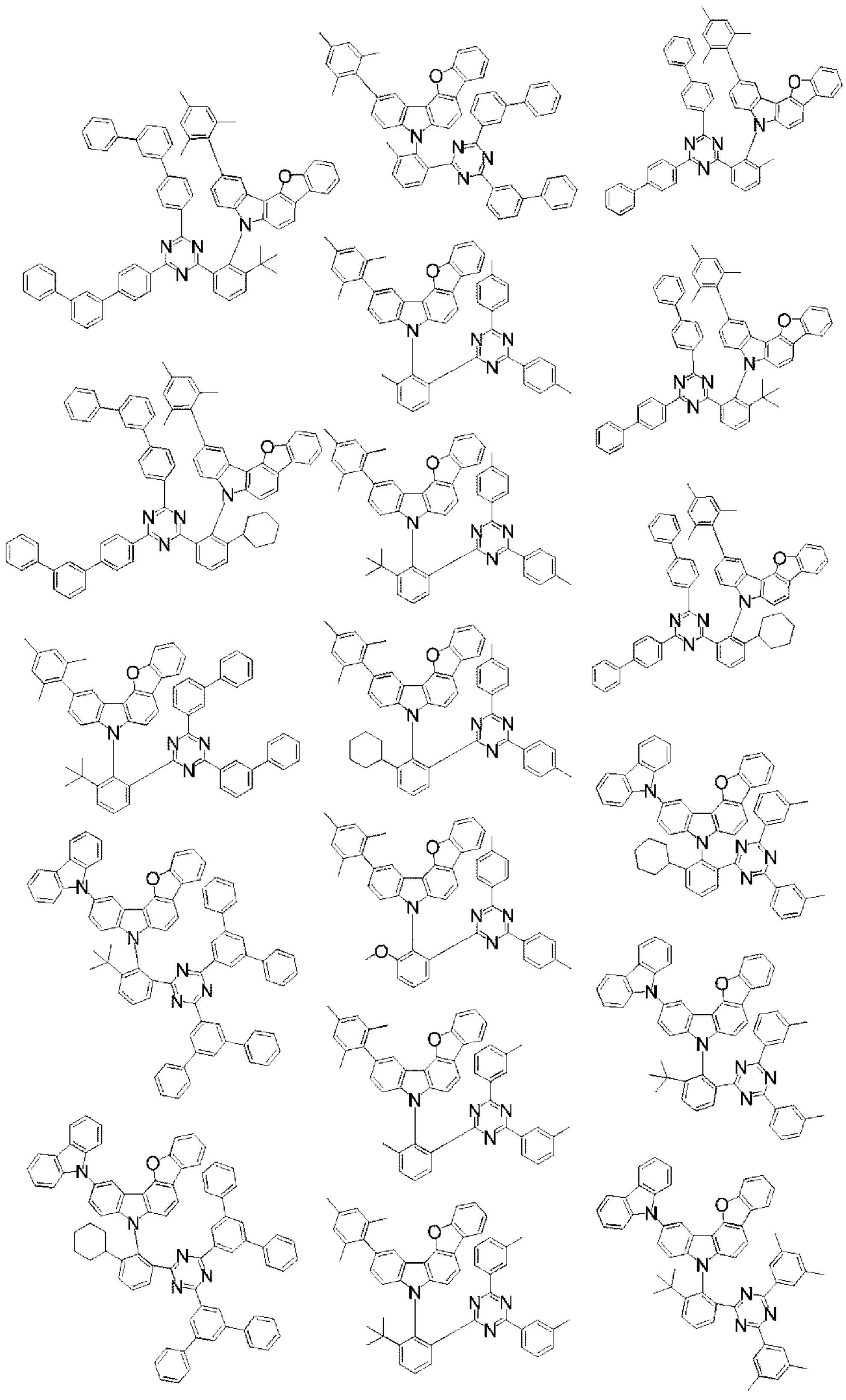

[Chem. 68]

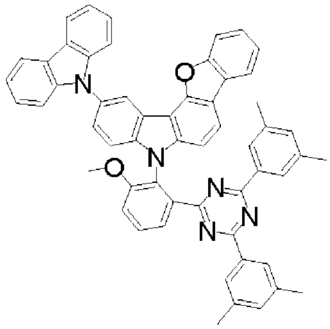
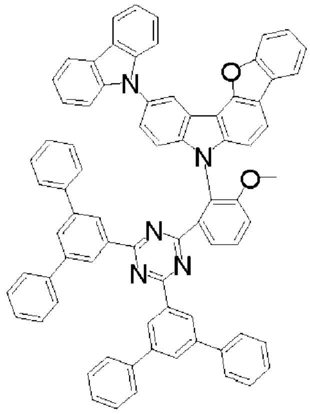
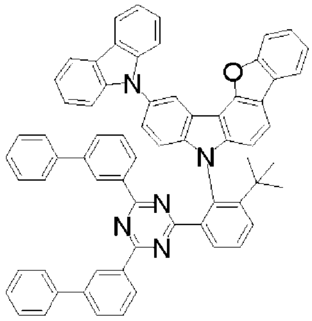
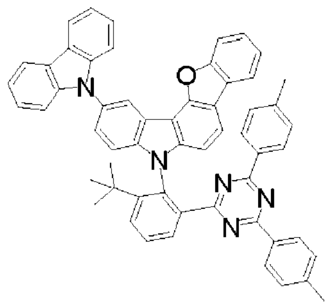
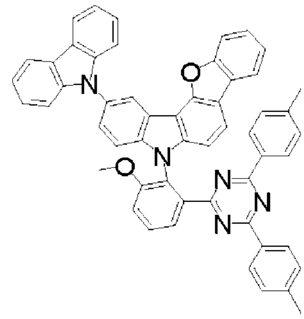
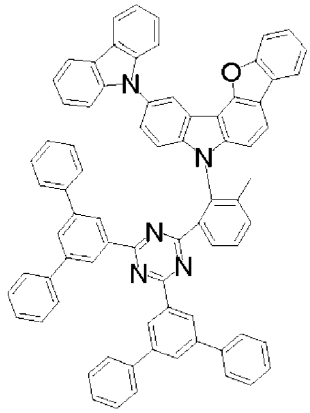
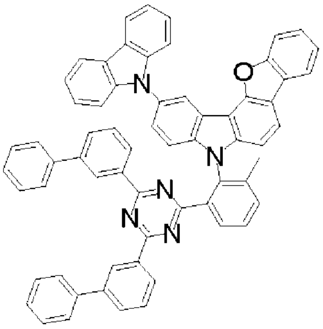
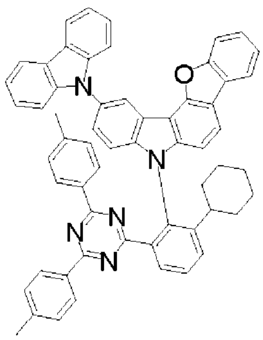
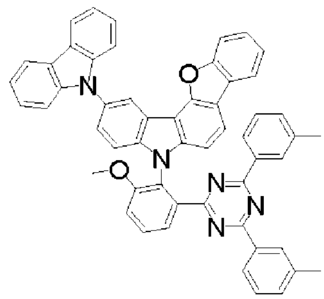
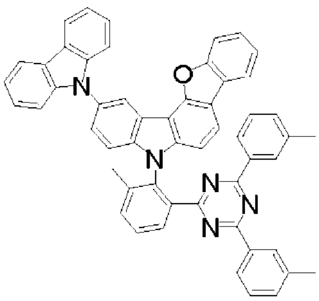
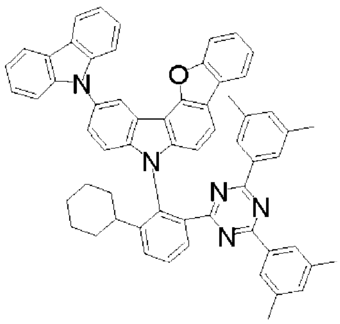
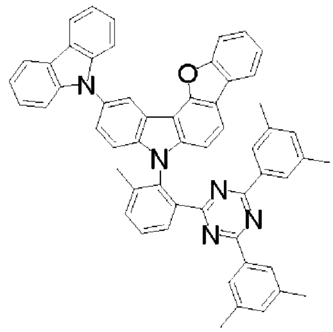
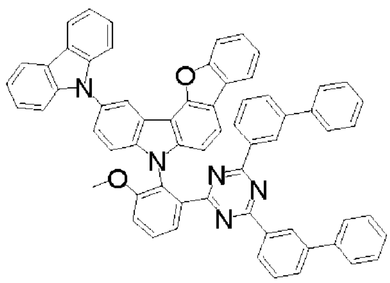

[Chem. 69]

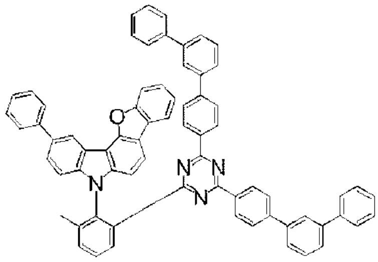
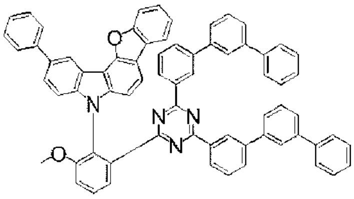
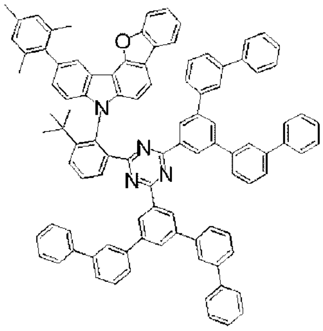
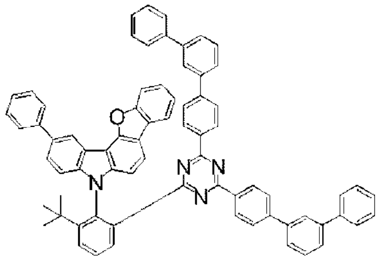
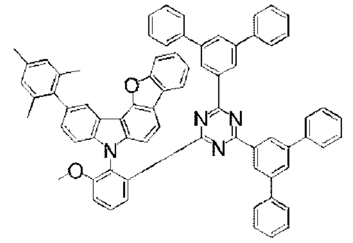
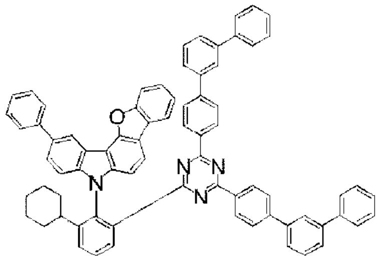
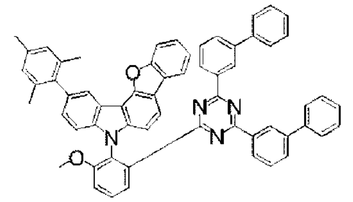
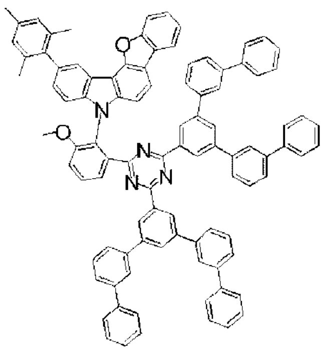
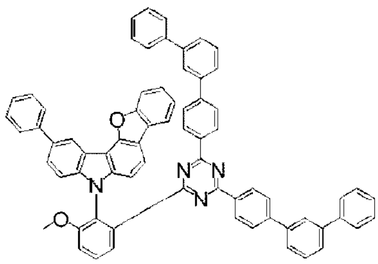
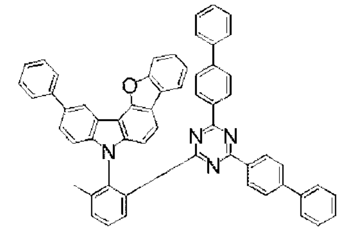
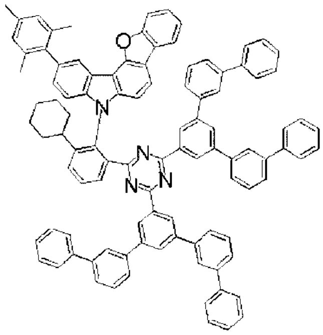
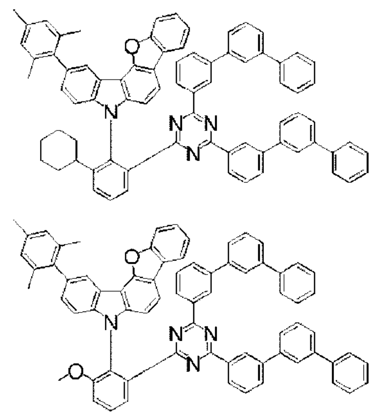
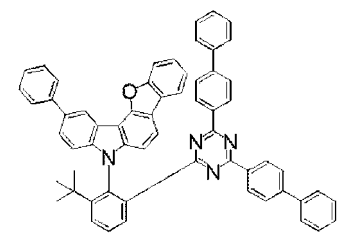
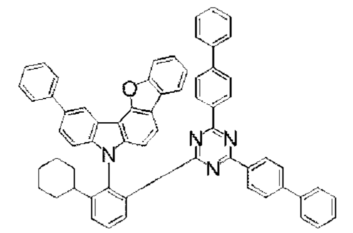
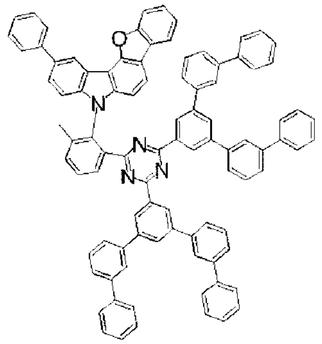

[Chem. 70]

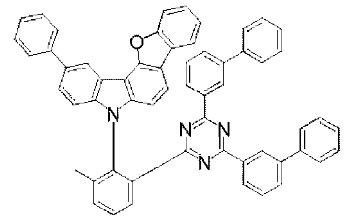

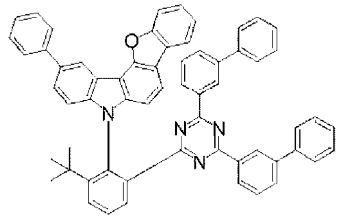

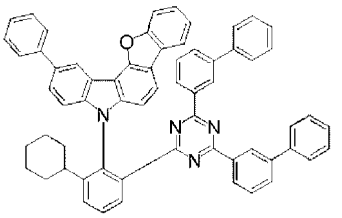

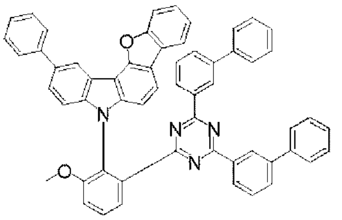

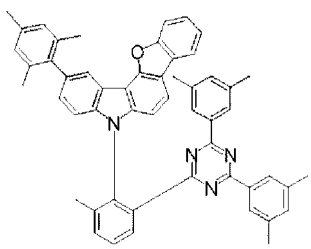

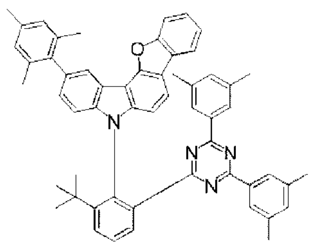

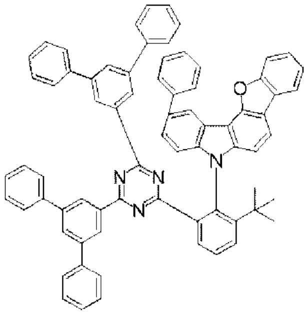

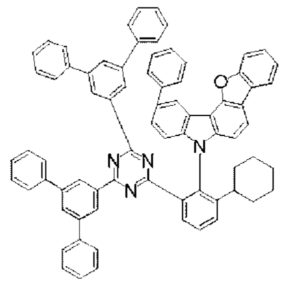

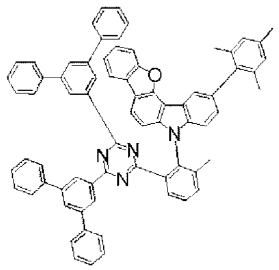

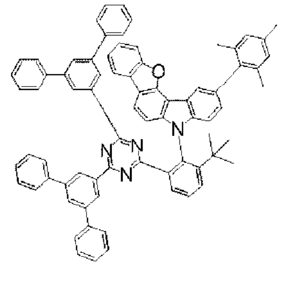

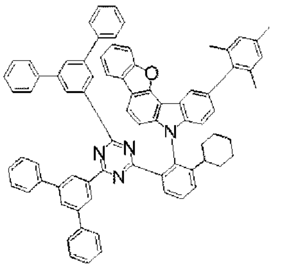

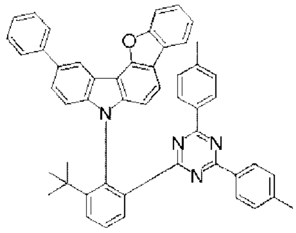

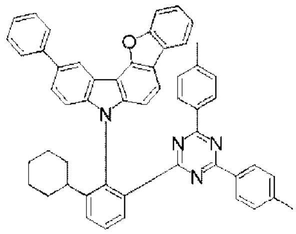

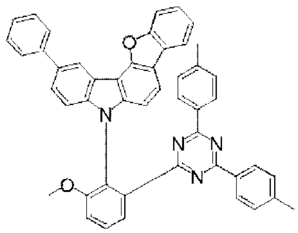

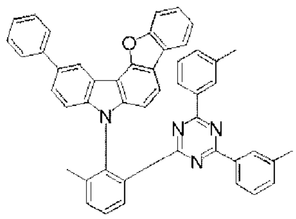

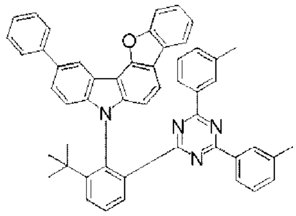

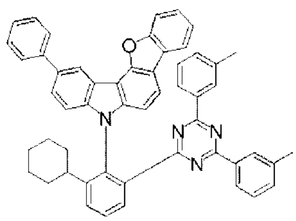

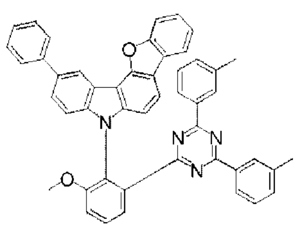

[Chem. 71]

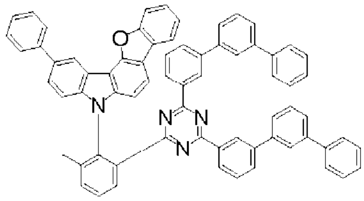
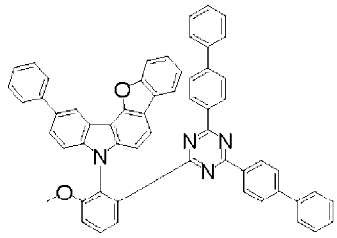
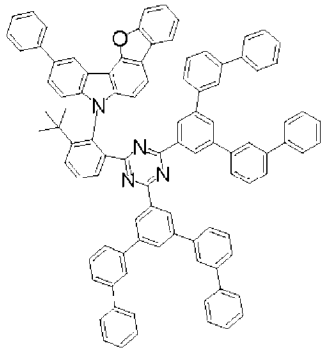
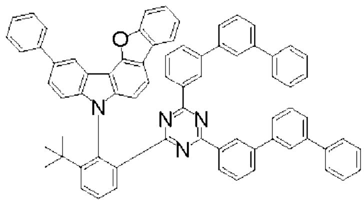
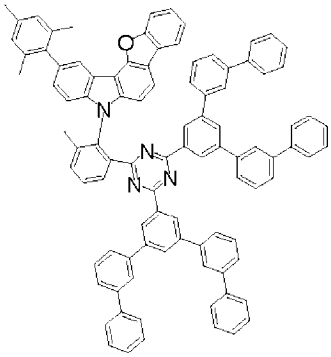
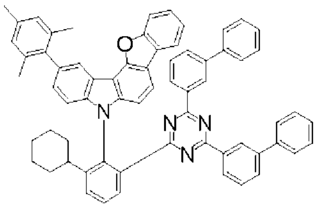
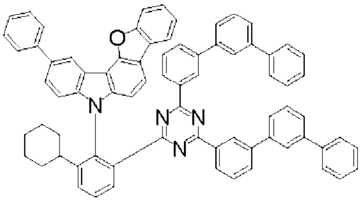
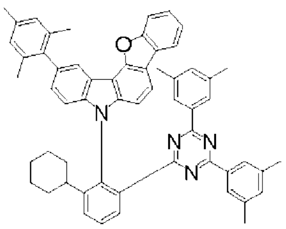
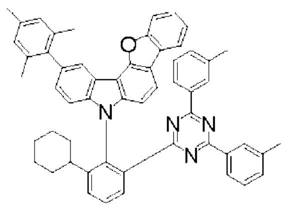
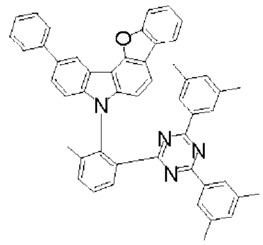
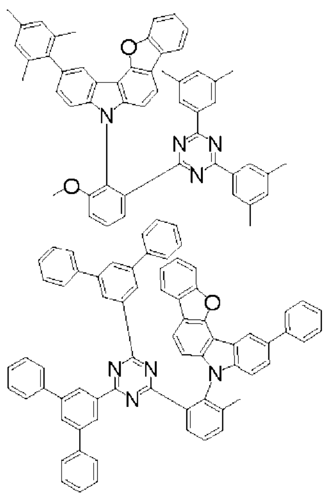
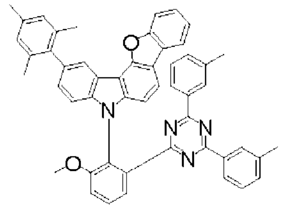
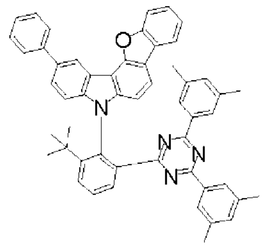
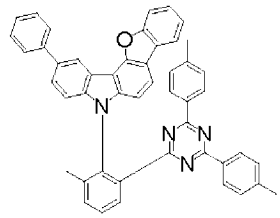
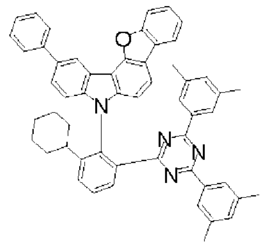

[Chem. 72]

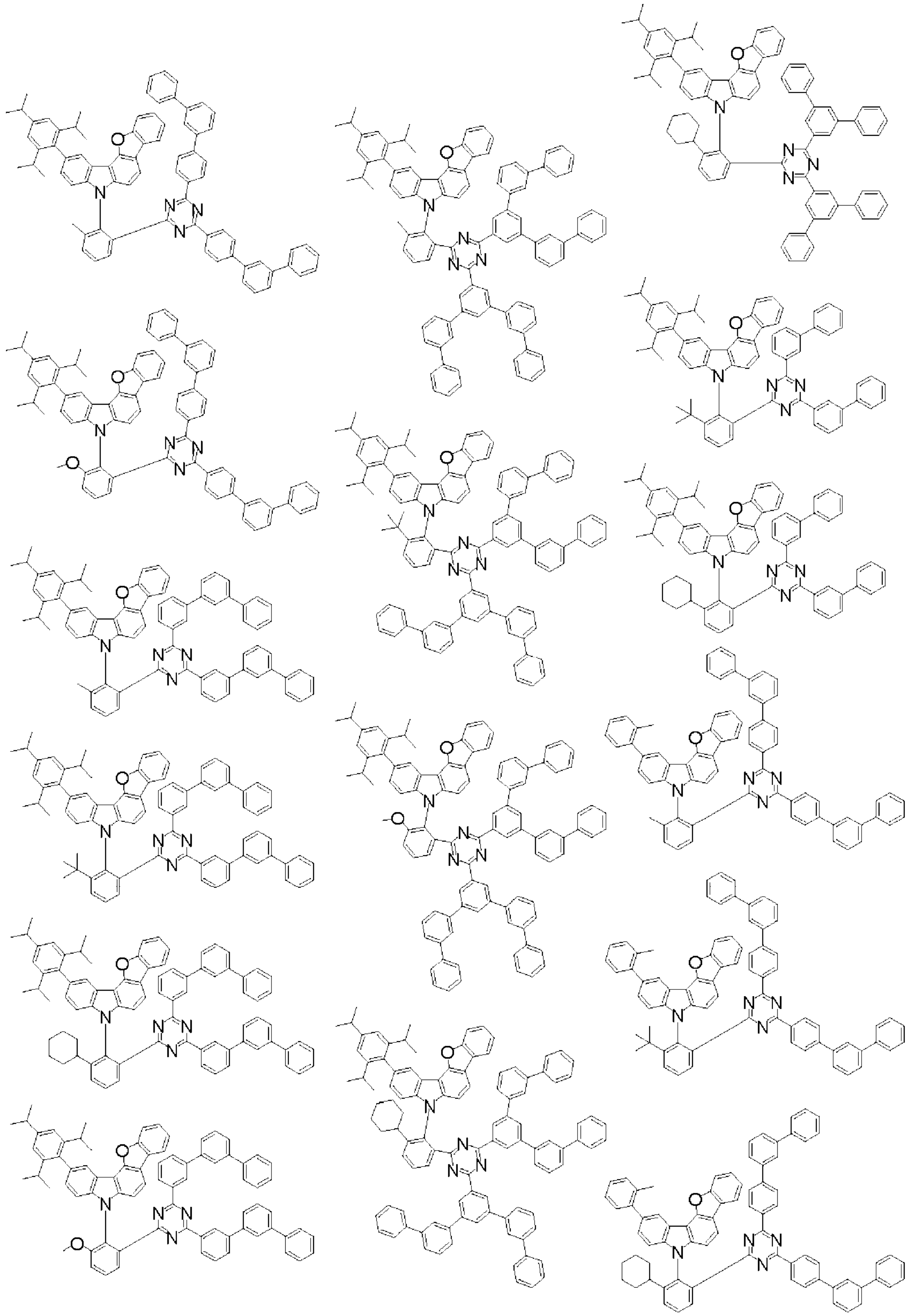

[Chem. 73]

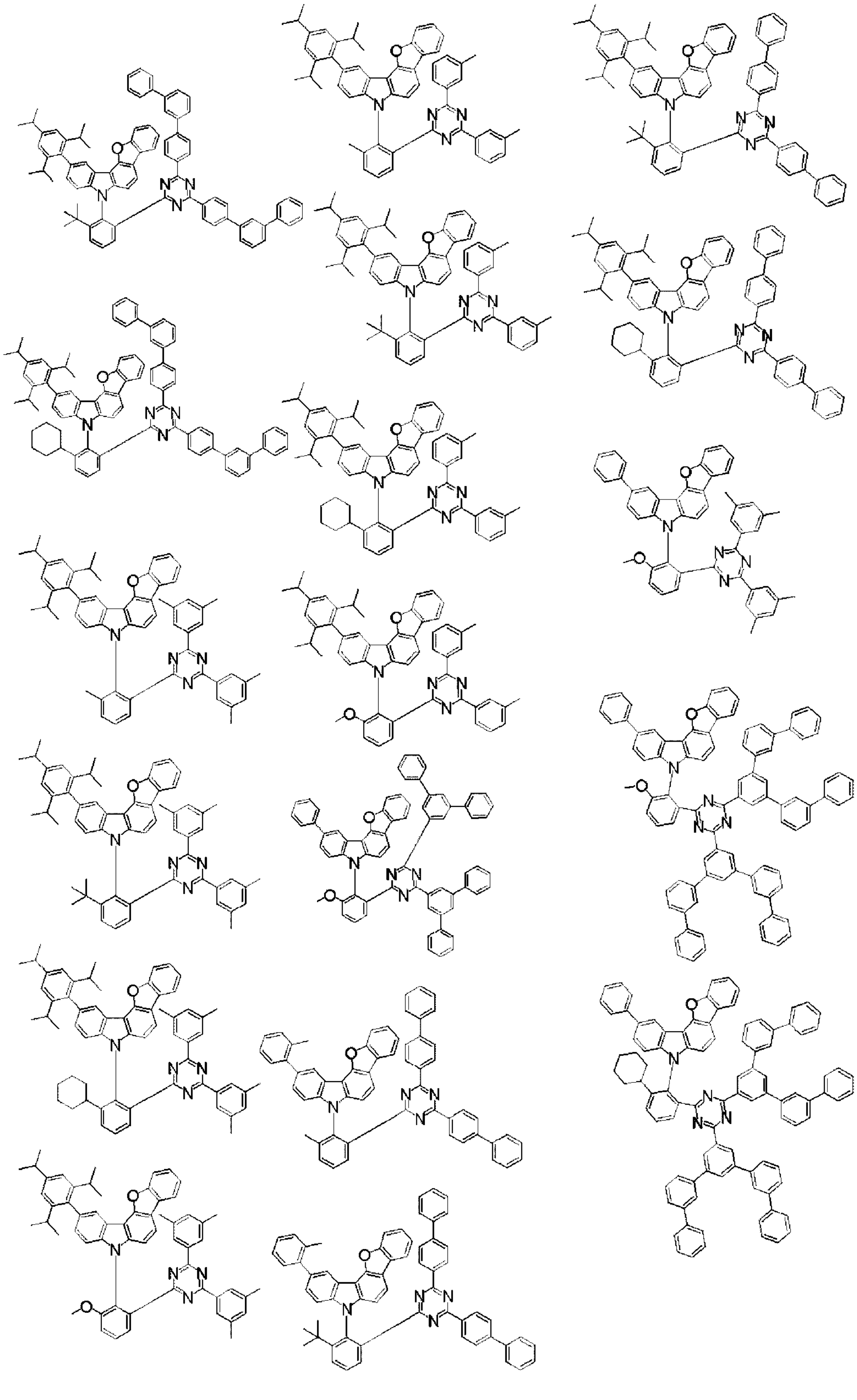

[Chem. 74]

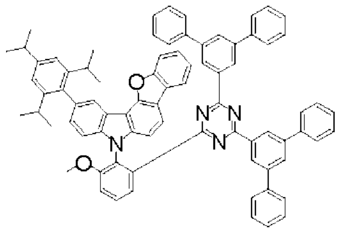
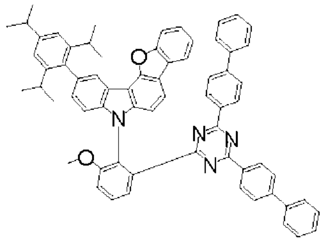
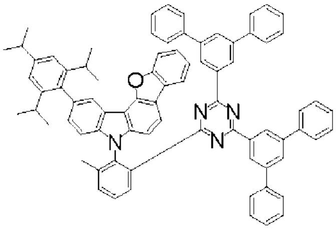
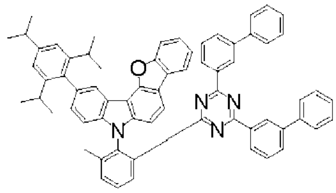
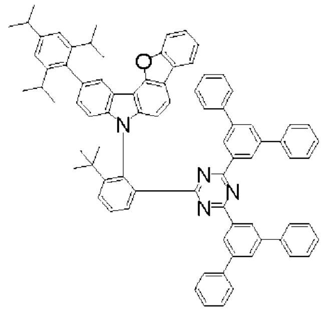
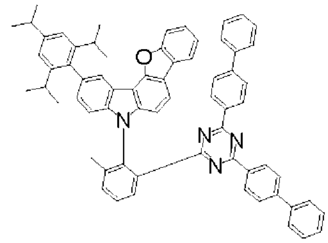
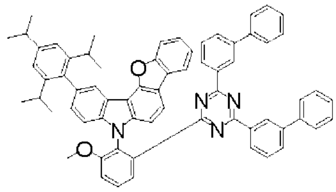
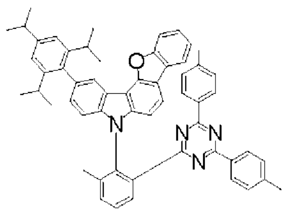
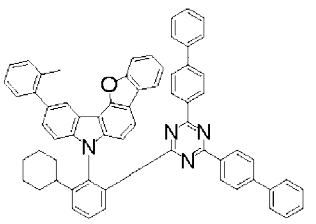
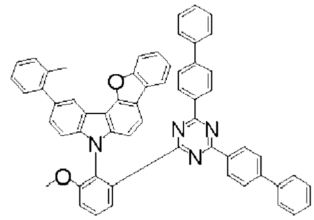
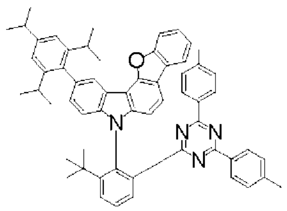
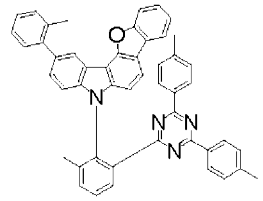
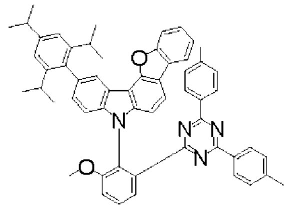
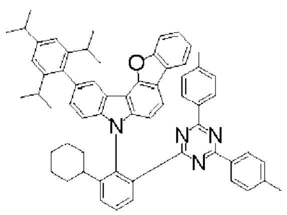
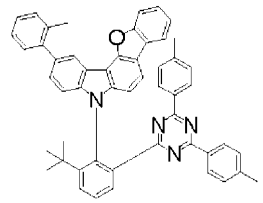
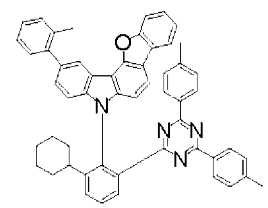

[Chem. 75]

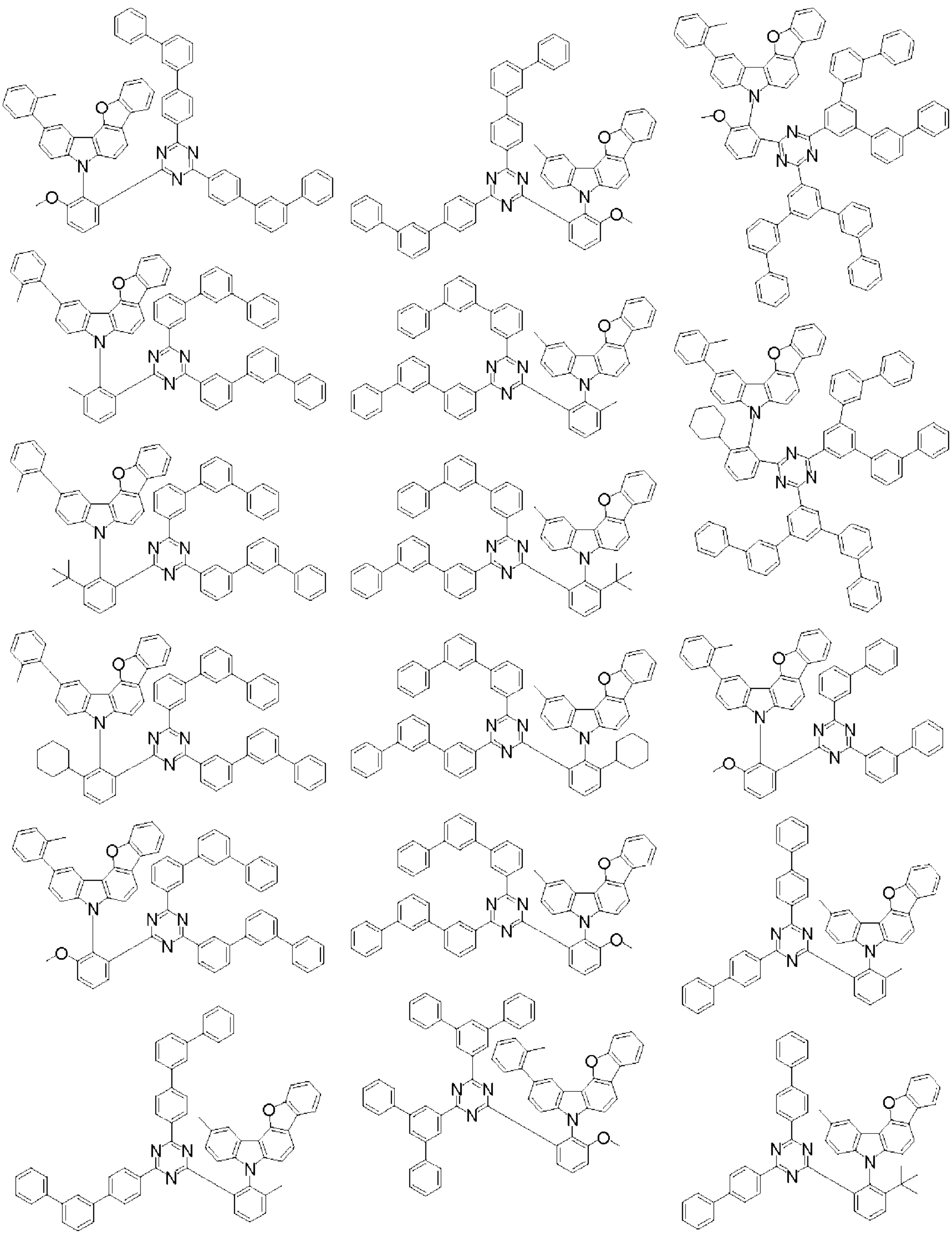

[Chem. 76]

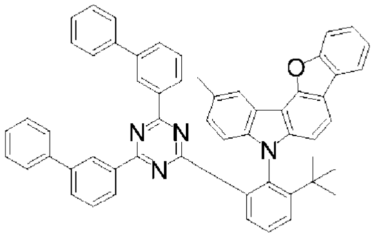
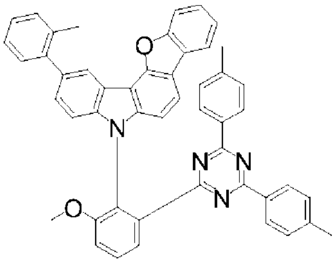
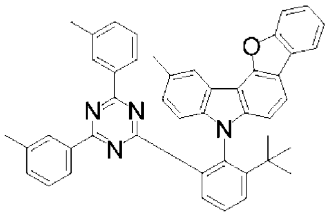
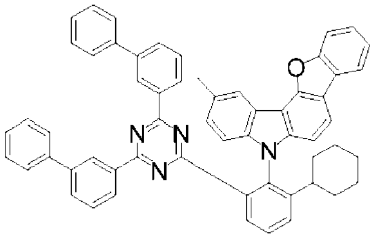
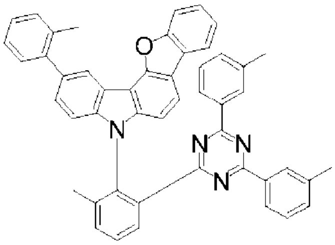
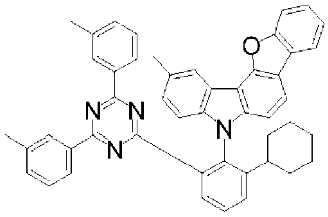
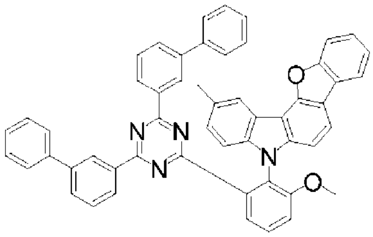
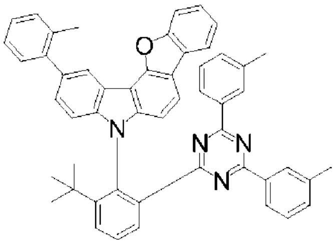
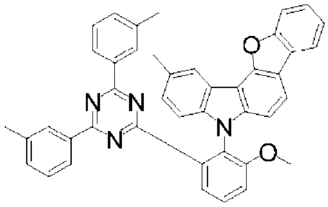
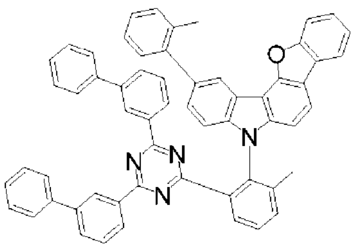
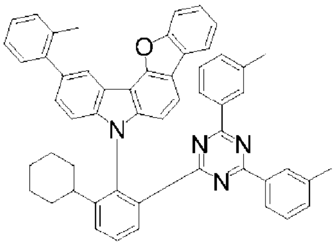
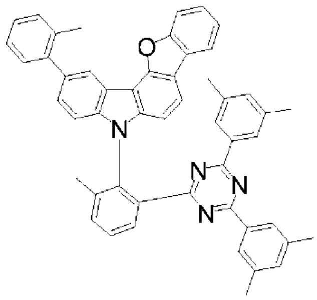
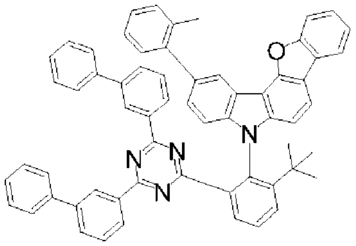
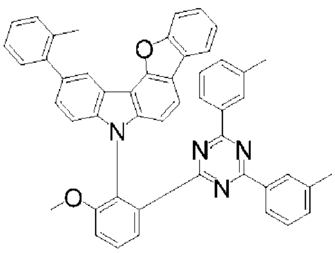
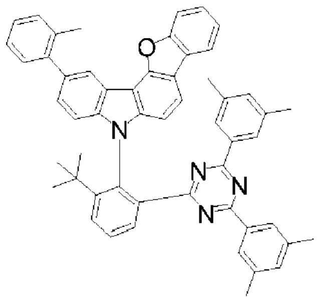
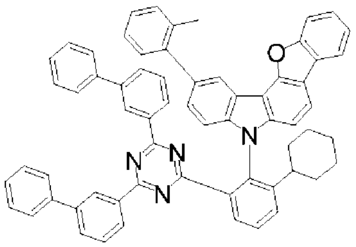
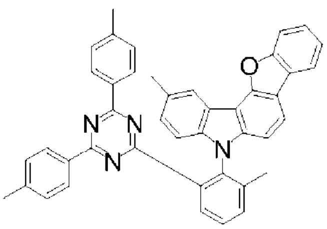

[Chem. 77]

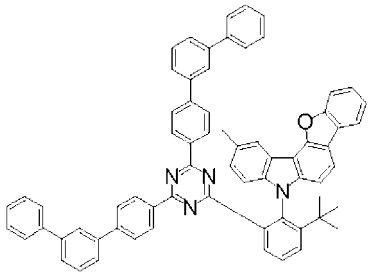
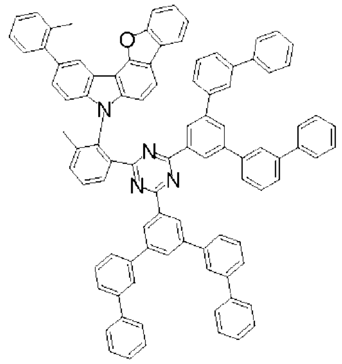
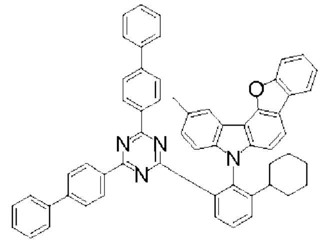
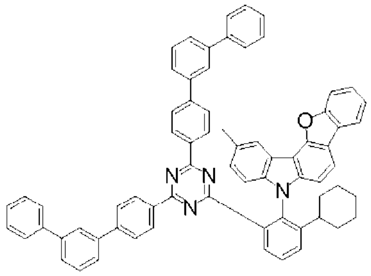
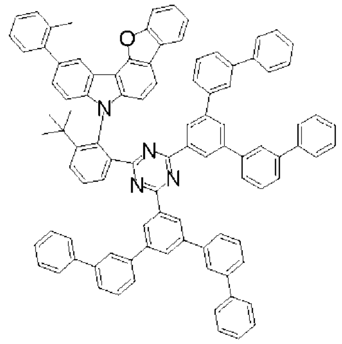
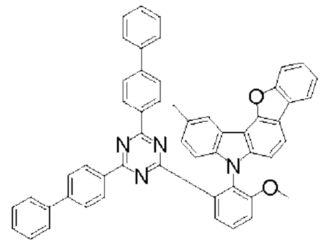
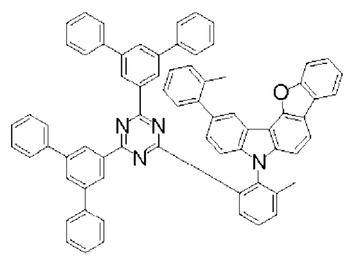
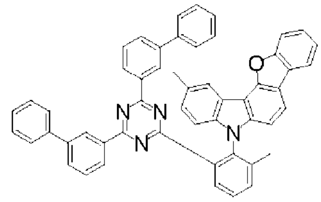
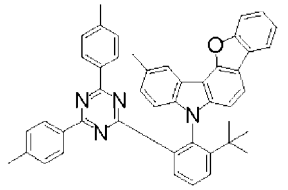
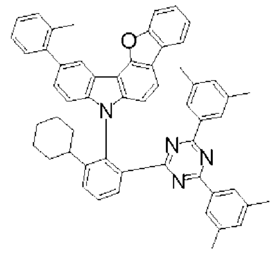
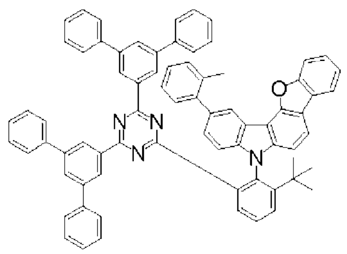
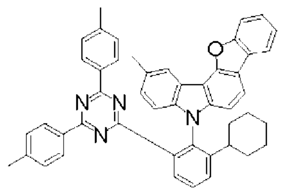
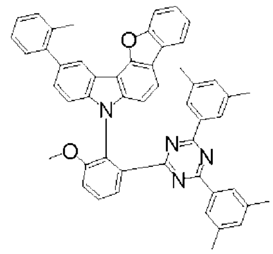
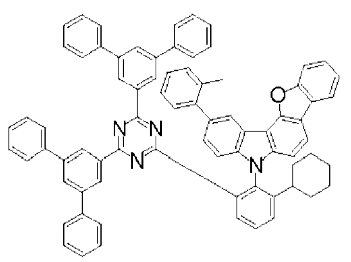
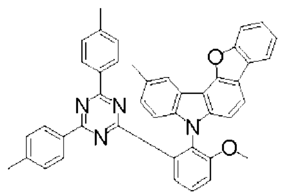
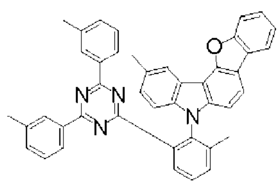

[Chem. 78]

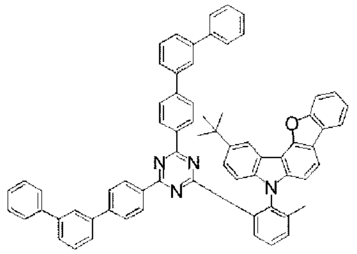
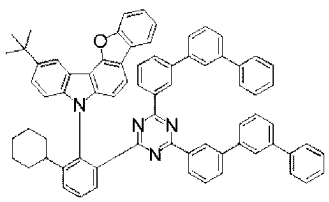
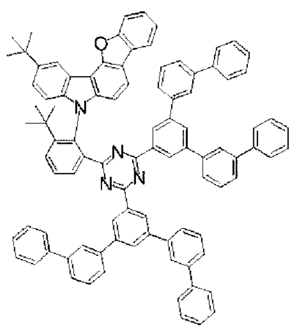
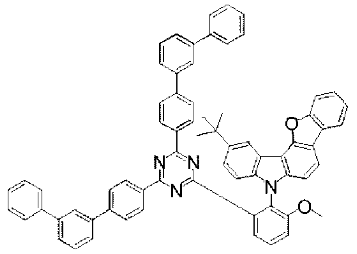
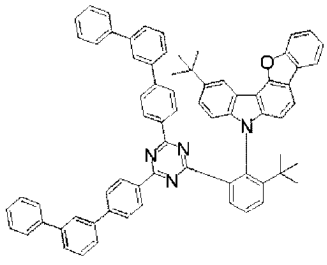
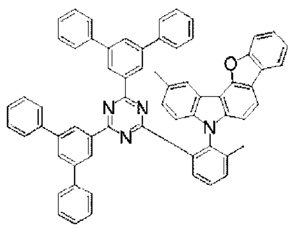
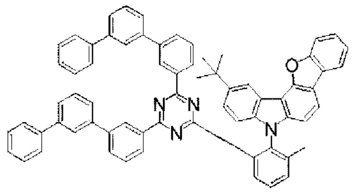
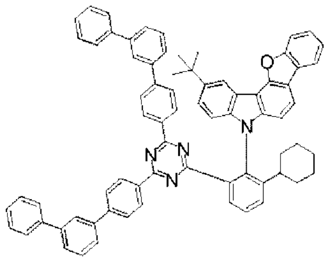
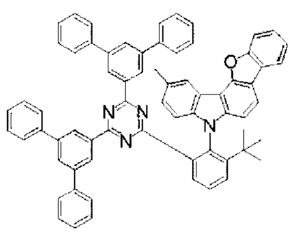
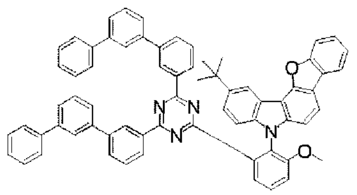
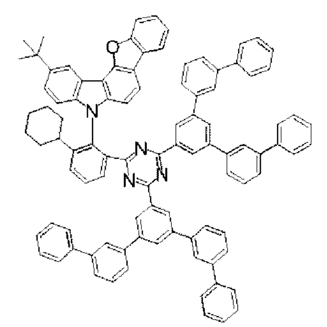
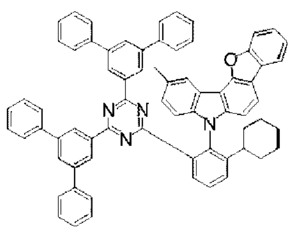
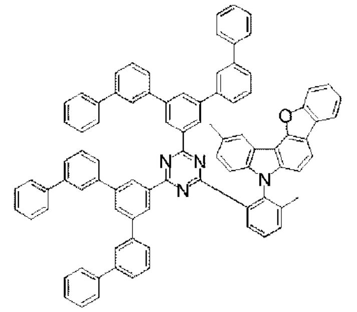
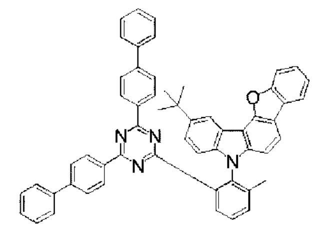
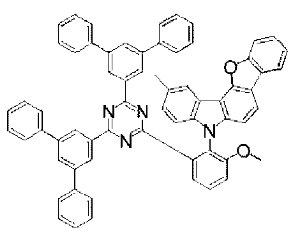
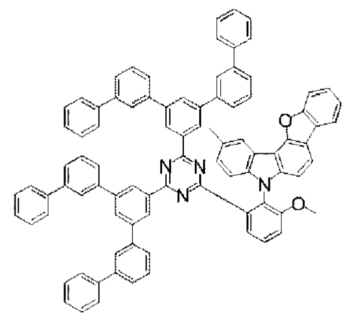
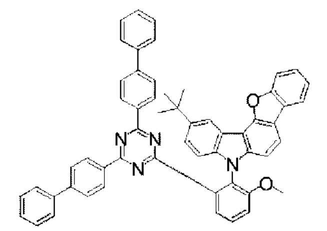
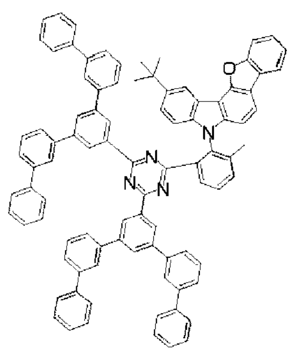

[Chem. 79]

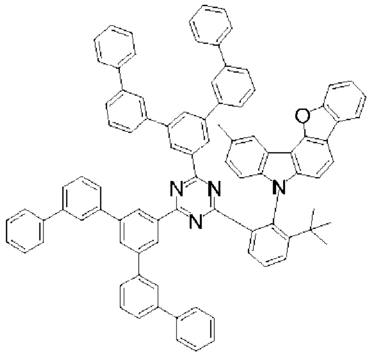
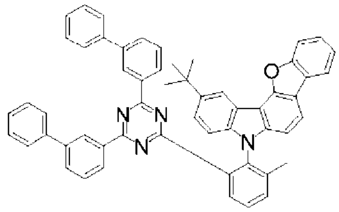
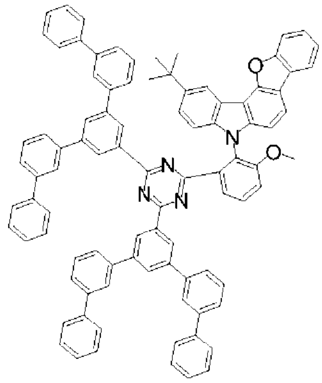
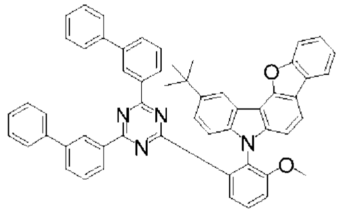
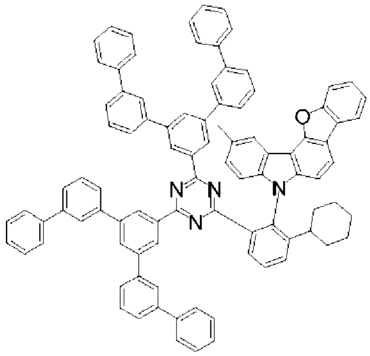
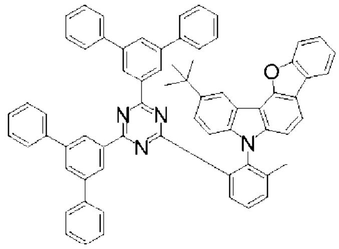
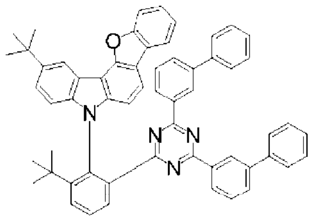
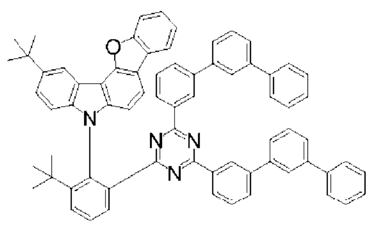
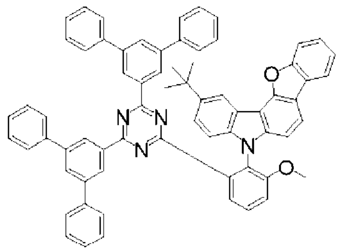
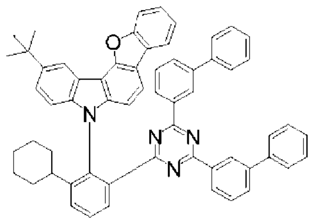
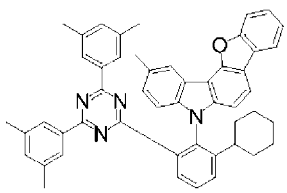
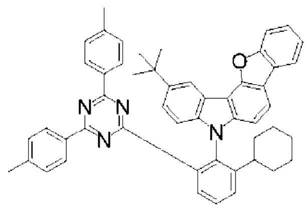
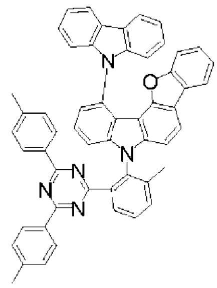
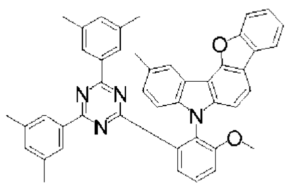

[Chem. 80]

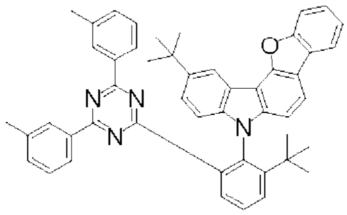

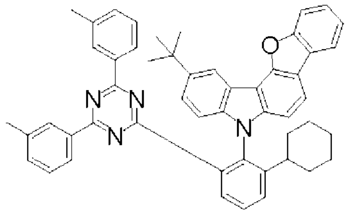

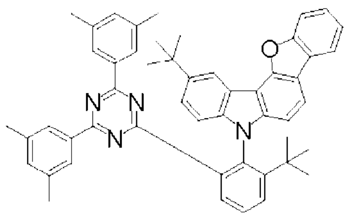

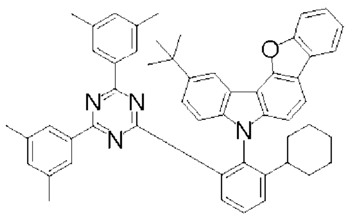

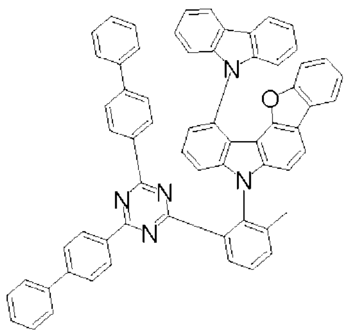

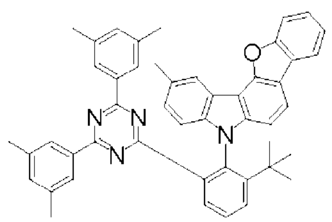

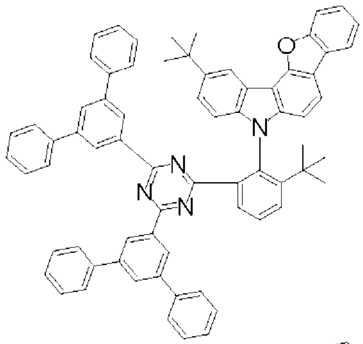

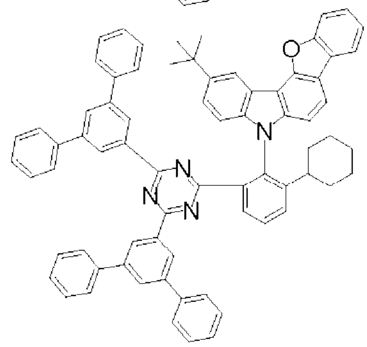

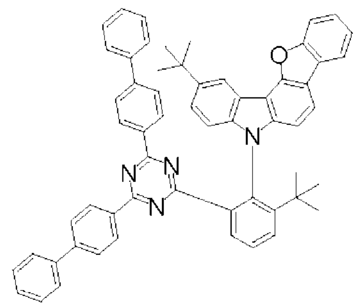

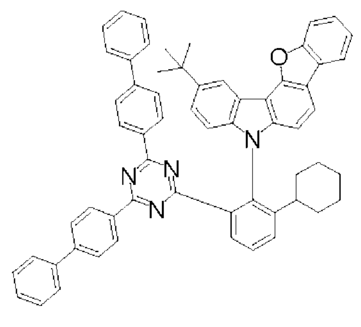

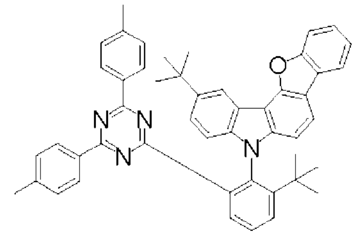

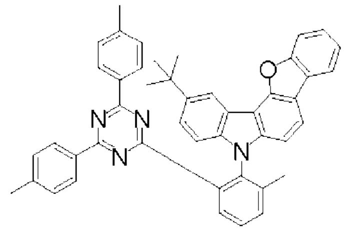

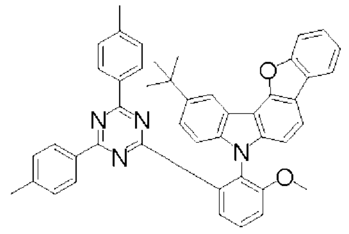

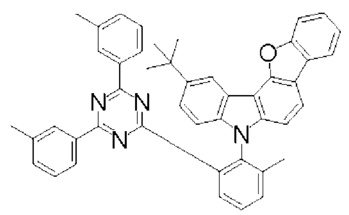

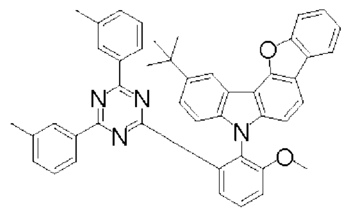

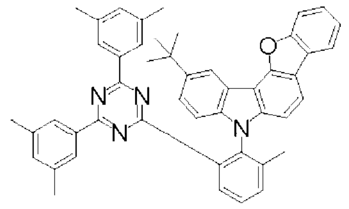

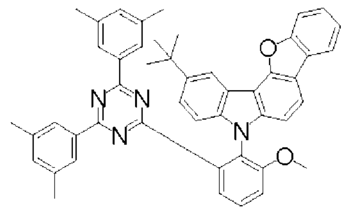

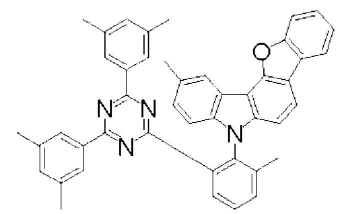

[Chem. 81]

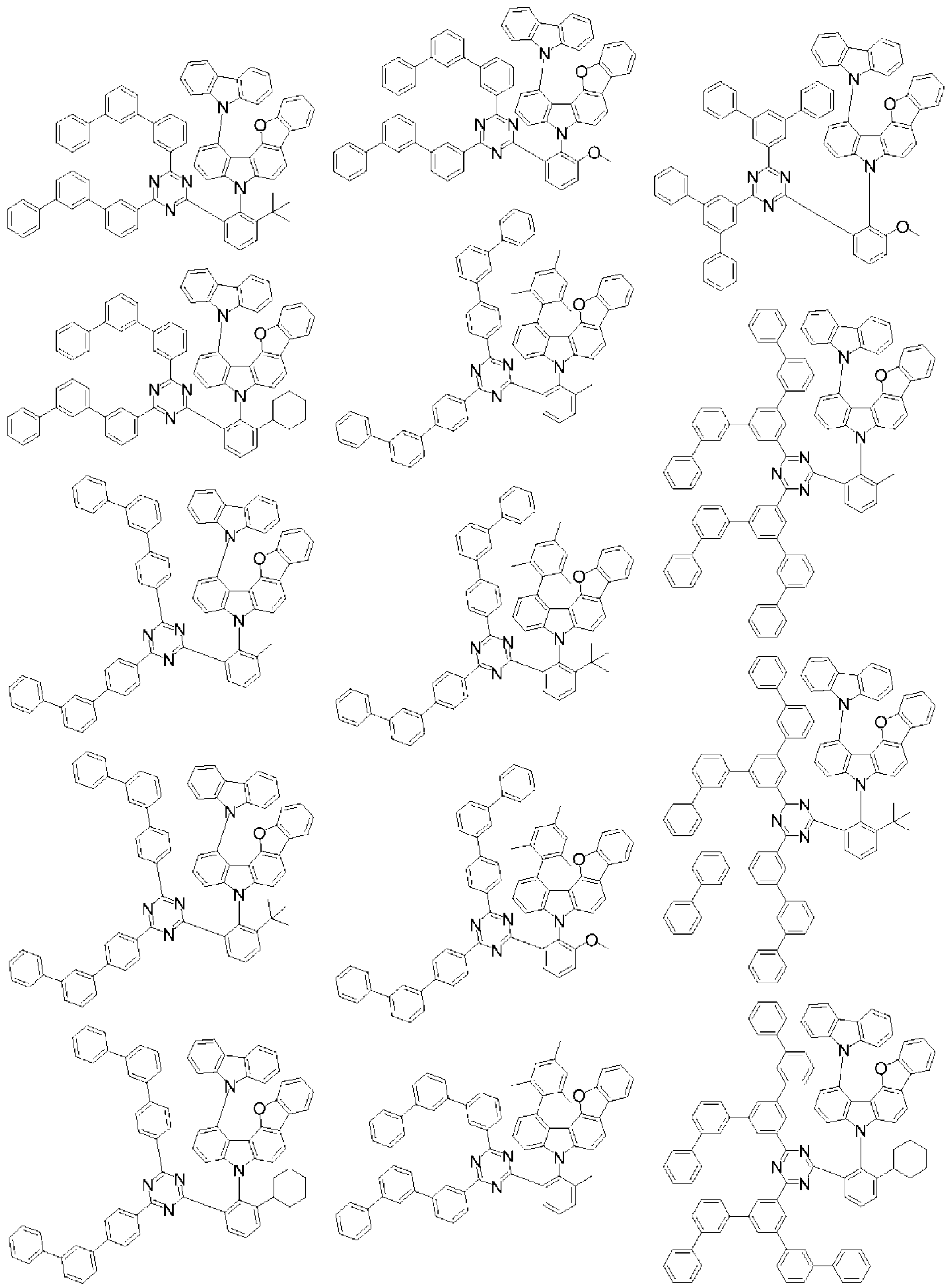

[Chem. 82]

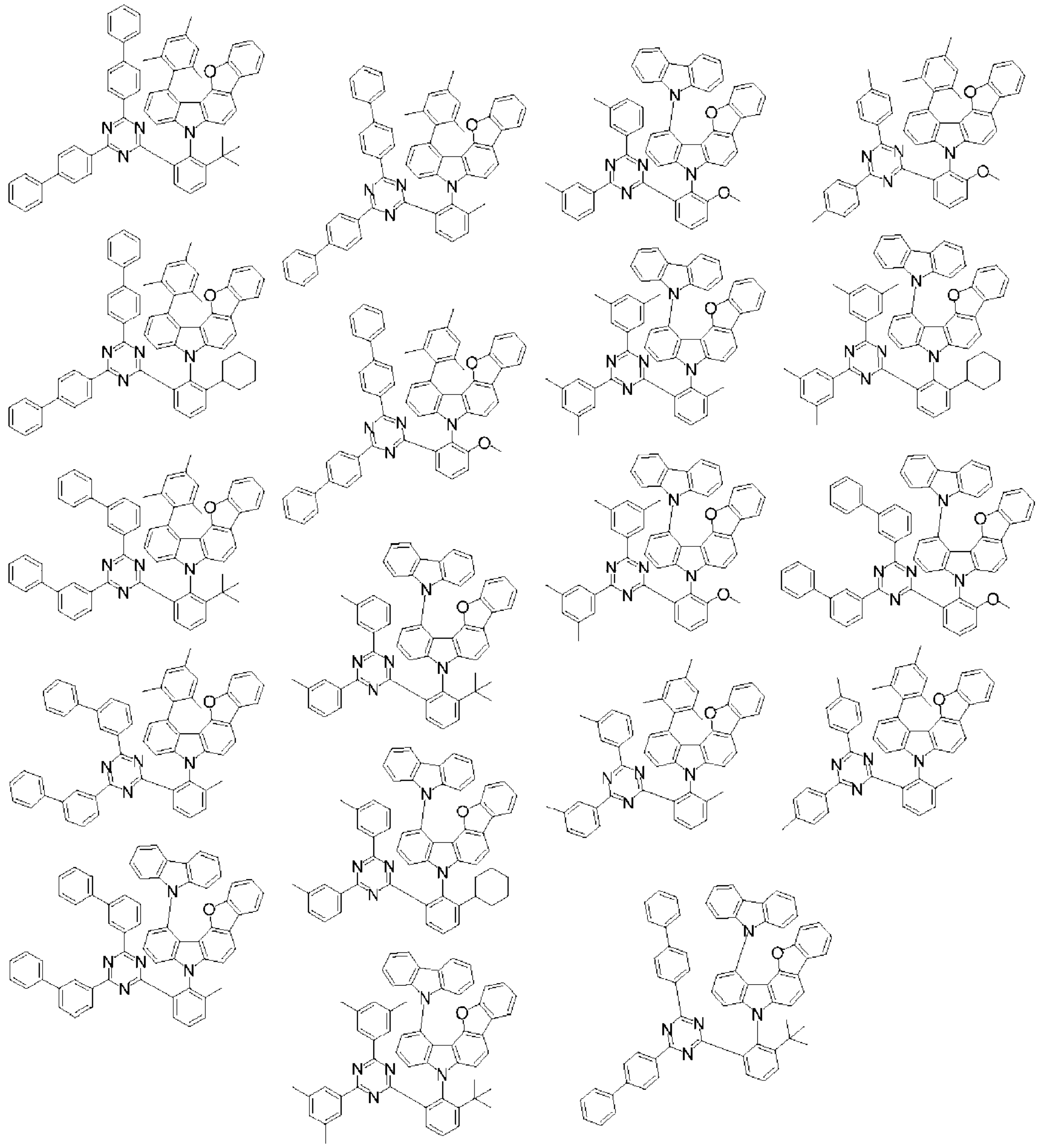

[Chem. 83]

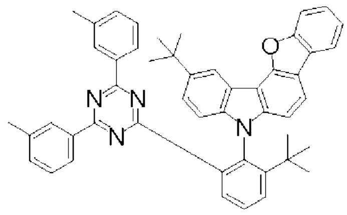
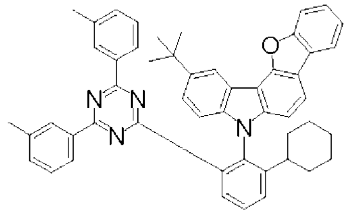
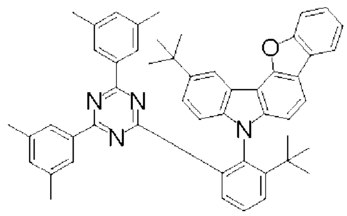
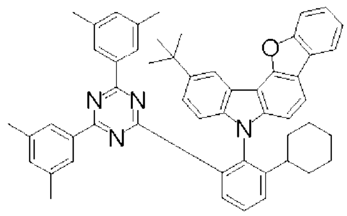
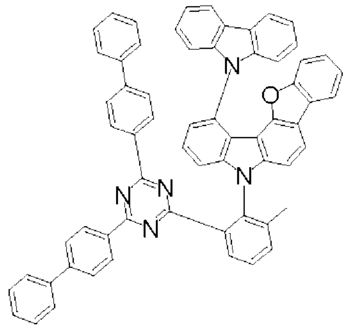
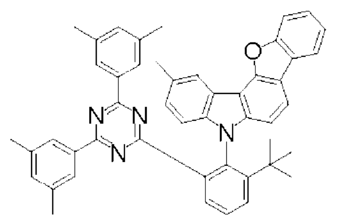
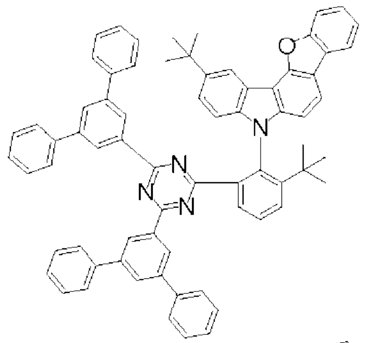
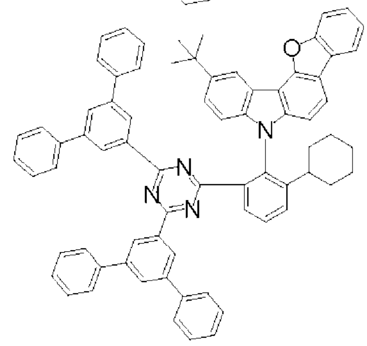
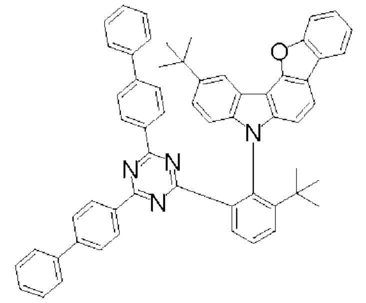
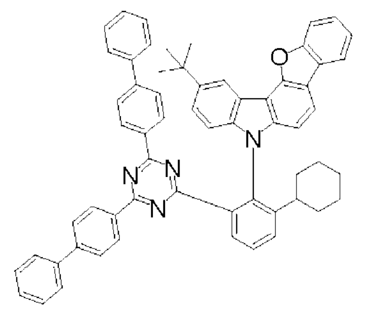
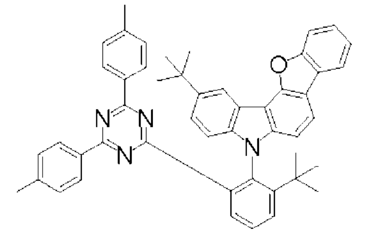
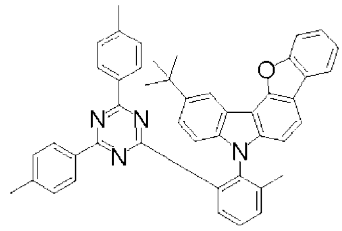
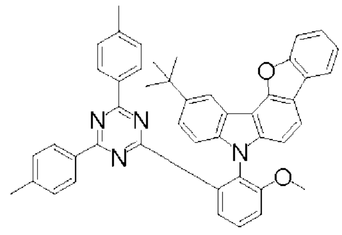
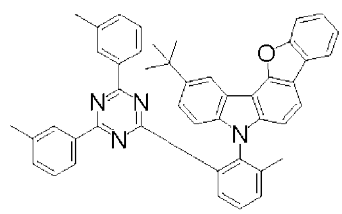
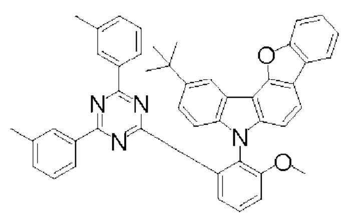
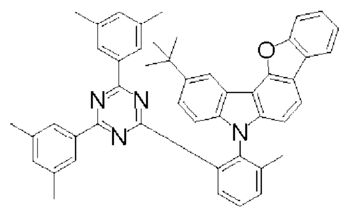
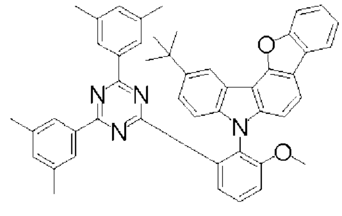
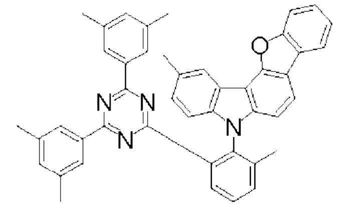

[Chem. 84]

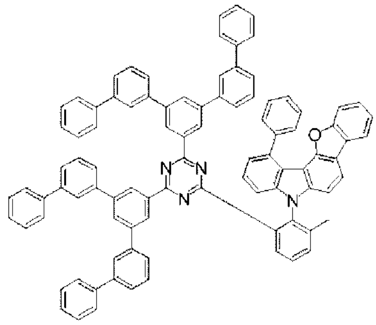
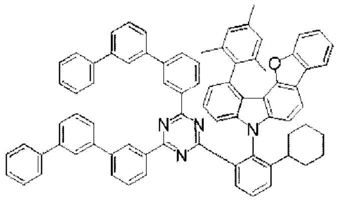
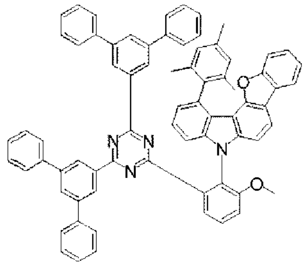
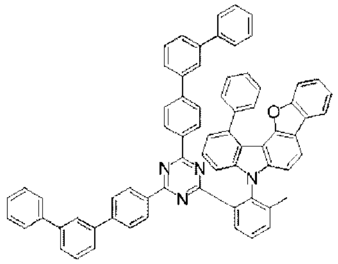
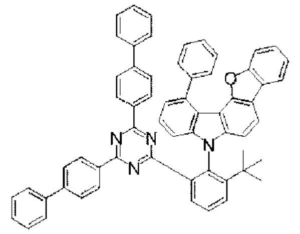
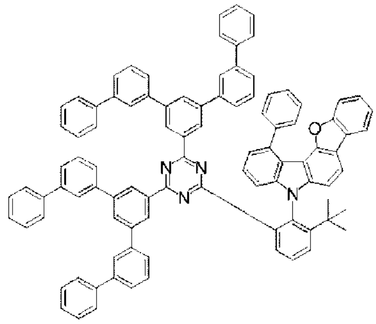
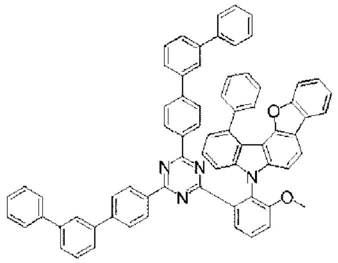
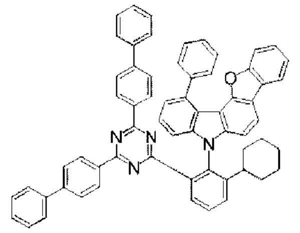
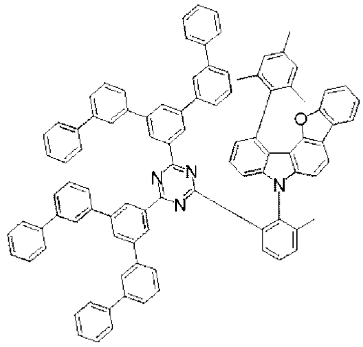
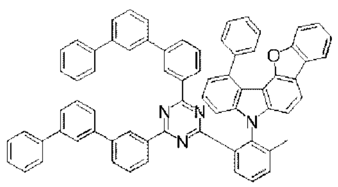
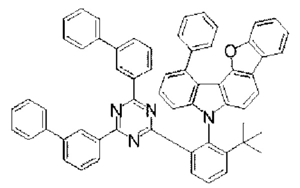
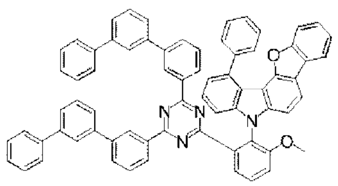
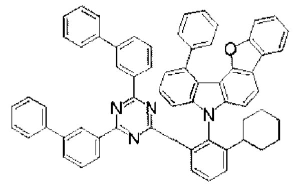
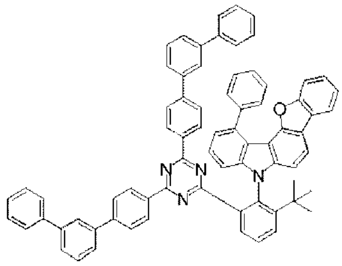
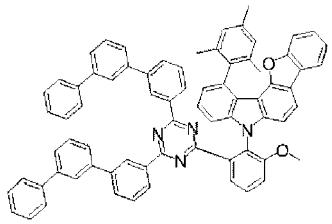
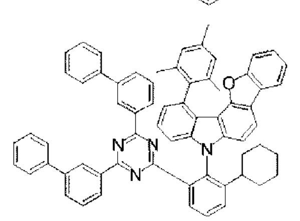
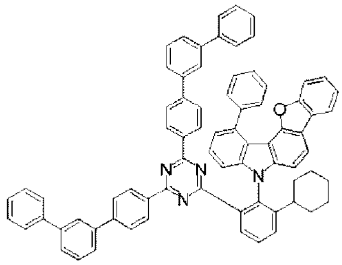
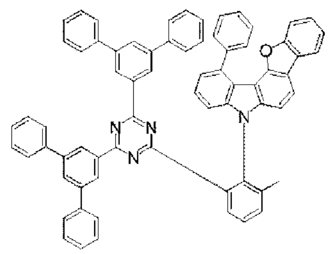
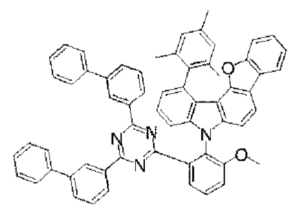

[Chem. 85]

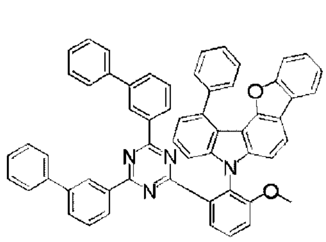

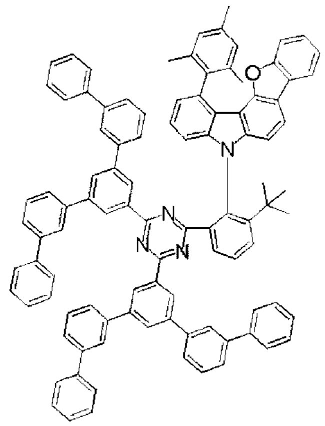

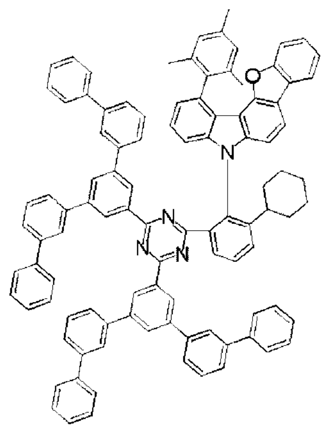

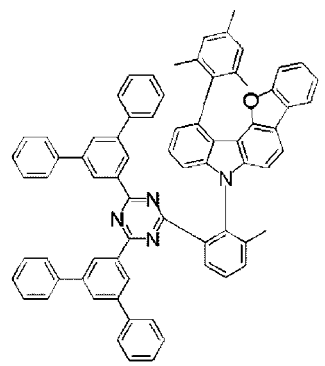

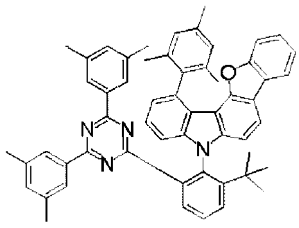

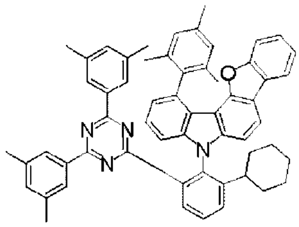

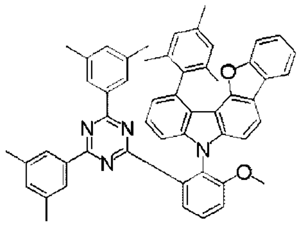

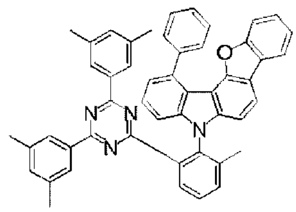

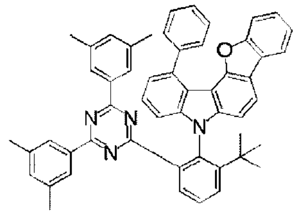

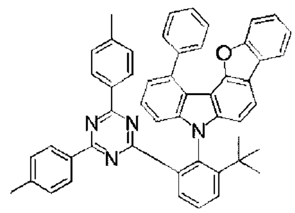

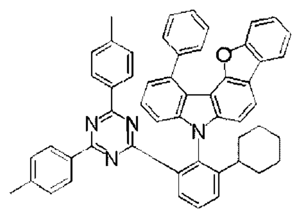

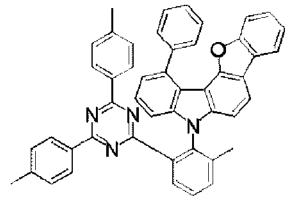

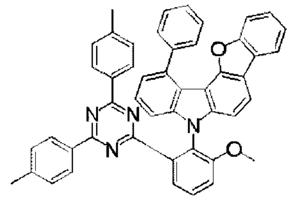

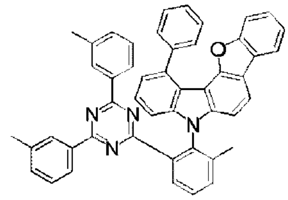

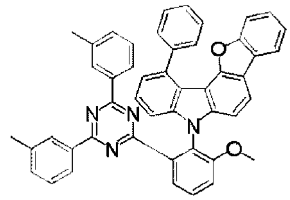

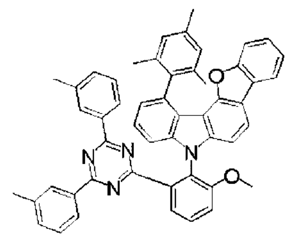

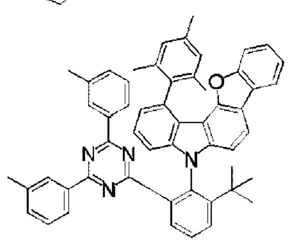

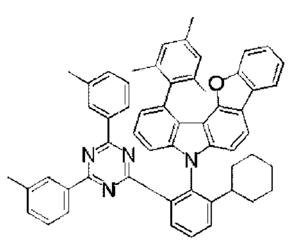

[Chem. 86]

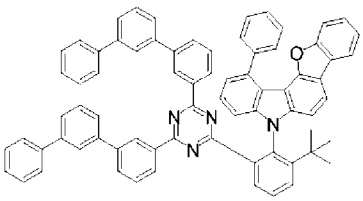
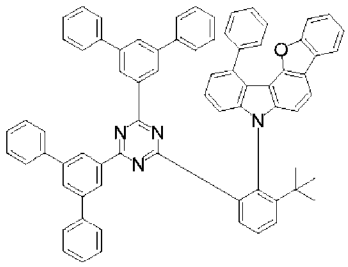
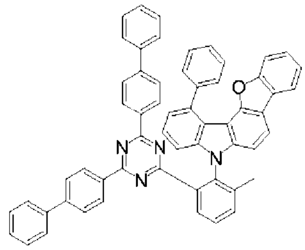
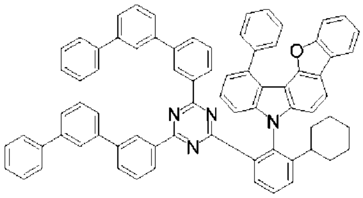
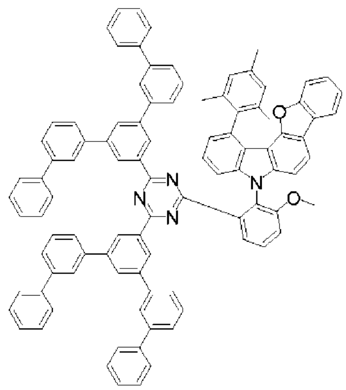
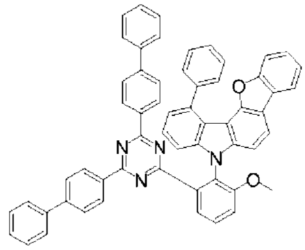
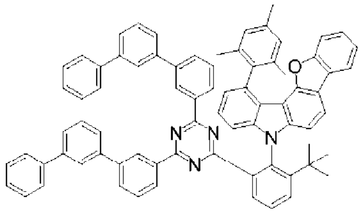
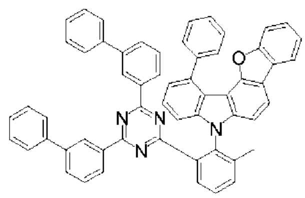
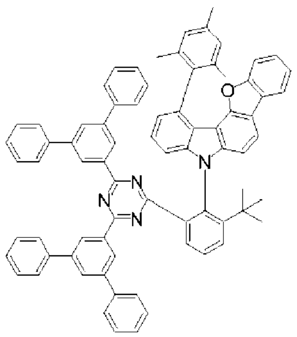
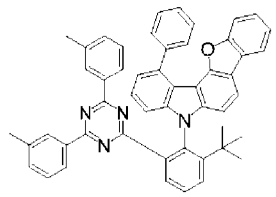
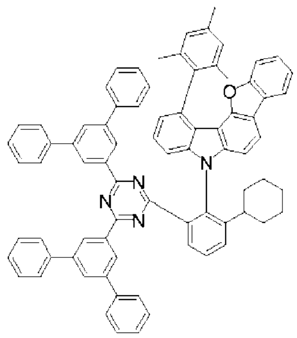
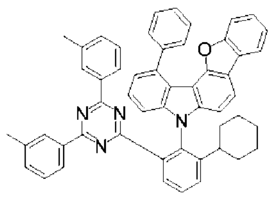
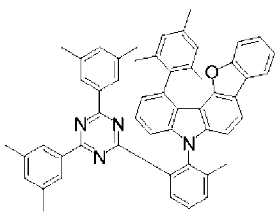

[Chem. 87]

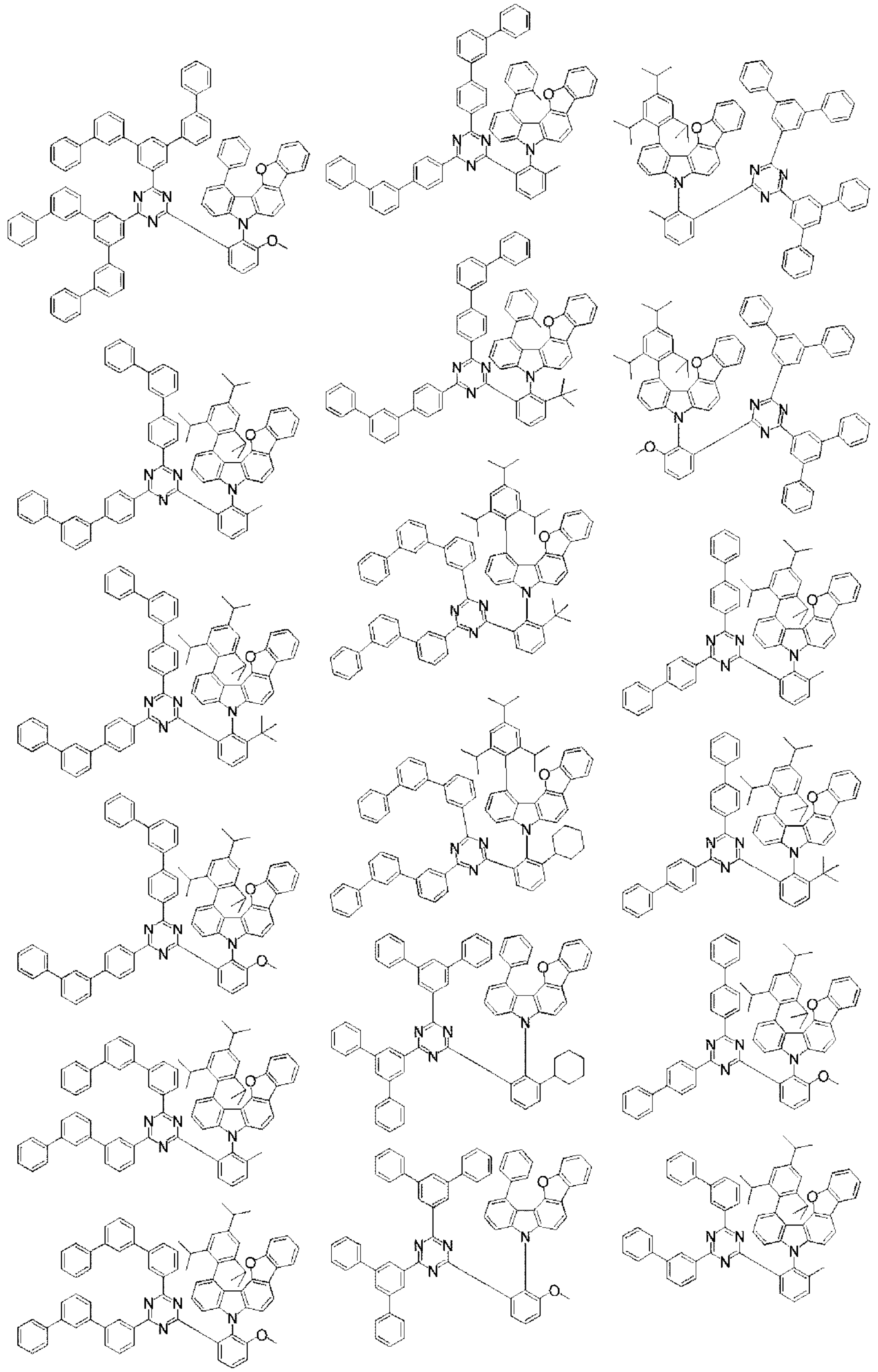

[Chem. 88]

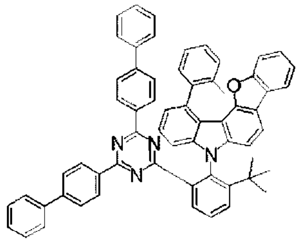

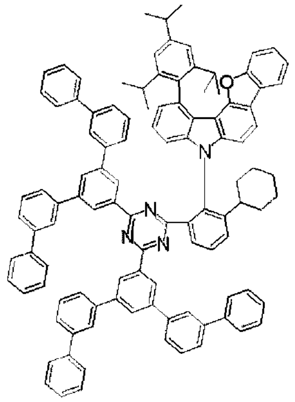

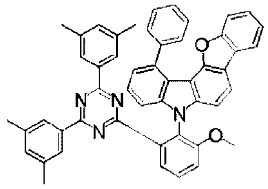

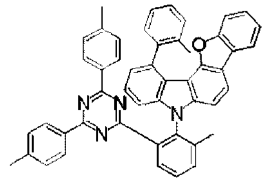

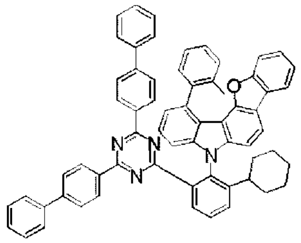

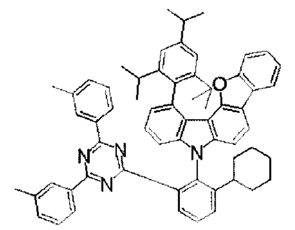

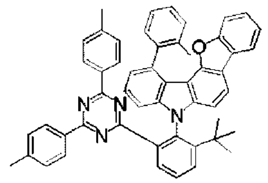

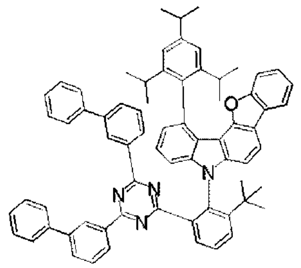

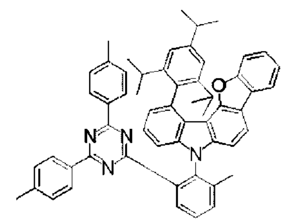

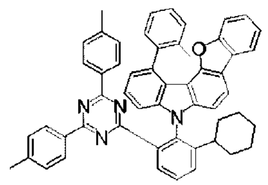

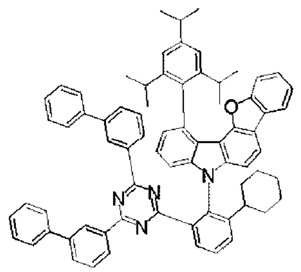

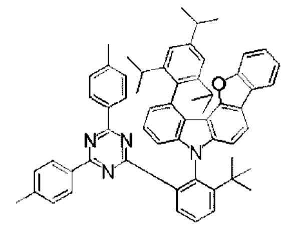

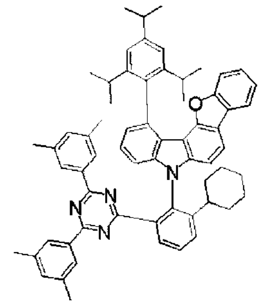

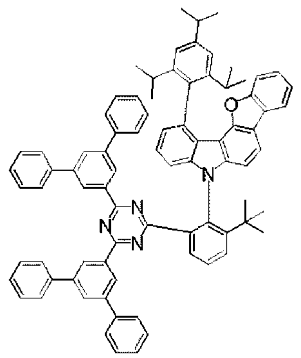

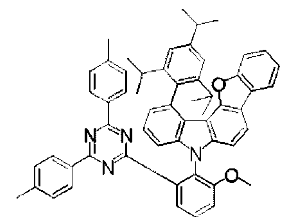

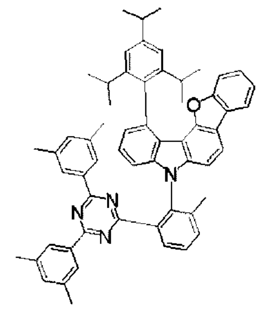

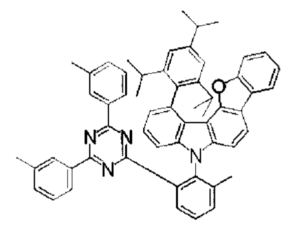

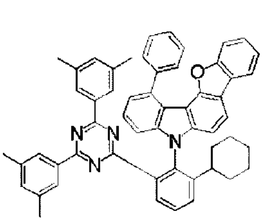

[Chem. 89]

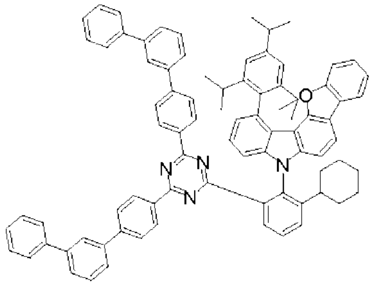
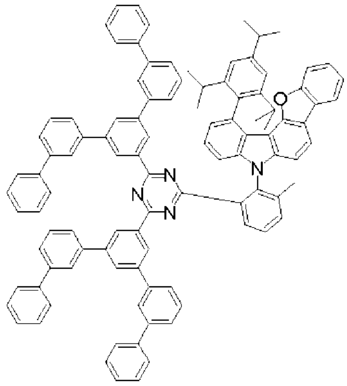
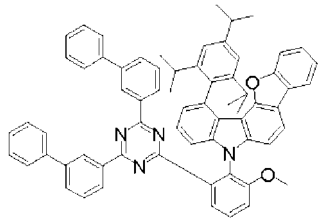
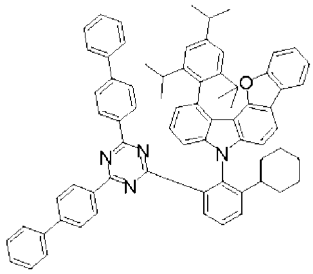
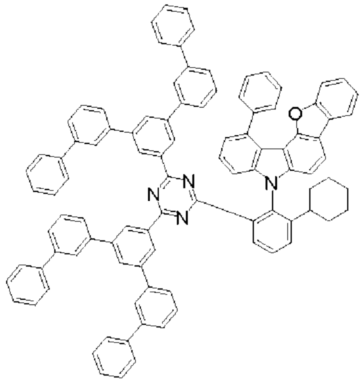
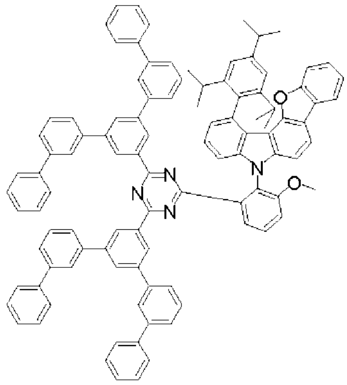
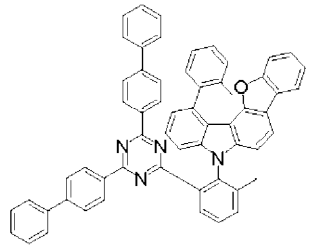
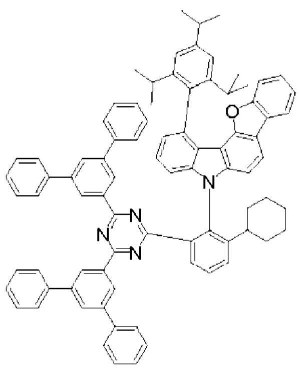
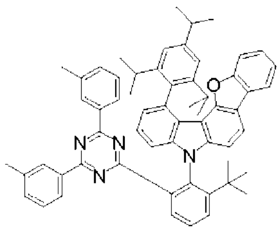
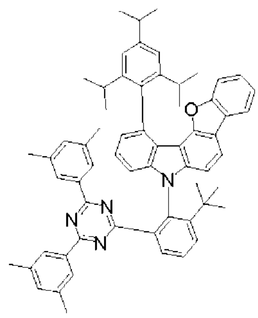
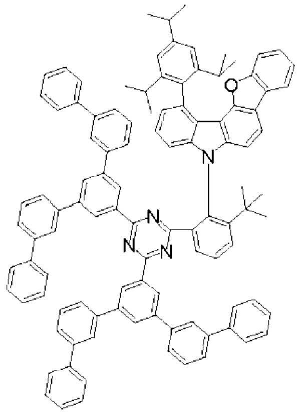
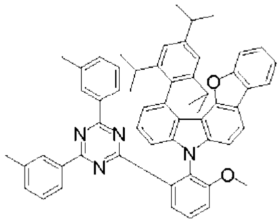
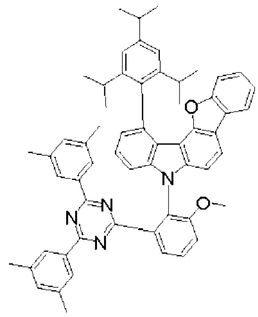
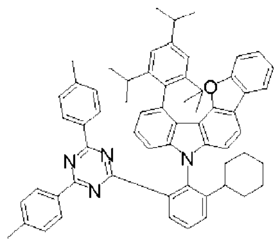

[Chem. 90]

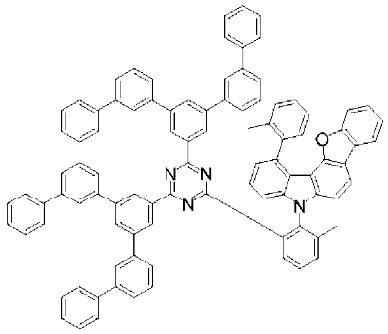

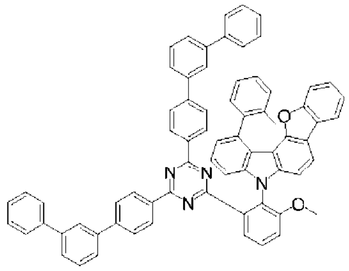

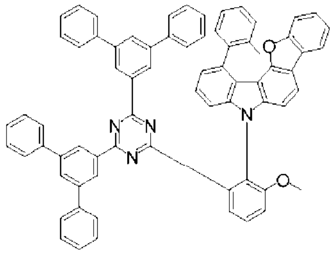

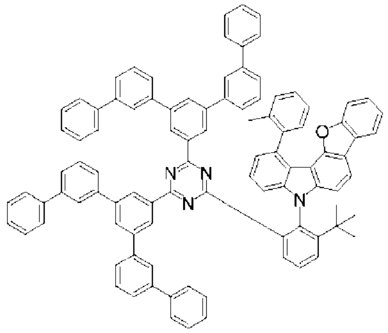

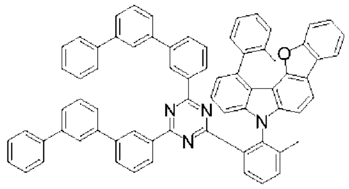

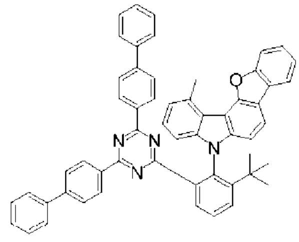

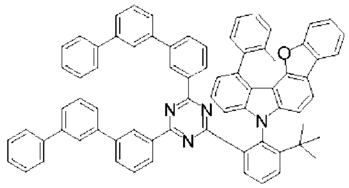

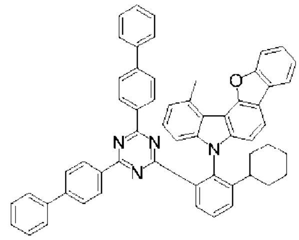

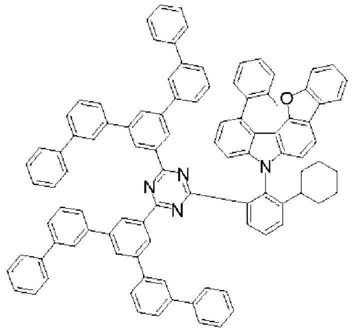

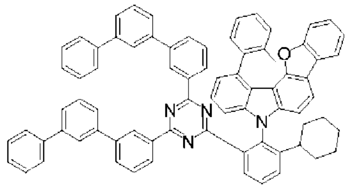

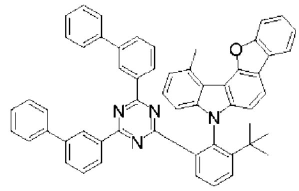

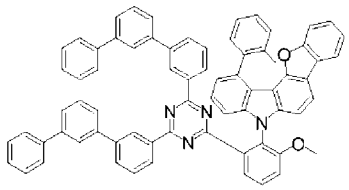

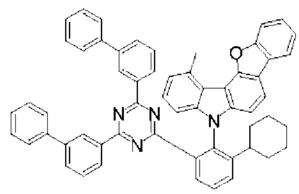

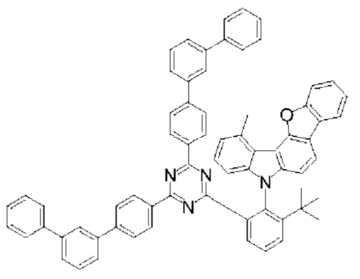

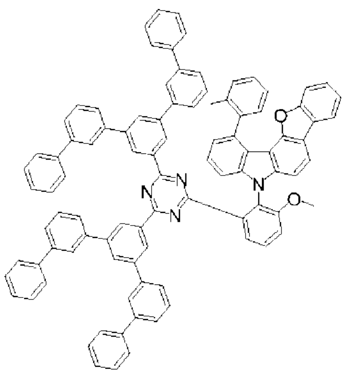

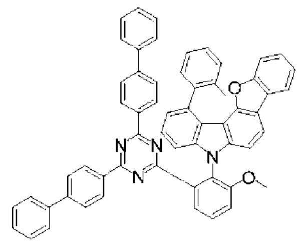

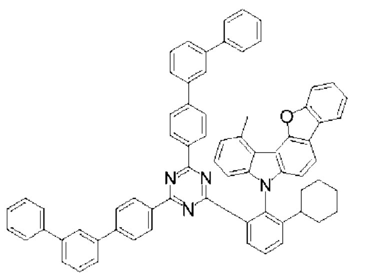

[Chem. 91]

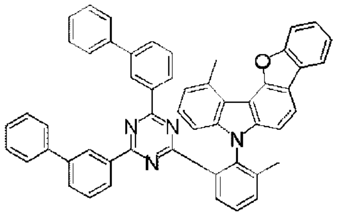
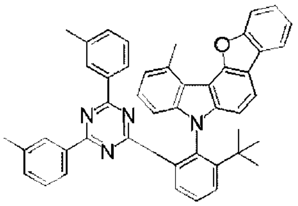
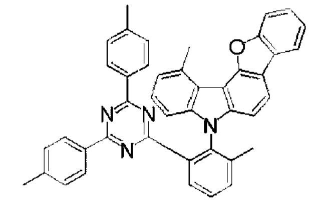
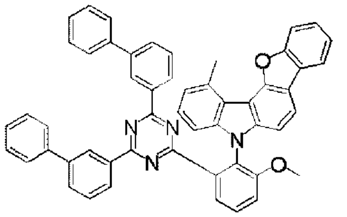
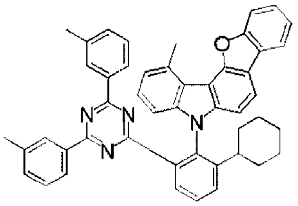
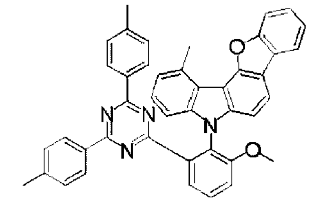
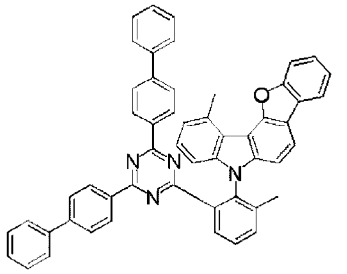
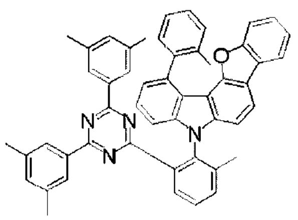
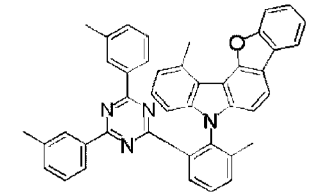
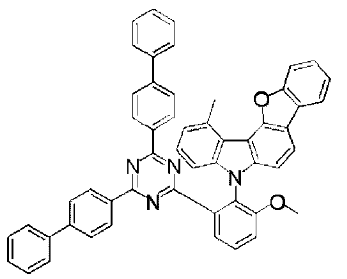
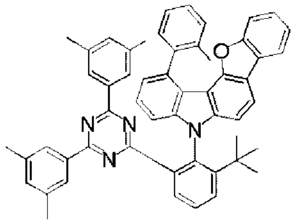
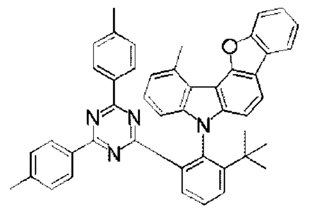
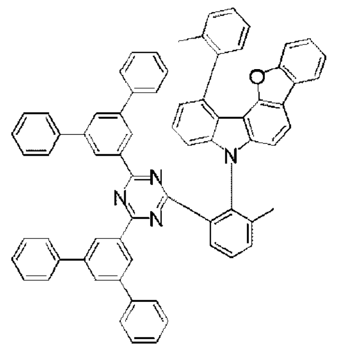
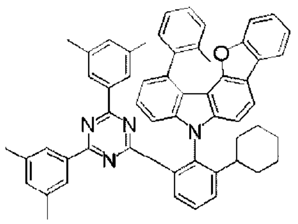
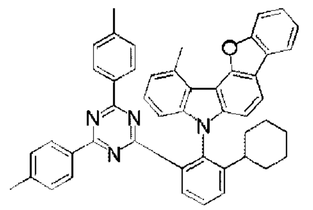
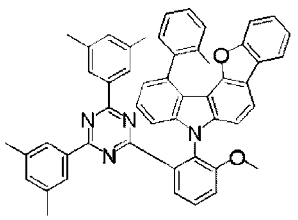
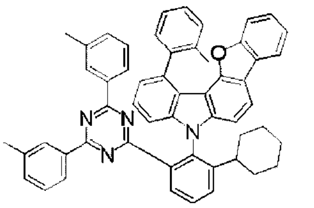
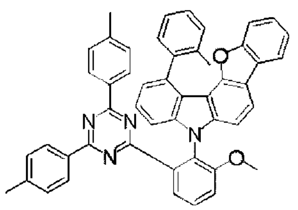
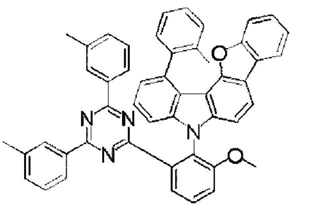

[Chem. 92]

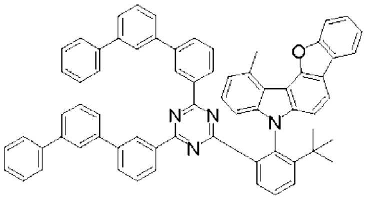

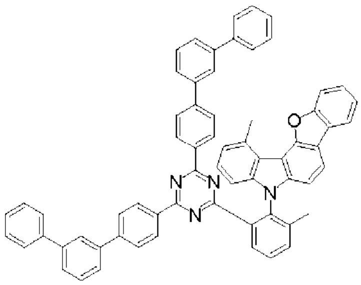

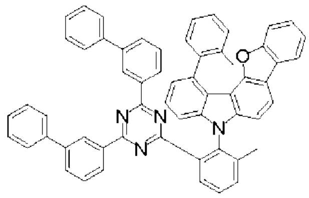

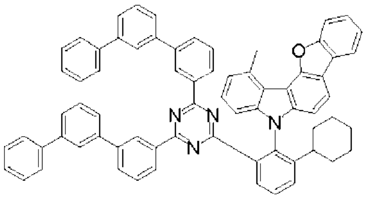

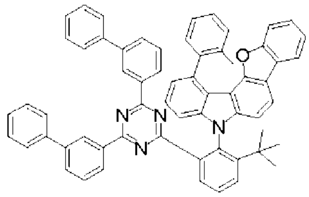

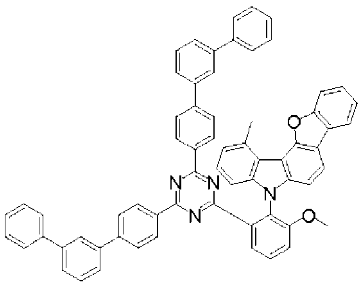

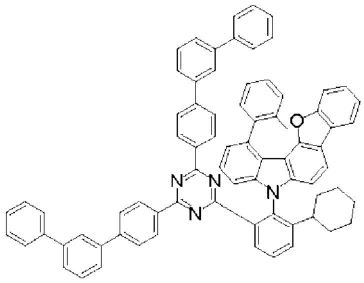

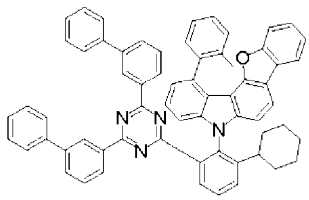

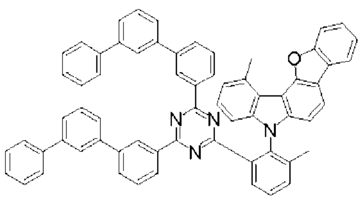

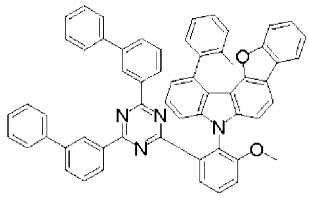

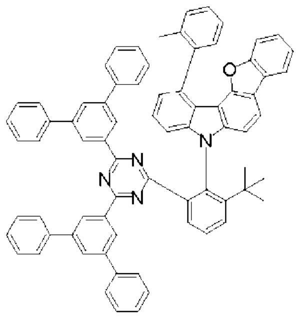

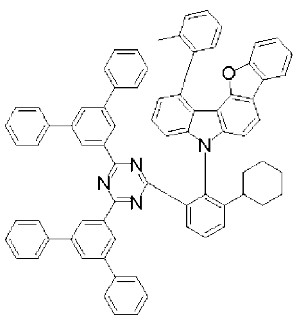

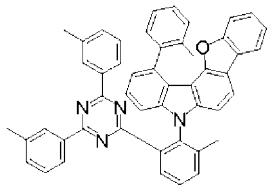

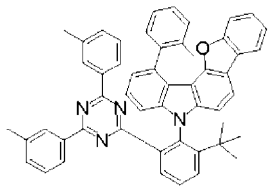

[Chem. 93]

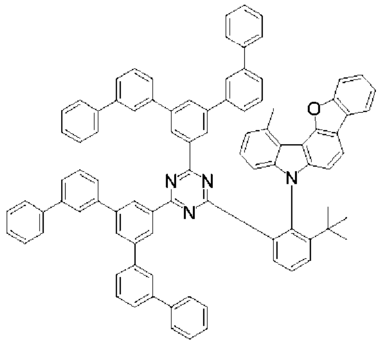
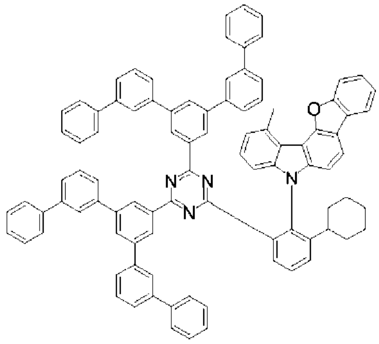
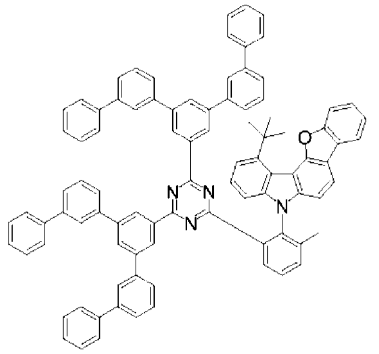
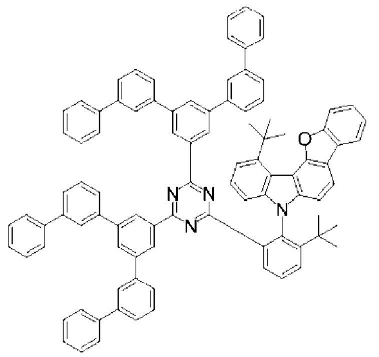
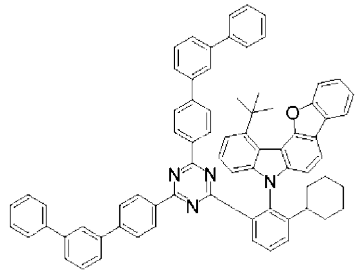
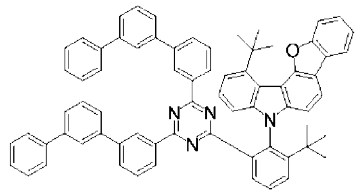
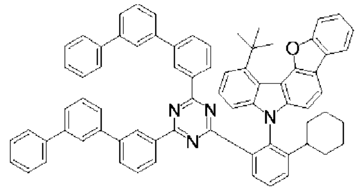
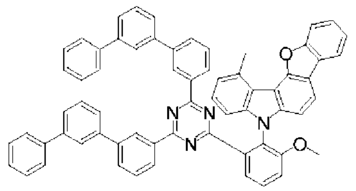
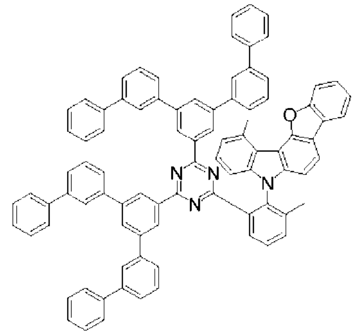
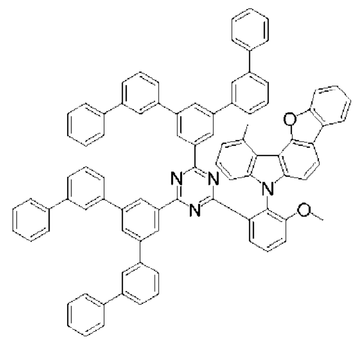
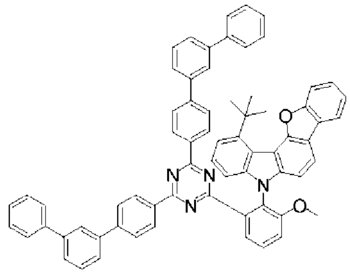
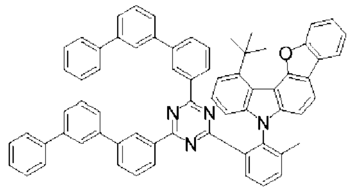
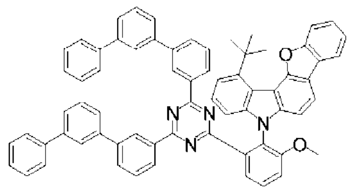
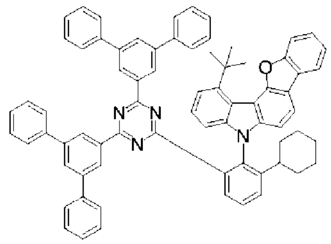
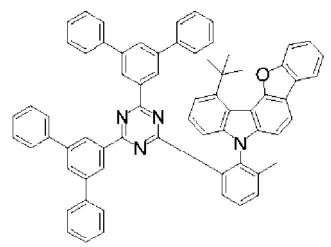
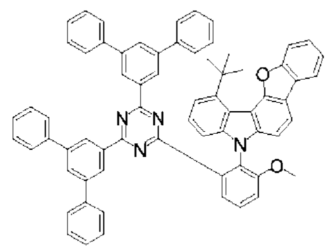

[Chem. 94]

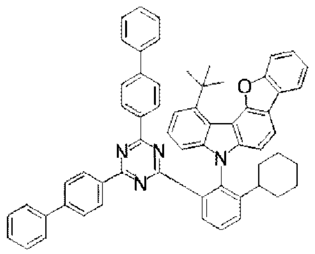
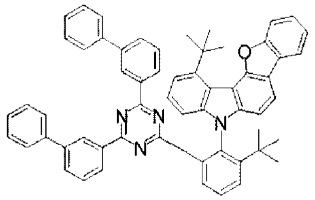
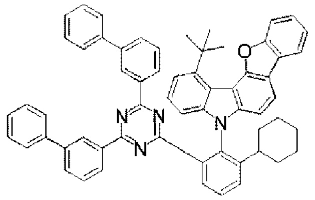
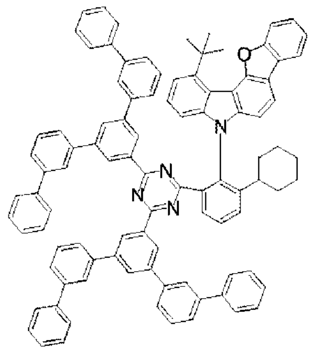
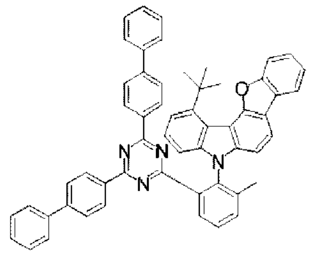
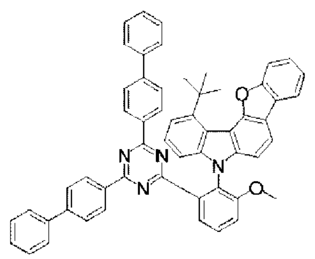
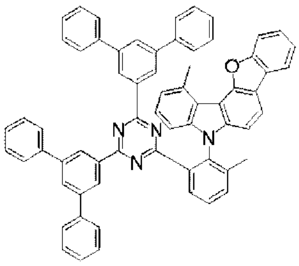
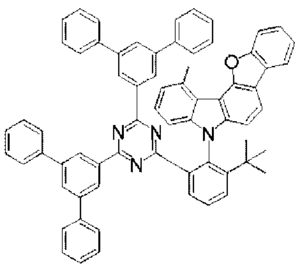
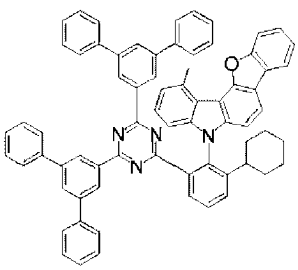
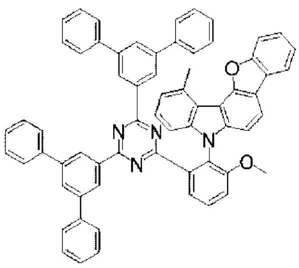
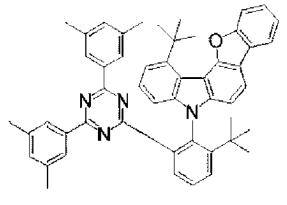
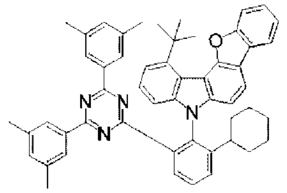
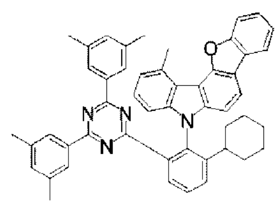
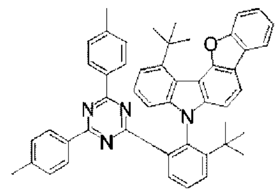
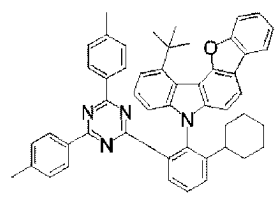
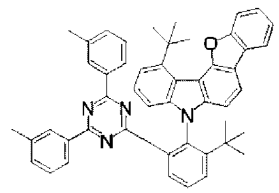
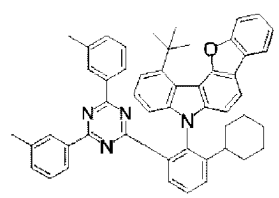
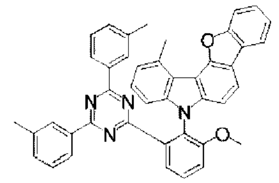
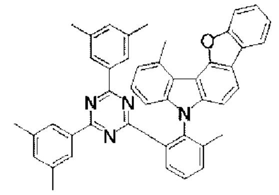

[Chem. 95]

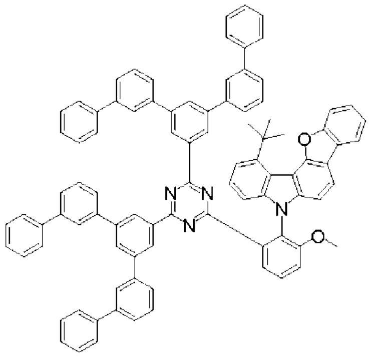

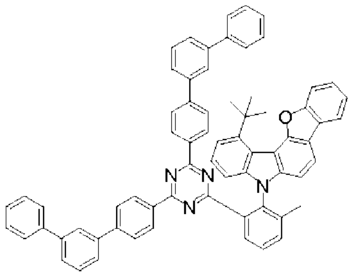

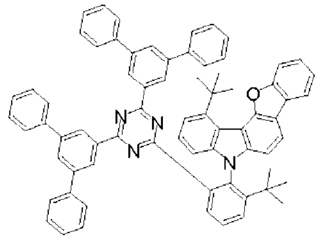

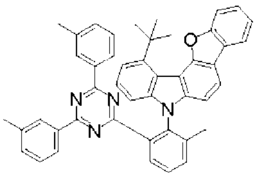

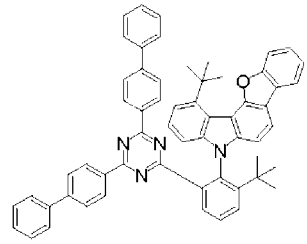

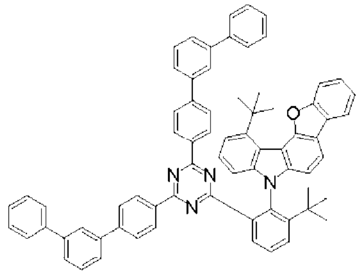

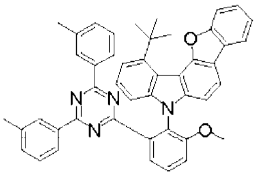

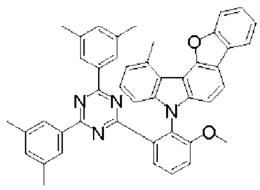

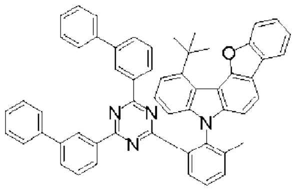

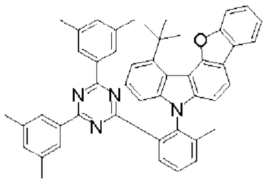

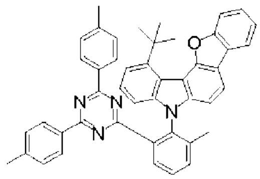

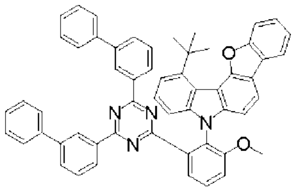

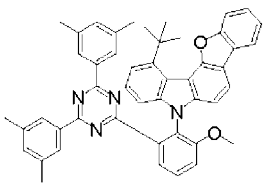

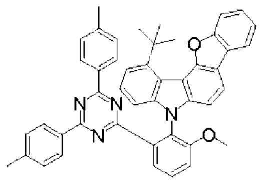

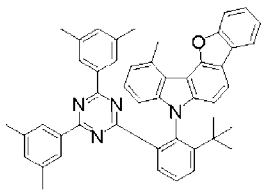

[Chem. 96]

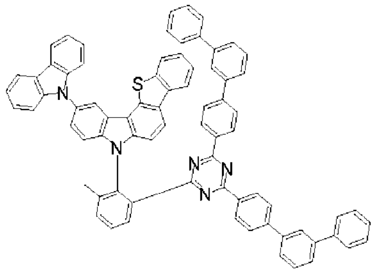
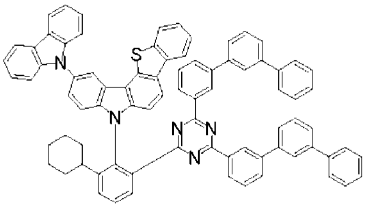
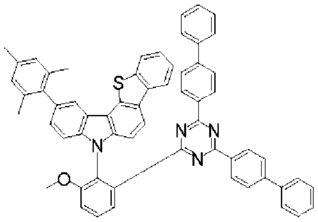
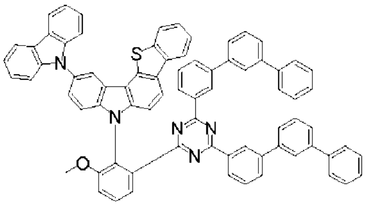
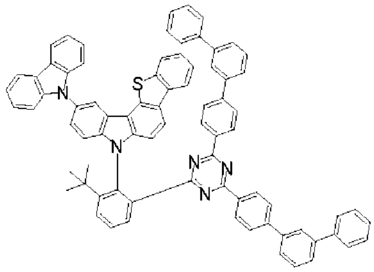
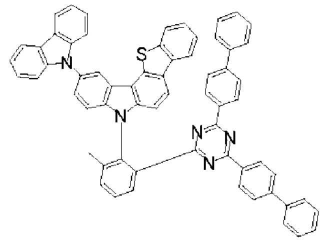
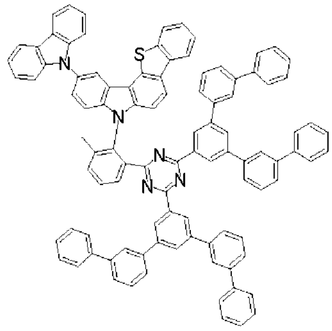
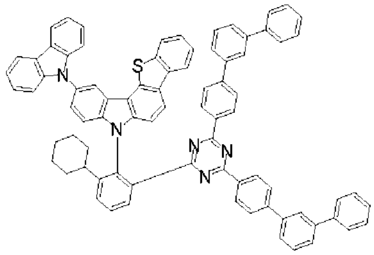
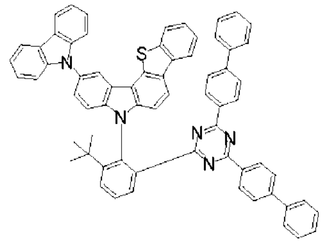
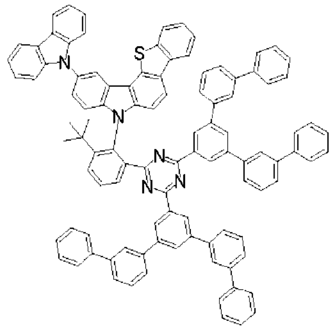
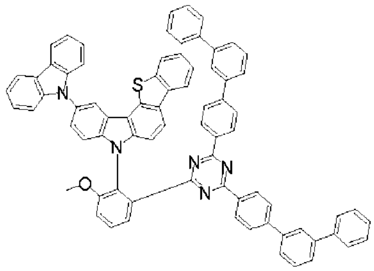
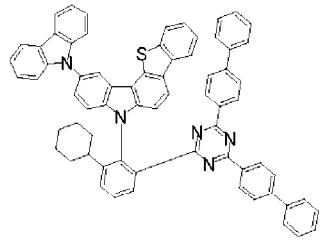
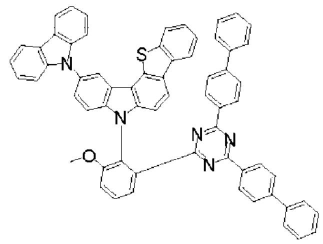
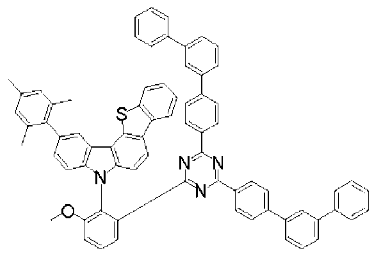
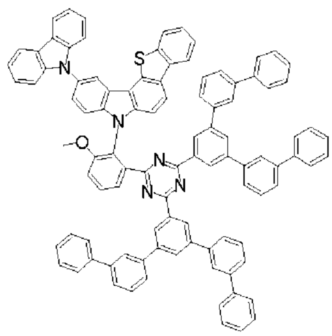
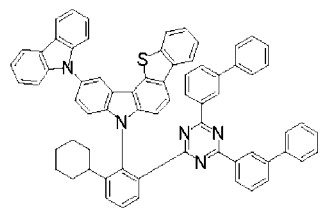
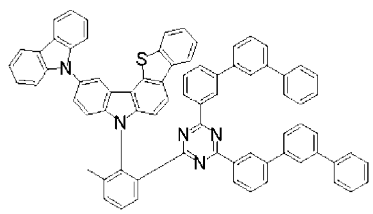

[Chem. 97]

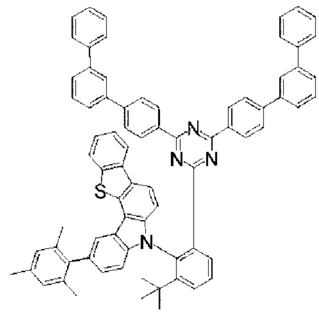

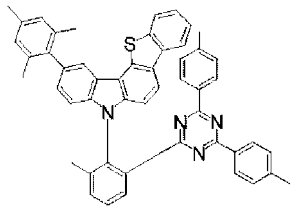

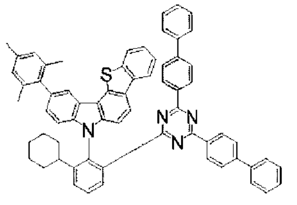

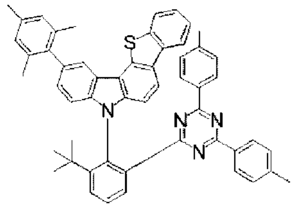

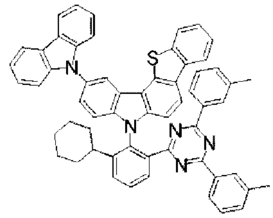

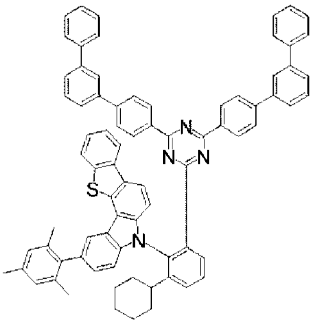

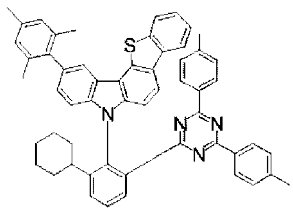

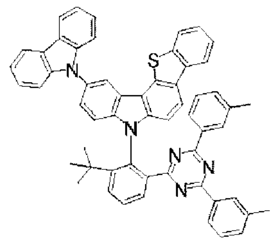

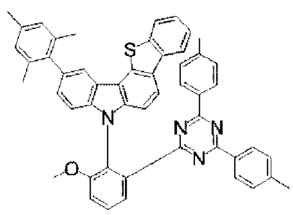

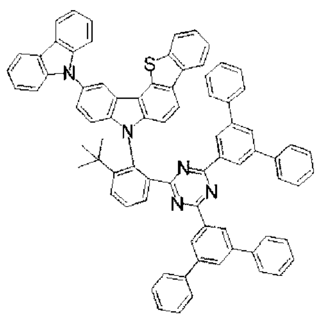

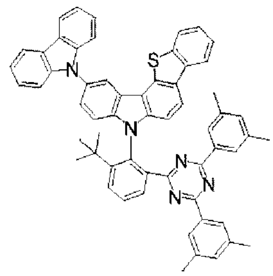

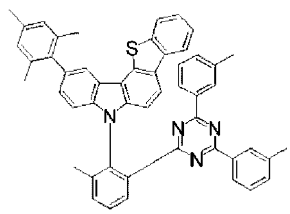

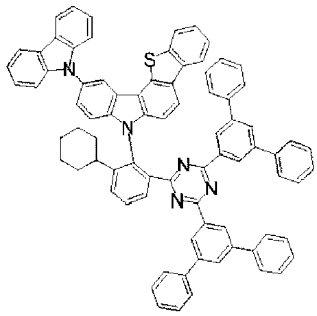

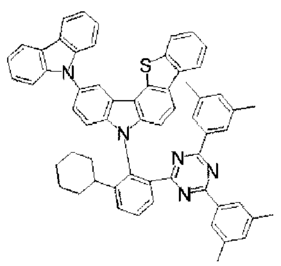

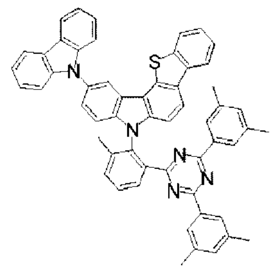

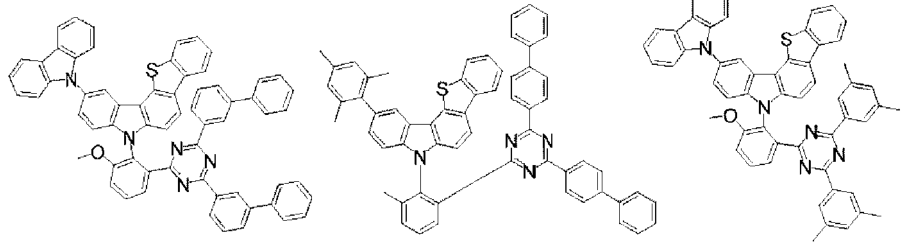

[Chem. 98]

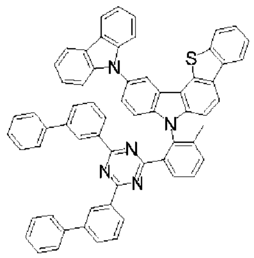
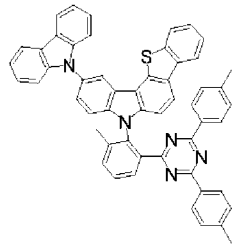
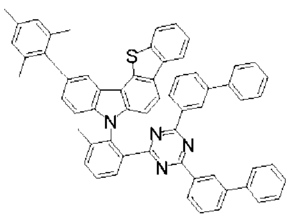
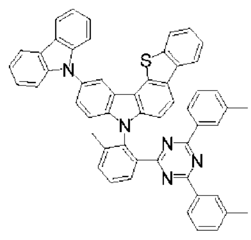
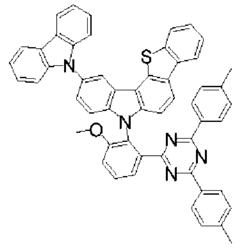
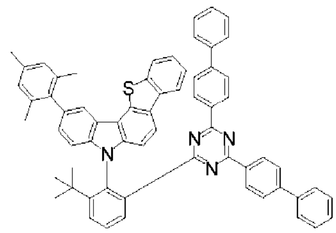
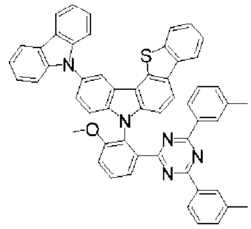
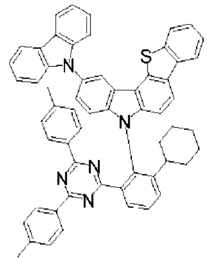
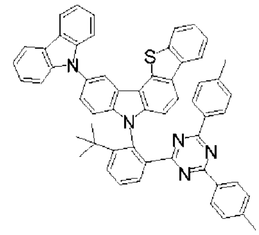
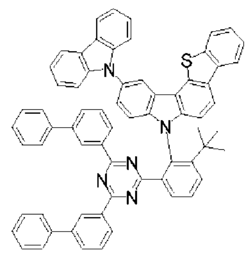
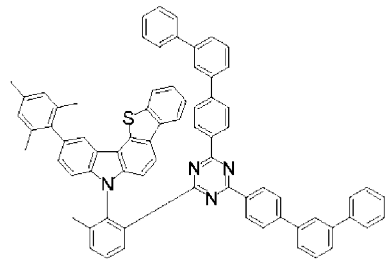
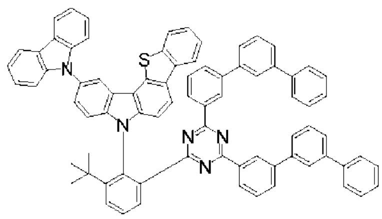
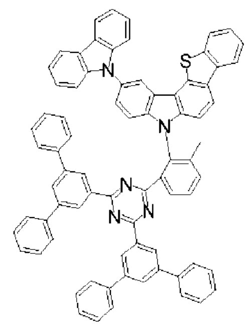
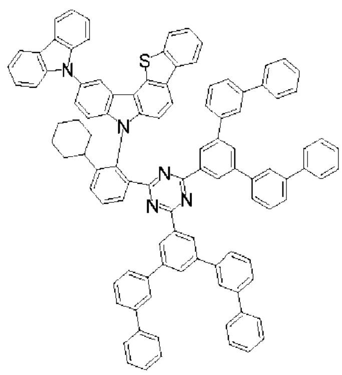
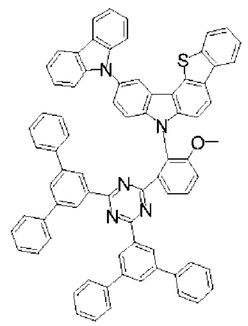

[Chem. 99]

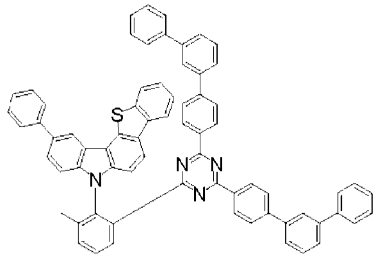

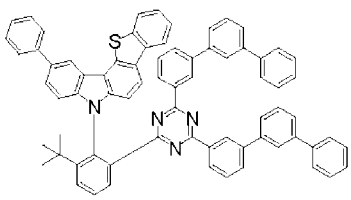

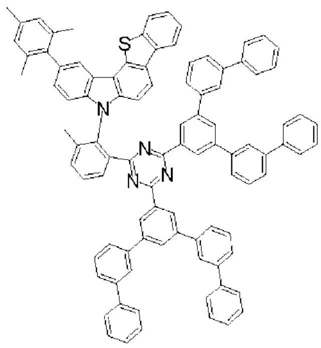

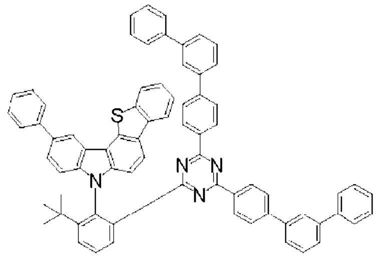

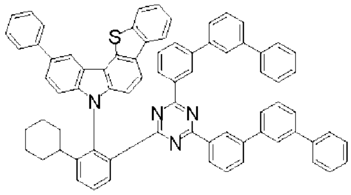

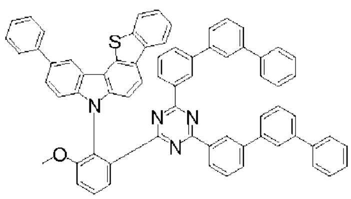

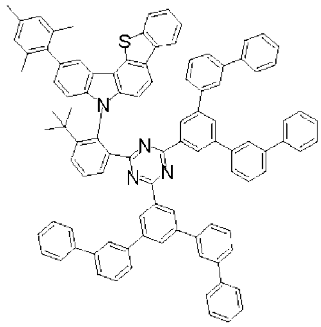

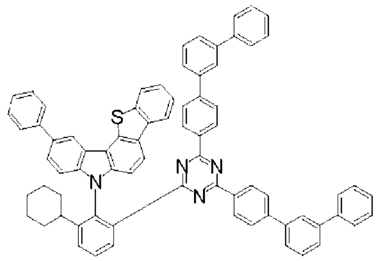

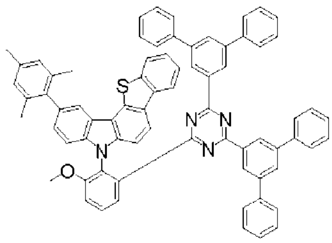

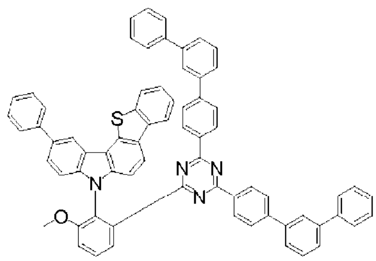

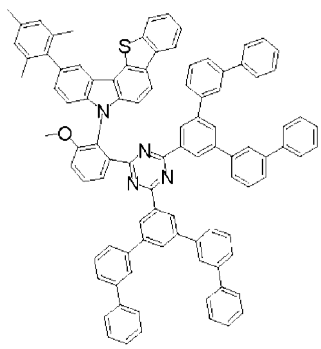

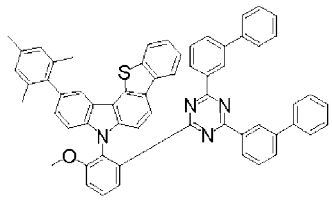

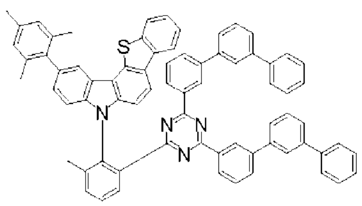

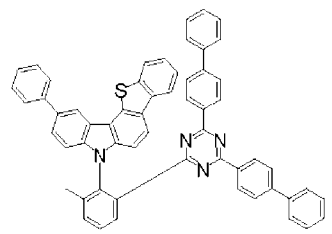

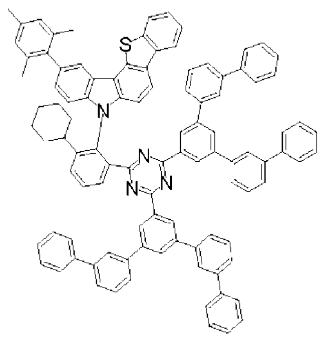

[Chem. 100]

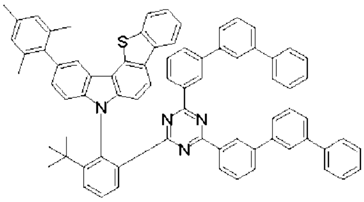
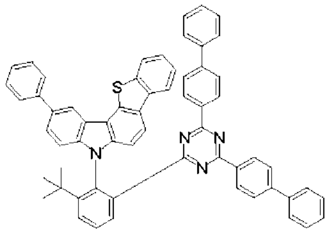
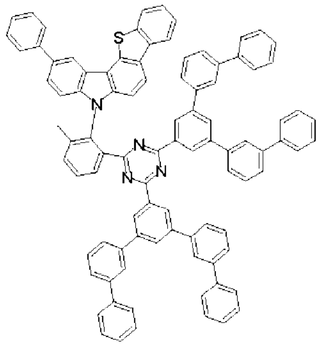
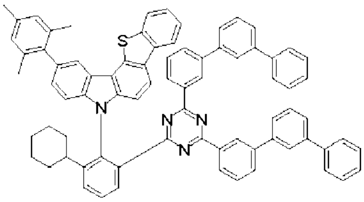
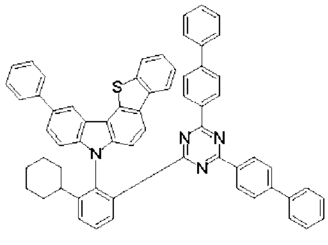
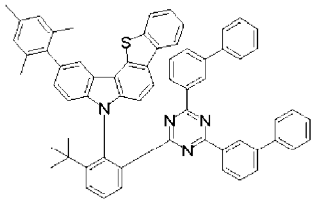
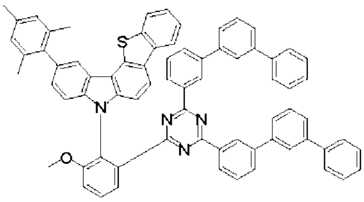
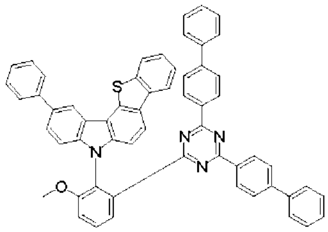
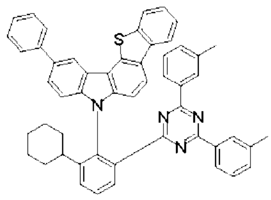
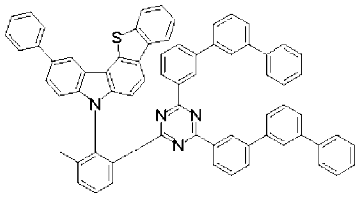
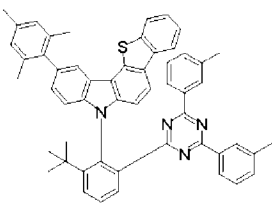
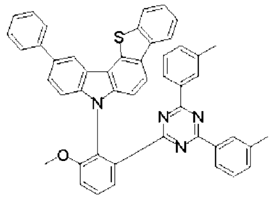
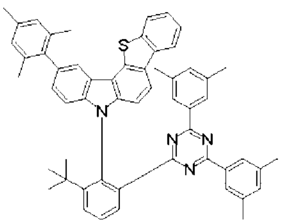
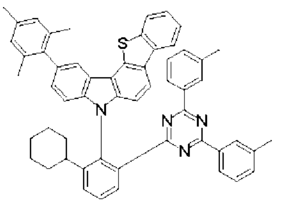
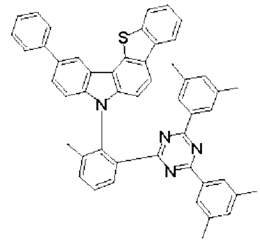
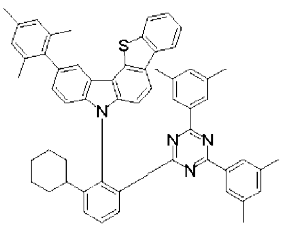
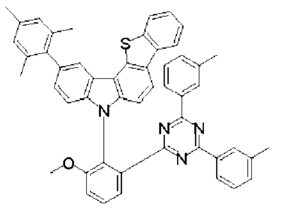
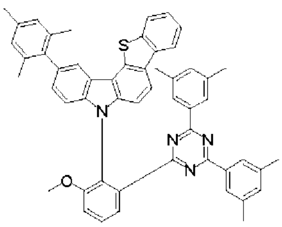

[Chem. 101]

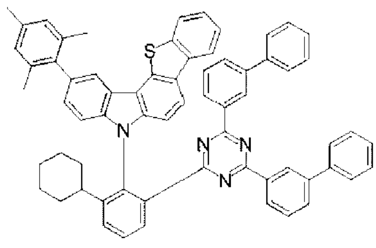

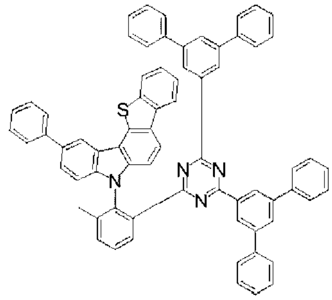

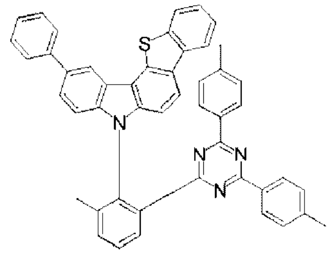

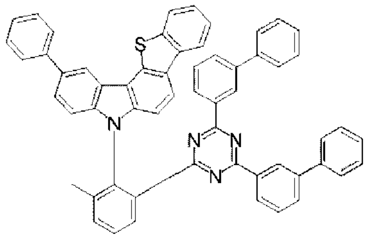

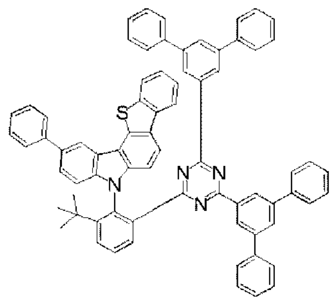

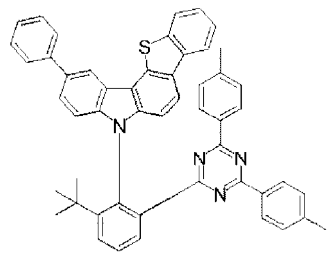

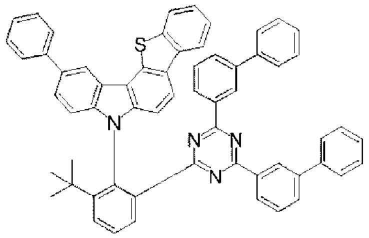

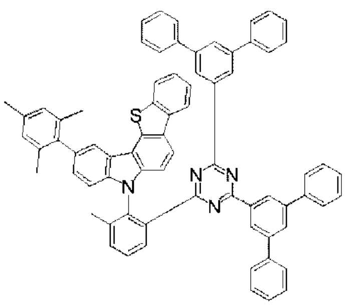

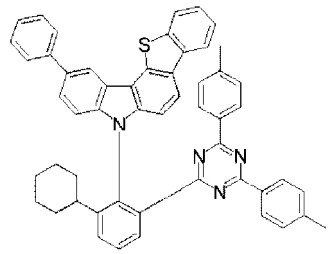

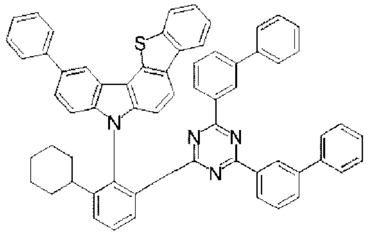

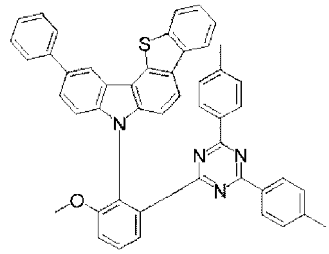

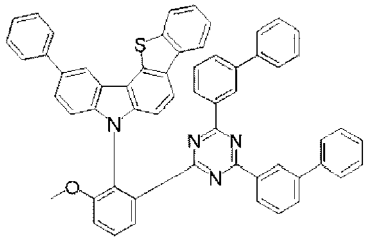

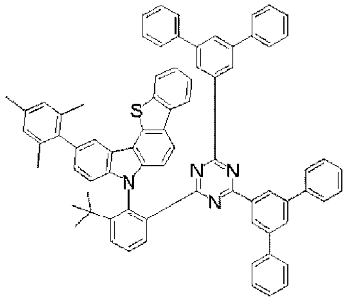

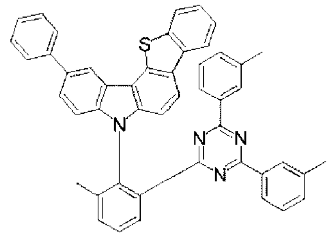

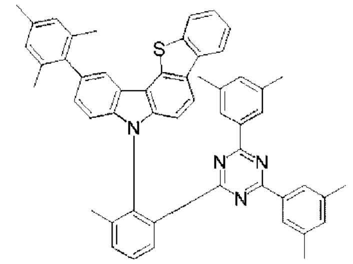

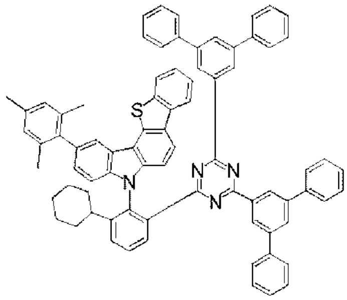

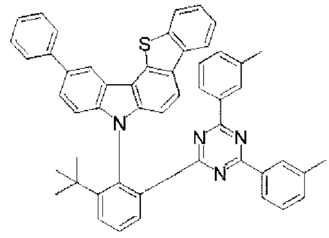

[Chem. 102]

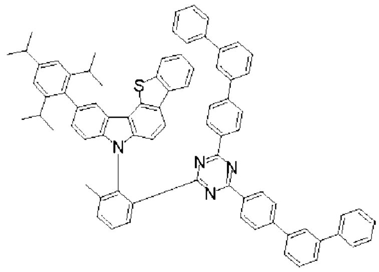
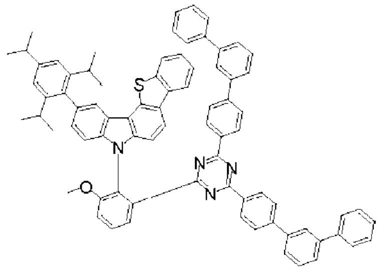
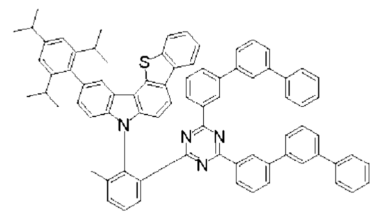
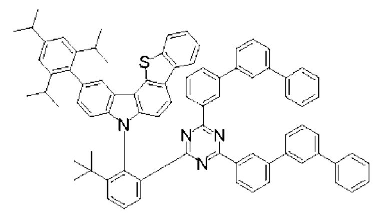
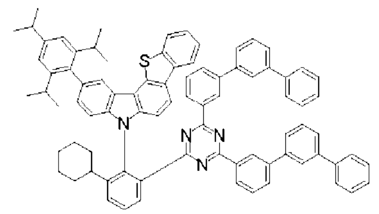
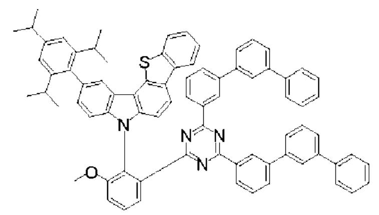
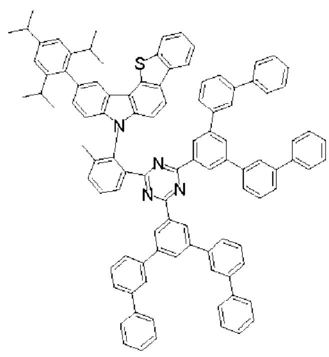
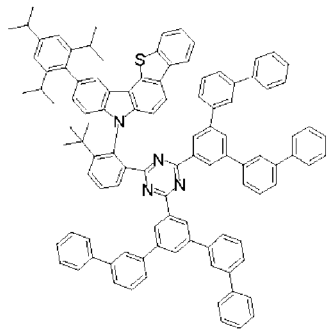
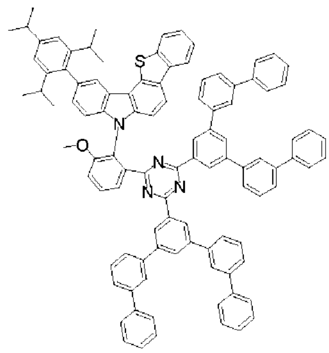
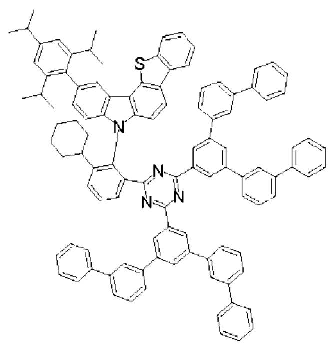
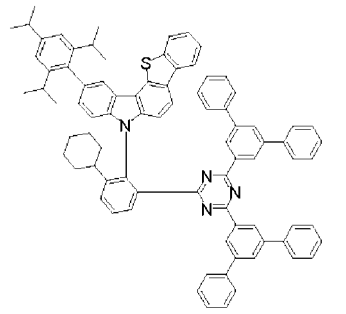
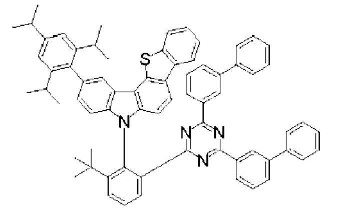
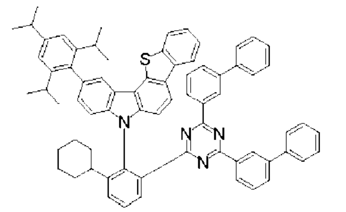
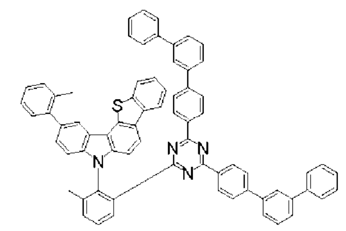
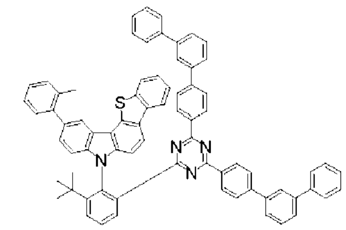

[Chem. 103]

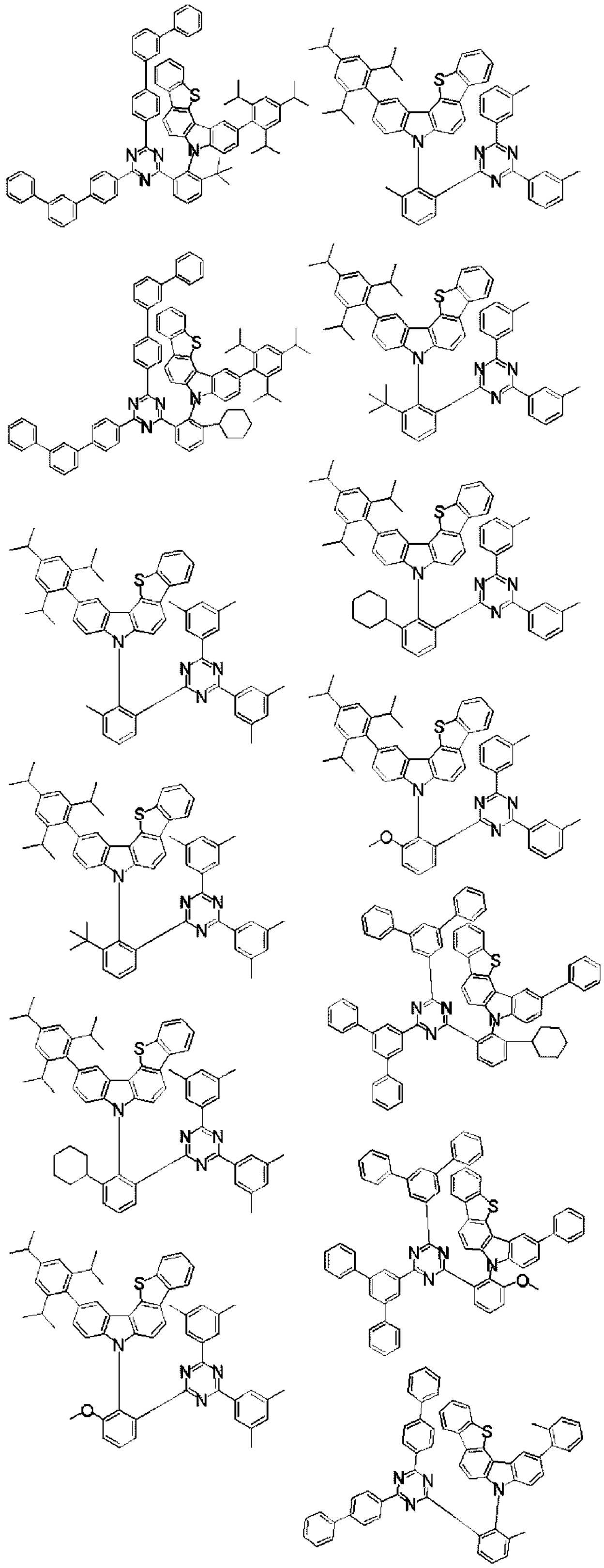

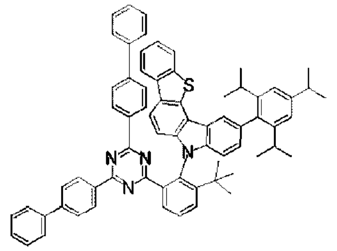

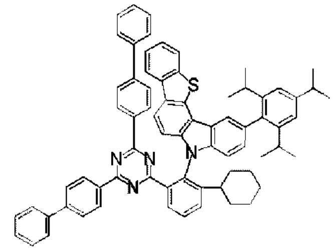

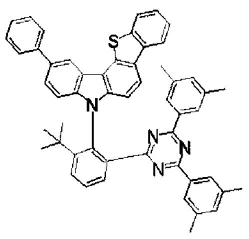

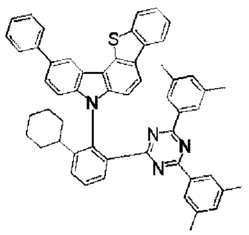

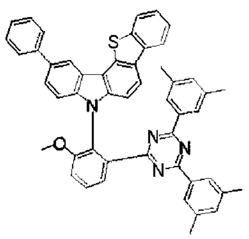

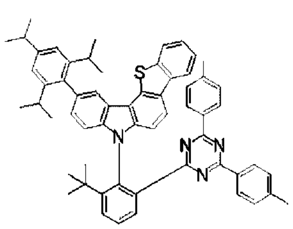

[Chem. 104]

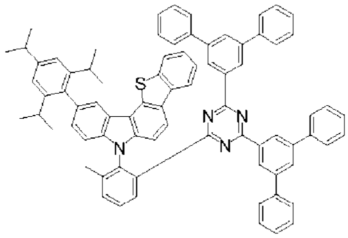
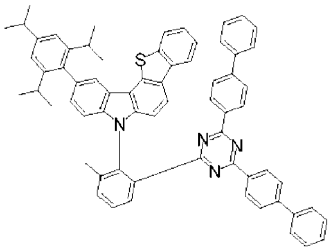
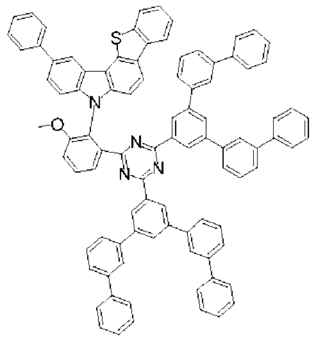
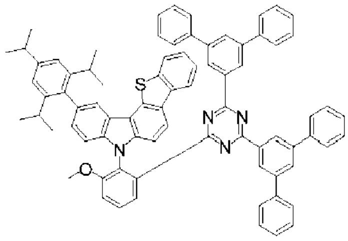
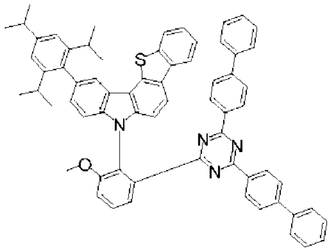
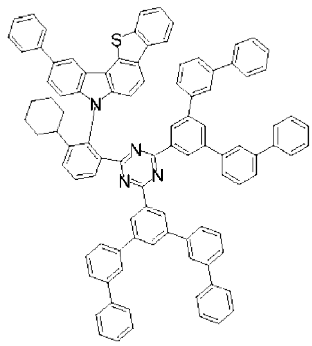
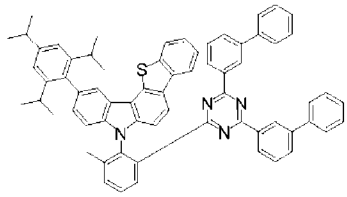
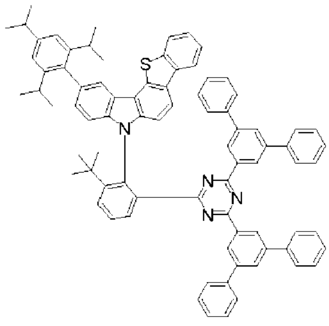
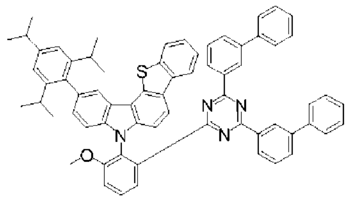
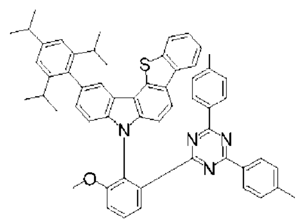
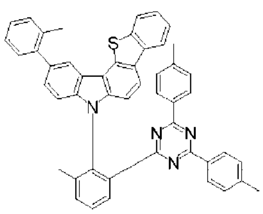
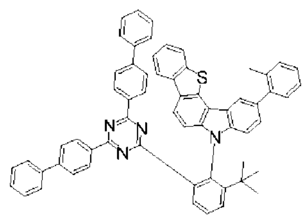
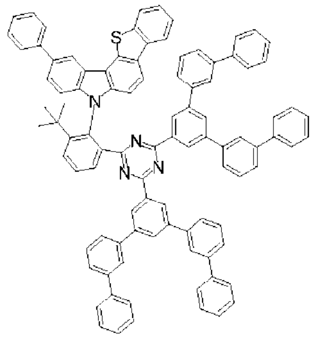
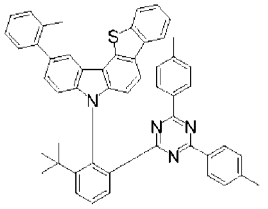
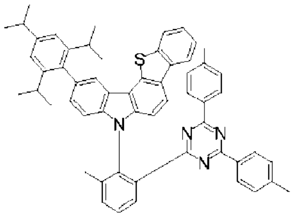
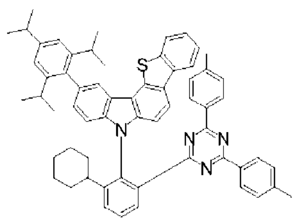

[Chem. 105]

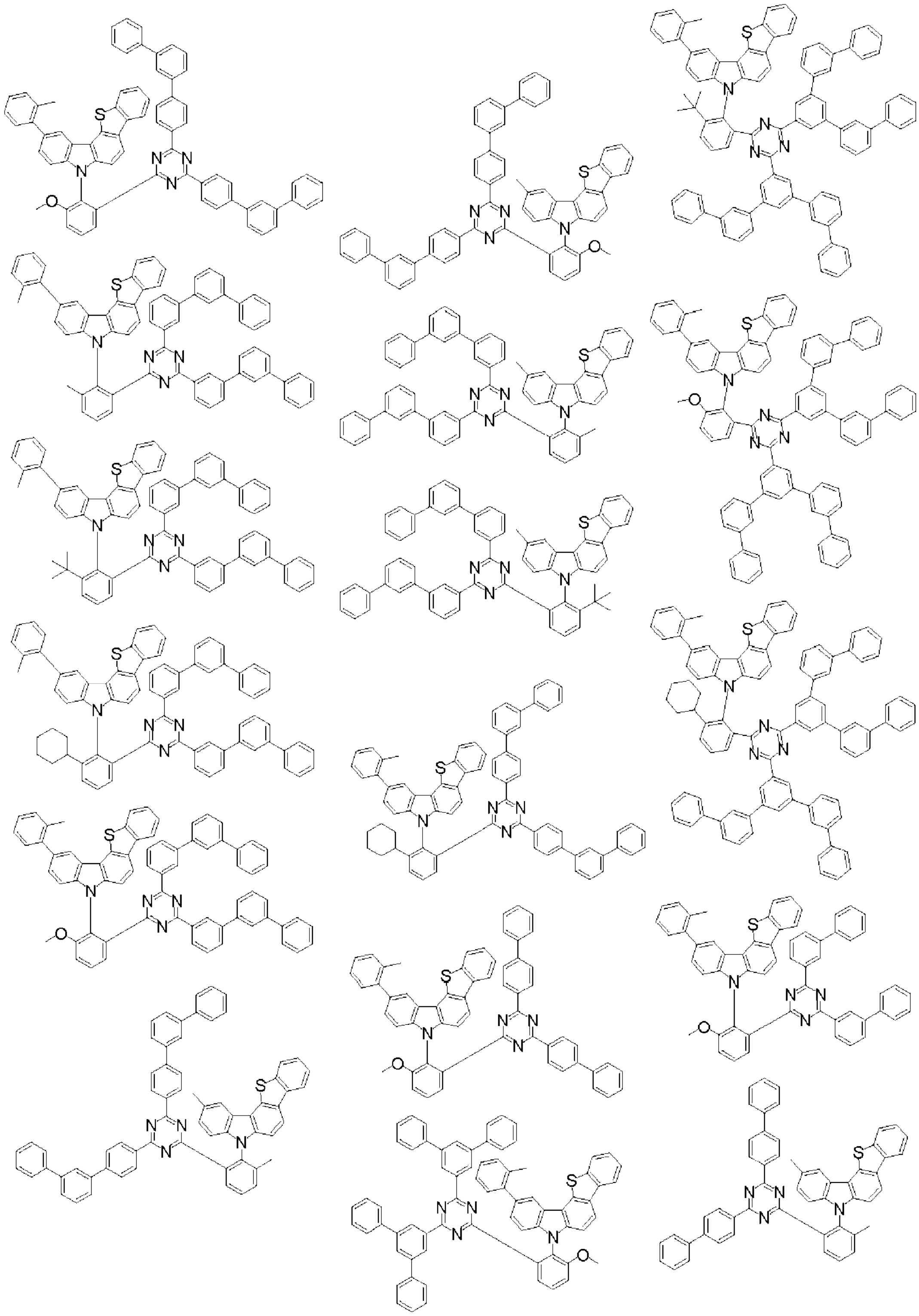

[Chem. 106]

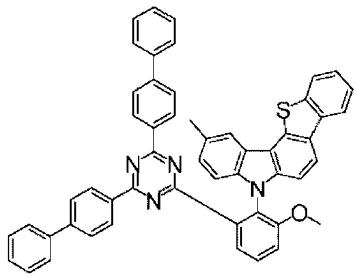

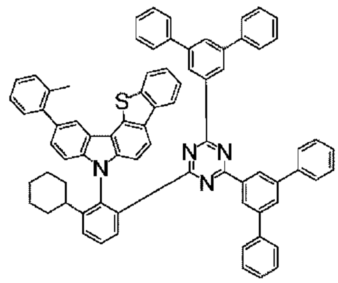

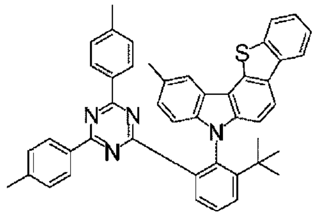

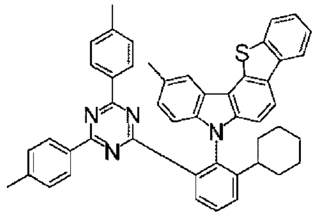

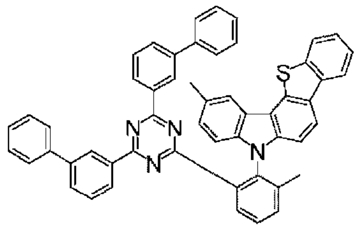

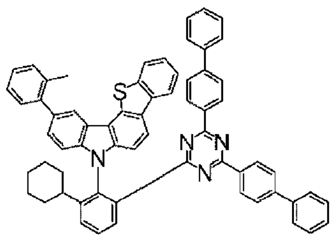

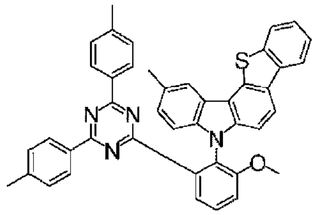

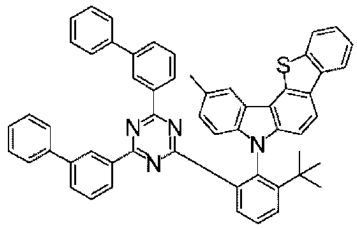

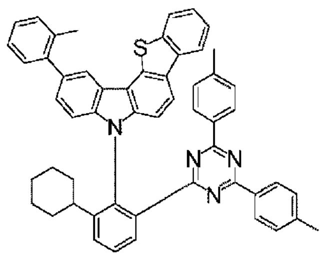

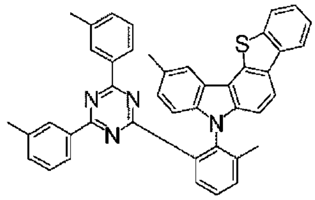

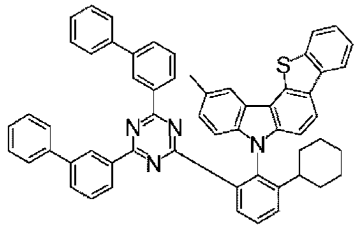

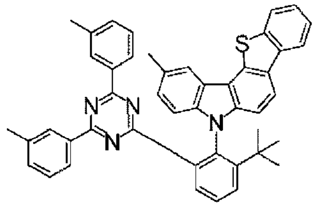

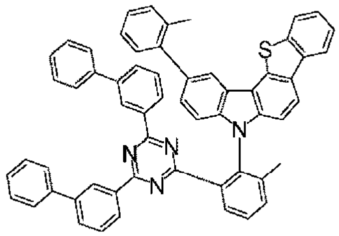

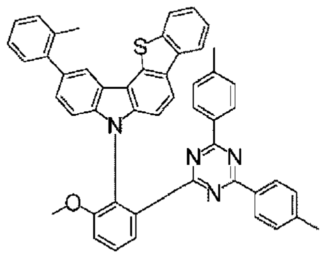

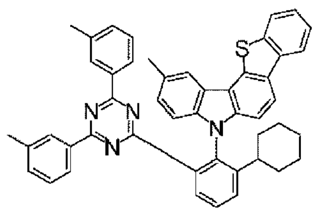

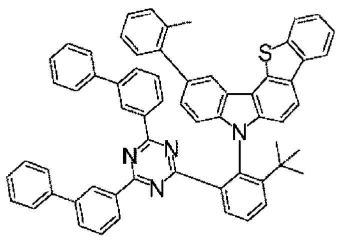

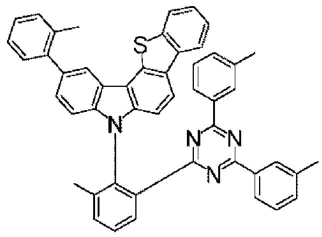

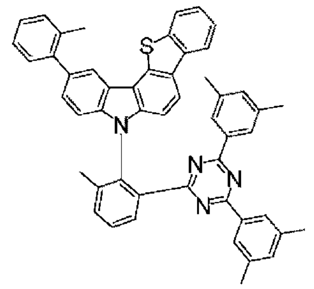

[Chem. 107]

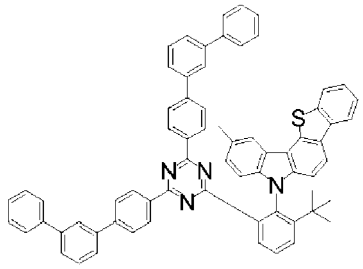
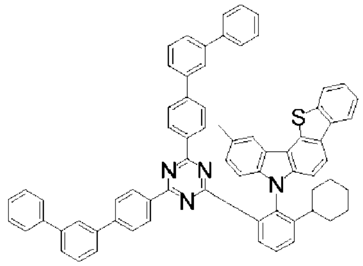
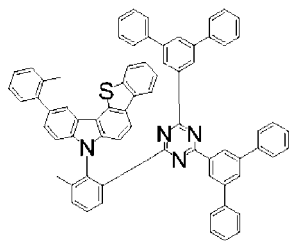
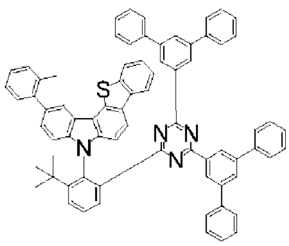
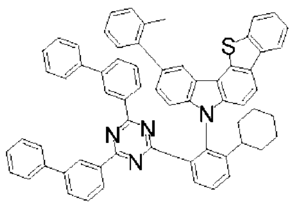
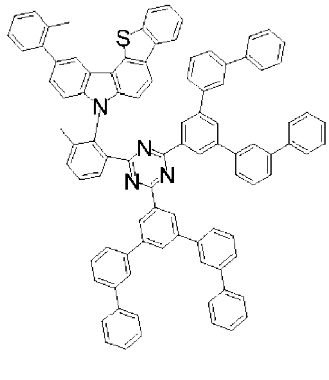
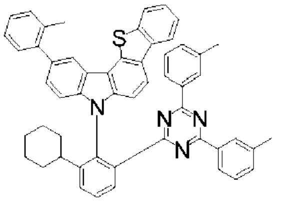
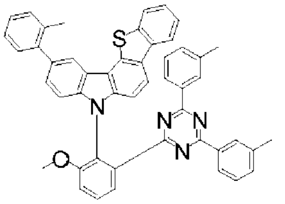
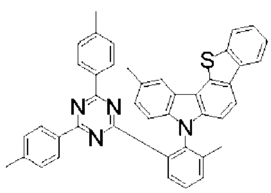
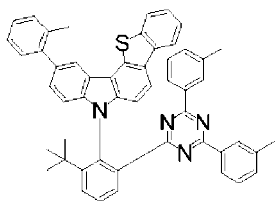
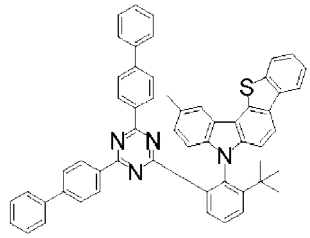
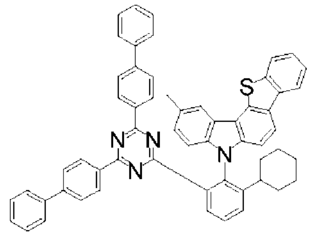
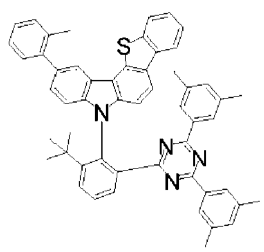
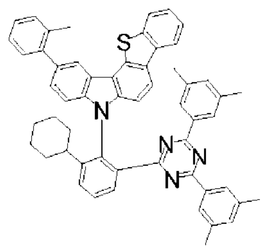
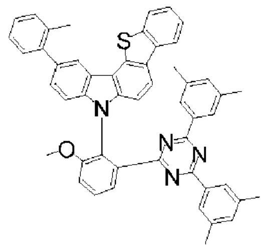

[Chem. 108]

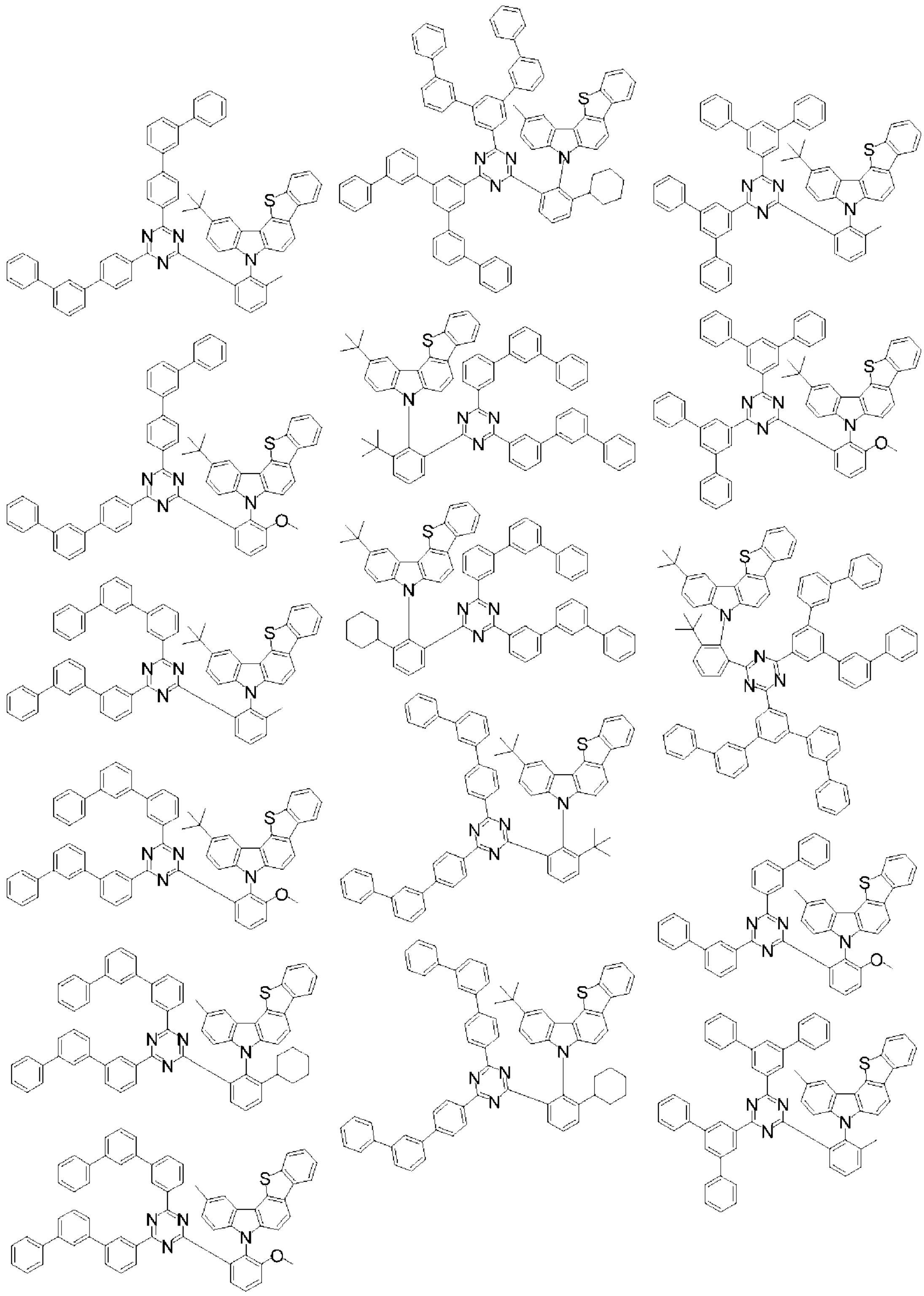

[Chem. 109]

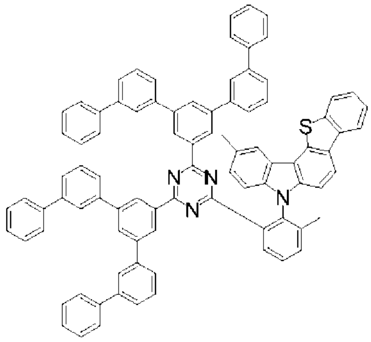
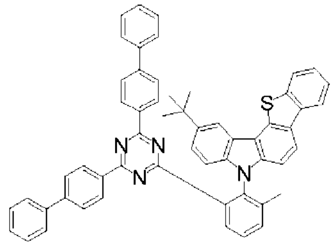
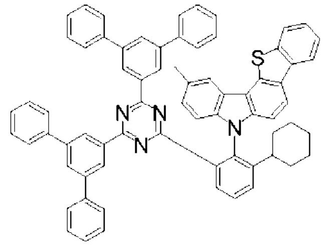
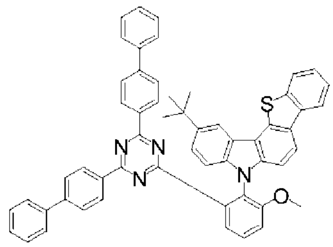
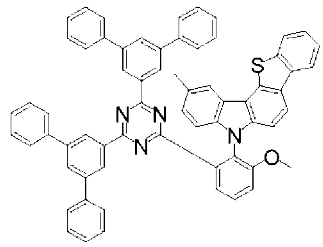
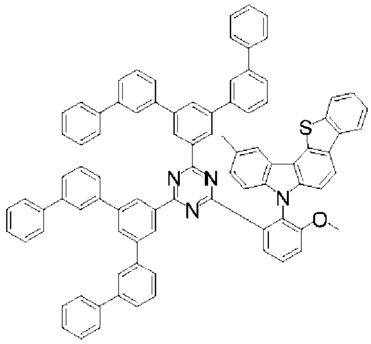
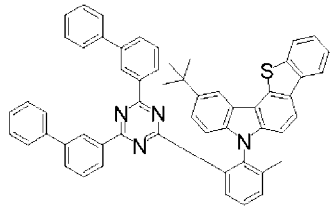
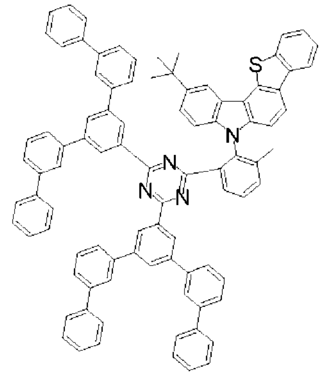
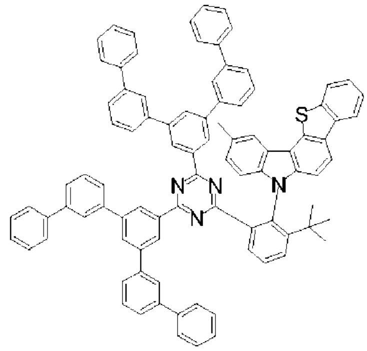
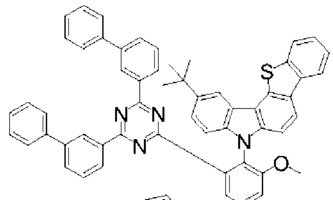
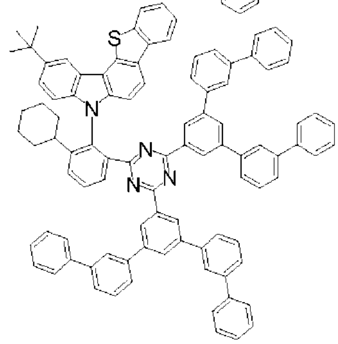
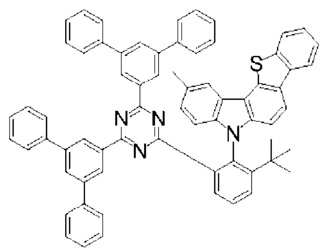
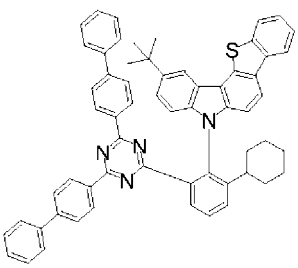
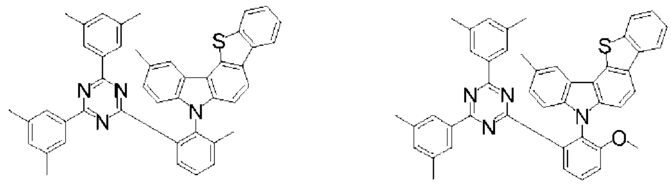

[Chem. 110]

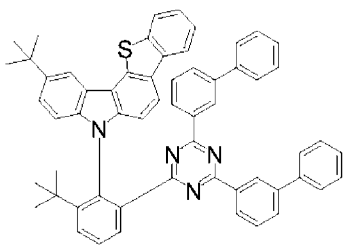
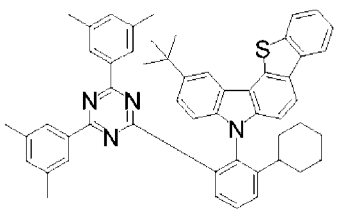
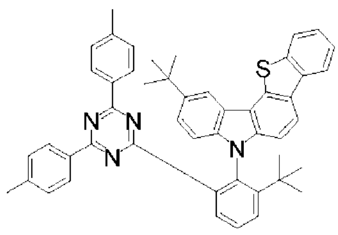
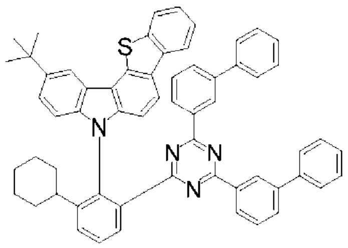
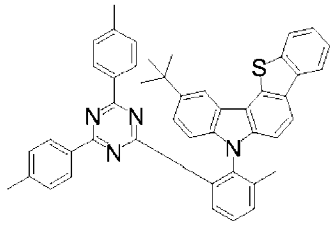
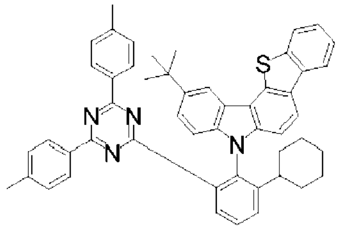
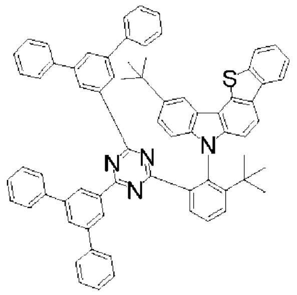
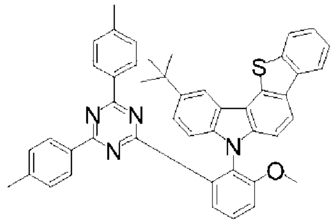
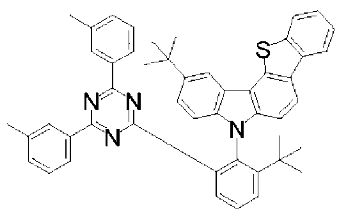
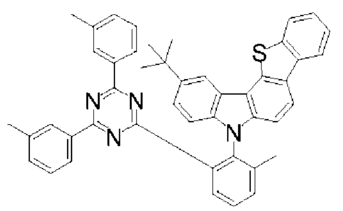
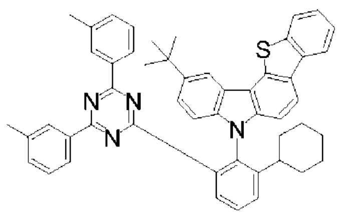
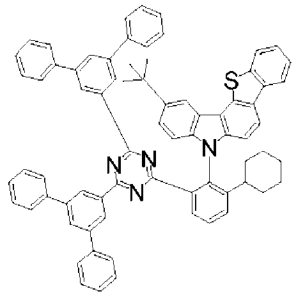
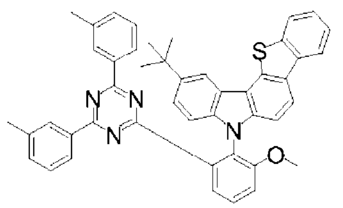
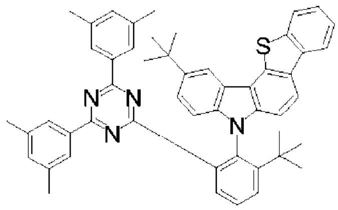
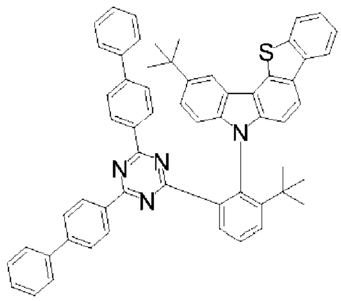
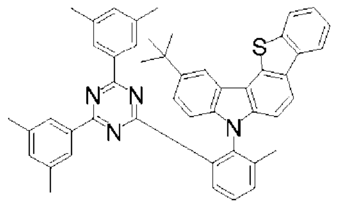
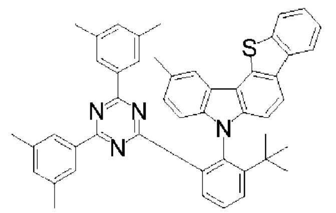
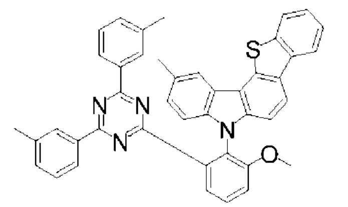
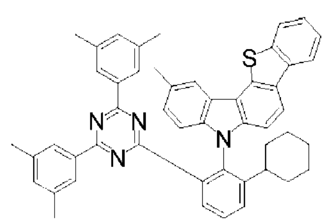

[Chem. 111]

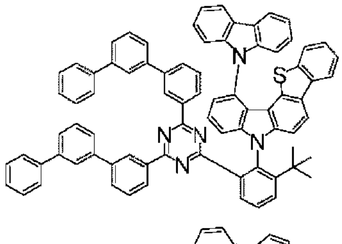

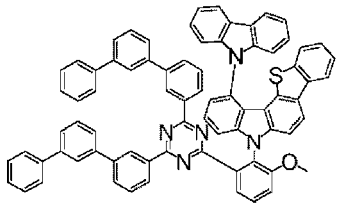

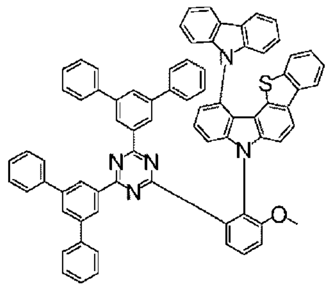

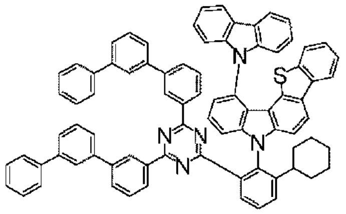

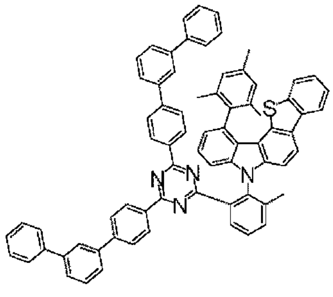

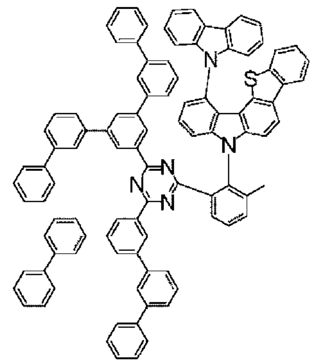

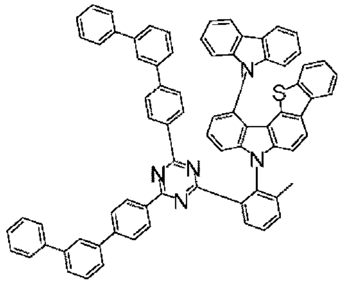

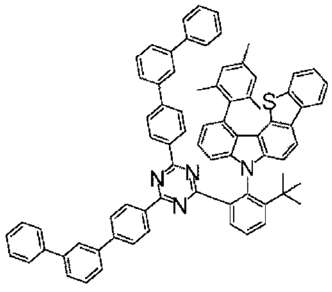

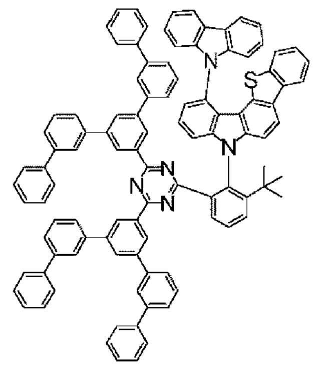

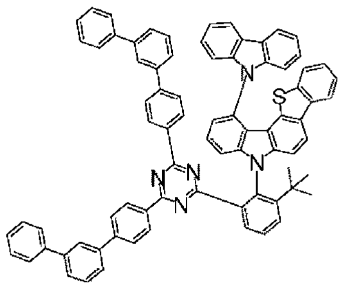

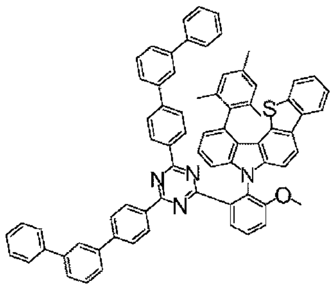

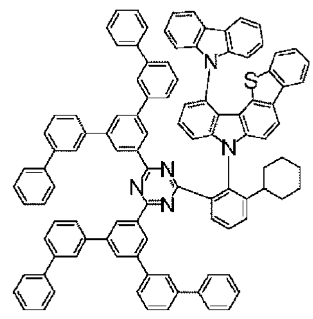

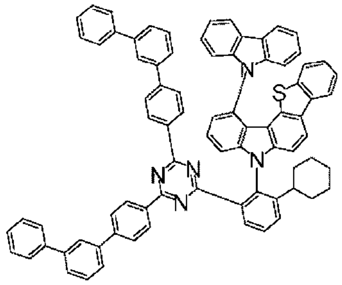

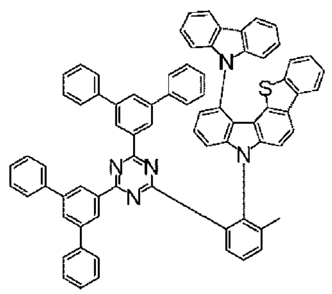

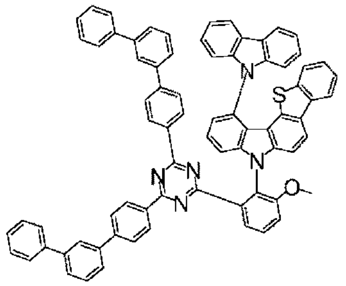

[Chem. 112]

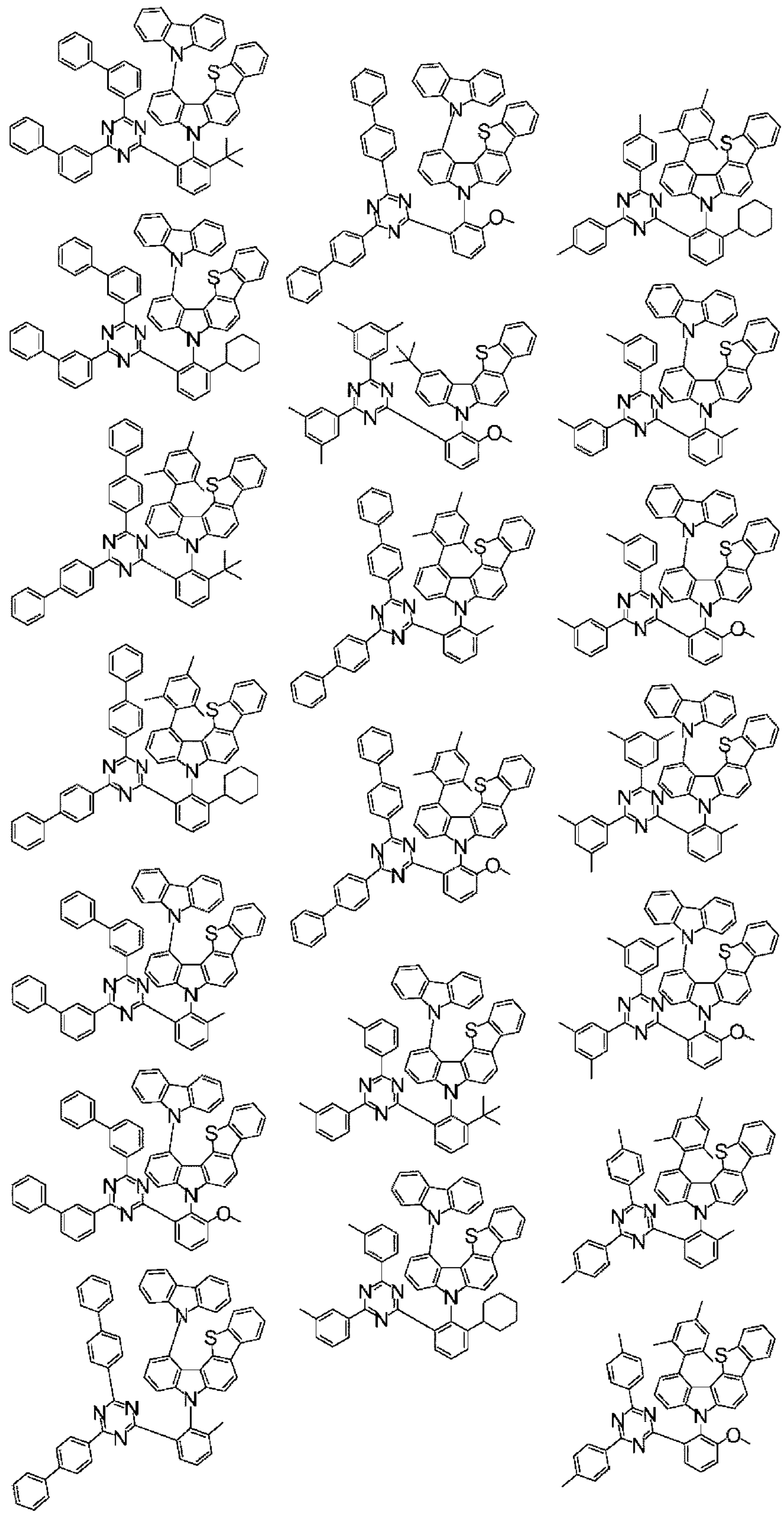

[Chem. 113]

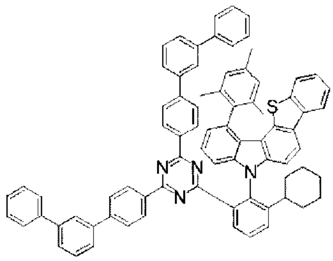
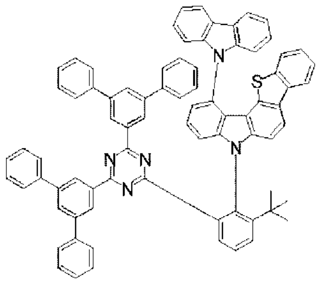
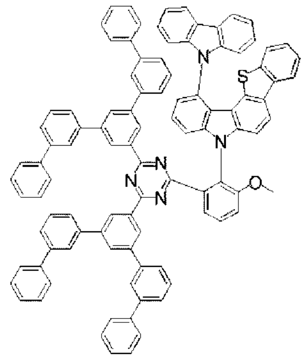
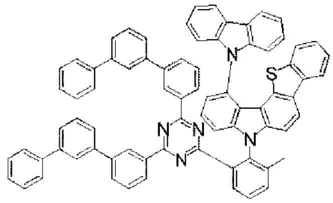
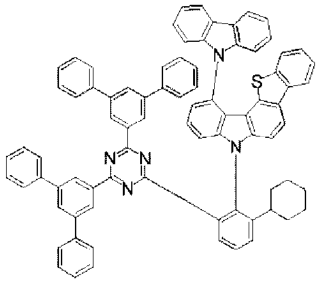
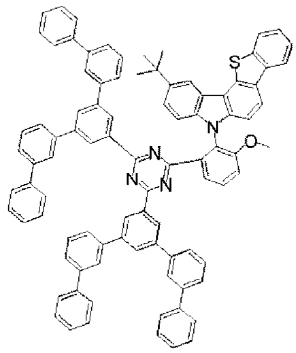
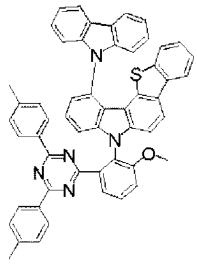
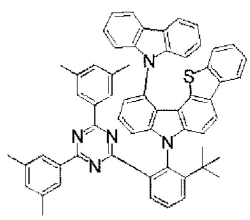
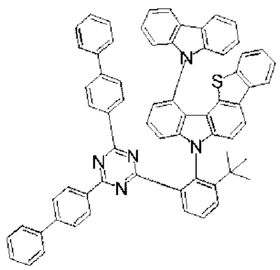
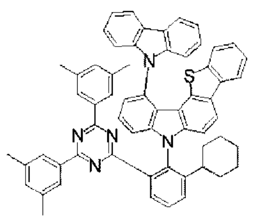
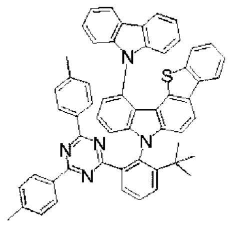
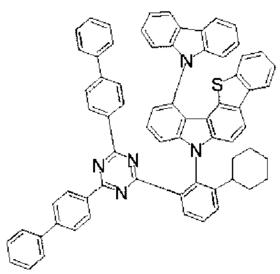
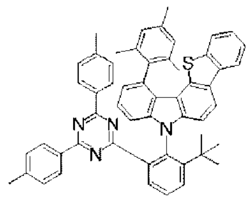
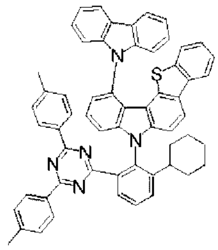
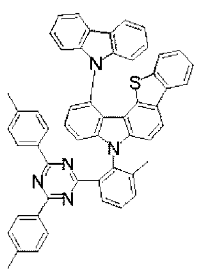

[Chem. 114]

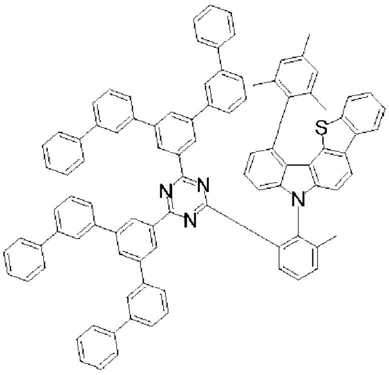
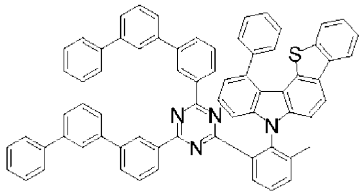
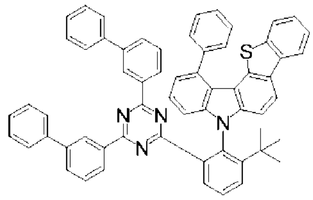
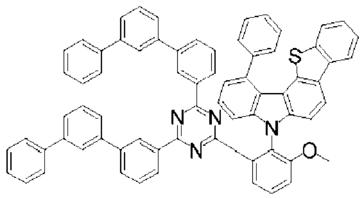
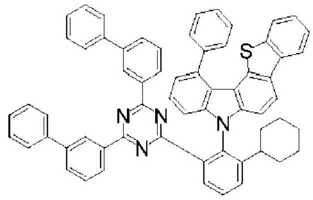
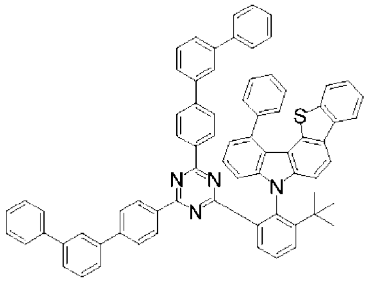
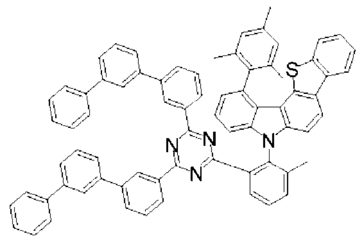
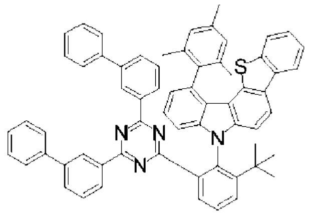
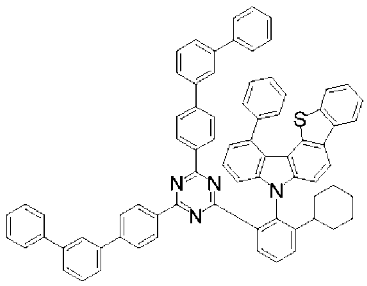
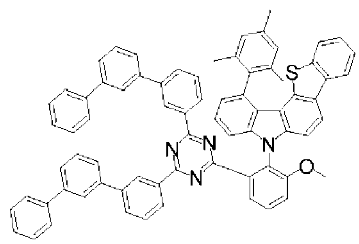
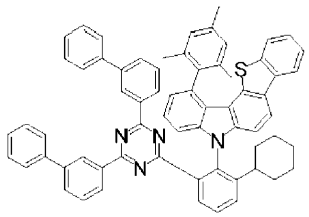
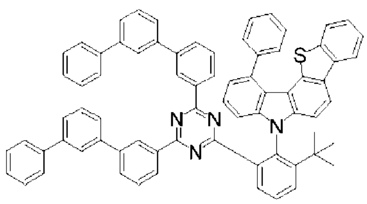
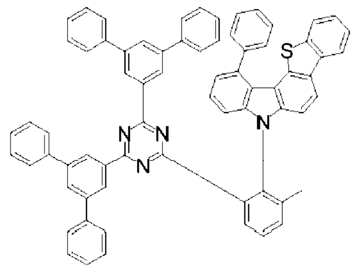
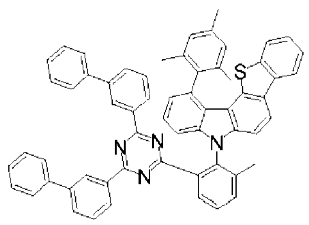
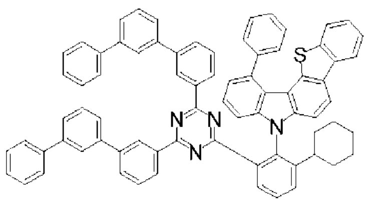
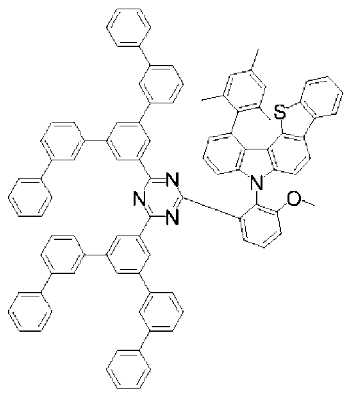
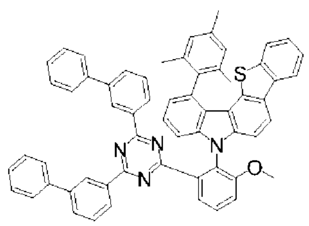
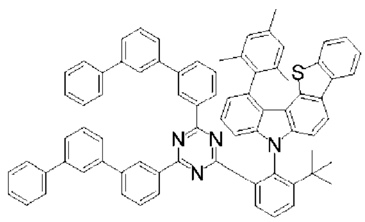

[Chem. 115]

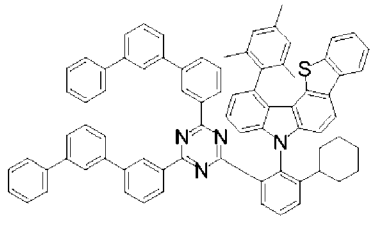
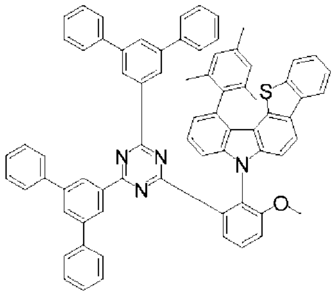
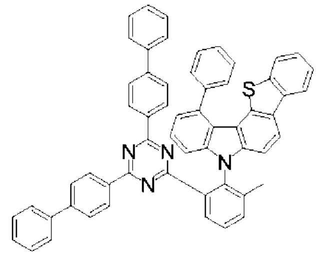
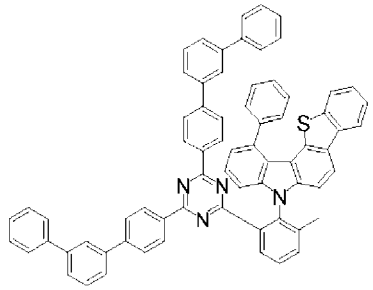
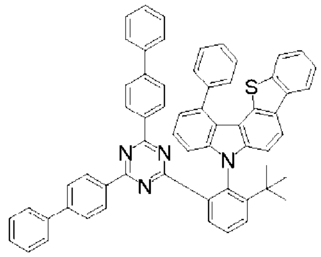
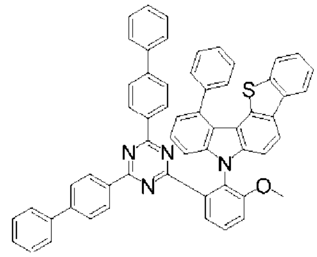
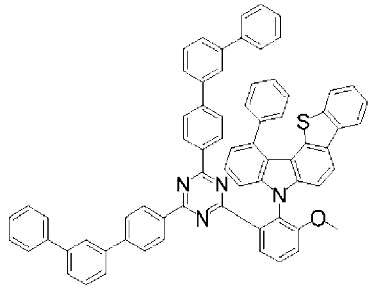
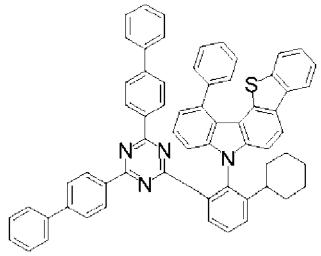
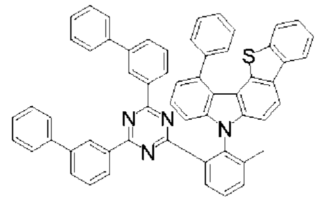
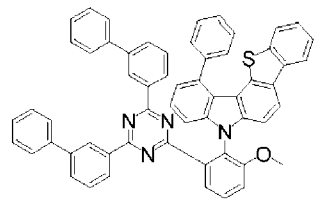
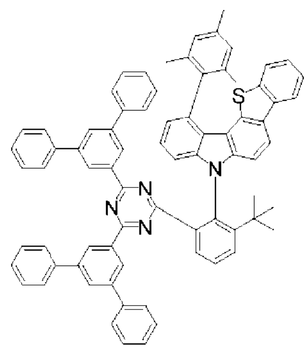
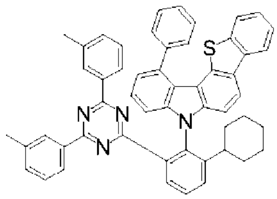
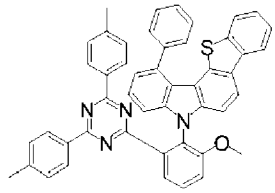
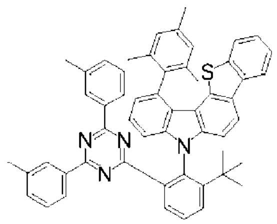
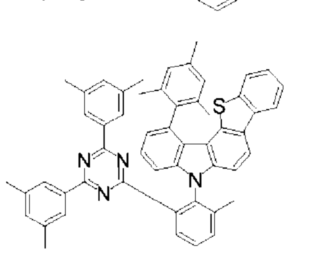
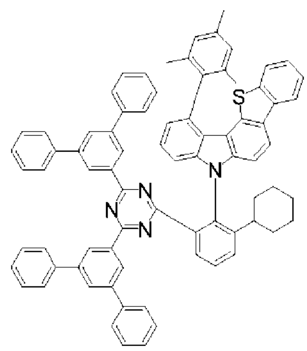
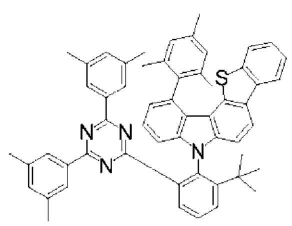

[Chem. 116]

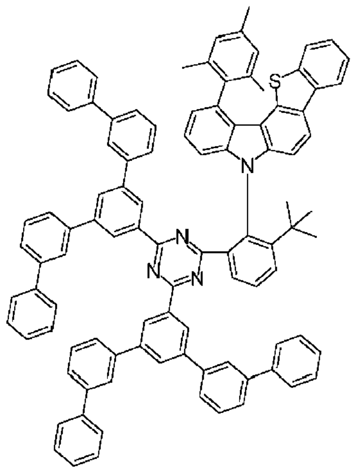
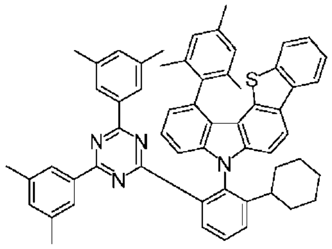
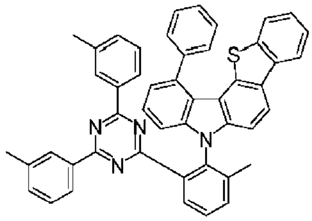
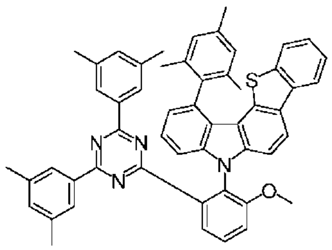
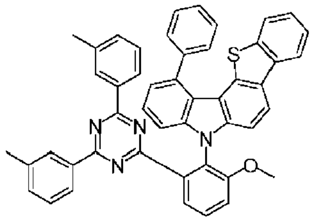
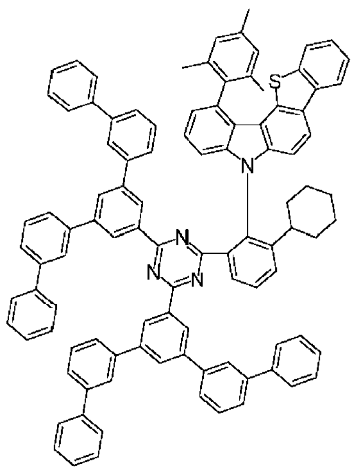
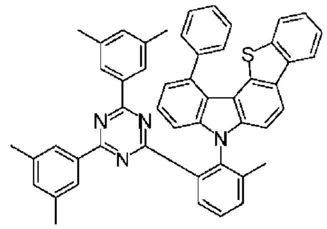
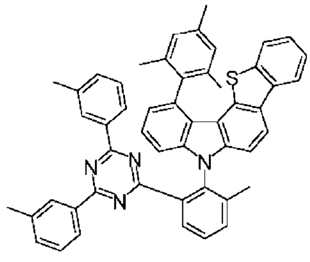
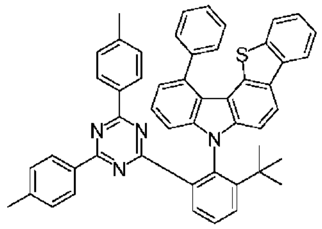
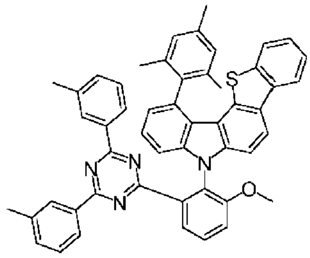
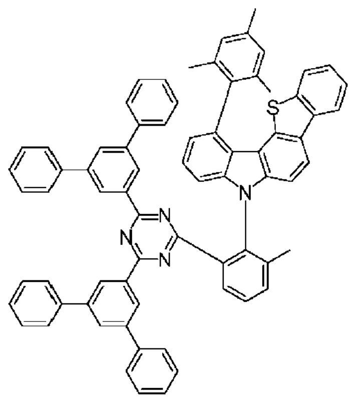
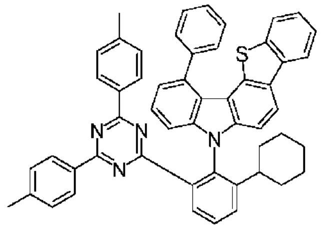
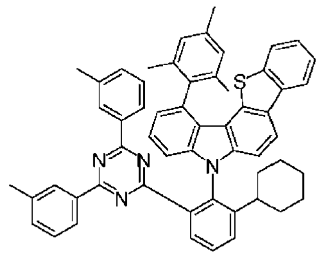
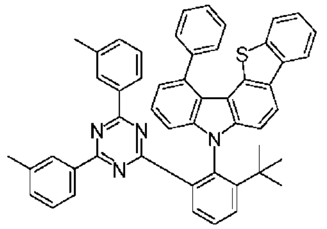
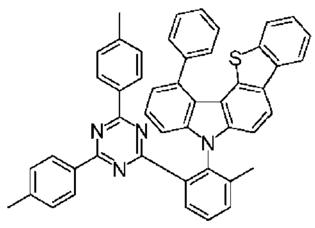

[Chem. 117]

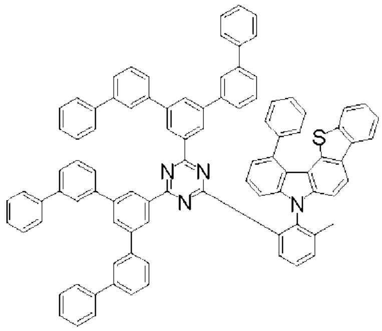
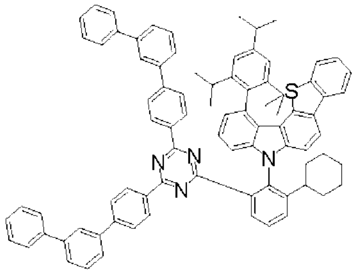
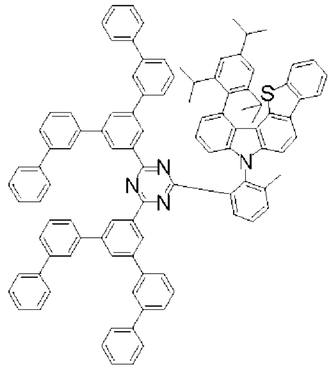
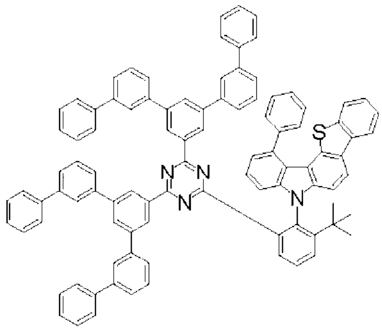
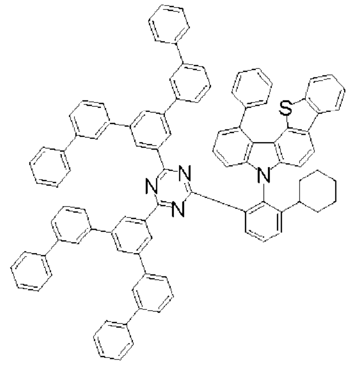
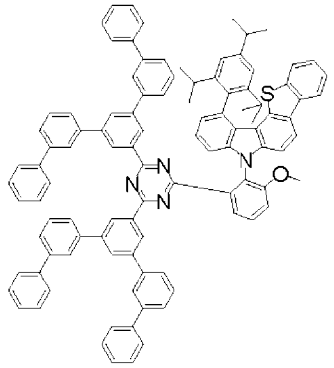
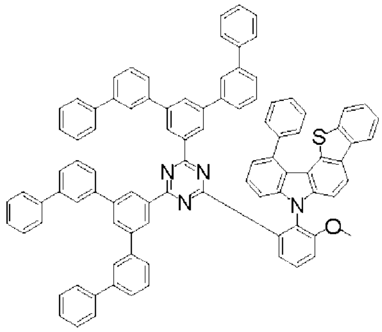
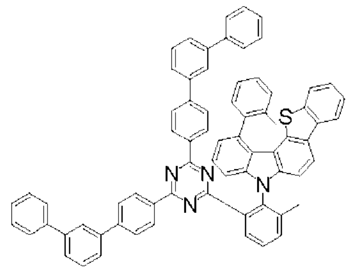
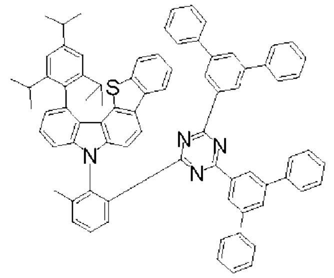
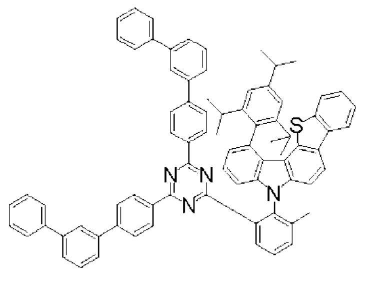
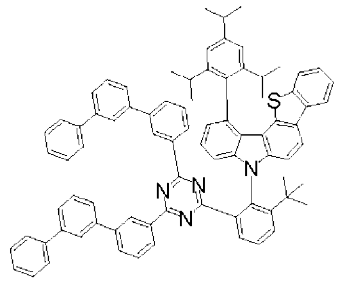
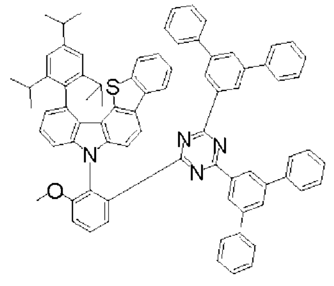
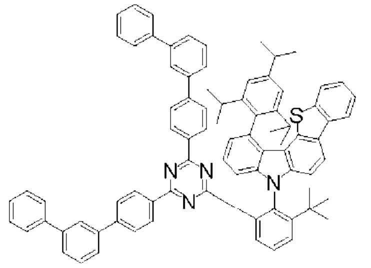
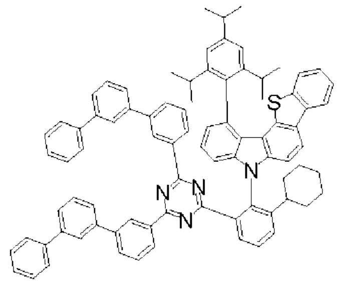
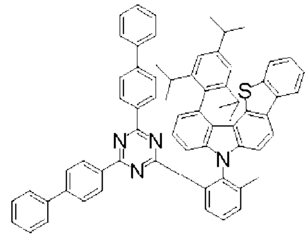

[Chem. 118]

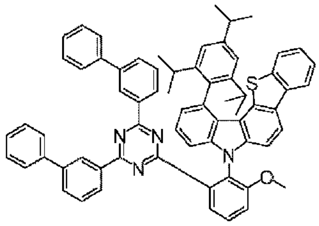

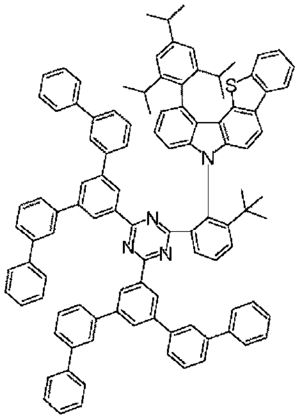

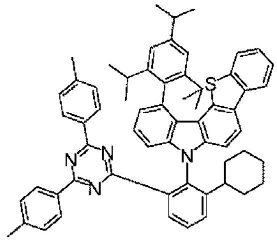

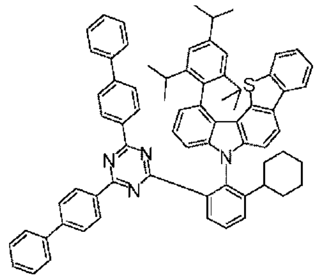

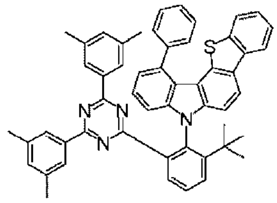

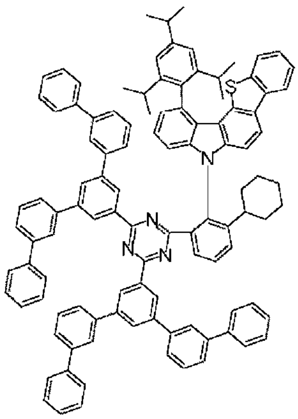

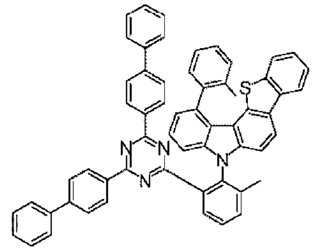

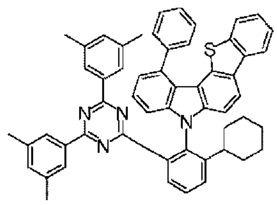

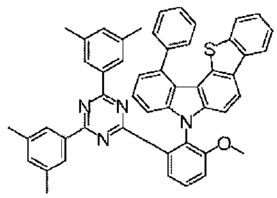

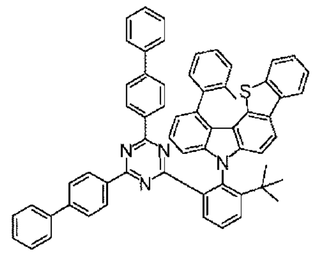

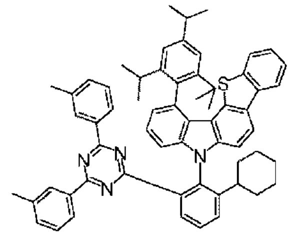

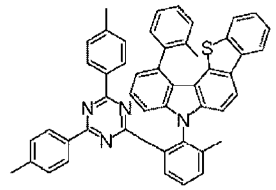

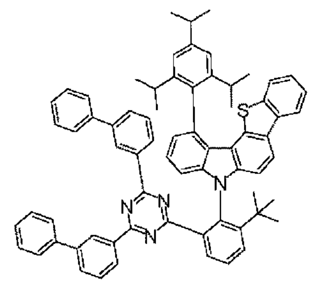

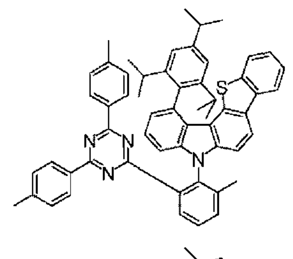

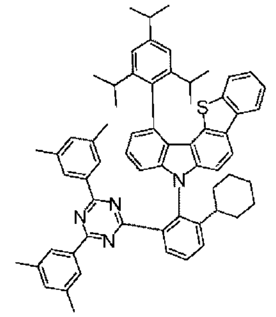

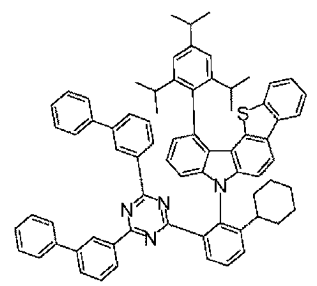

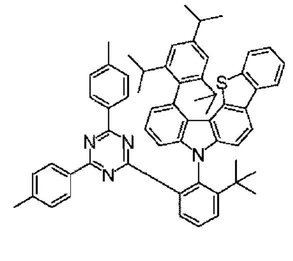

[Chem. 119]

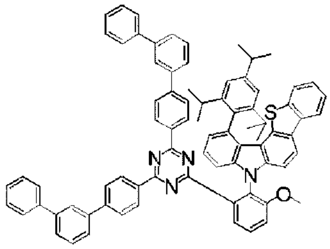
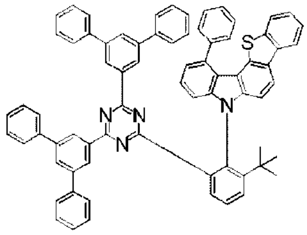
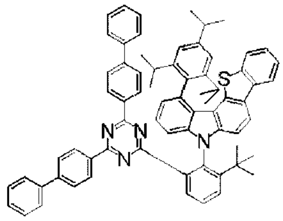
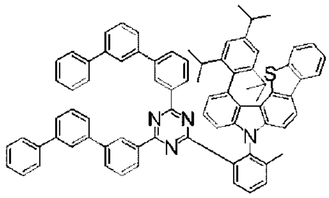
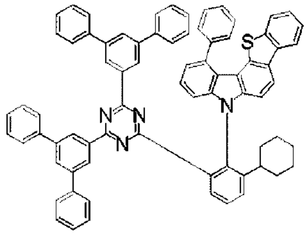
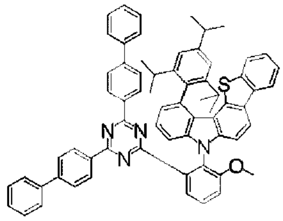
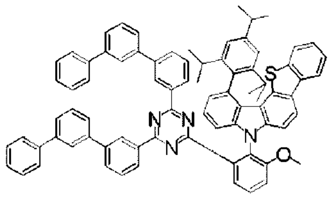
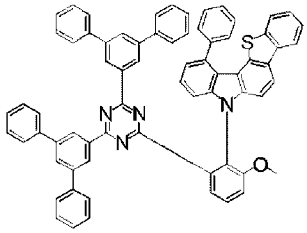
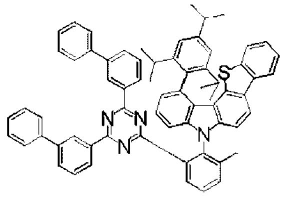
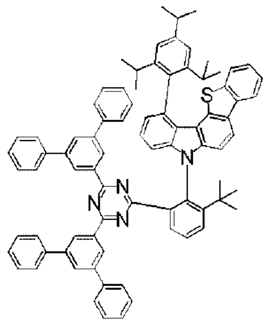
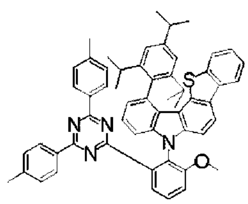
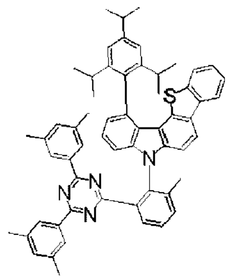
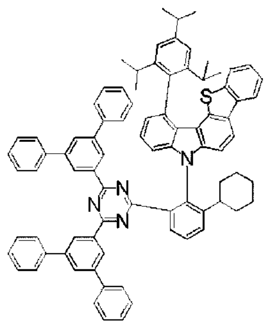
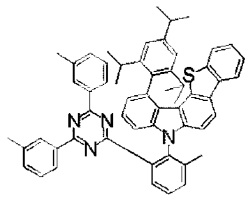
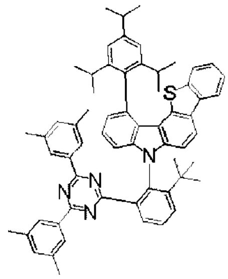
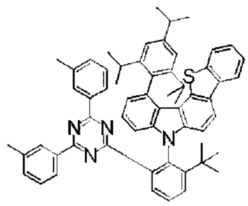
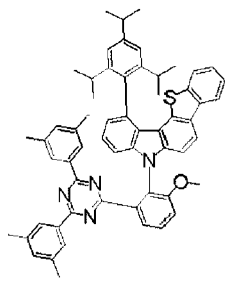
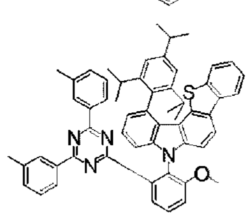

[Chem. 120]

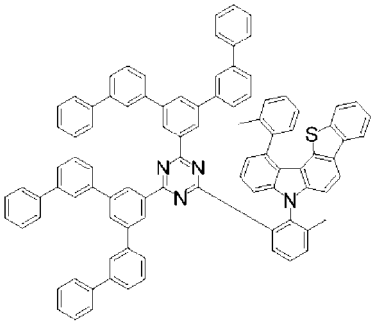
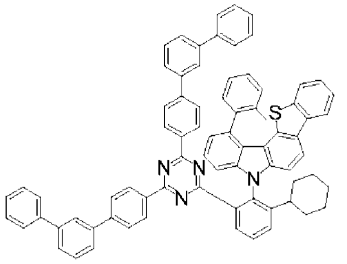
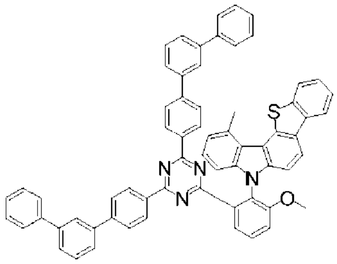
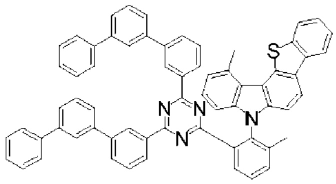
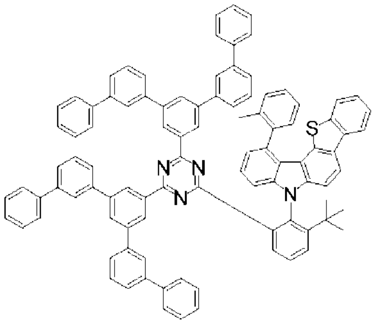
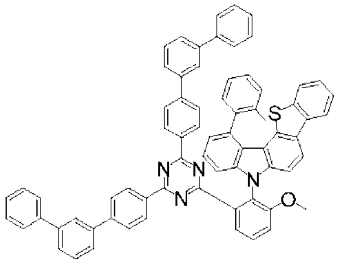
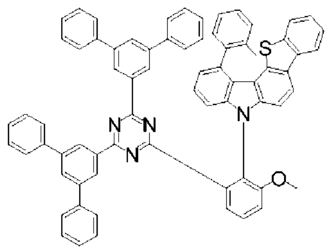
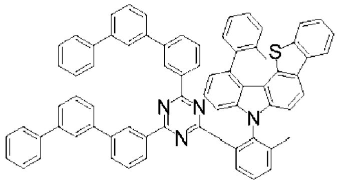
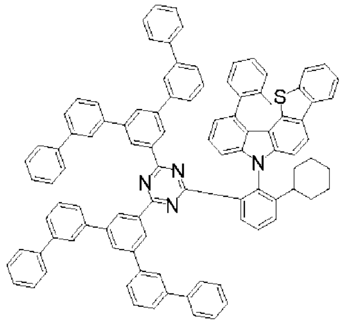
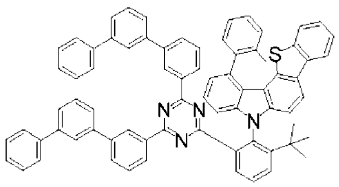
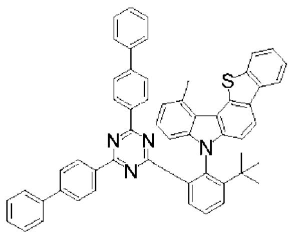
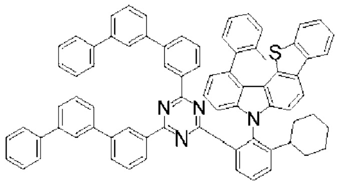
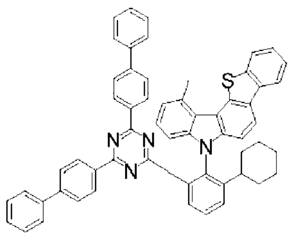
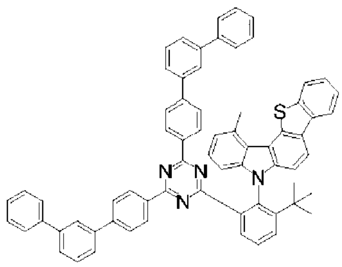
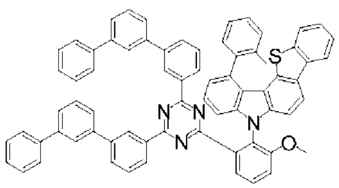
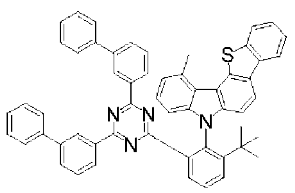
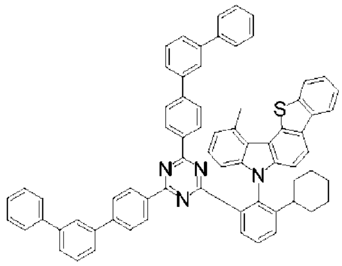

[Chem. 121]

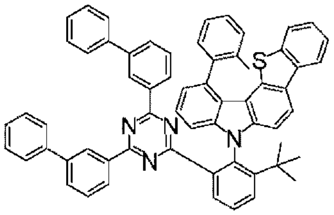
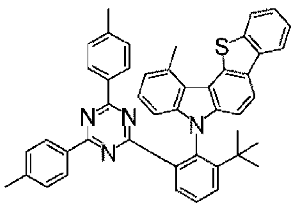
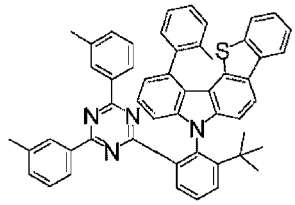
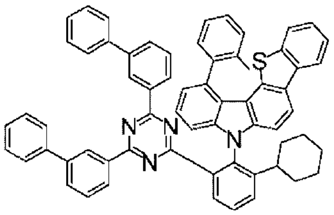
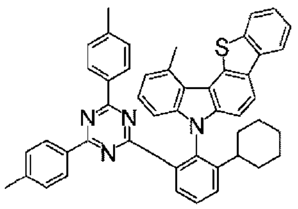
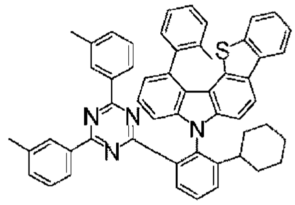
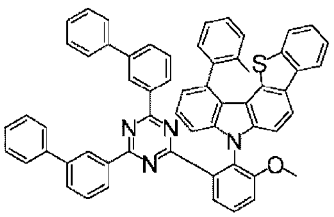
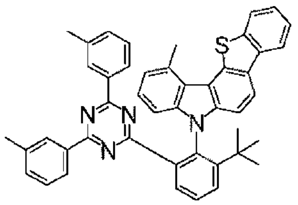
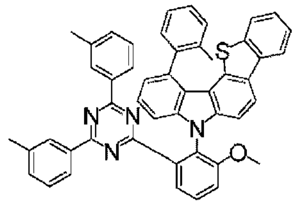
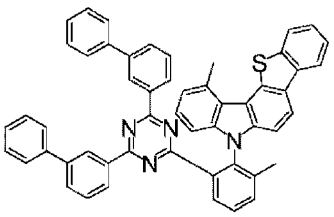
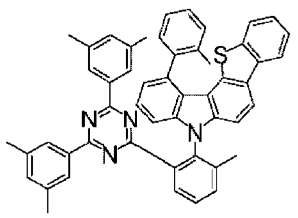
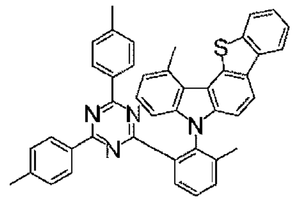
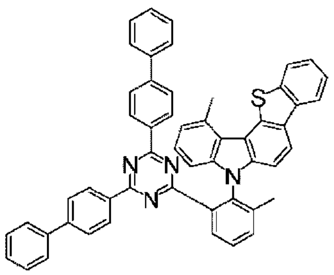
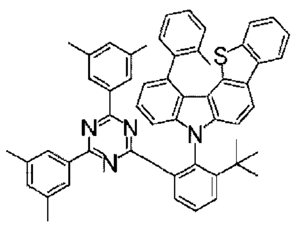
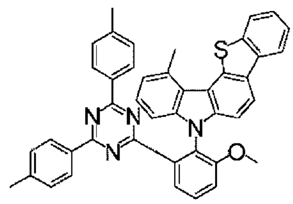
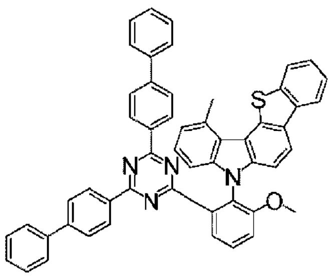
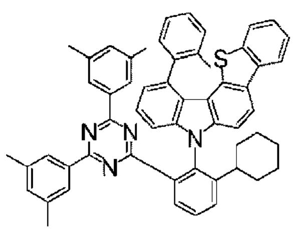
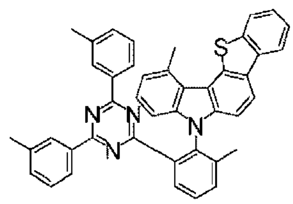
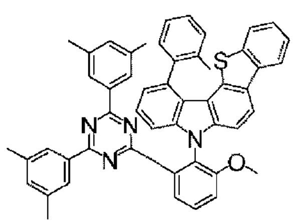
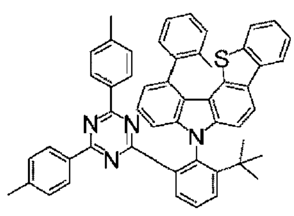

[Chem. 122]

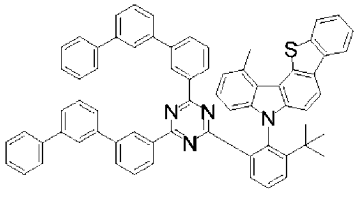

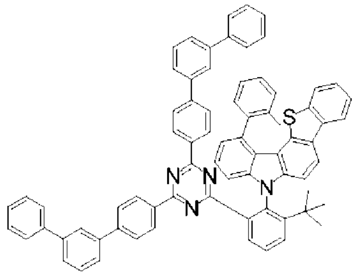

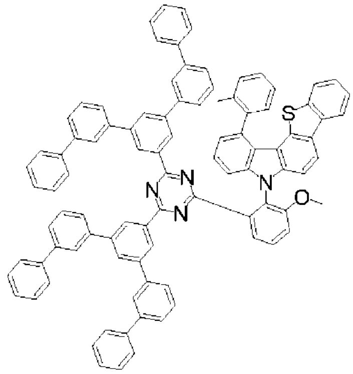

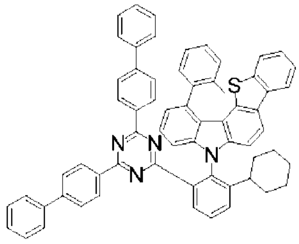

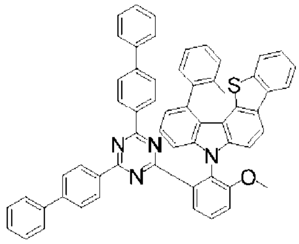

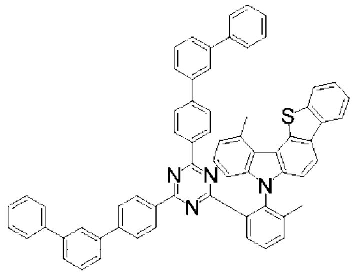

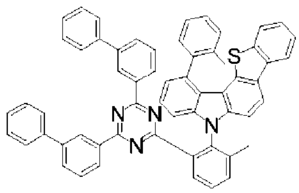

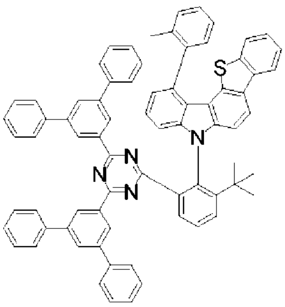

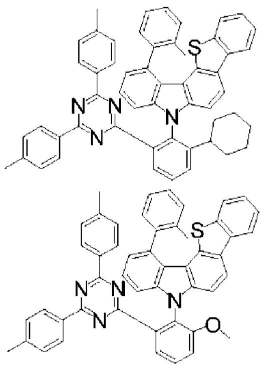

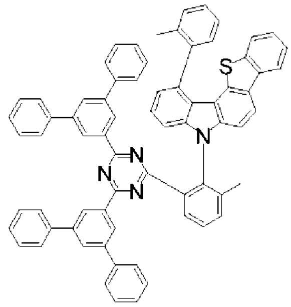

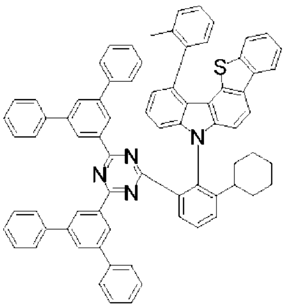

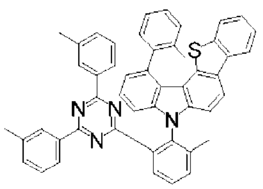

[Chem. 123]

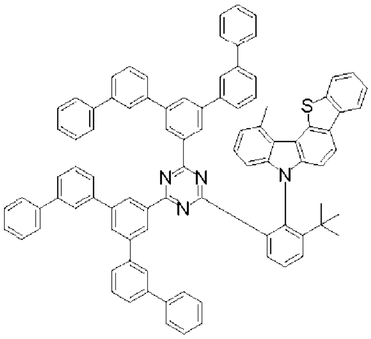
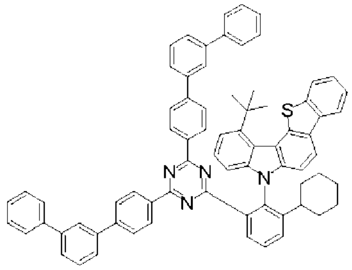
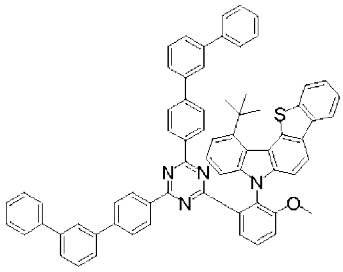
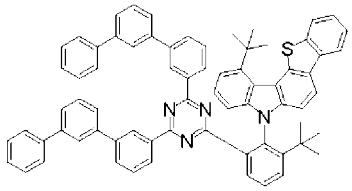
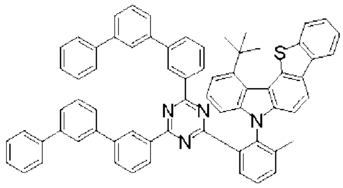
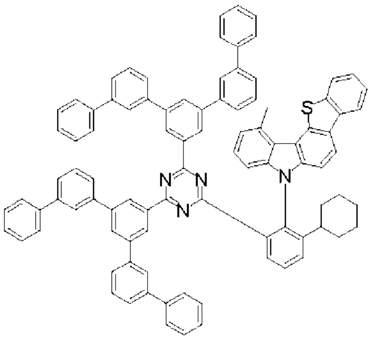
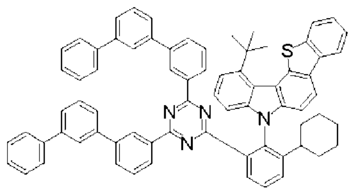
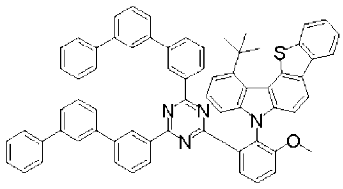
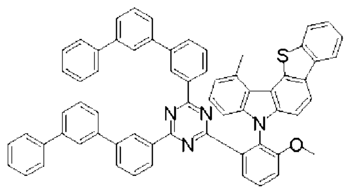
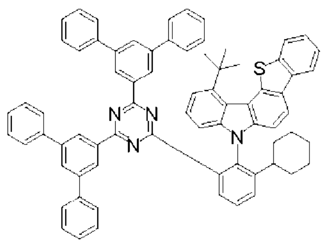
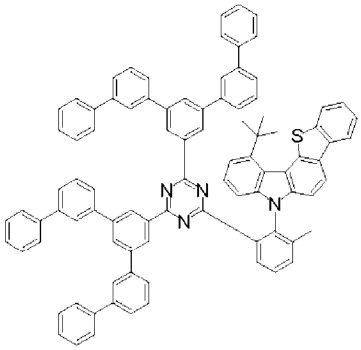
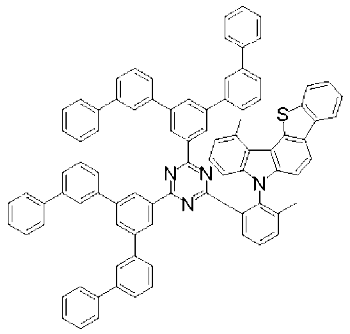
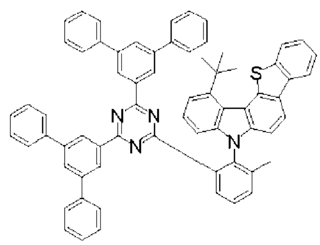
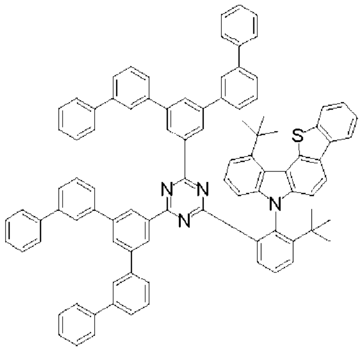
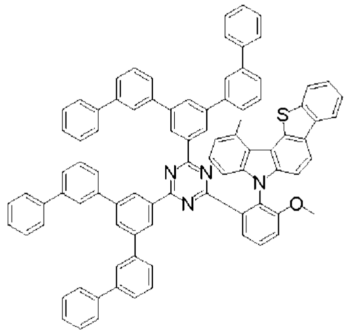
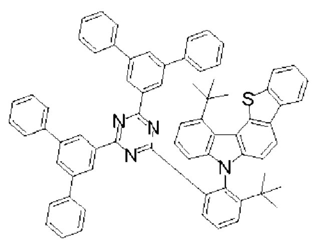
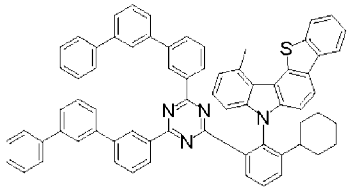
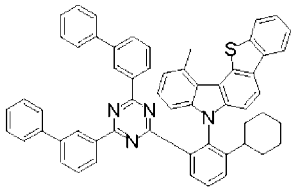

[Chem. 124]

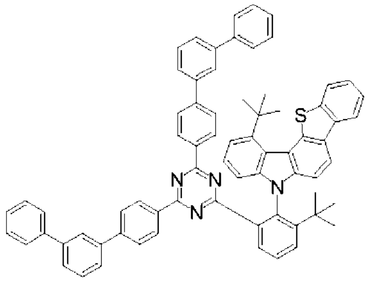
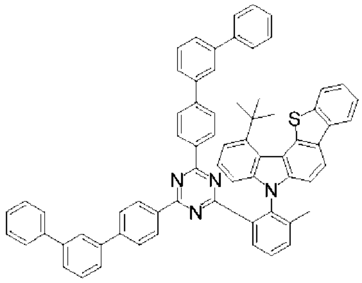
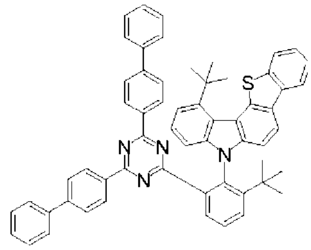
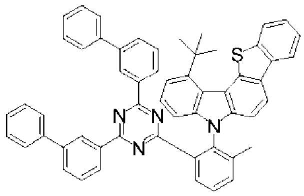
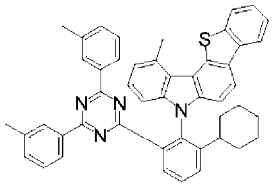
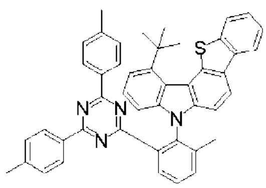
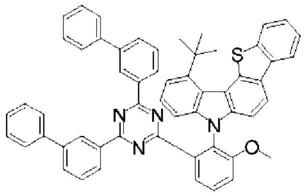
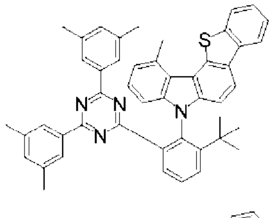
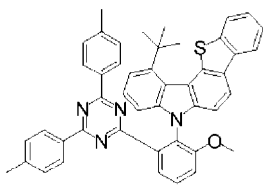
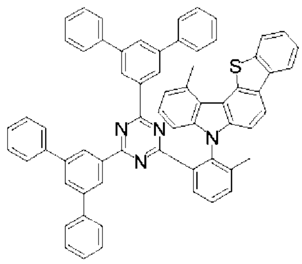
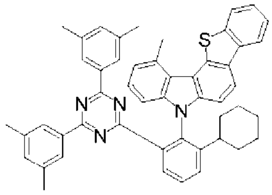
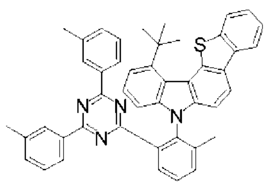
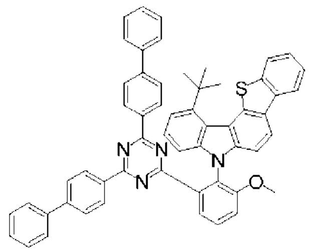
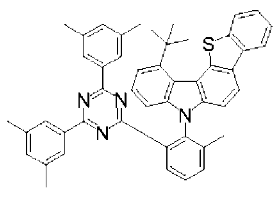
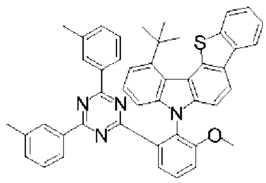
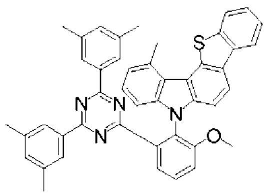

[Chem. 125]

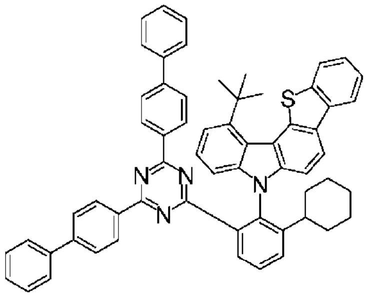
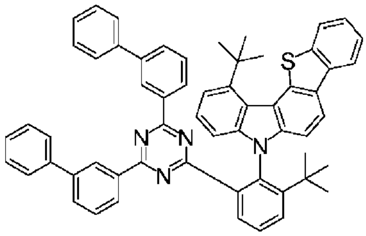
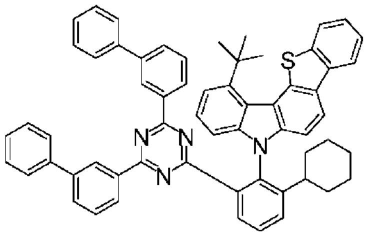
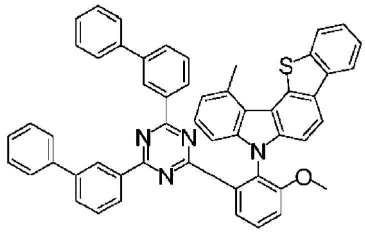
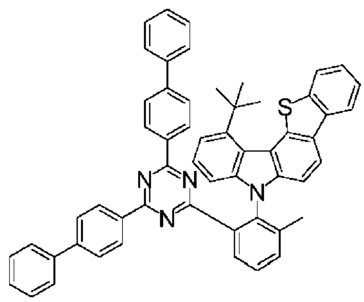
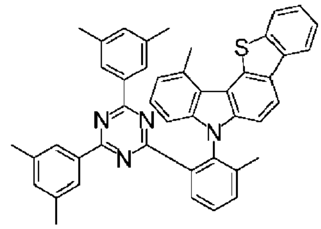
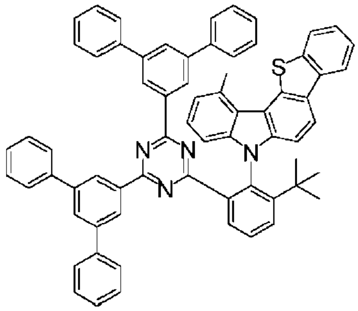
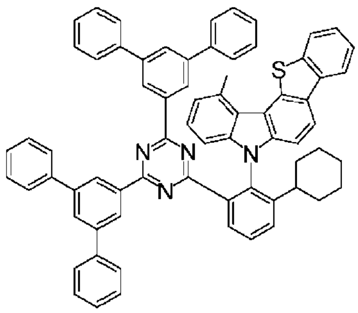
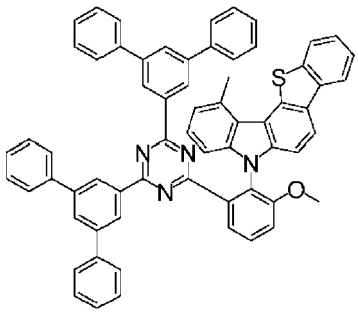
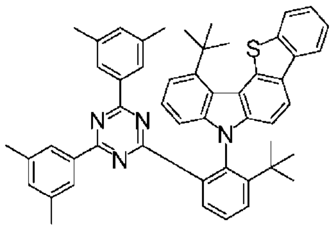
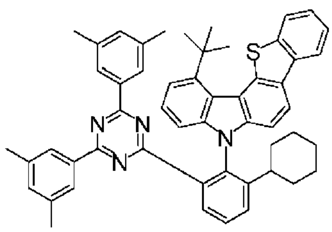
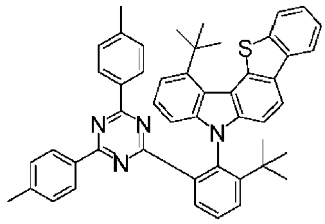
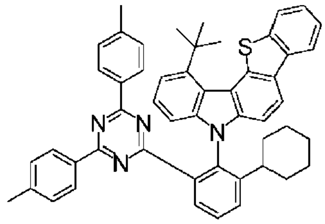
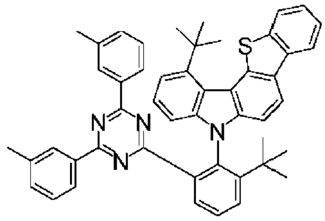
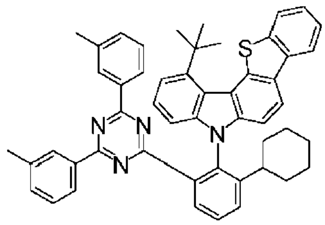
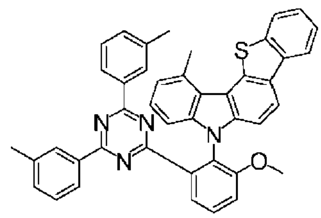

[Chem. 126]

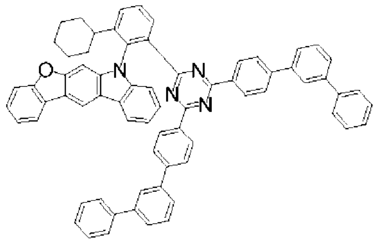
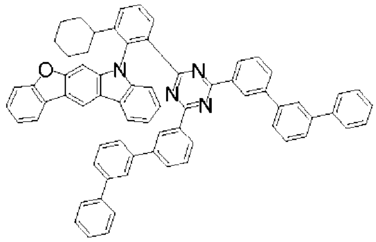
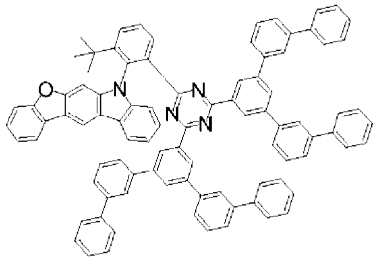
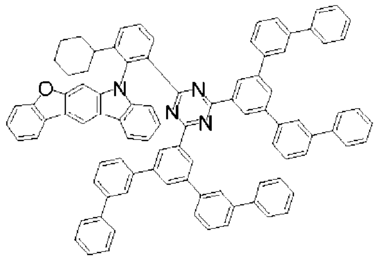
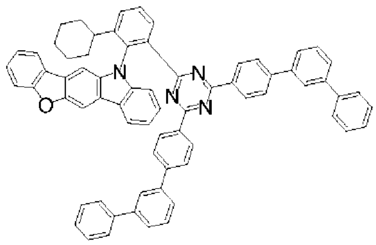
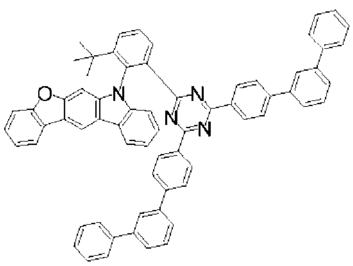
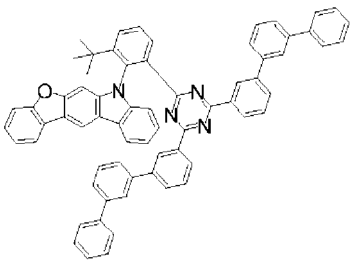
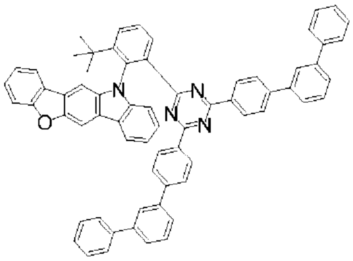
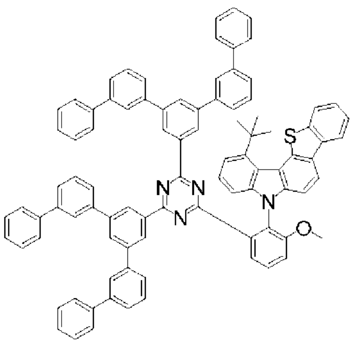
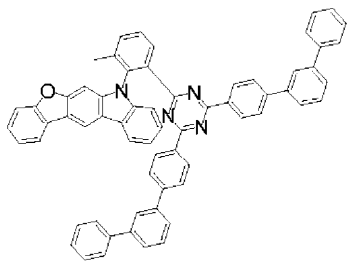
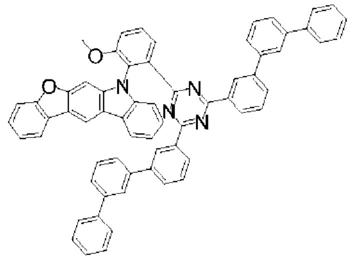
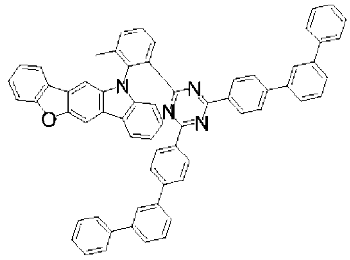
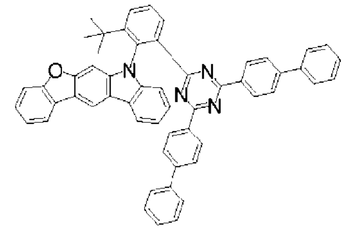
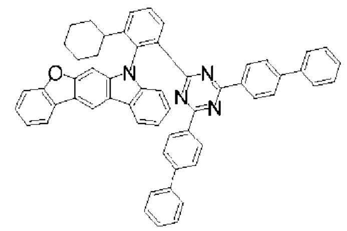
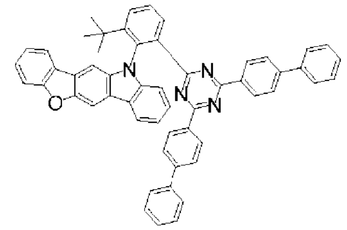
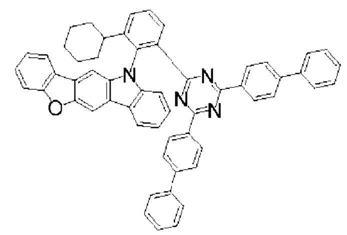

[Chem. 127]

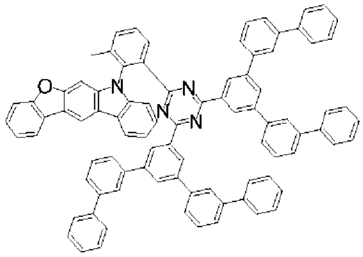
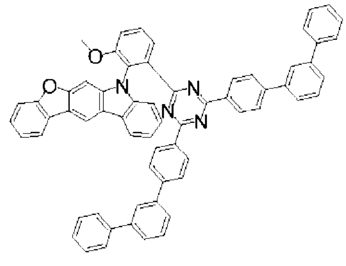
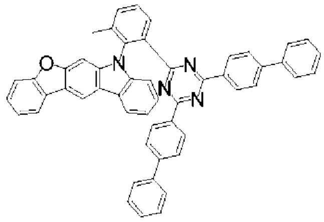
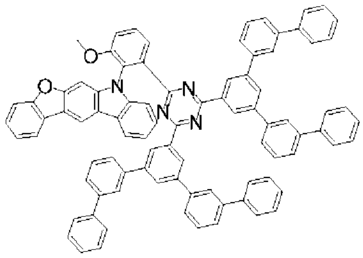
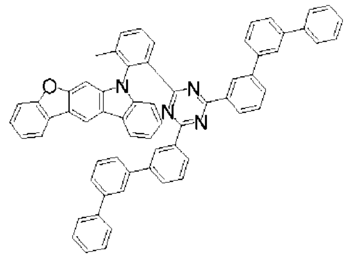
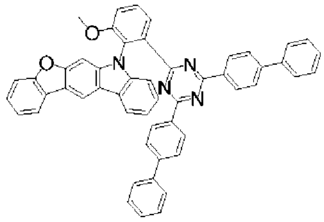
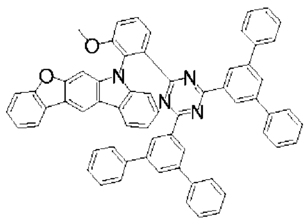
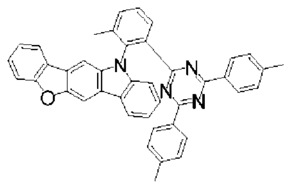
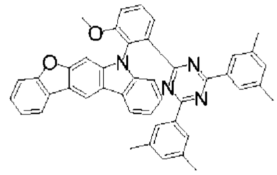
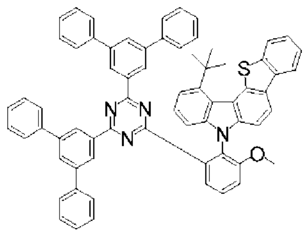
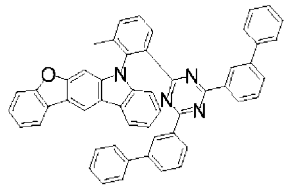
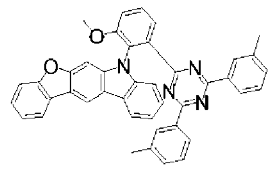
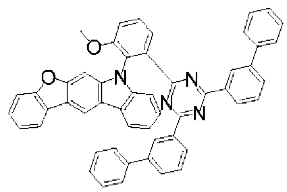
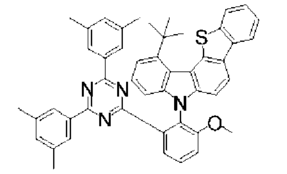

[Chem. 128]

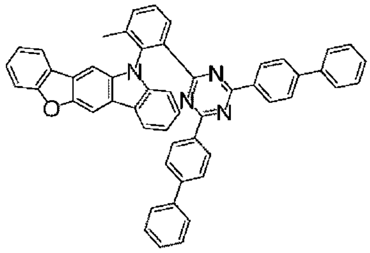

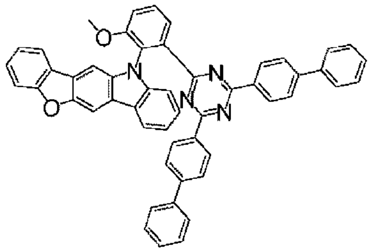

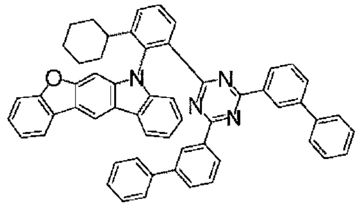

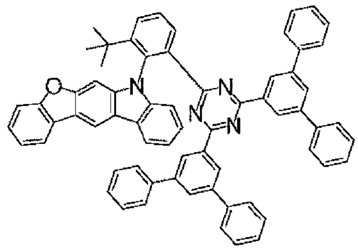

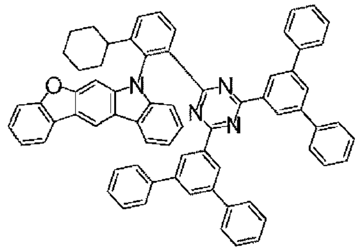

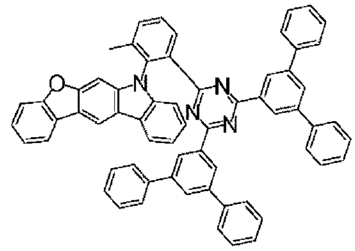

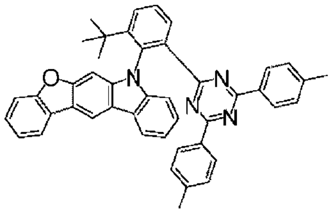

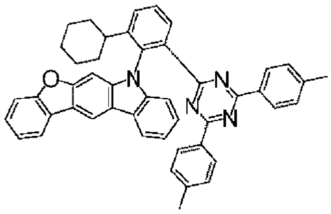

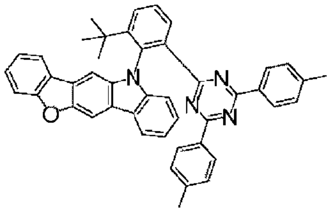

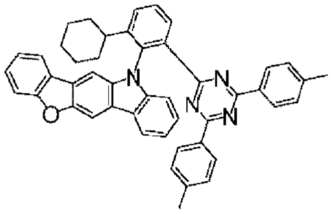

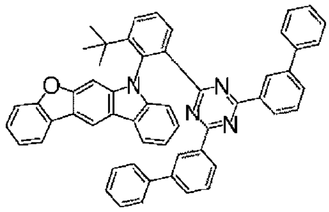

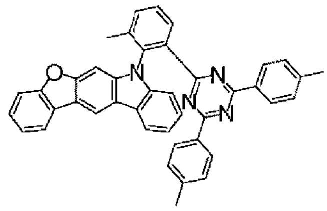

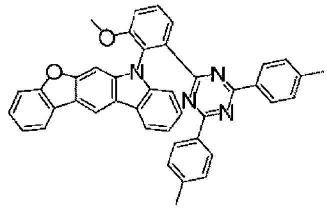

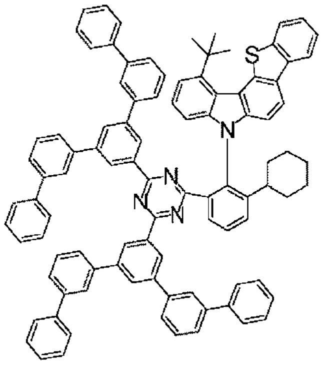

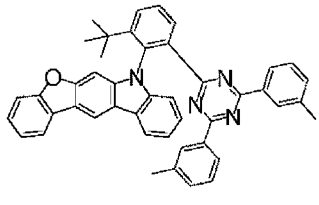

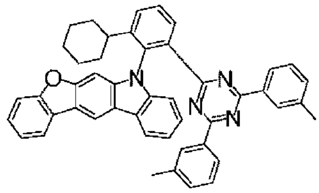

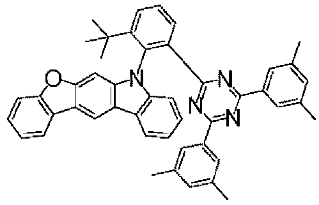

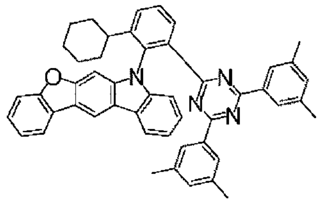

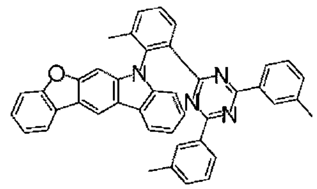

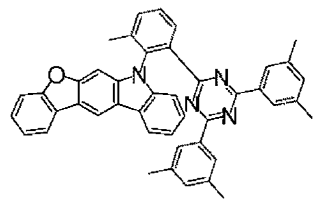

[Chem. 129]

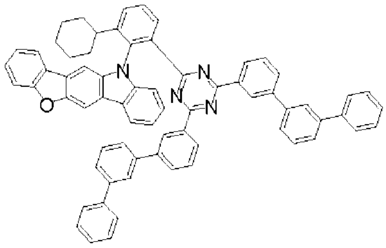
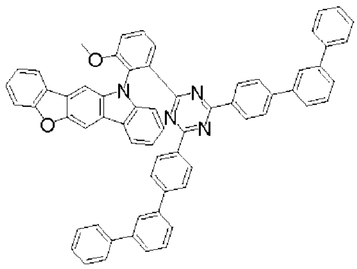
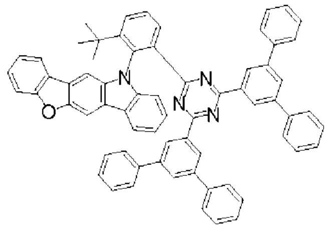
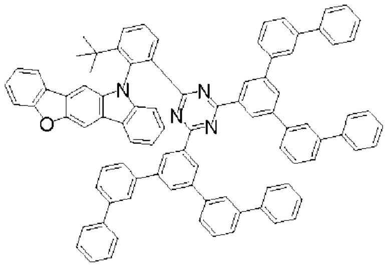
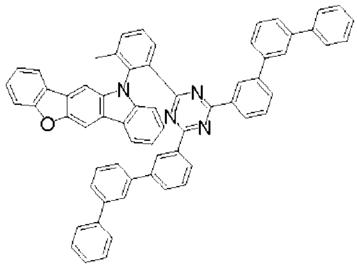
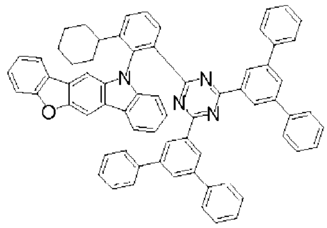
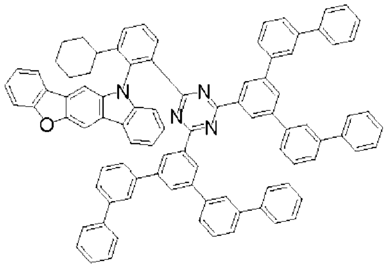
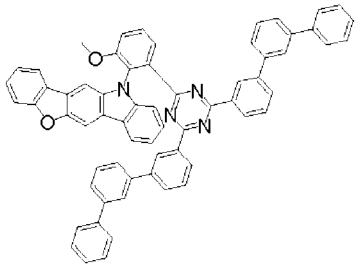
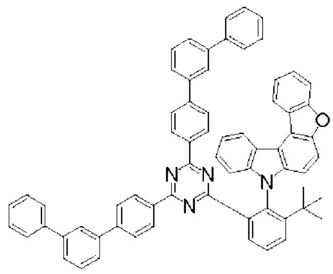
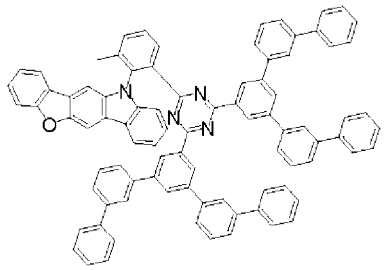
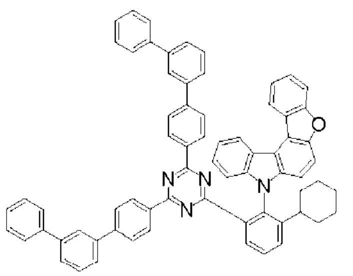
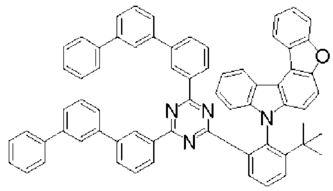
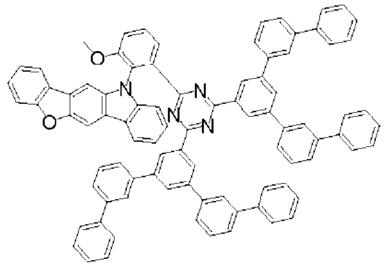
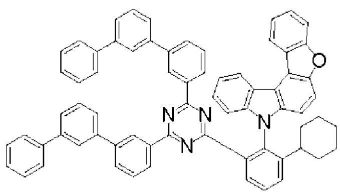
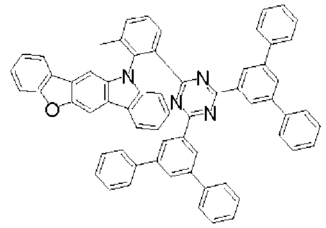

[Chem. 130]

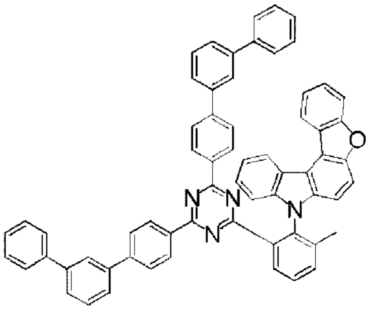
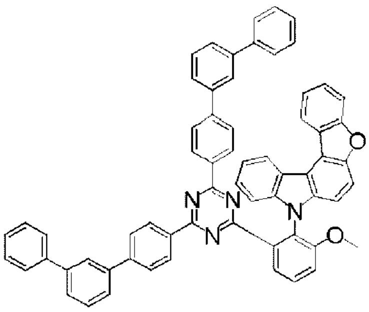
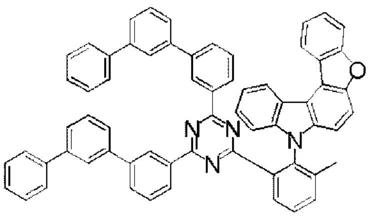
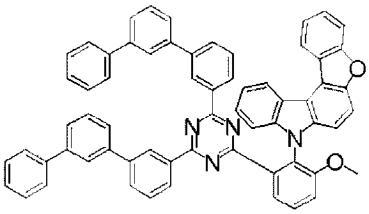
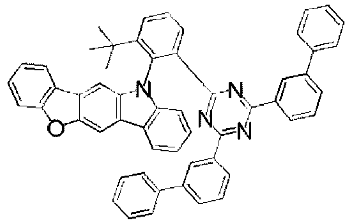
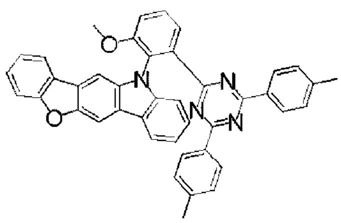
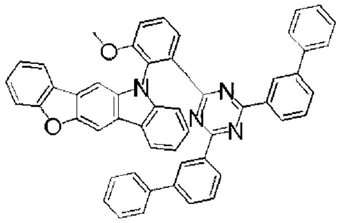
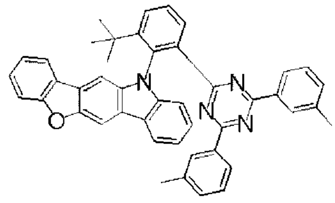
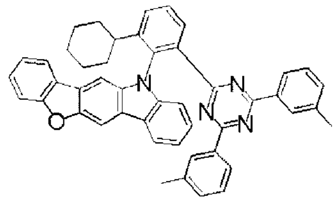
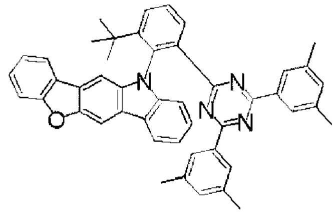
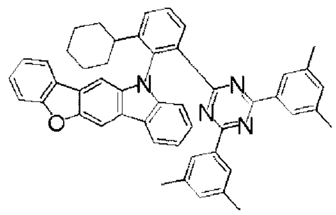
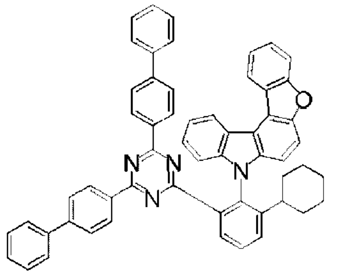
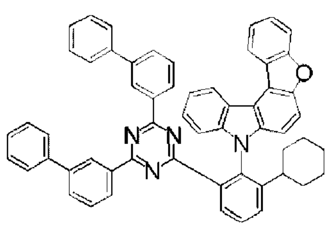
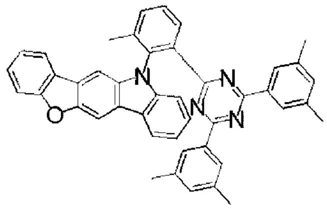
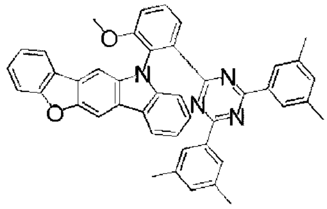
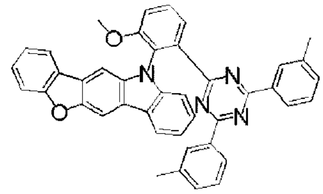
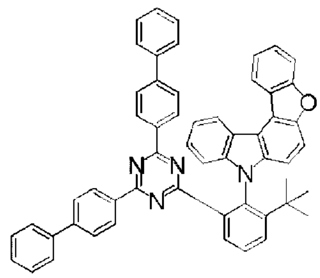
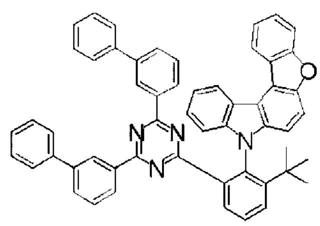
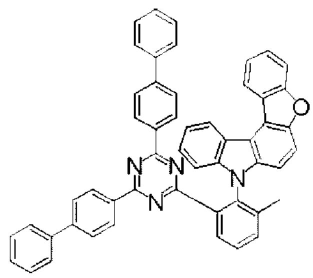

[Chem. 131]

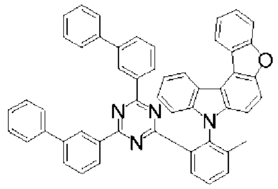
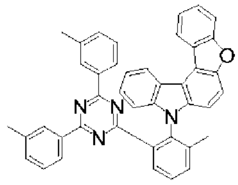
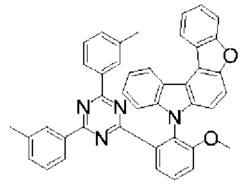
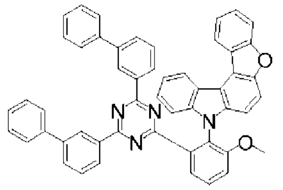
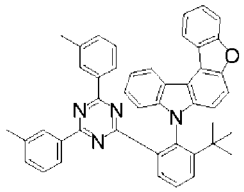
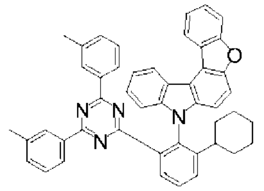
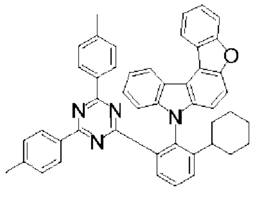
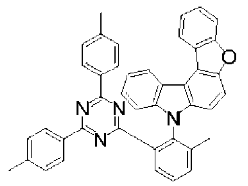
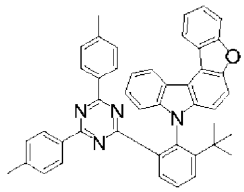
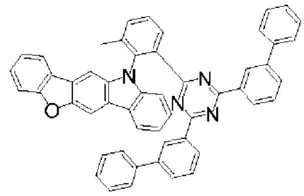
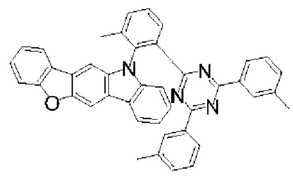
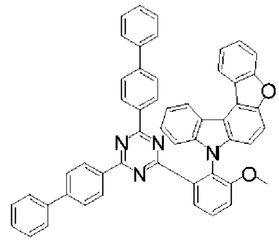
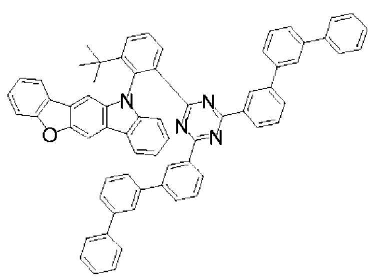
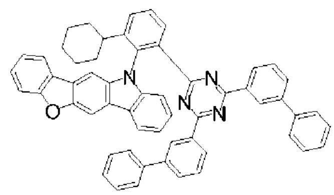
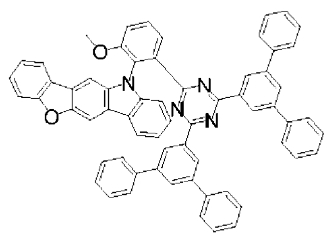
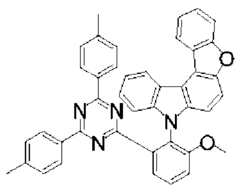

[Chem. 132]

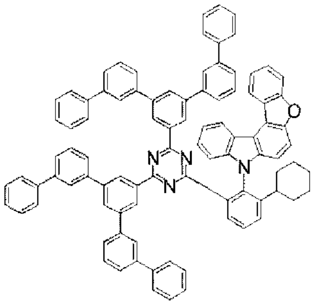
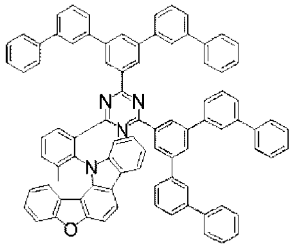
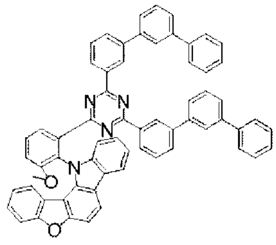
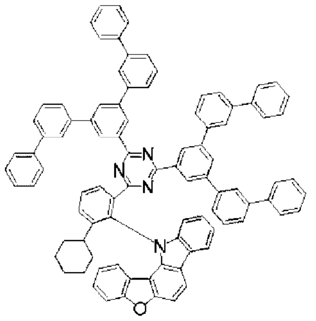
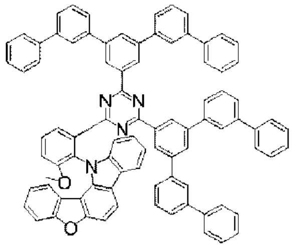
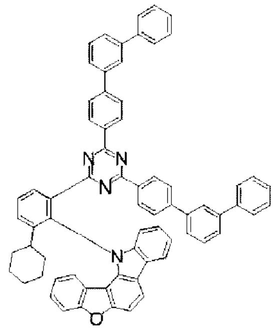
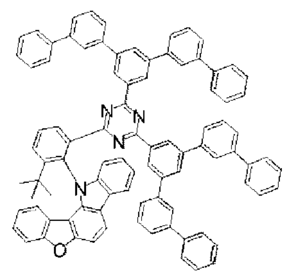
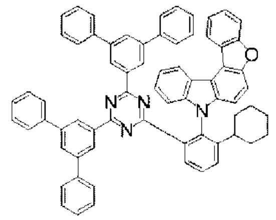
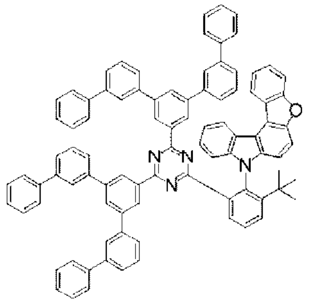
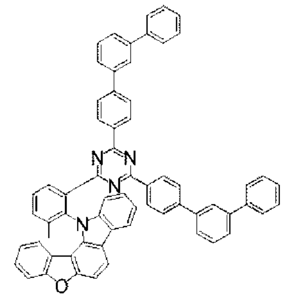
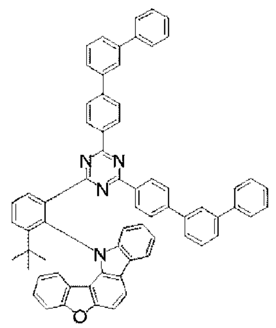
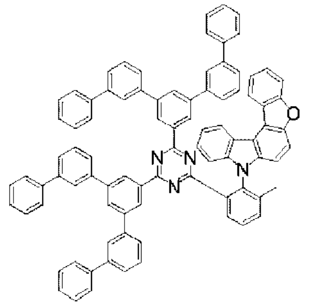
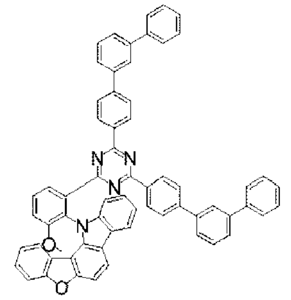
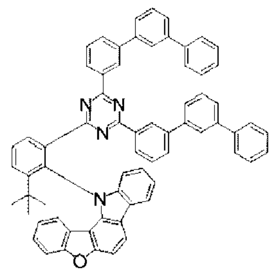
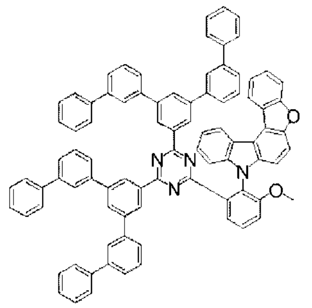
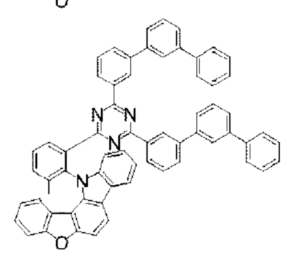
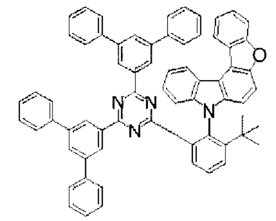

[Chem. 133]

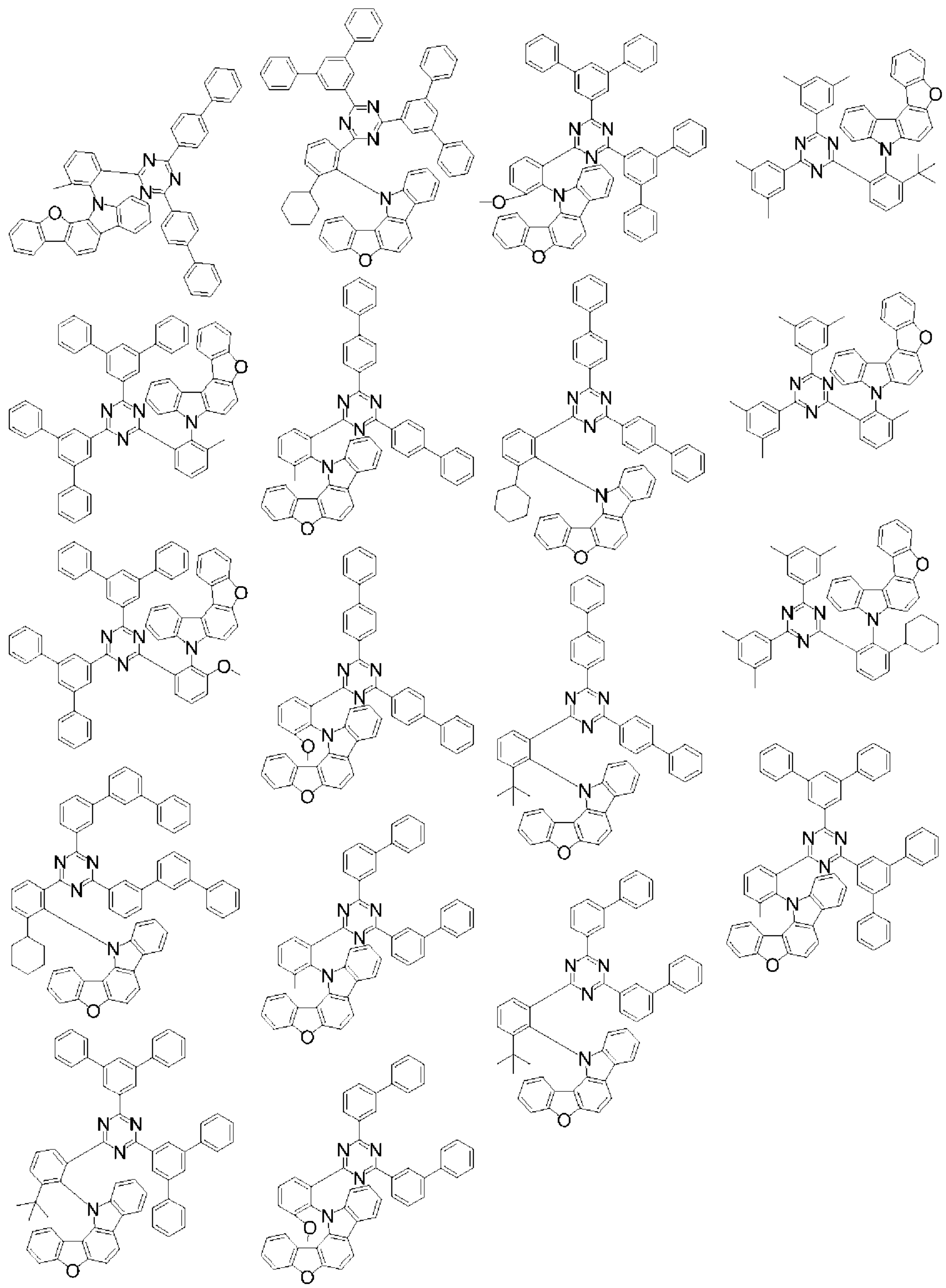

[Chem. 134]

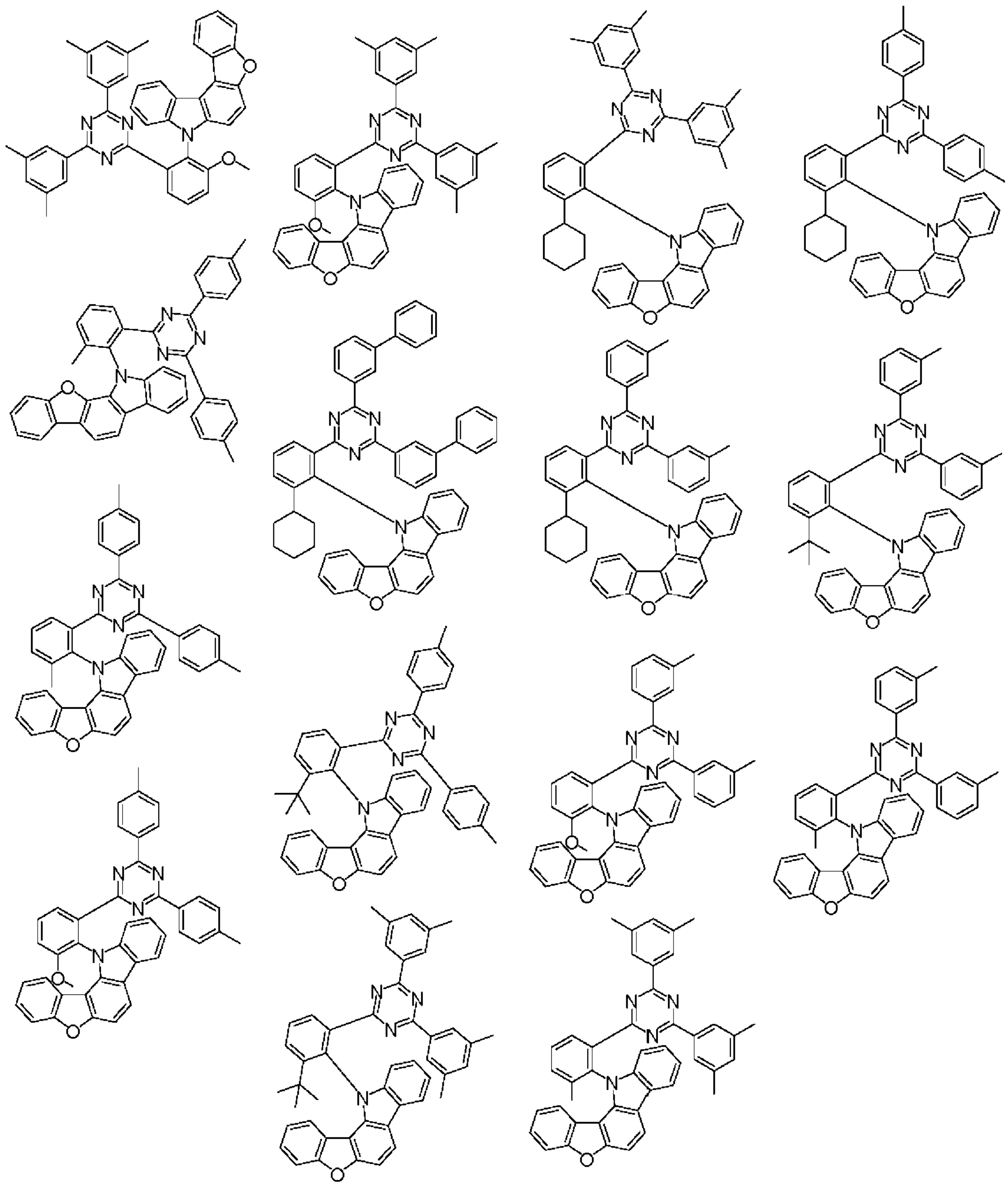

[Chem. 135]

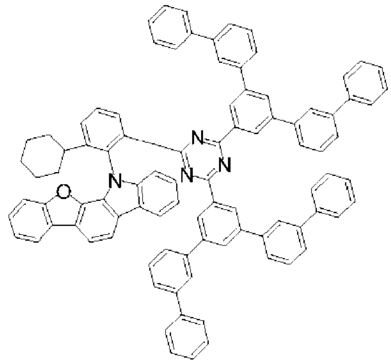

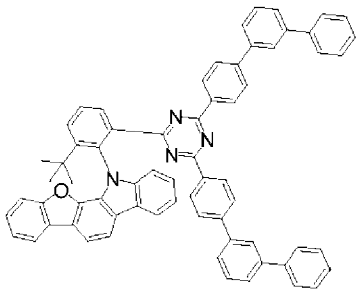

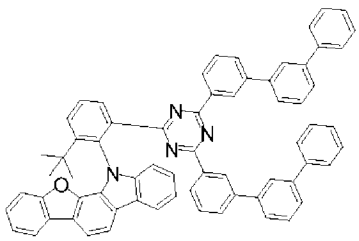

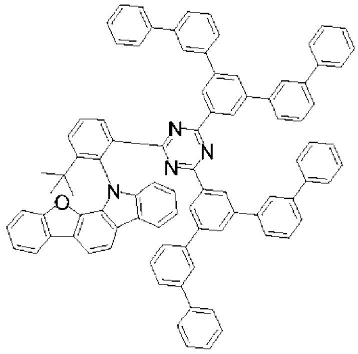

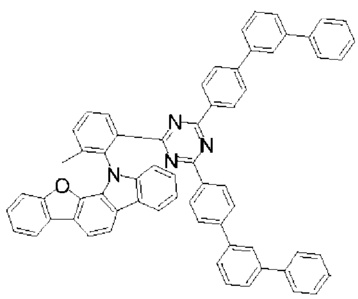

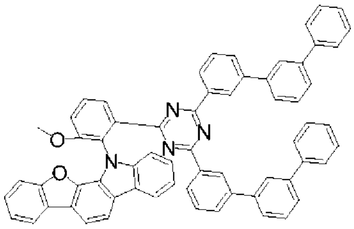

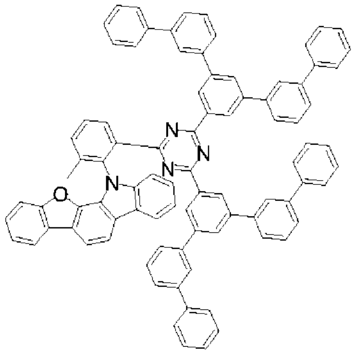

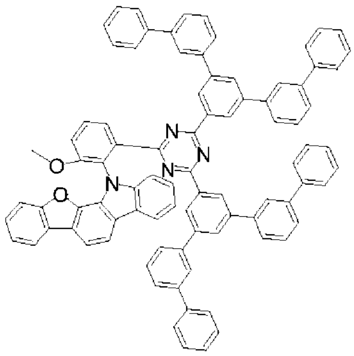

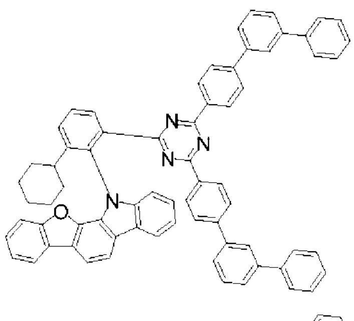

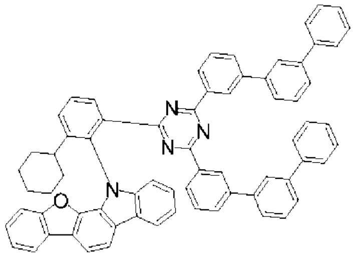

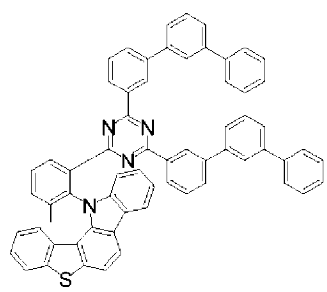

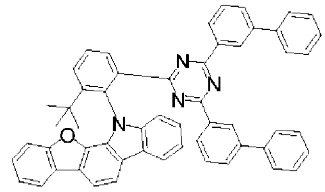

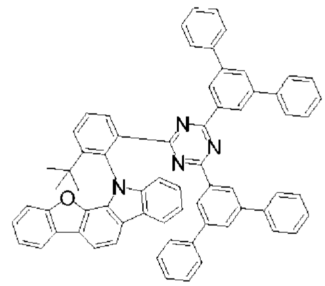

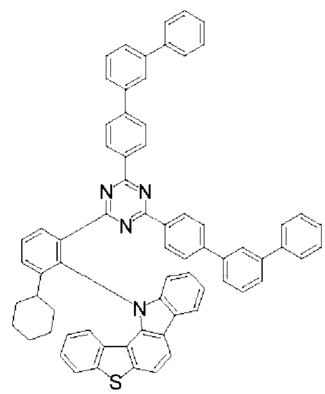

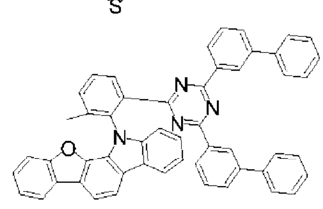

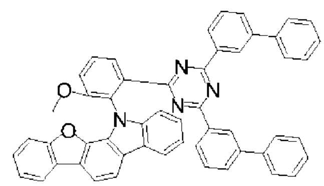

[Chem. 136]

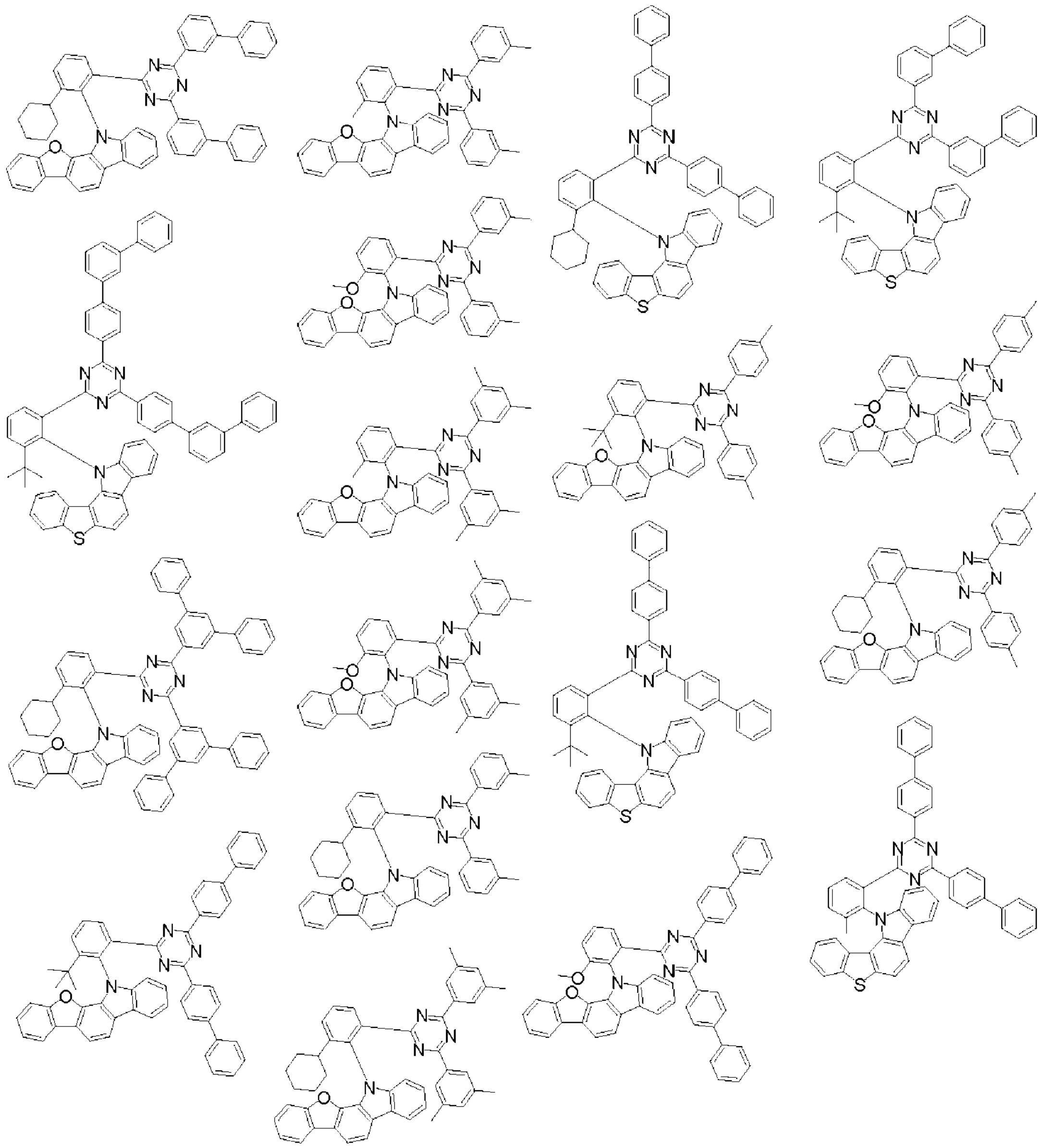

[Chem. 137]

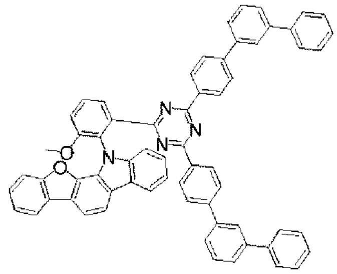
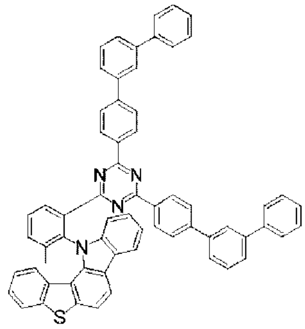
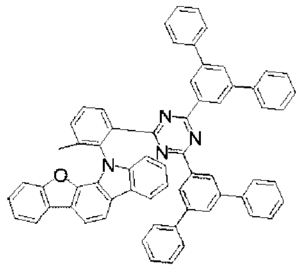

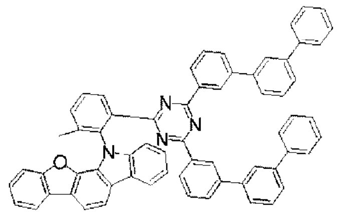
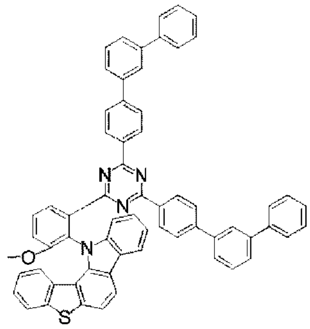
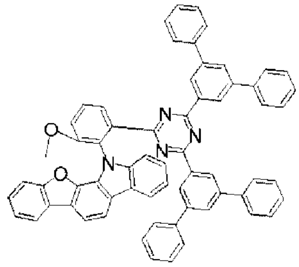

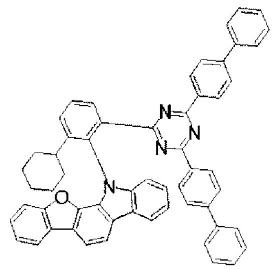
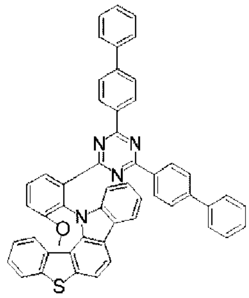
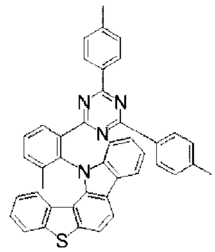

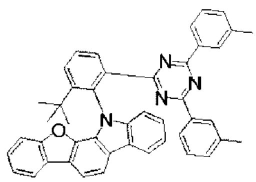
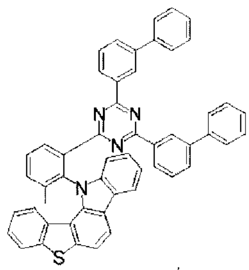
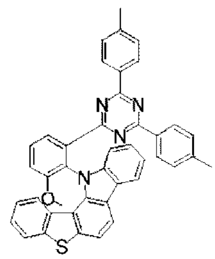

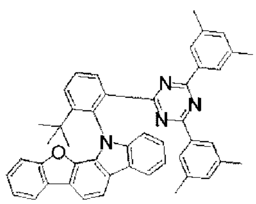
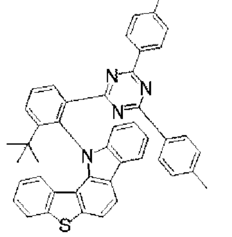
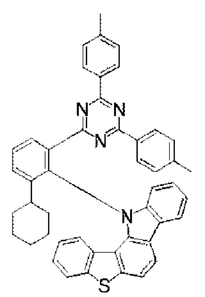

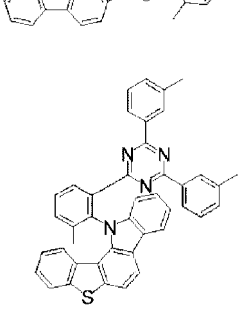

[Chem. 138]

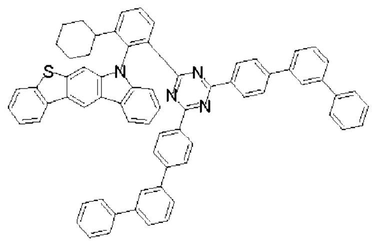
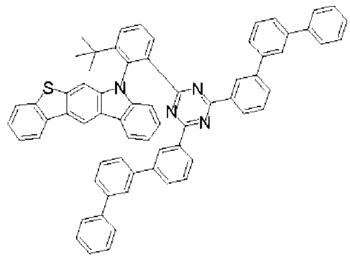
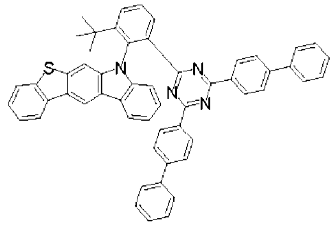
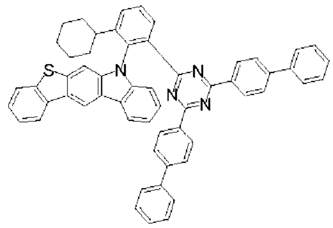
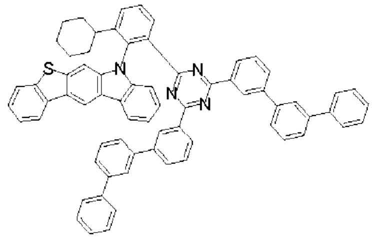
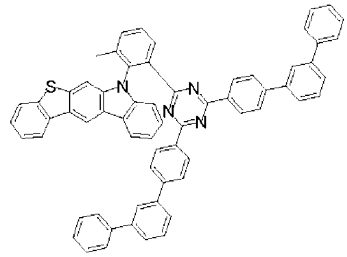
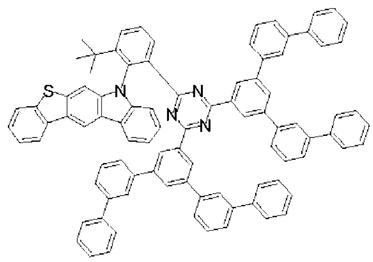
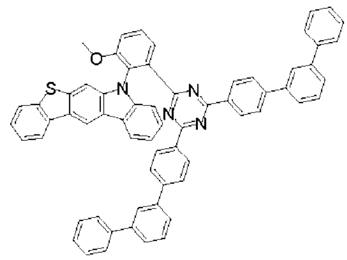
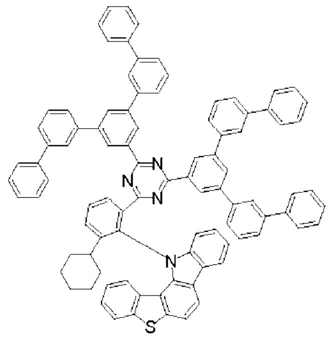
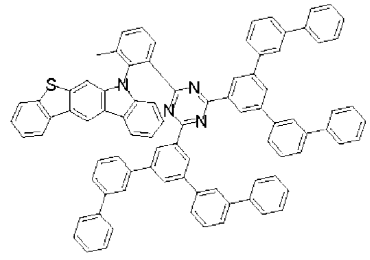
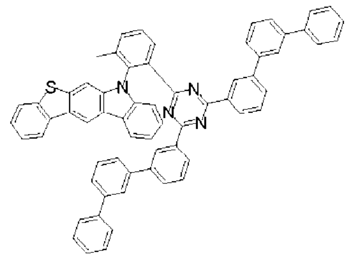
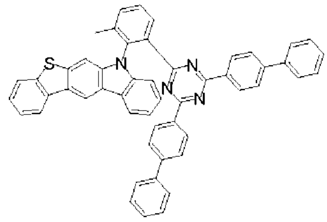
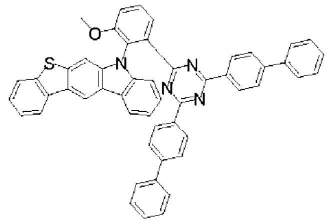
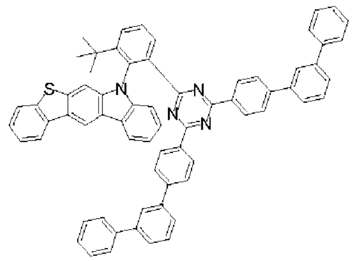
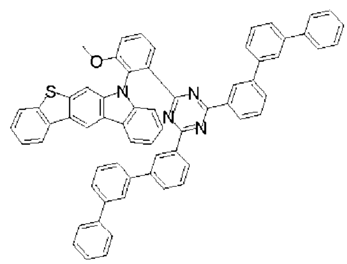
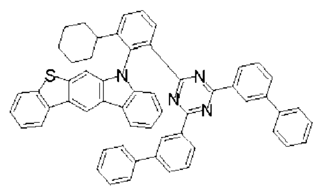

[Chem. 139]

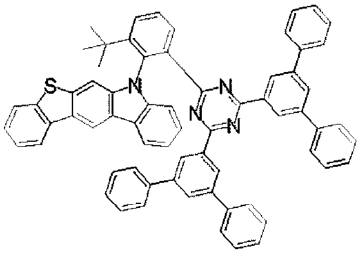
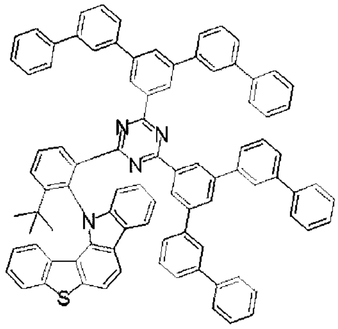
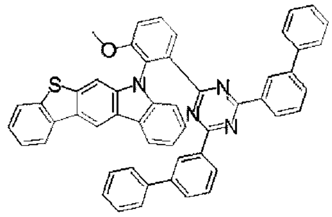
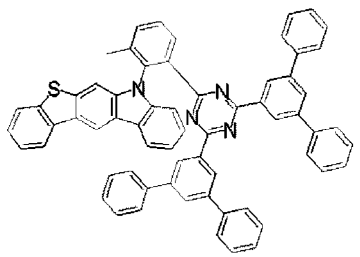
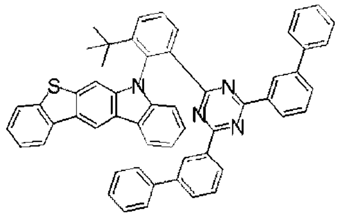
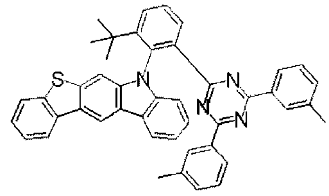
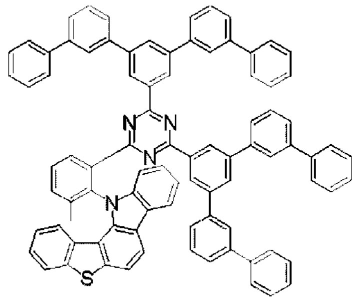
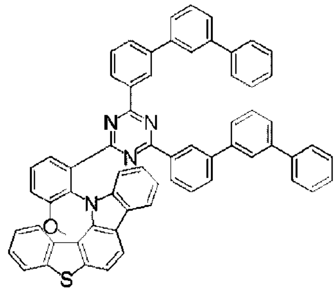
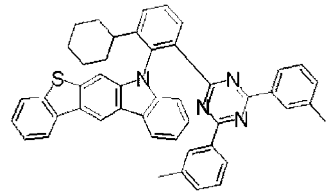
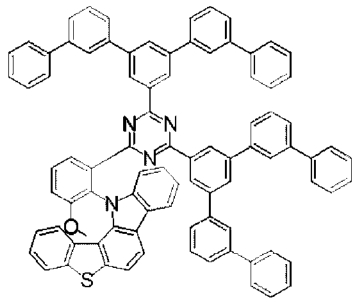
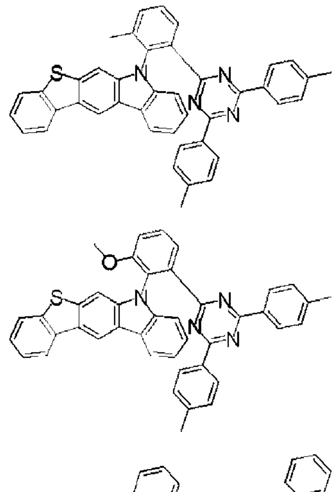
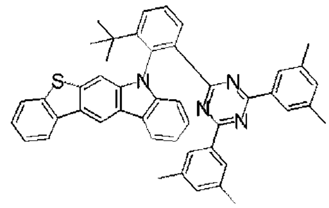
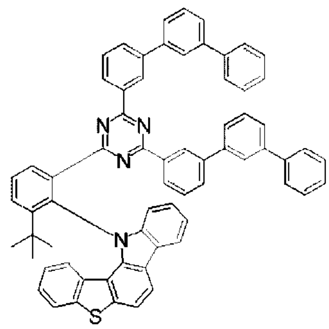
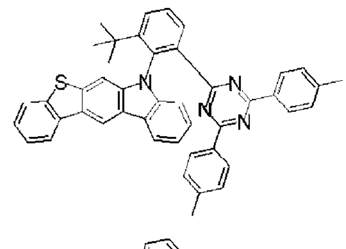
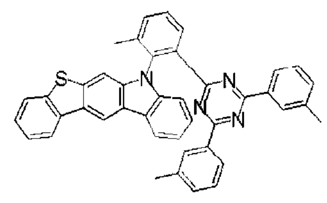
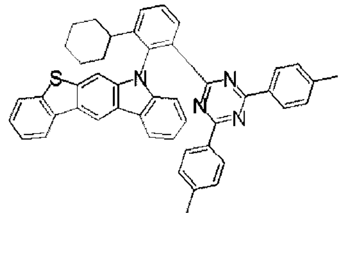
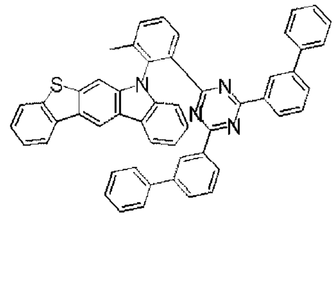
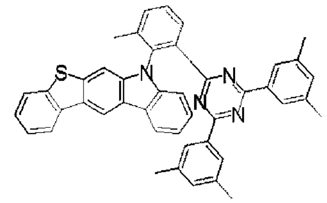

[Chem. 140]

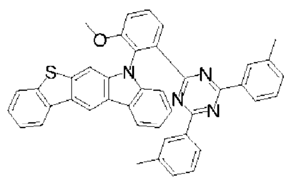
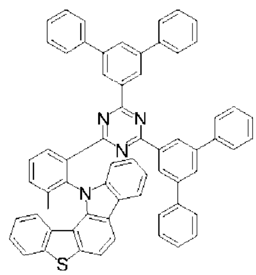
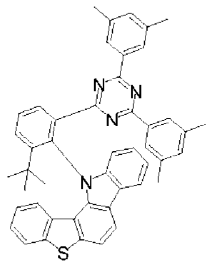
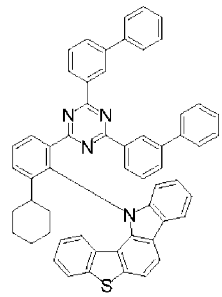
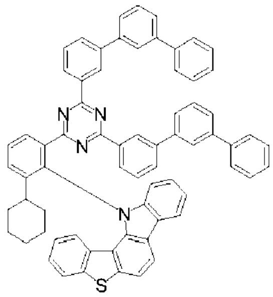
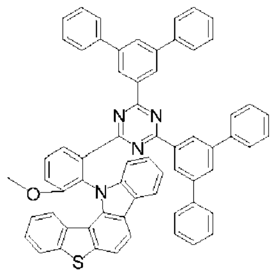
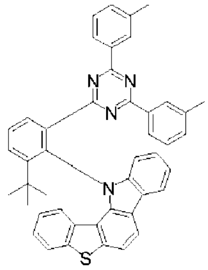
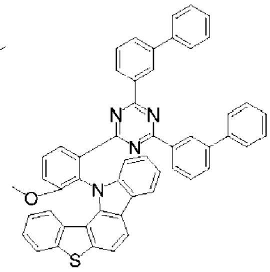
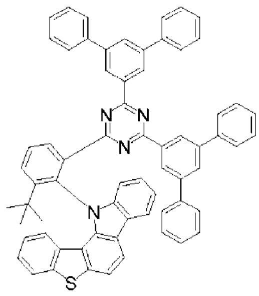
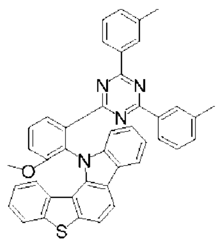
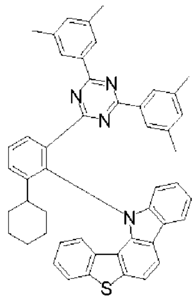
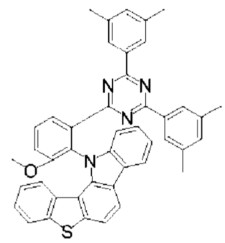
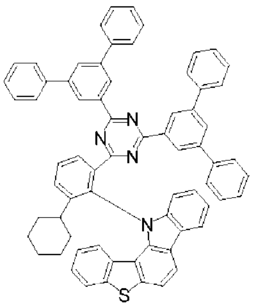
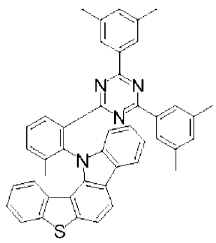
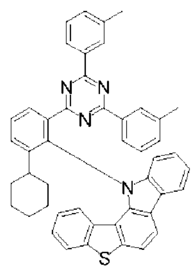

[Chem. 141]

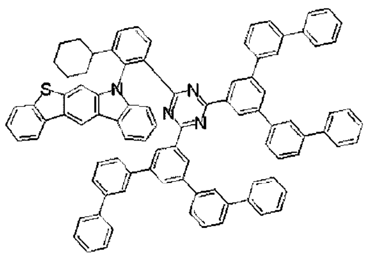
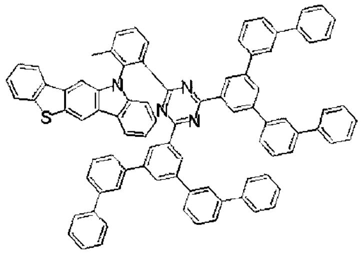
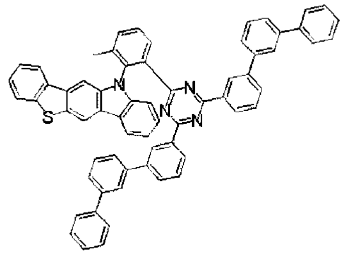
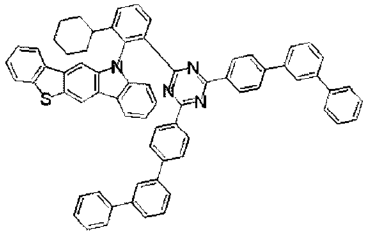
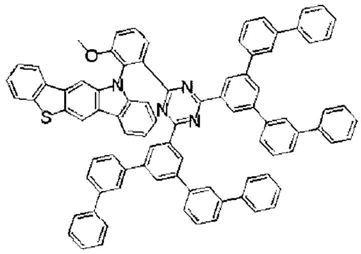
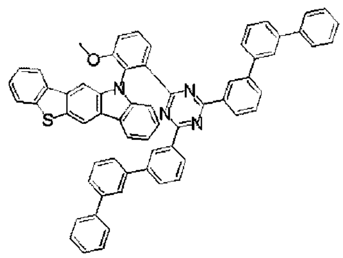
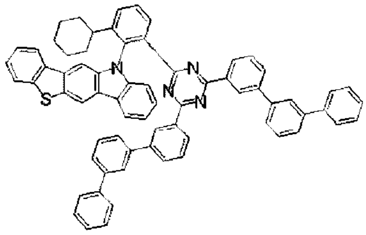
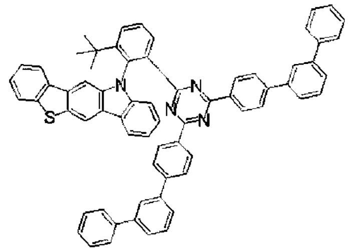
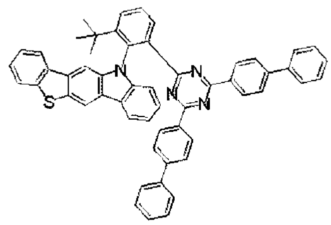
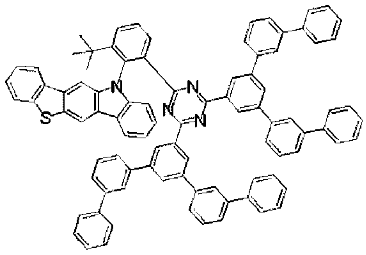
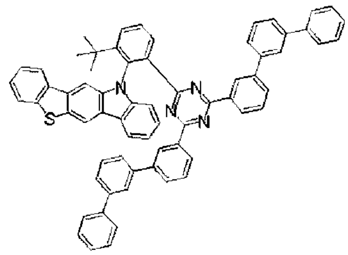
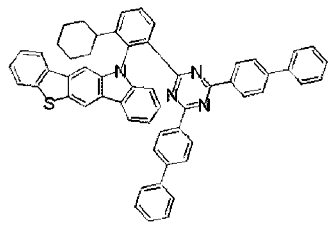
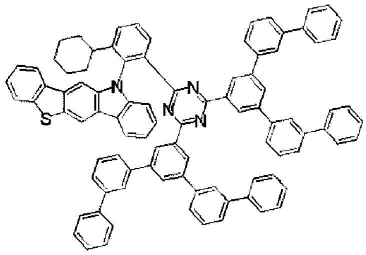
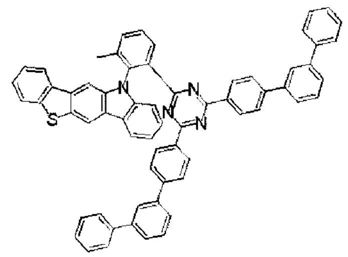
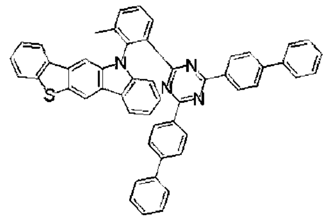

[Chem. 142]

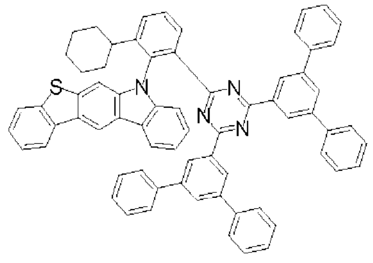

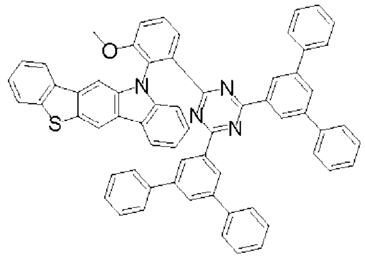

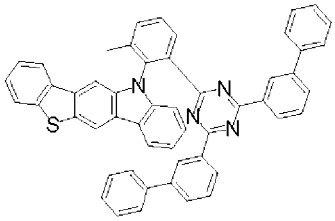

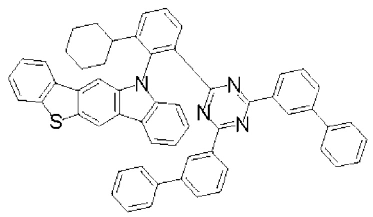

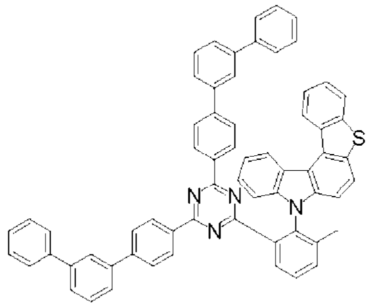

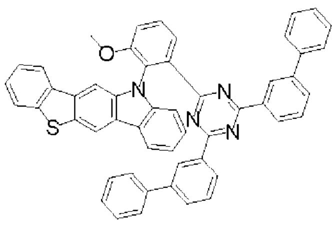

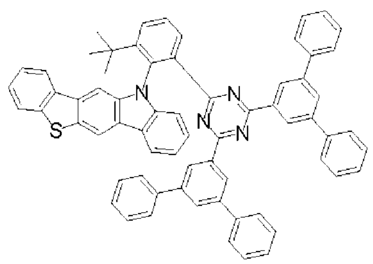

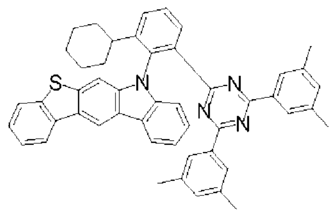

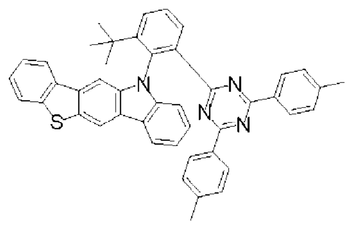

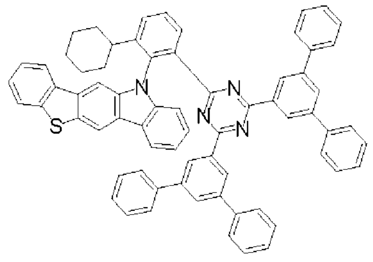

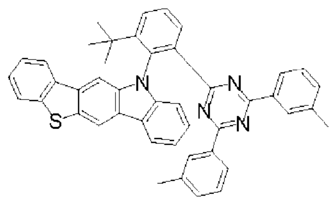

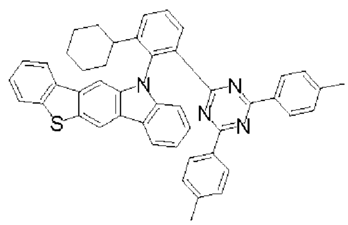

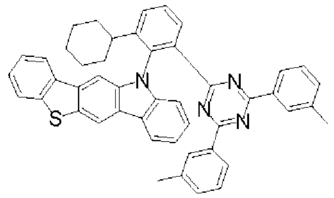

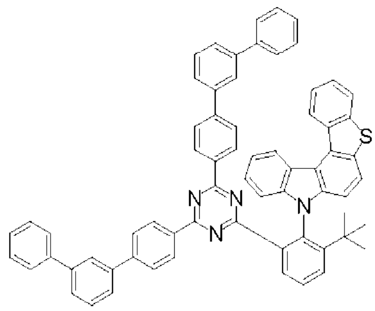

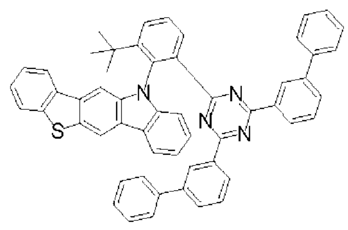

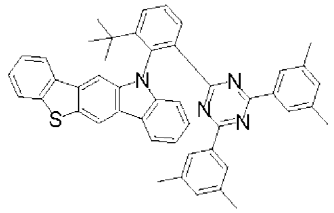

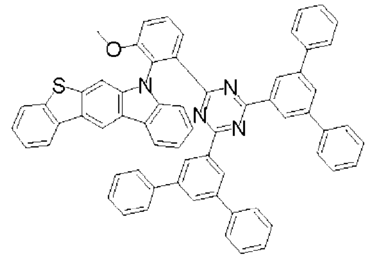

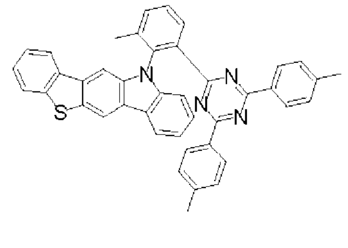

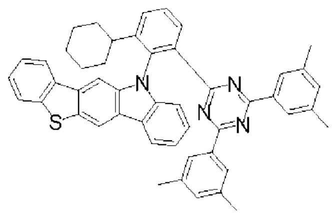

[Chem. 143]

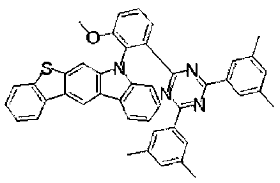
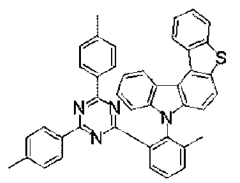
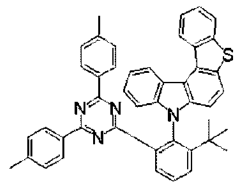
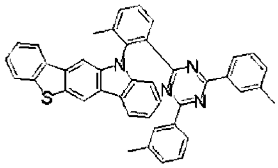
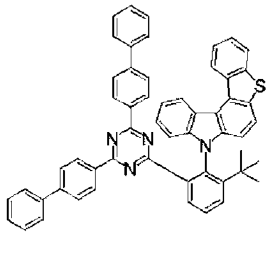
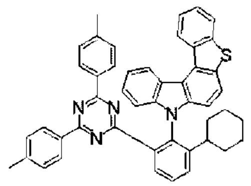
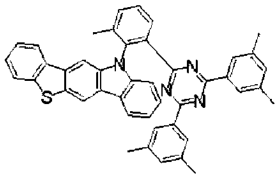
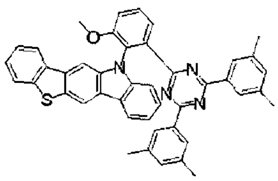
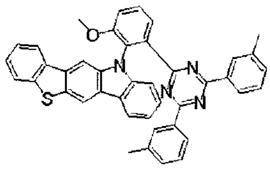
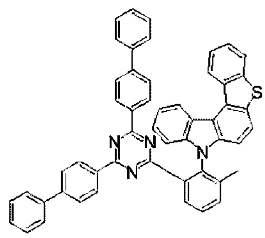
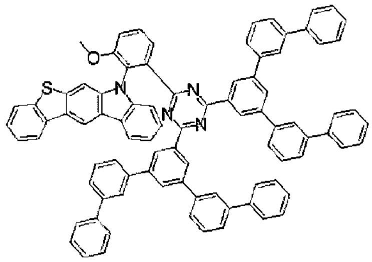
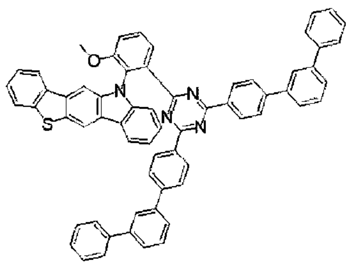
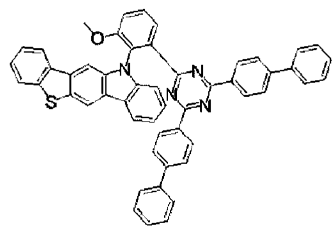
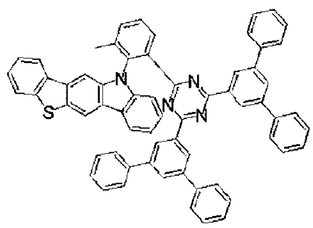
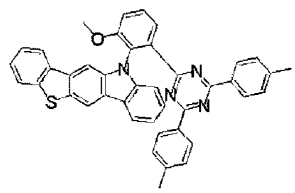
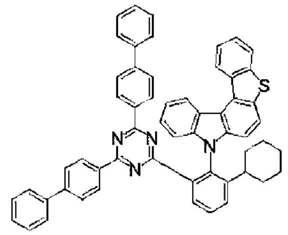

[Chem. 144]

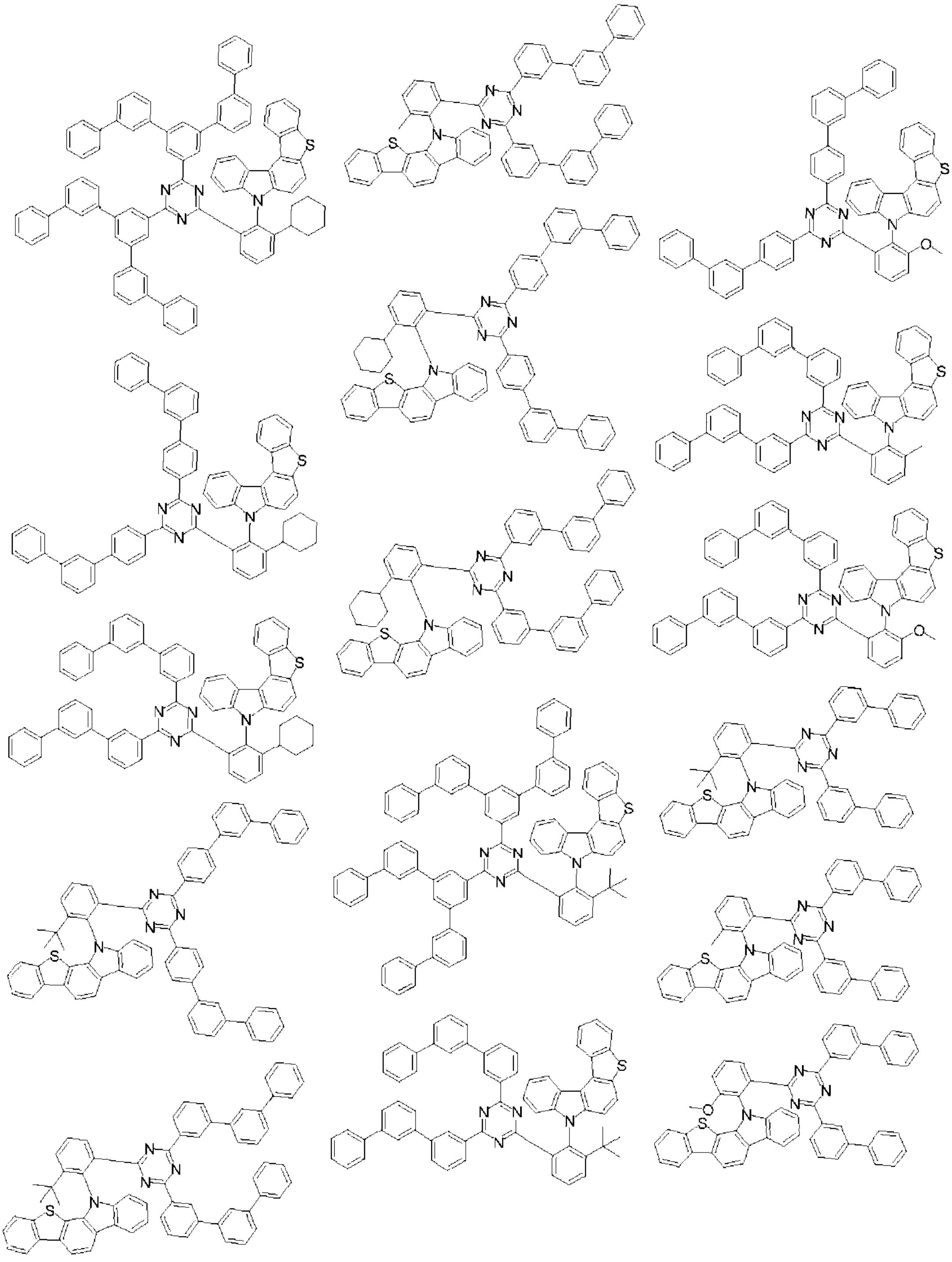

[Chem. 145]

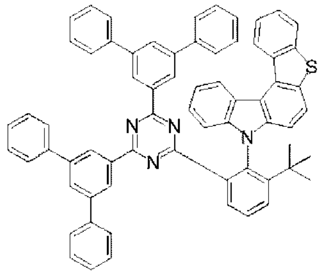
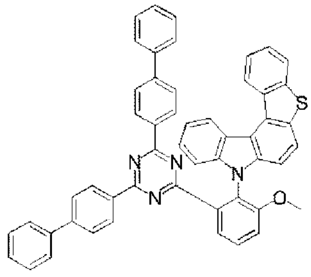
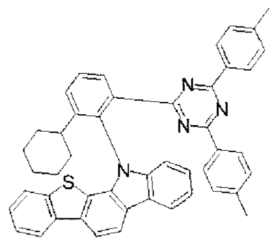
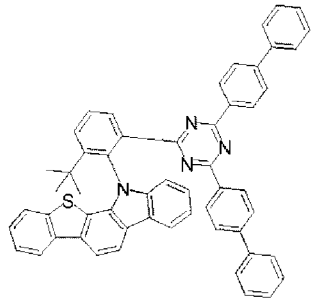
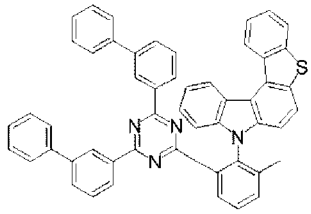
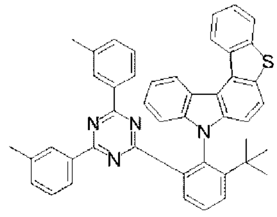
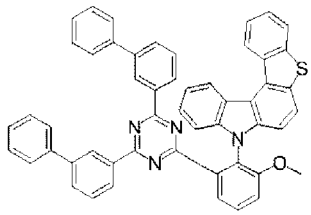
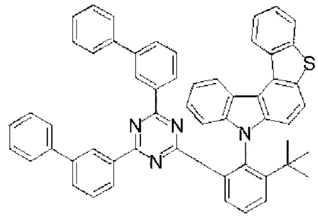
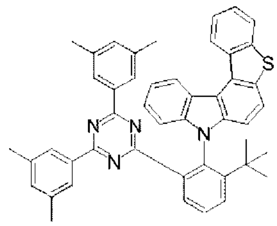
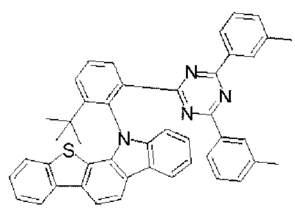
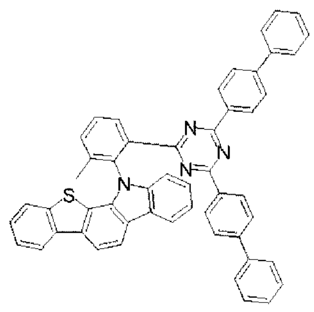
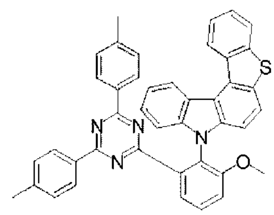
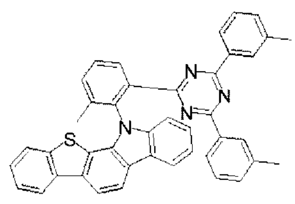
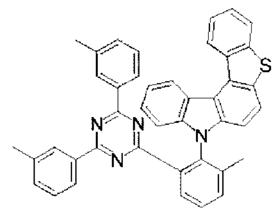
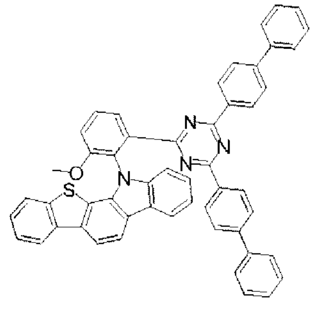
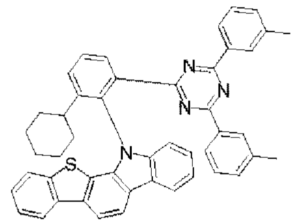
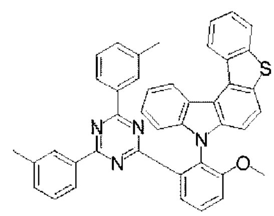
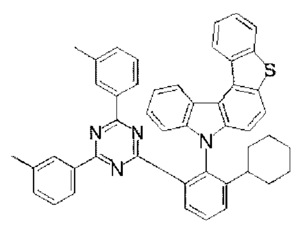

[Chem. 146]

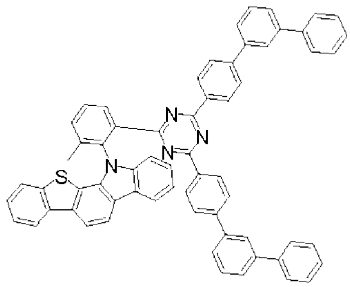
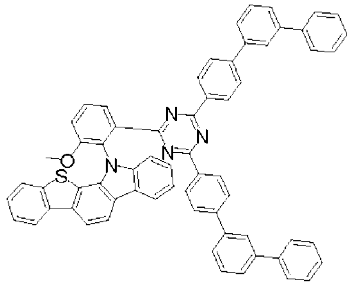
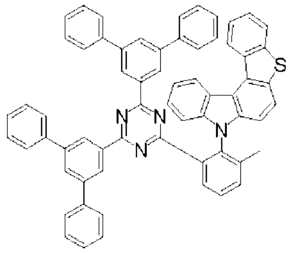
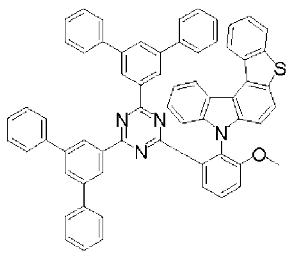
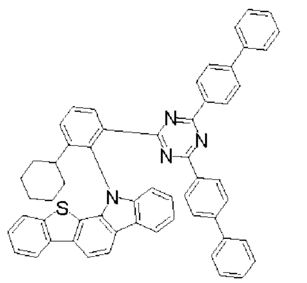
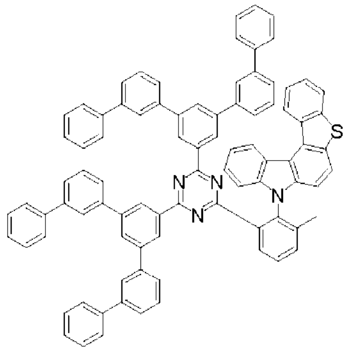
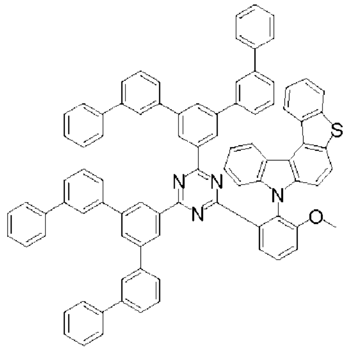
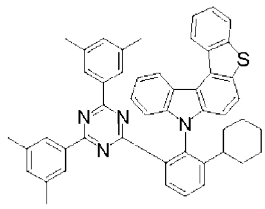
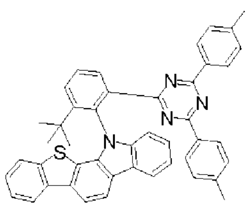
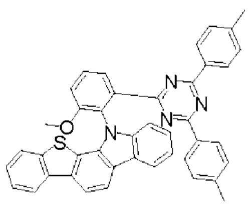
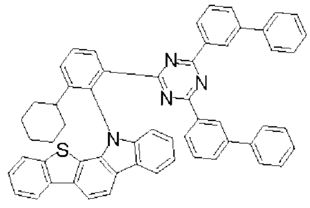
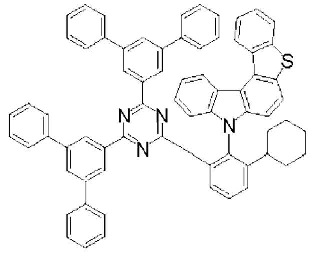
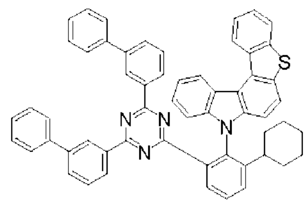
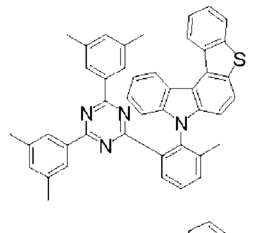
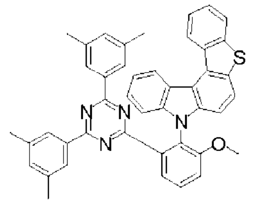
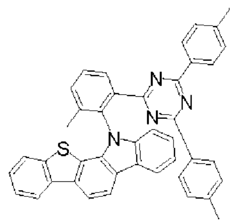

[Chem. 147]

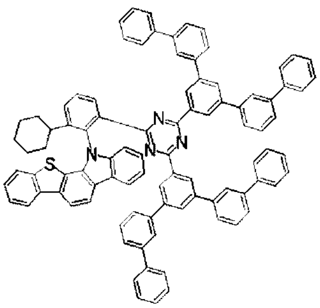
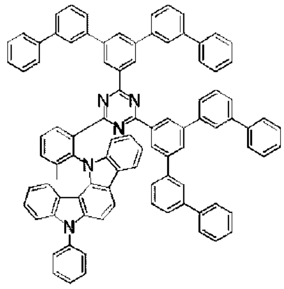
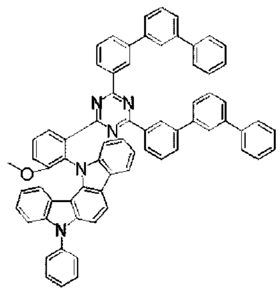
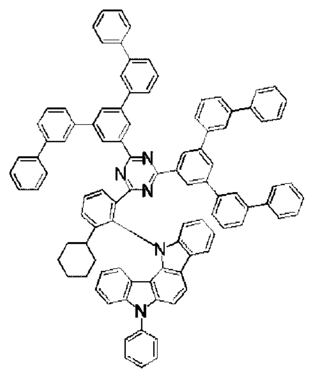
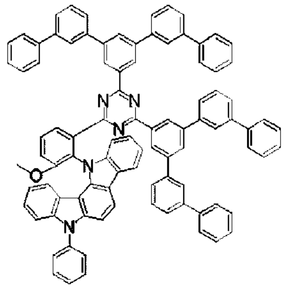
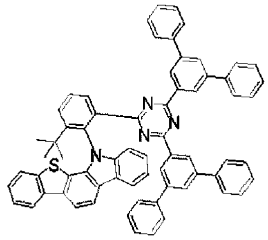
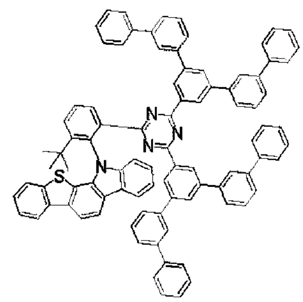
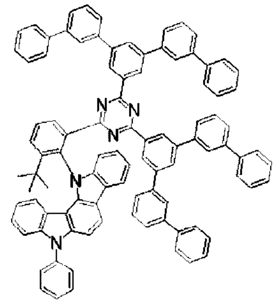
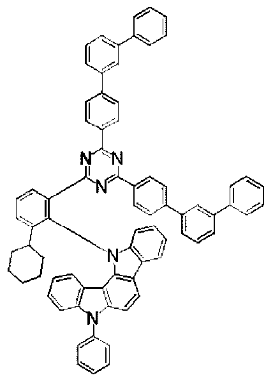
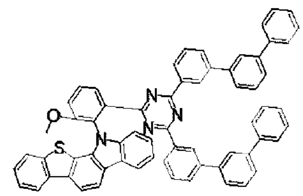
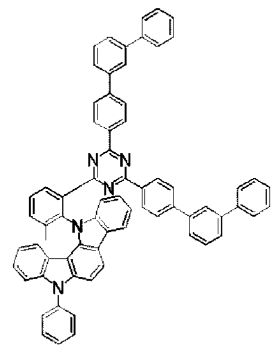
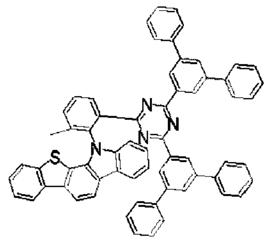
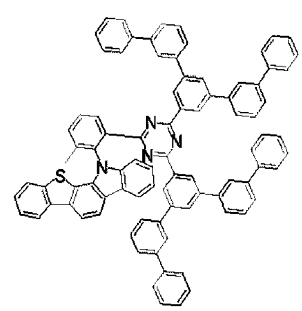
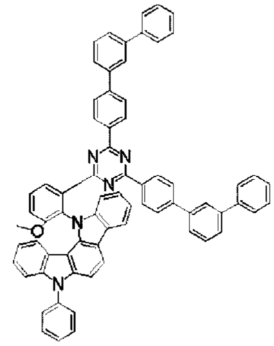
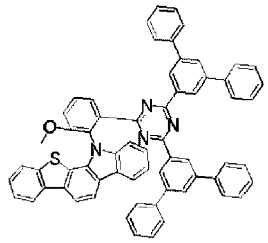

[Chem. 148]

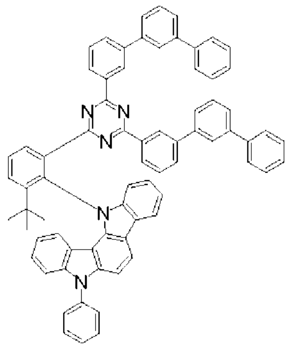
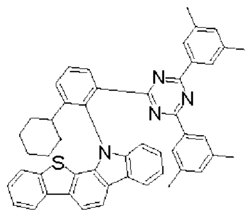
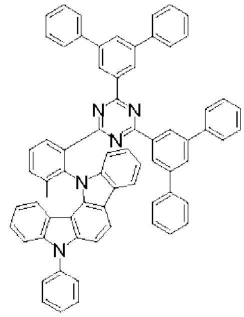
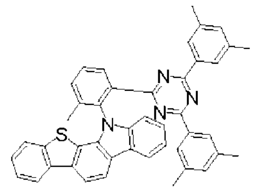
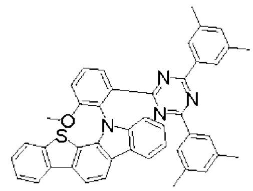
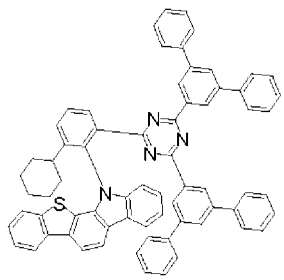
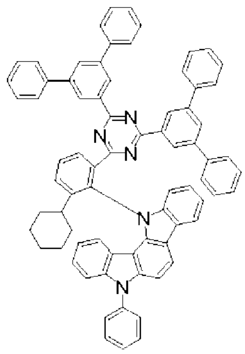
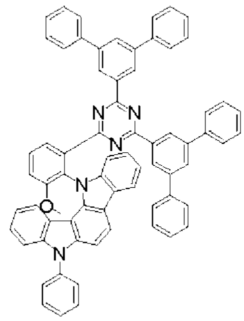
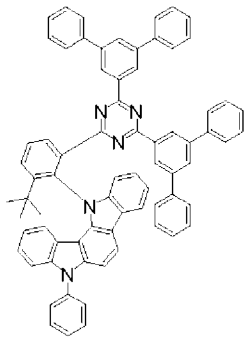
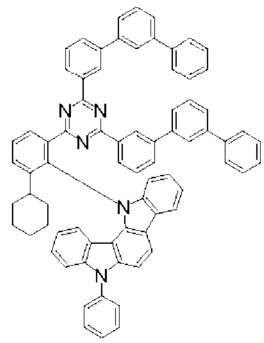
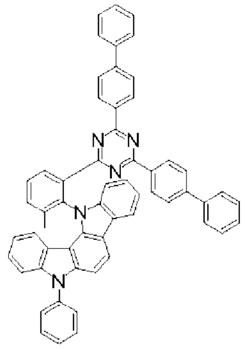
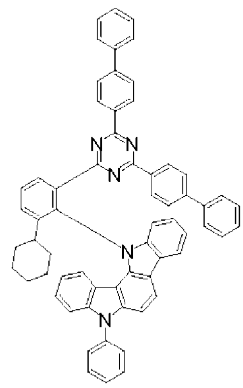
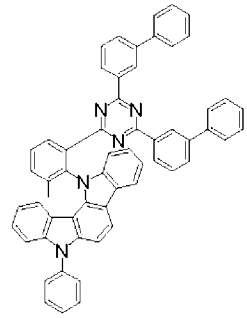
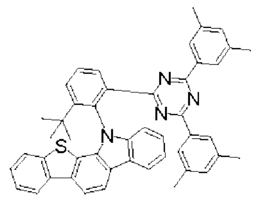
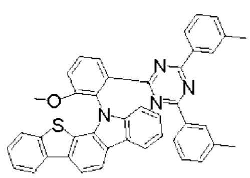
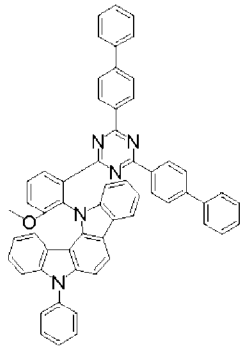
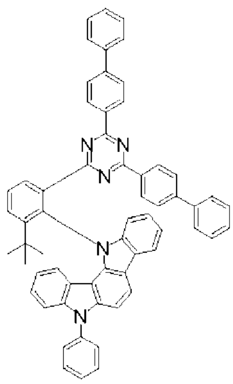
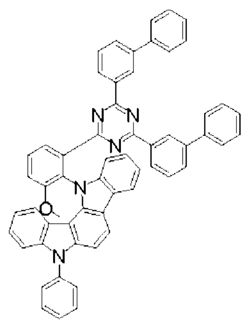

[Chem. 149]

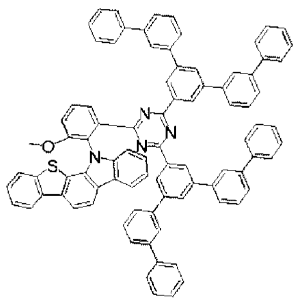
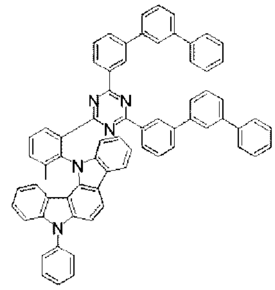
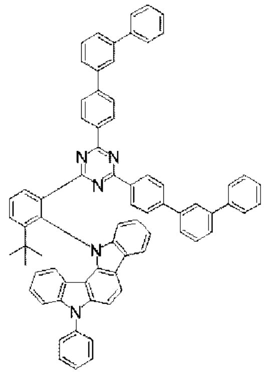
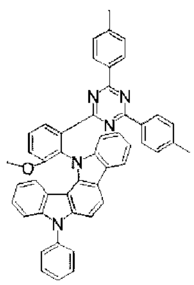
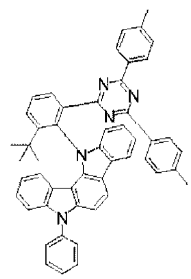
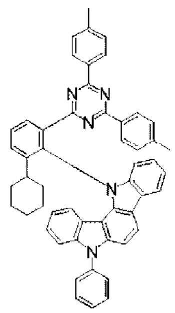
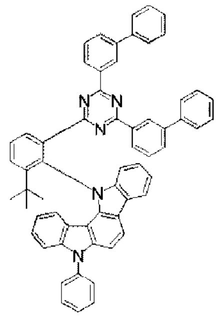
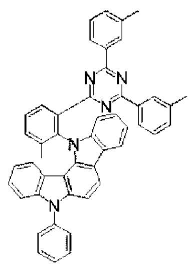
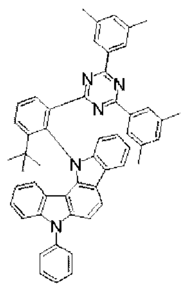
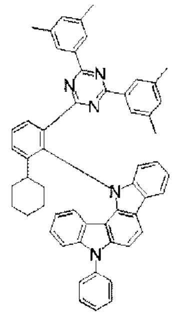
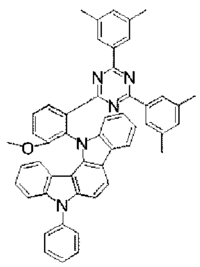
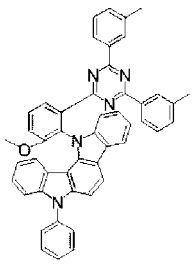
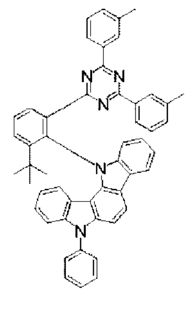
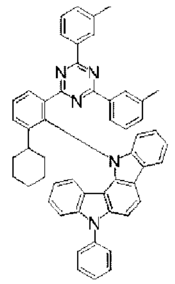
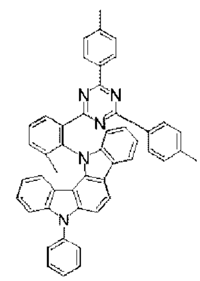
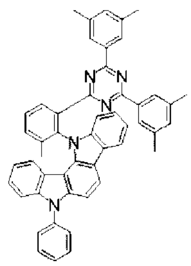
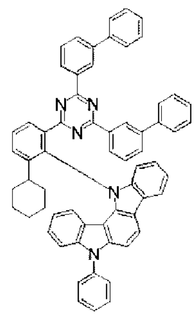

[Chem. 150]

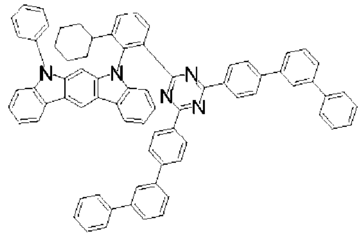
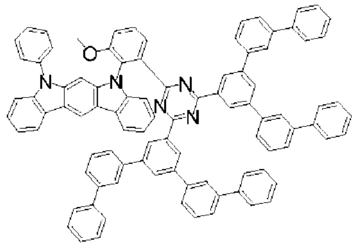
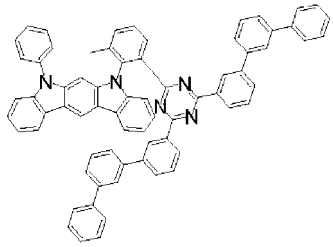
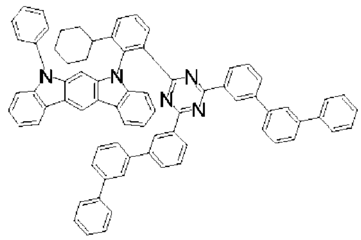
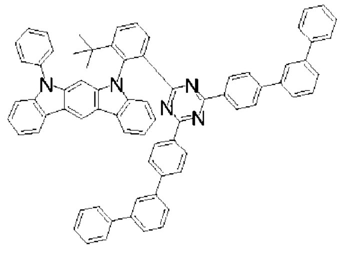
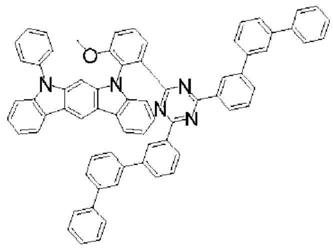
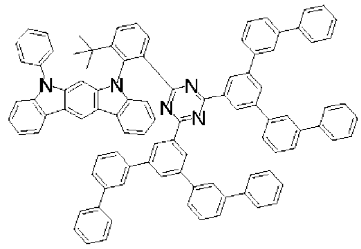
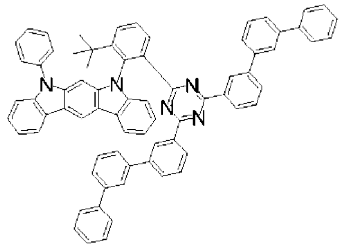
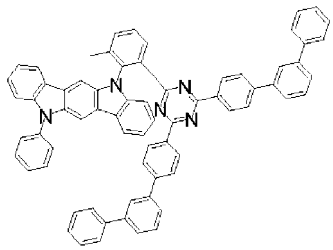
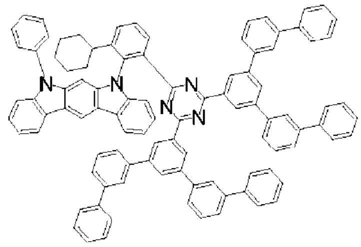
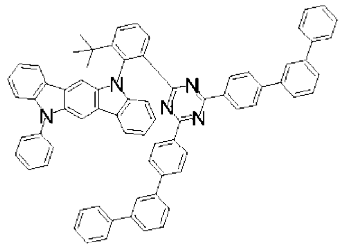
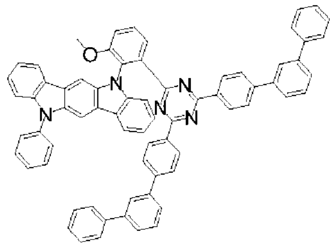
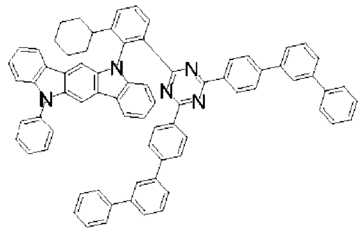
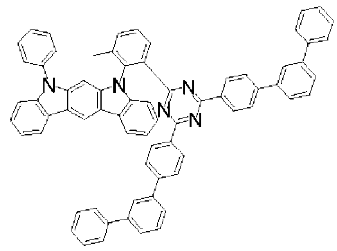
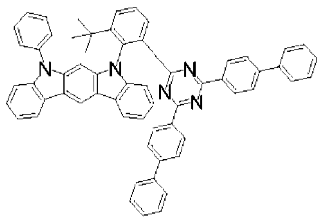
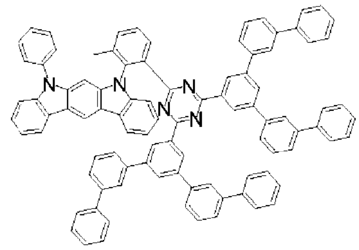
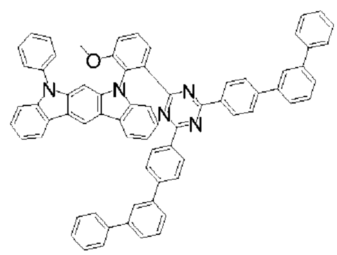
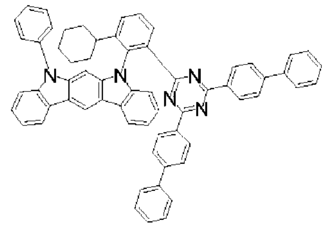

[Chem. 151]

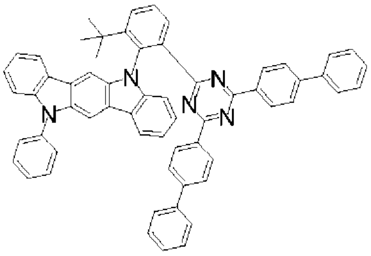

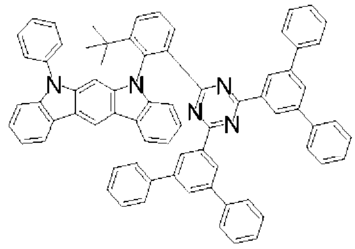

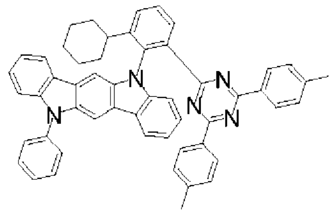

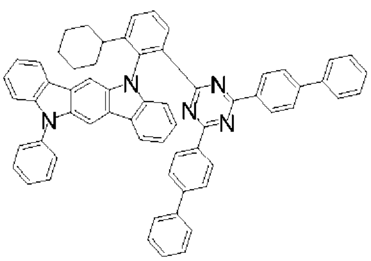

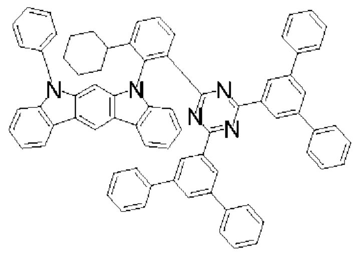

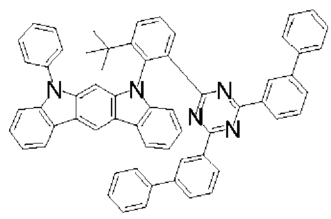

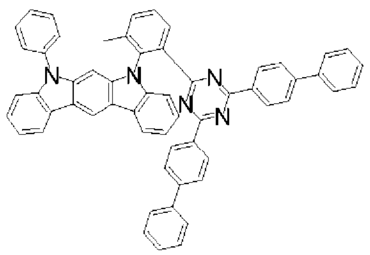

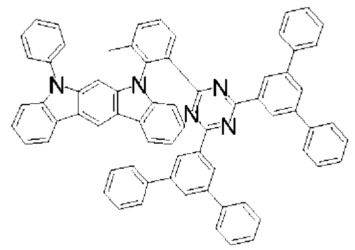

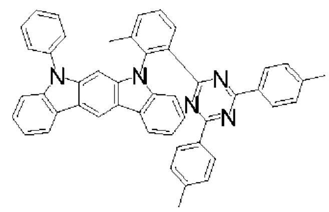

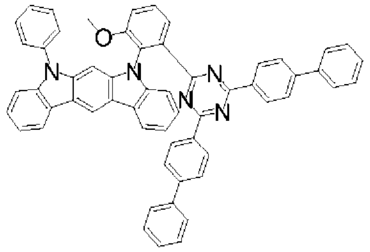

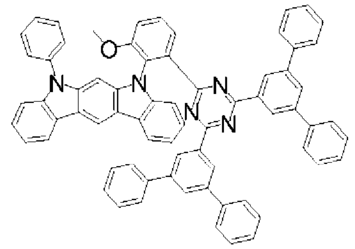

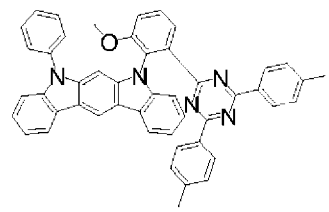

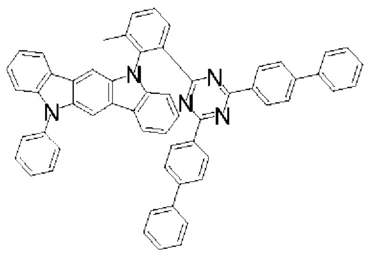

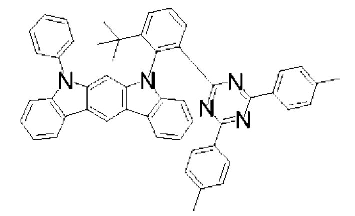

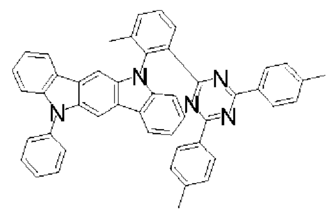

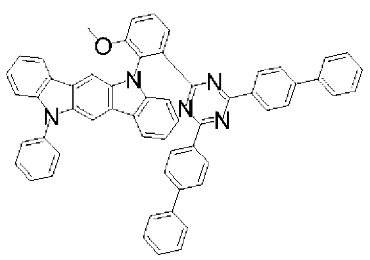

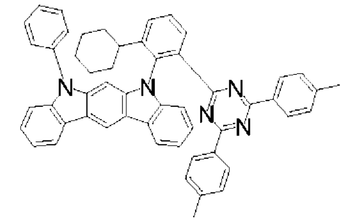

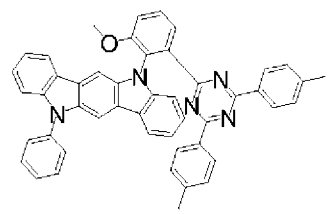

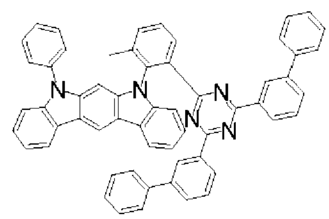

[Chem. 152]

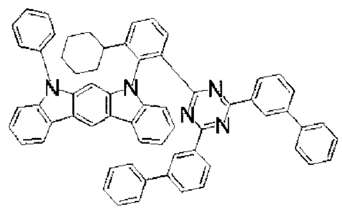
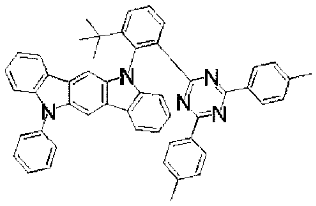
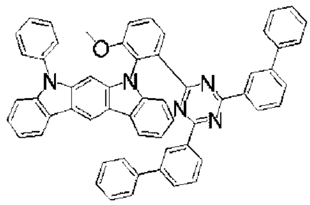
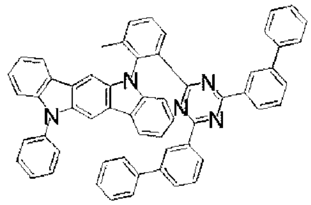
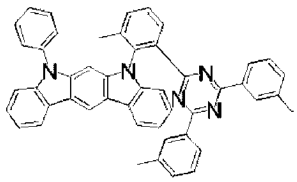
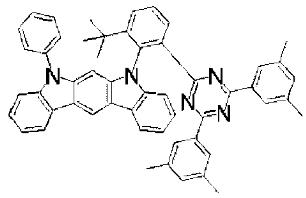
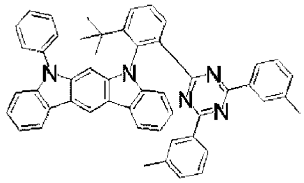
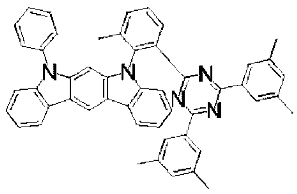
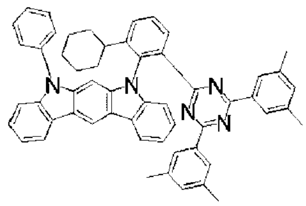
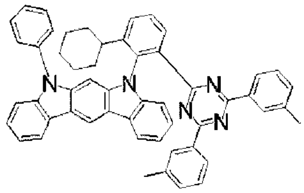
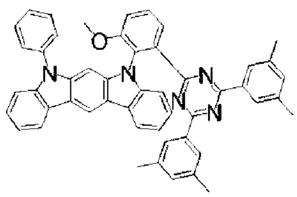
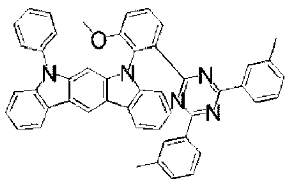
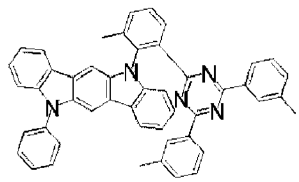

[Chem. 153]

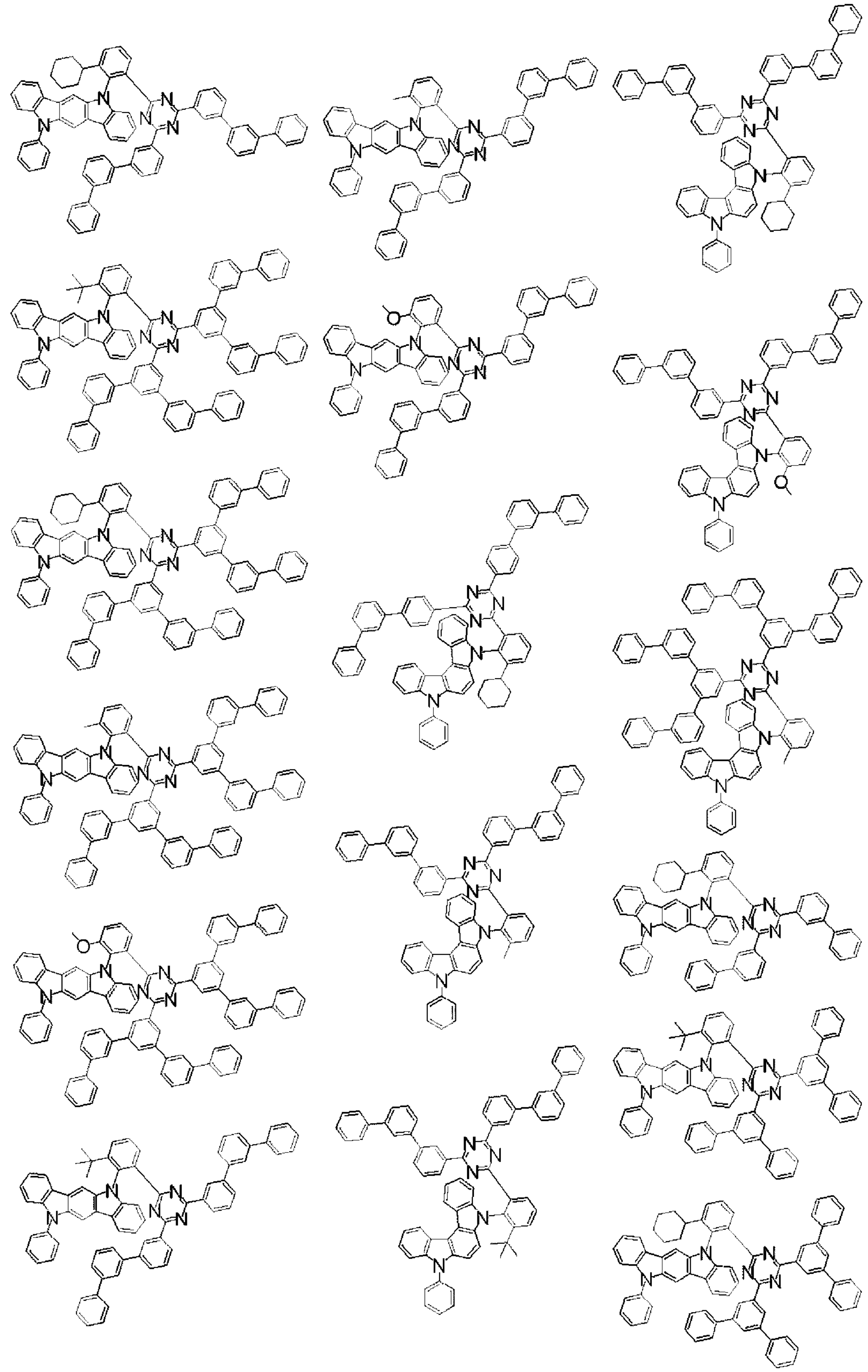

[Chem. 154]

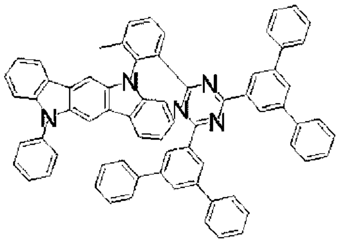
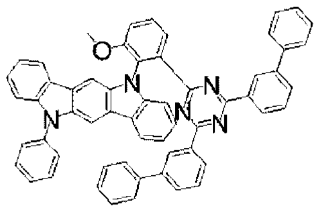
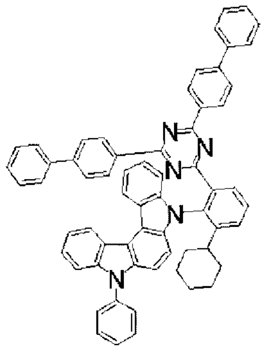
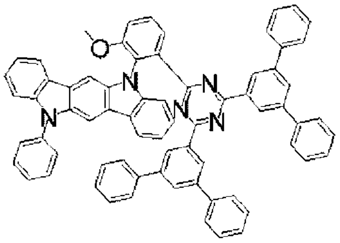
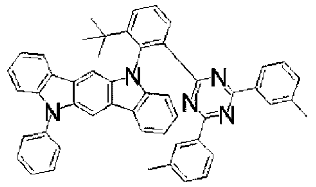
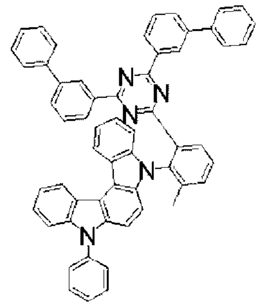
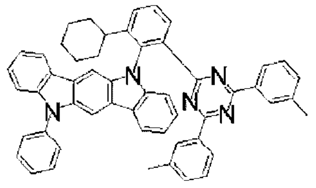
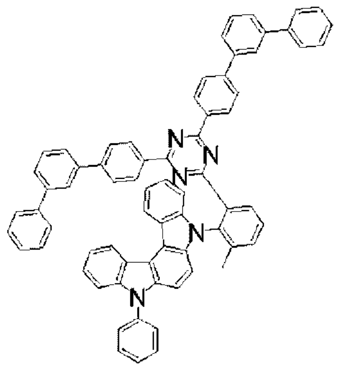
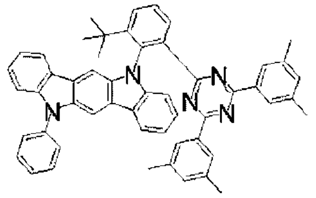
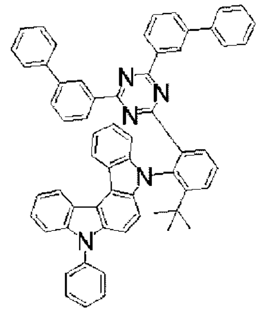
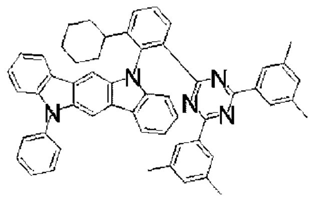
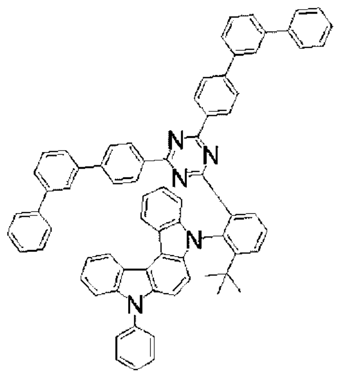
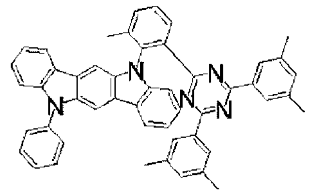
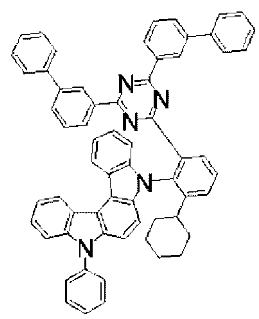
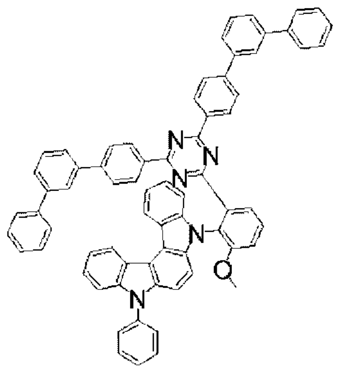
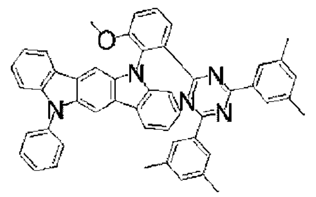

[Chem. 155]

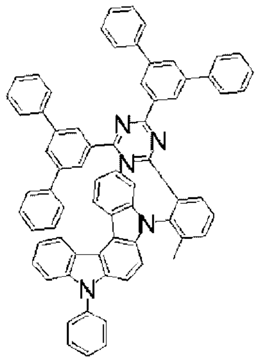
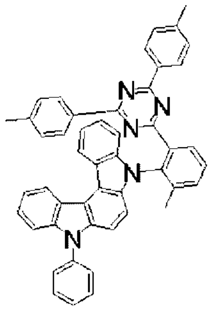
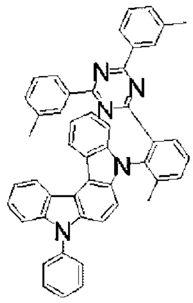
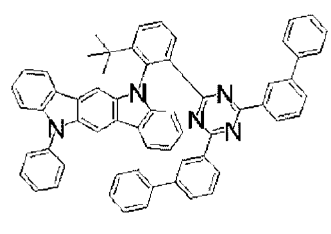
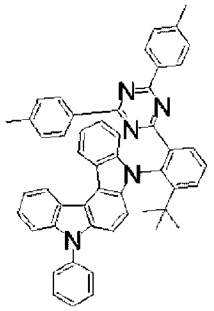
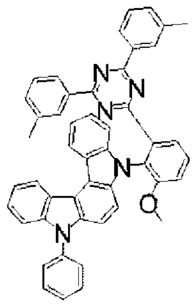
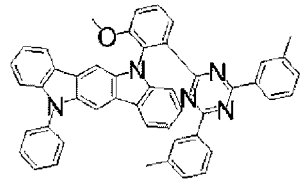
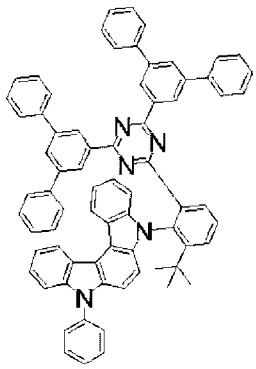
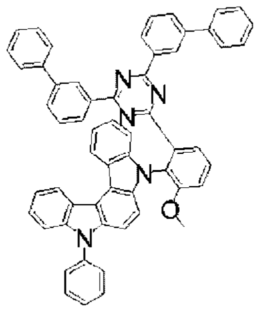
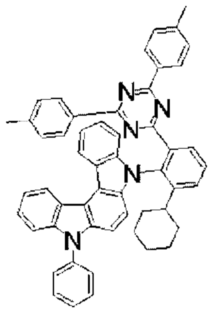
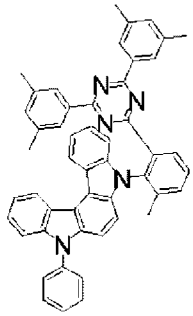
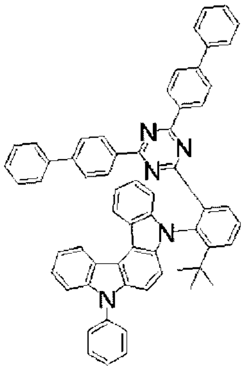
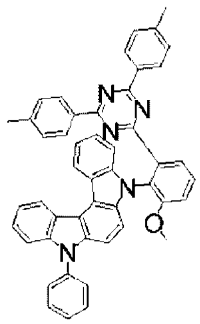
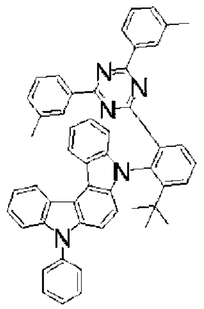
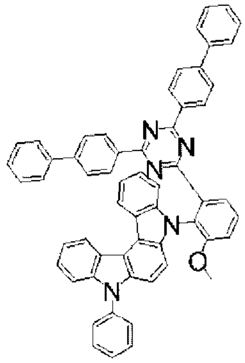
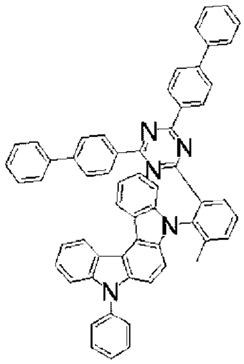
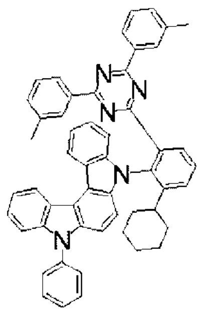

[Chem. 156]

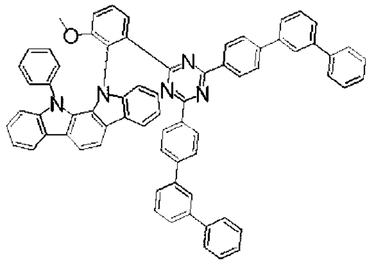
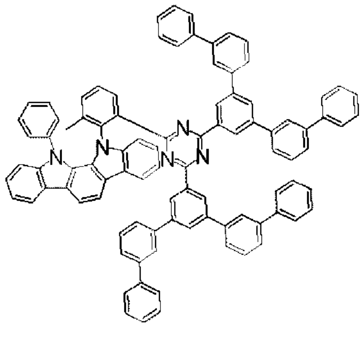
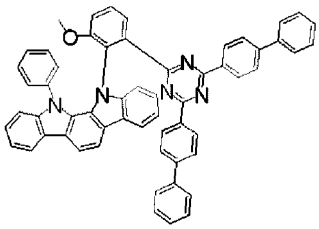
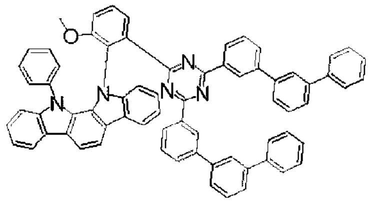
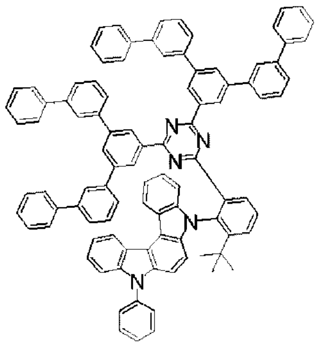
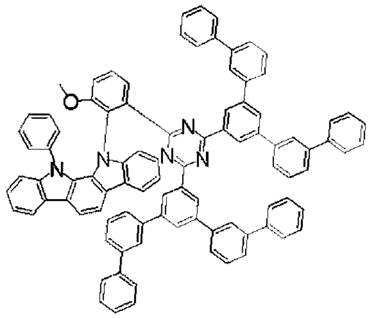
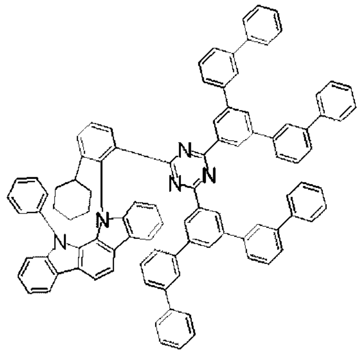
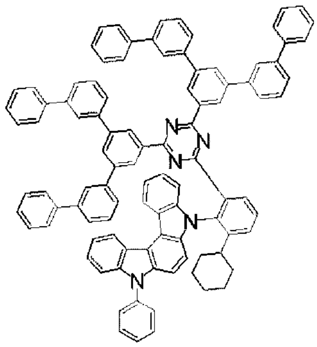
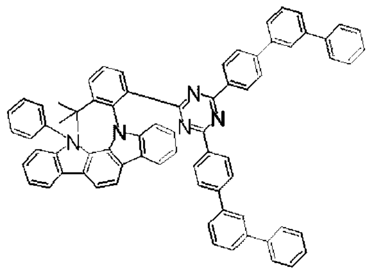
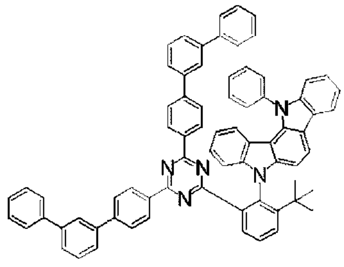
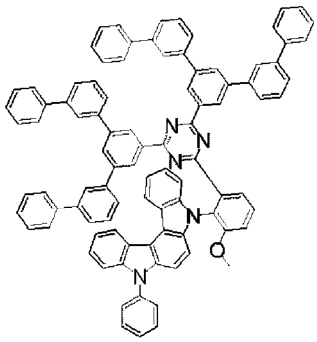
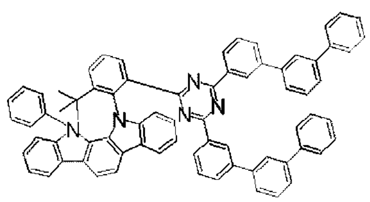
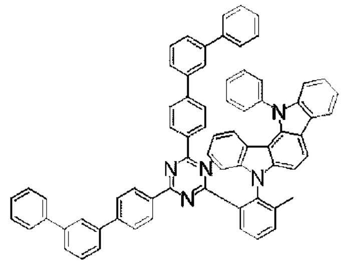
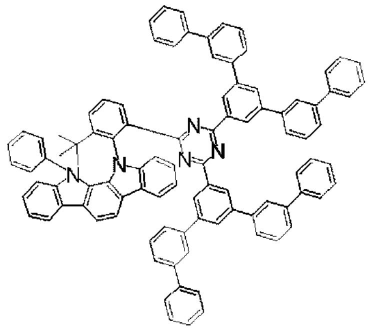
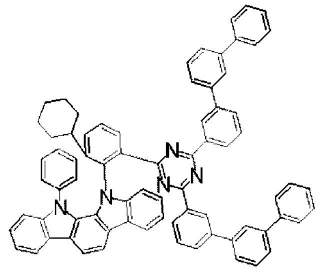
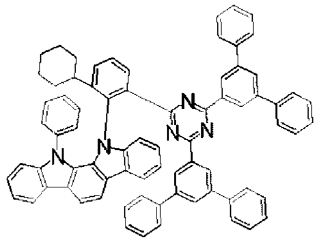

[Chem. 157]

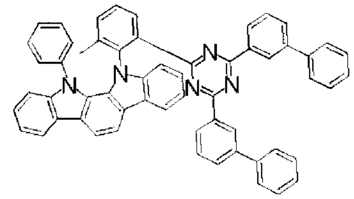
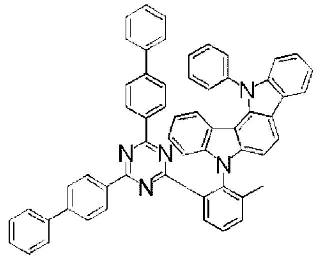
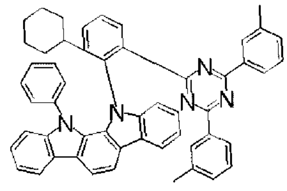
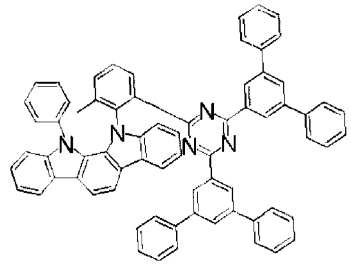
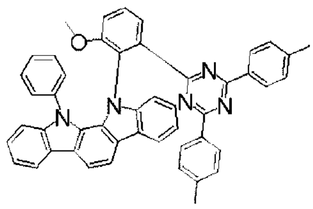
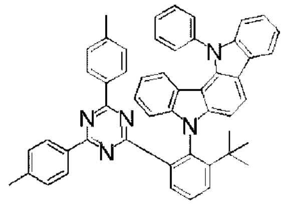
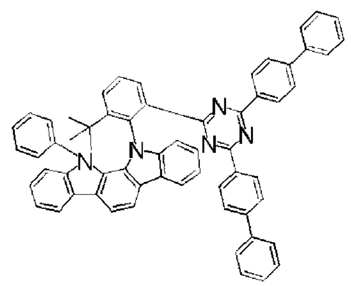
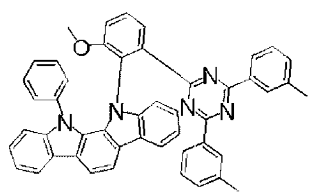
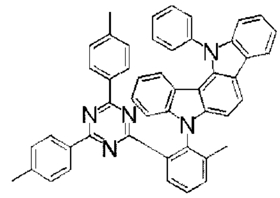
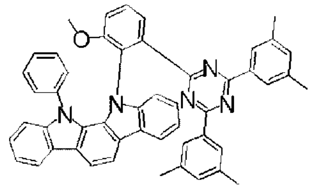
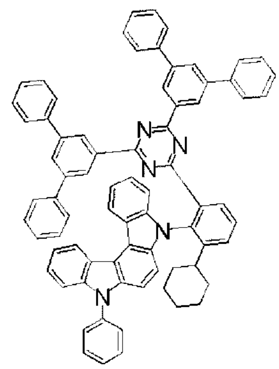
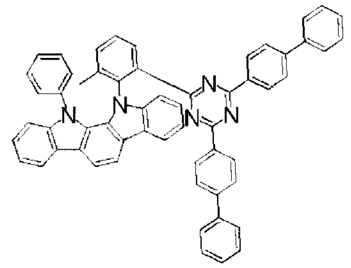
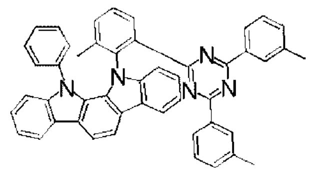
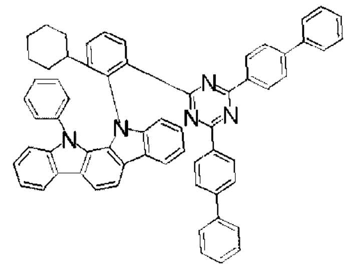
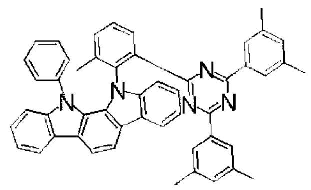
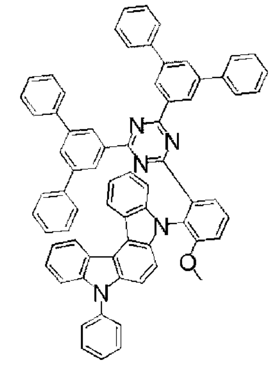
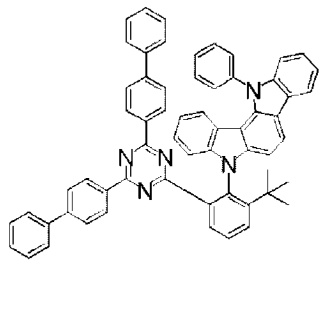
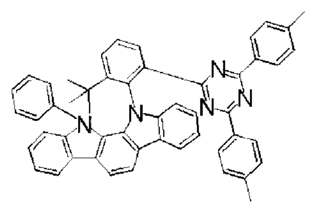

[Chem. 158]

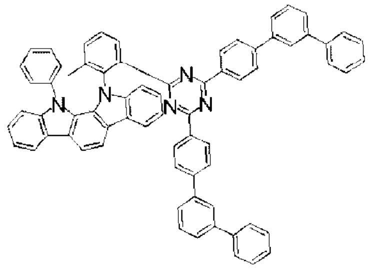
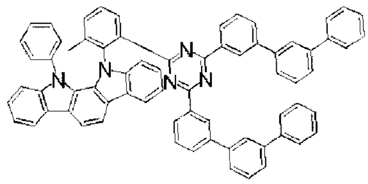
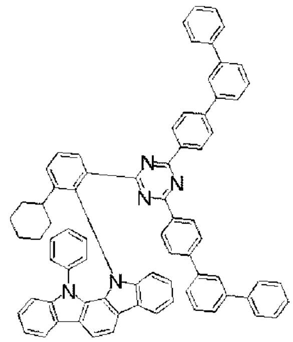
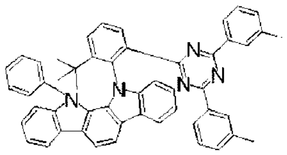
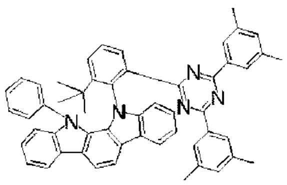
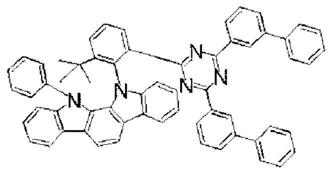
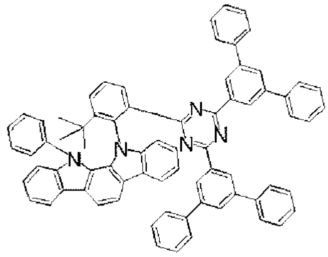
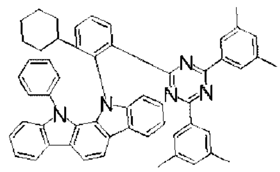
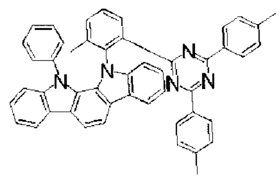
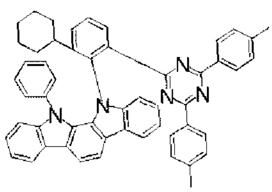
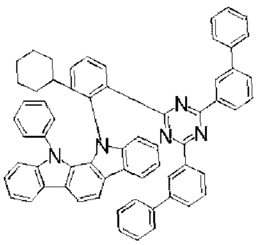
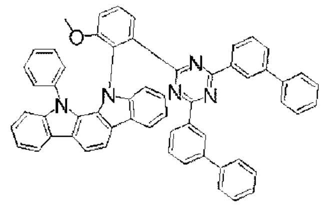
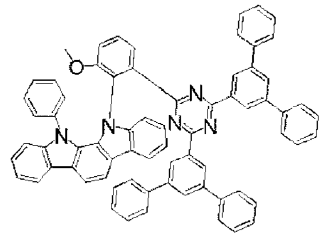
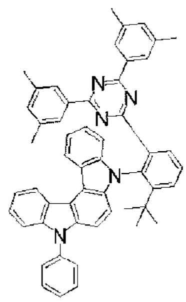
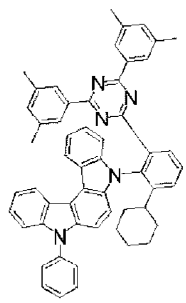
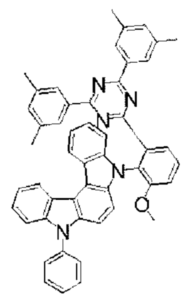

[Chem. 159]

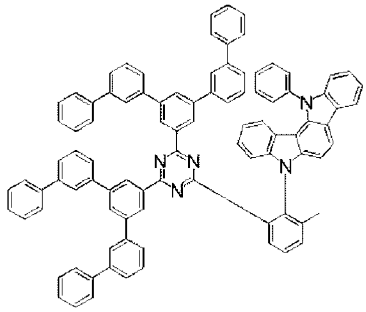
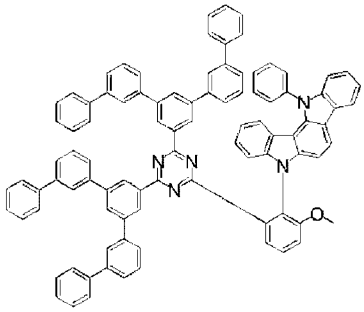
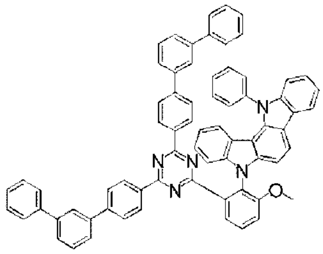
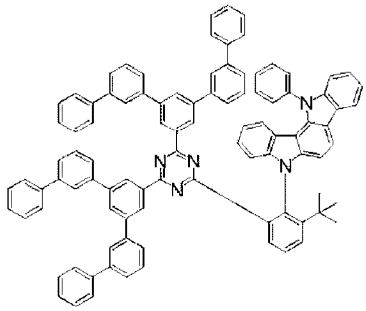
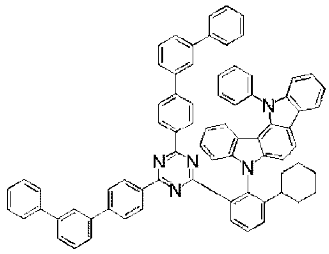
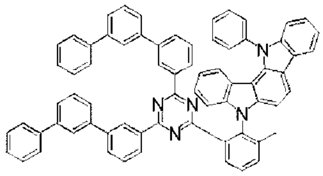
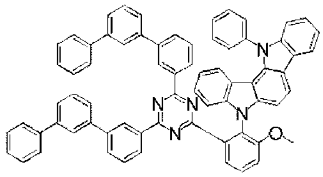
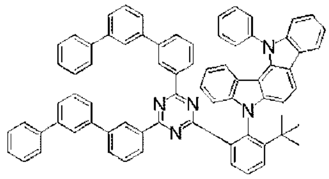
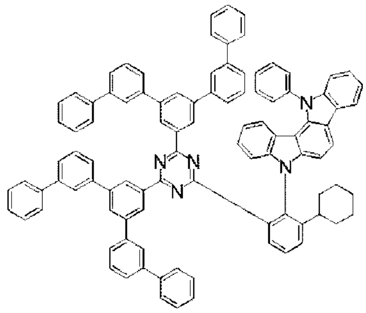
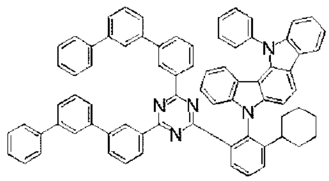
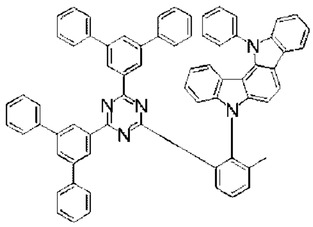

[Chem. 160]

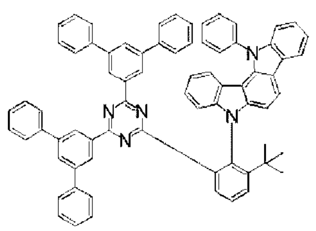
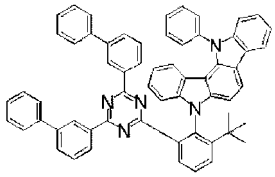
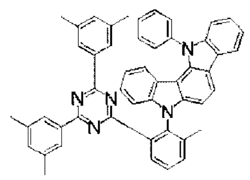
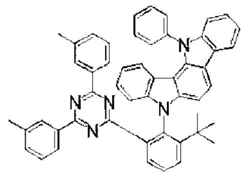
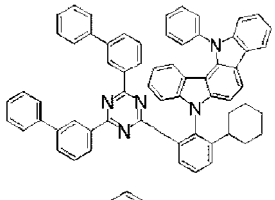
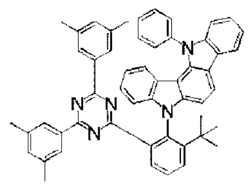
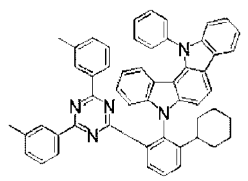
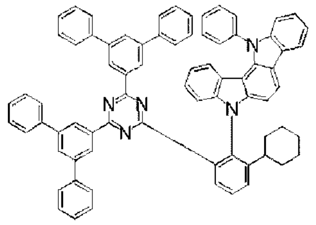
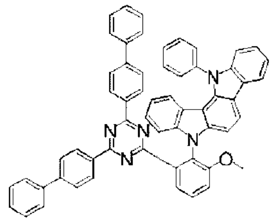
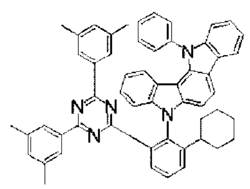
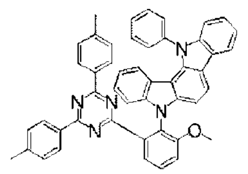
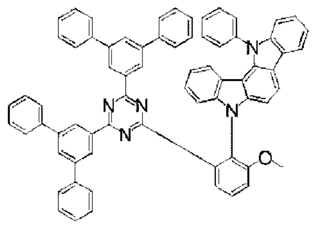
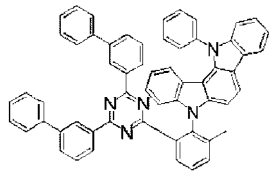
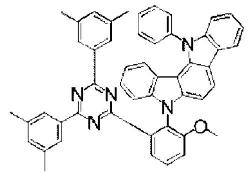
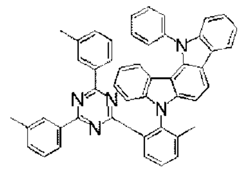
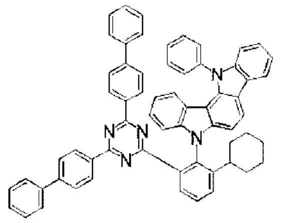
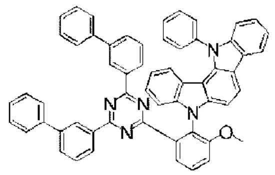
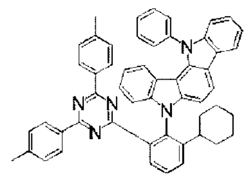
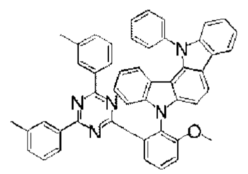

[Chem. 161]

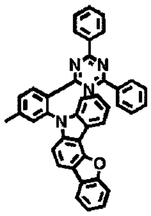
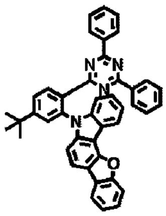
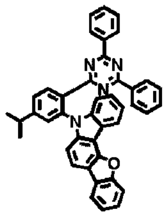
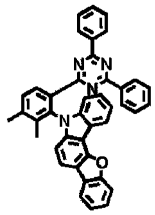
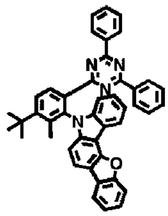
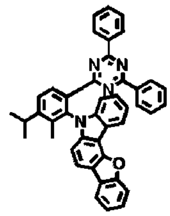

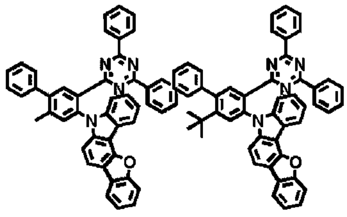
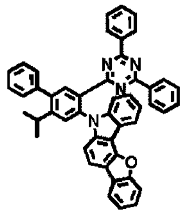
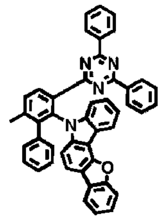
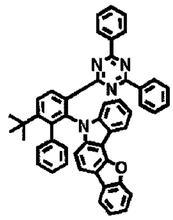

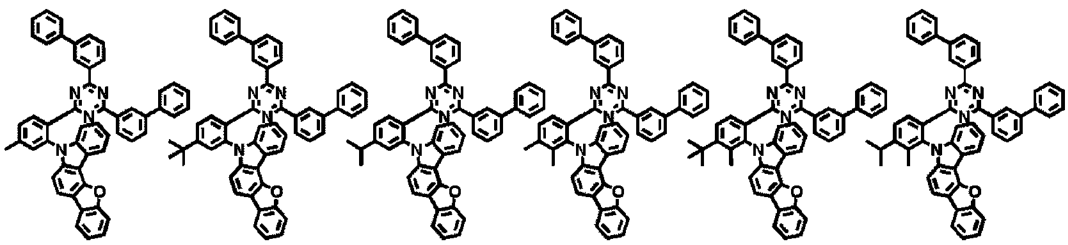

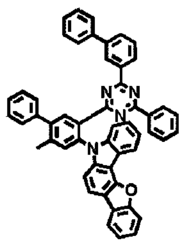
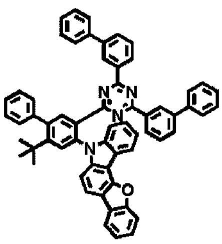
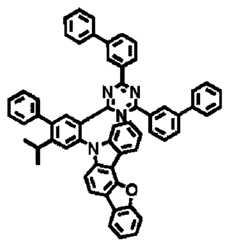
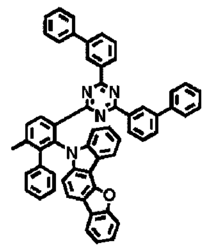
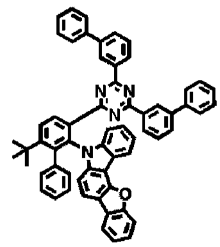

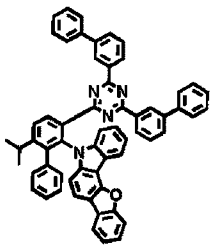
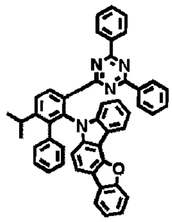
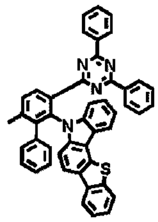
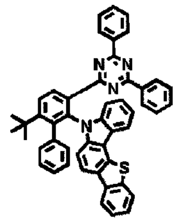
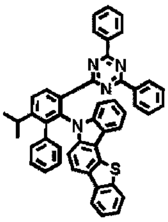

(2) Organic Light-Emitting Element

Next, an organic light-emitting element according to the present embodiment will be described. The organic light-emitting element according to the present embodiment includes a first electrode, a second electrode, and an organic compound layer disposed between the electrodes. One of the first electrode and the second electrode is an anode, and the other is a cathode. In the organic light-emitting element according to the present embodiment, the organic compound layer may be a single layer or a stack of a plurality of layers as long as the organic compound layer includes a light-emitting layer. The organic compound according to the present invention may be contained in the organic compound layer, and is preferably contained in the light-emitting layer. When the organic compound layer is a stack of a plurality of layers, the organic compound layer may include, in addition to the light-emitting layer, a hole injection layer, a hole transport layer, an electron blocking layer, a hole/exciton blocking layer, an electron transport layer, an electron injection layer, and the like. The light-emitting layer may be a single layer or a stack of a plurality of layers. When the light-emitting layer includes a plurality of layers, a charge generation layer may be disposed between the light-emitting layers. The charge generation layer may be composed of a compound having a LUMO energy level lower than the LUMO energy level of the hole transport layer, and the LUMO energy level of the charge generation layer may be lower than the HOMO energy level of the

hole transport layer. The HOMO energy level and the LUMO energy level of the organic compound layer may be the HOMO energy level and the LUMO energy level of an organic compound having the highest weight ratio in the organic compound layer.

HOMO energy levels and LUMO energy levels closer to the vacuum level are described as being "higher". When the LUMO energy level of the charge generation layer is lower than the HOMO energy level of the hole transport layer, it indicates that the LUMO energy level of the charge generation layer is farther from the vacuum level than the HOMO energy level of the hole transport layer is.

In the present specification, the HOMO energy level and the LUMO energy level can be calculated using molecular orbital calculations. The molecular orbital calculations are performed by Density Functional Theory (DFT) or the like, and may be performed using, for example, the B3LYP functional and the 6-31G* basis set. The molecular orbital calculations can be performed using, for example, Gaussian 09 (Gaussian 09, Revision C.01, M. J. Frisch, G. W. Trucks, H. B. Schlegel, G. E. Scuseria, M. A. Robb, J. R. Cheeseman, G. Scalmani, V. Barone, B. Mennucci, G. A. Petersson, H. Nakatsuji, M. Caricato, X. Li, H. P. Hratchian, A. F. Izmaylov, J. Bloino, G. Zheng, J. L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J. A. Montgomery, Jr., J. E. Peralta, F. Ogliaro, M. Bearpark, J. J. Heyd, E. Brothers, K. N. Kudin, V. N. Staroverov, T. Keith, R. Kobayashi, J. Normand, K. Raghavachari, A. Rendell, J. C. Burant, S. S. Iyengar, J. Tomasi, M. Cossi, N. Rega, J. M. Millam, M. Klene, J. E. Knox, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, R. L. Martin, K. Morokuma, V. G. Zakrzewski, G. A. Voth, P. Salvador, J. J. Dannenberg, S. Dapprich, A. D. Daniels, O. Farkas, J. B. Foresman, J. V. Ortiz, J. Cioslowski, and D. J. Fox, Gaussian, Inc., Wallingford CT, 2010.).

The HOMO energy level and the LUMO energy level in the present specification can be calculated using an ionization potential and a band gap. The HOMO energy level can be estimated by measuring an ionization potential. The ionization potential can be measured with a measurement apparatus such as AC-3 after a compound to be measured has been dissolved in a solvent such as toluene or a vapor-deposited film of the compound to be measured has been formed on a substrate such as glass. The band gap can be measured by a measurement in which a compound to be measured is dissolved in a solvent such as toluene and irradiated with excitation light. The band gap can be determined by measuring the absorption edge of an absorption spectrum of the excitation light. Alternatively, the band gap can be measured by depositing the compound to be measured on a substrate such as glass and applying excitation light to the vapor-deposited film. In the measurement, the absorption edge of an absorption spectrum in which the vapor-deposited film absorbs the excitation light is measured, whereby the band gap can be determined.

The LUMO energy level can be calculated using values of the band gap and the ionization potential. By subtracting the value of the ionization potential from the value of the band gap, the LUMO energy level can be estimated.

The LUMO energy level can also be estimated from a reduction potential. For example, a one-electron reduction potential is estimated using CV (cyclic voltammetry) measurement. The CV measurement can be performed, for example, in a 0.1 M solution of tetrabutylammonium perchlorate in DMF using $Ag/Ag^+$ as a reference electrode, Pt as a counter electrode, and glassy carbon as a working electrode. The LUMO energy level can be estimated by adding the difference between the obtained reduction potential of the compound and the reduction potential of ferrocene to - 4.8 eV.

In the organic light-emitting element according to an embodiment of the present invention, when the organic compound according to the present invention is contained in the light-emitting layer, the light-emitting layer may be a layer formed only of the organic compound according to the present invention or a layer formed of the organic compound according to the present invention and other compounds. When the light-emitting layer is a layer formed of the organic compound according to the present invention and other compounds, the organic compound according to the present invention may be used as a host material or a guest material of the light-emitting layer. The organic compound may also be used as an assist material that can be contained in the light-emitting layer. The host material, which is also referred to as a "host" or a "first compound", is a compound having the largest mass ratio among the compounds constituting the light-emitting layer. The guest material, which is also referred to as a "guest", a "dopant material", a "dopant", or a "third compound", is a compound having a mass ratio smaller than that of the host among the compounds constituting the light-emitting layer and is a compound responsible for main light emission. Thus, the guest material may be referred to as a light-emitting material. In the present embodiment, the guest material may be a fluorescent material. The assist material, which is also referred to as an "assist" or a "second compound", is a compound that has a mass ratio smaller than that of the host material among the compounds constituting the light-emitting layer and that assists the light emission of the guest material. The assist material is also referred to as a second host.

Here, the lowest excited singlet energy of the host material is defined as S1 (H), the lowest excited singlet energy of the guest material as S1 (D), and the lowest excited singlet energy of the assist material as S1 (A). The organic compound according to the present invention may be any of the guest material, the assist material, and the host material. At this time, the organic light-emitting element according to the present embodiment preferably satisfies S1 (H) > S1 (D) or S1 (H) > S1 (A) > S1 (D). When the lowest excited singlet energies of the compounds contained in the organic light-emitting element according to the present embodiment satisfy the above relationship, excitons can be efficiently transferred to the

guest material, so that the organic light-emitting element has higher luminous efficiency.

When the organic compound according to the present invention is used in the light-emitting layer, the concentration of the organic compound according to the present invention may be 0.01 mass% or more and 99 mass% or less relative to the whole light-emitting layer, and is preferably 10 mass% or more and 50 mass% or less. When the light-emitting layer contains the guest material, the assist material, and the host material, the mass of the organic compound according to the present invention may be 0.01 mass% or more and 99 mass% or less relative to the whole light-emitting layer, and is preferably 10 mass% or more and 50 mass% or less.

The present inventors have conducted various studies and found that when the organic compound according to the present invention is used in the light-emitting layer, an element that outputs light with high efficiency and high luminance and has very high durability can be provided. This light-emitting layer may have a single-layer structure or a multilayer structure and can also contain a light-emitting material having any other emission color so as to emit a mixed color. The multilayer structure refers to a state in which the light-emitting layer and another light-emitting layer are stacked on top of each other. In this case, the emission color of the organic light-emitting element is not limited to a single color. More specifically, the emission color may be white or an intermediate color. In the case of white, when the light-emitting layer emits blue light, the other light-emitting layer emits light of a color other than blue, that is, red or green. The light-emitting layer is formed by vapor deposition or coating.

The organic compound according to the present invention can be used as a constituent material of an organic compound layer other than the light-emitting layer constituting the organic light-emitting element according to the present embodiment. Specifically, the organic compound may be used as a constituent material of, for example, the electron transport layer, the electron injection layer, the hole transport layer, the hole injection layer, or the hole blocking layer. In this case, the emission color of the organic light-emitting element is not limited to a single color. More specifically, the emission color may be white or an intermediate color.

(3) Other Compounds

In addition to the organic compound according to the present invention, conventionally known low-molecular-weight and high-molecular-weight hole injection compounds or hole transport compounds, host materials, luminescent compounds, electron injection compounds or electron transport compounds, and the like can optionally be used in combination. Examples of these compounds will be described below.

As hole injection and transport materials, materials that facilitate injection of holes from the anode and that have so high hole mobility that enables injected holes to be transported to the light-emitting layer are preferred. To suppress, for example, crystallization of the organic compound in the organic light-emitting element, materials having high glass-transition temperatures are preferred. Examples of low-molecular-weight and high-molecular-weight materials having hole injection and transport properties include triarylamine derivatives, arylcarbazole derivatives, phenylenediamine derivatives, stilbene derivatives, phthalocyanine derivatives, porphyrin derivatives, poly(vinylcarbazole), poly(thiophene), and other conductive polymers. These hole injection and transport materials are also suitable for use in the electron blocking layer. When the hole injection layer is formed by a coating method, a mixture of polyethylenedioxythiophene and polystyrene sulfonate (PEDOT:PSS), which is commonly used as a hole injection material, may be used. Non-limiting specific examples of compounds usable as hole injection and transport materials are shown below.

[Chem. 162]

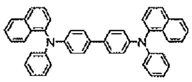
HT1

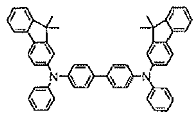
HT2

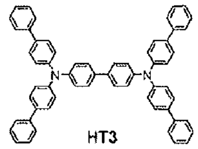
HT3

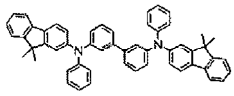
HT4

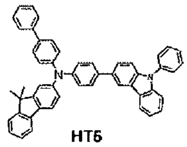
HT5

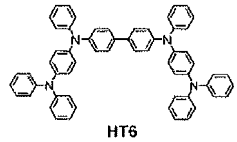
HT6

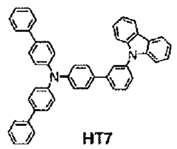
HT7

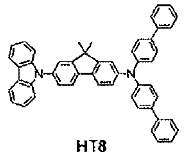
HT8

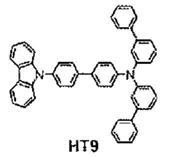
HT9

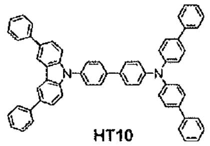
HT10

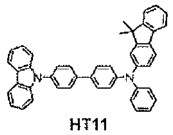
HT11

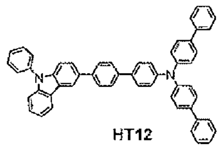
HT12

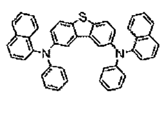
HT13

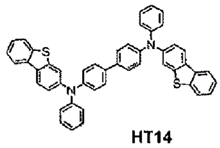
HT14

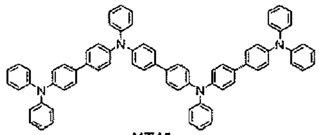
HT15

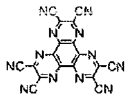
HT16

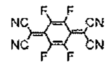
HT17

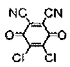
HT18

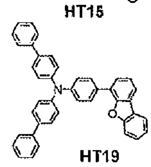
HT19

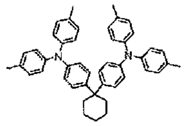
HT20

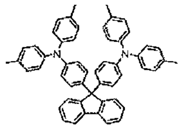
HT21

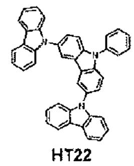
HT22

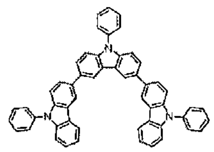
HT23

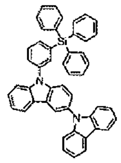
HT24

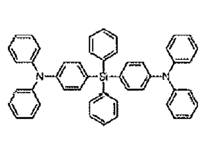
HT26

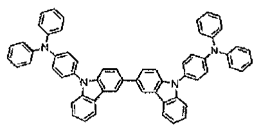
HT26

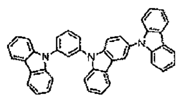
HT27

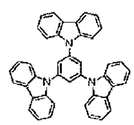
HT28

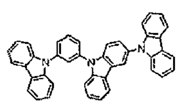
HT29

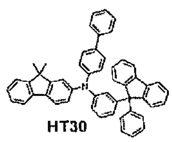
HT30

Among the hole injection and transport materials given above, HT16 to HT18 can be used for a layer in contact with the anode to achieve a lower driving voltage. HT16 is widely used in organic light-emitting elements. HT2 to HT7, HT10, HT12, and HT22 to 28 may be used for an organic compound layer adjacent to HT16. Hole transport polymer compounds such as polyphenylene vinylene (PPV), polyfluorene (PF), polyvinyl carpazole (PVK), and derivatives thereof may be used. Alternatively, for example, insulator layers of inorganics such as SiO2 and SiN and organic silicon polymers

such as siloxane can also be used. A plurality of materials may be used for one organic compound layer.

Examples of guest materials mainly involved in the light-emitting function include donor-acceptor type organic compounds, boron-containing complexes, indocarbazole fused-ring compounds, fused-ring compounds (e.g., fluorene derivatives, naphthalene derivatives, pyrene derivatives, perylene derivatives, tetracene derivatives, anthracene derivatives, and rubrene), quinacridone derivatives, coumarin derivatives, stilbene derivatives, organic aluminum complexes such as tris(8-quinolinolato)aluminum, iridium complexes, platinum complexes, rhenium complexes, copper complexes, europium complexes, ruthenium complexes, and polymer derivatives such as poly(phenylenevinylene) derivatives, poly(fluorene) derivatives, and poly(phenylene) derivatives. When the light-emitting layer is produced by a coating method, a polymer compound having light-emitting properties is mainly used. This is because polymer compounds tend to exhibit high glass transition temperatures and thus are less likely to undergo crystallization than low-molecular-weight compounds. Examples of materials that are specifically used include polymer compounds such as polyphenylene vinylene (PPV), polyfluorene (PF), polyvinyl carpazole (PVK), and derivatives thereof.

Non-limiting specific examples of compounds usable as light-emitting materials are shown below.

[Chem. 163]

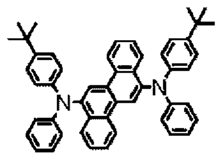
BD1
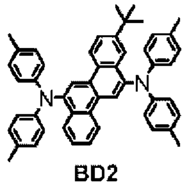
BD2
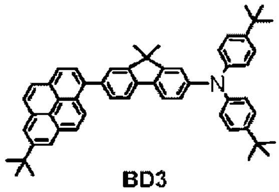
BD3
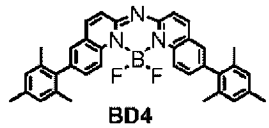
BD4

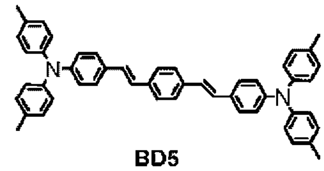
BD5
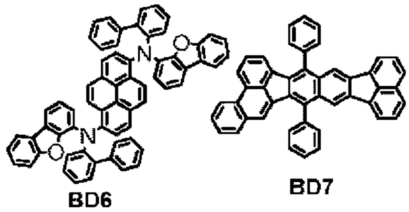
BD6
BD7
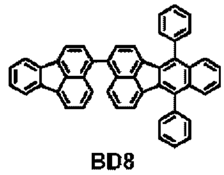
BD8

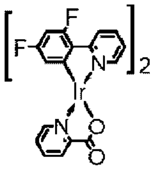
BD9
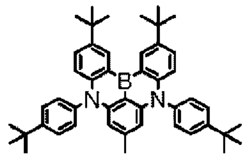
BD10
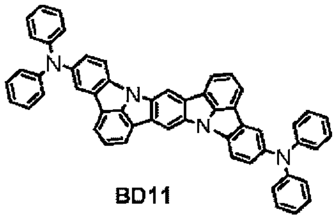
BD11

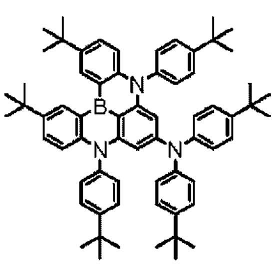
BD12
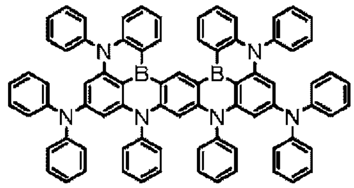
BD13
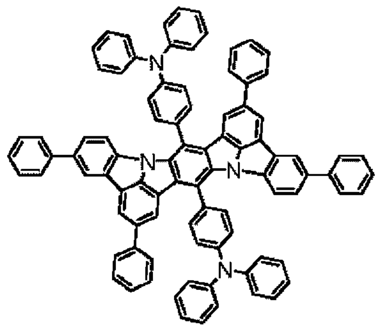
BD14

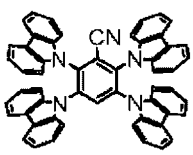
BD15
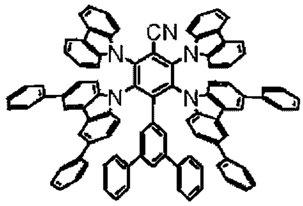
BD16
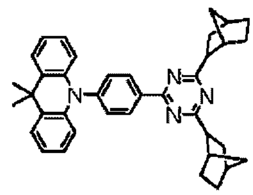
BD17

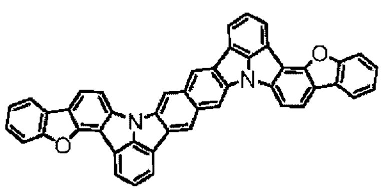
BD18
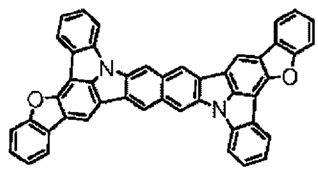
BD19

[Chem. 164]

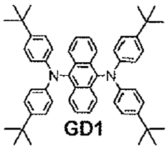
GD1
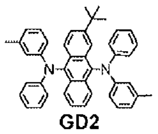
GD2
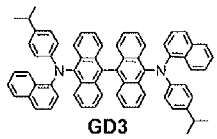
GD3
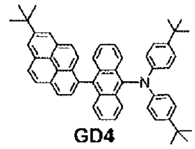
GD4
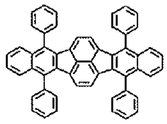
GD5
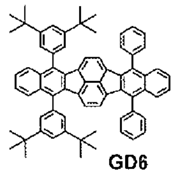
GD6
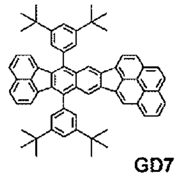
GD7
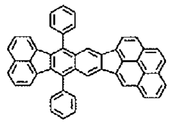
GD8
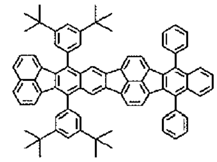
GD9
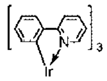
GD10
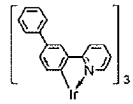
GD11
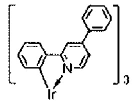
GD12
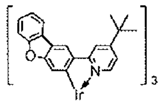
GD13
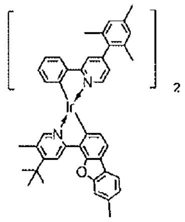
GD14
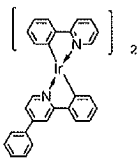
GD15
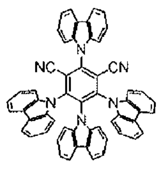
GD16
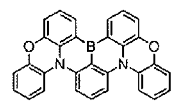
GD17
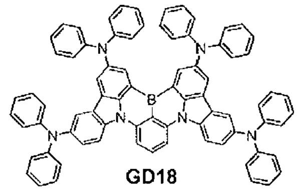
GD18
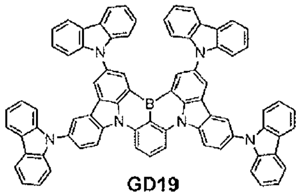
GD19
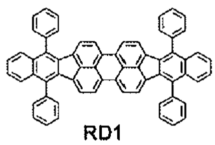
RD1
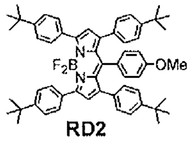
RD2
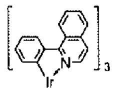
RD3
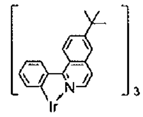
RD4
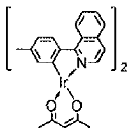
RD5
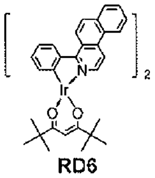
RD6
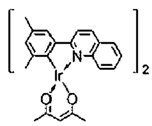
RD7
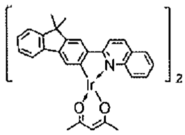
RD8
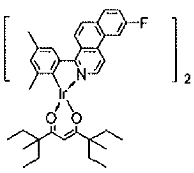
RD9
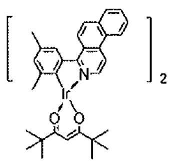
RD10
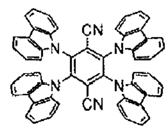
RD11

Non-limiting specific examples of compounds usable as host materials or assist materials contained in the light-emitting layer are shown below.

[Chem. 165]

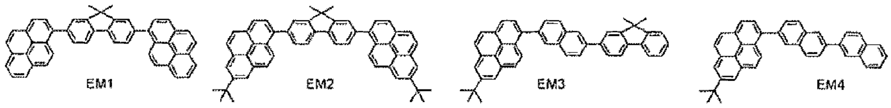

EM1 EM2 EM3 EM4

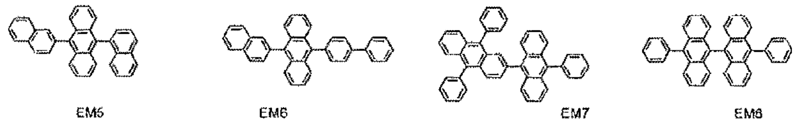

EM5 EM6 EM7 EM8

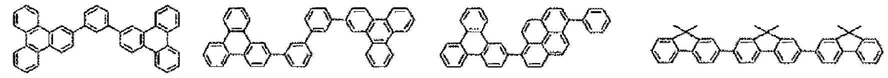

EM9 EM10 EM11 EM12

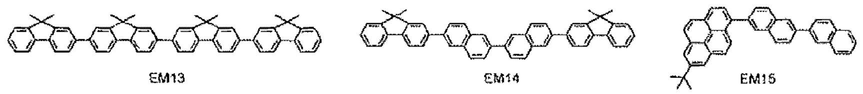

EM13 EM14 EM15

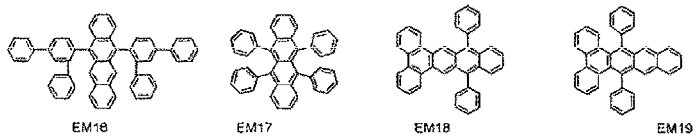

EM16 EM17 EM18 EM19

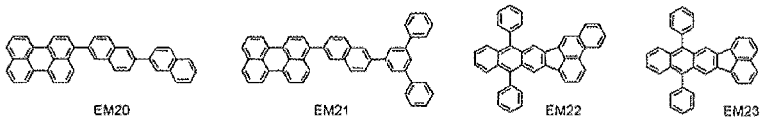

EM20 EM21 EM22 EM23

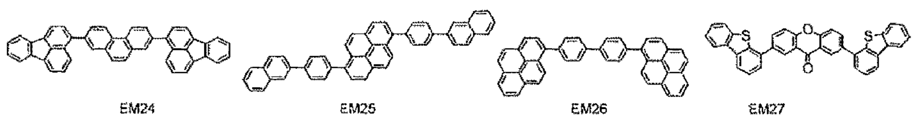

EM24 EM25 EM26 EM27

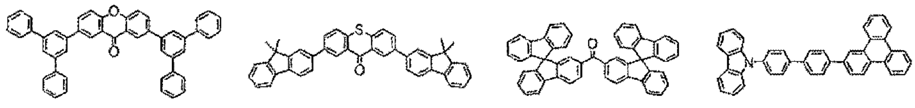

EM28 EM29 EM30 EM31

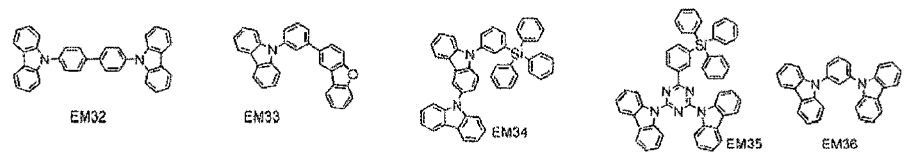

EM32 EM33 EM34 EM35 EM36

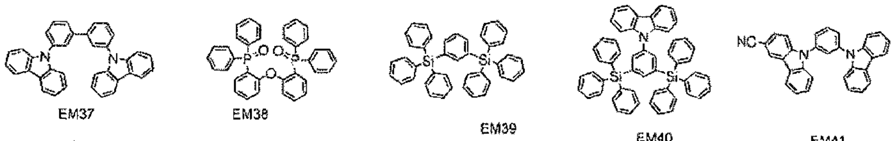

EM37 EM38 EM39 EM40 EM41

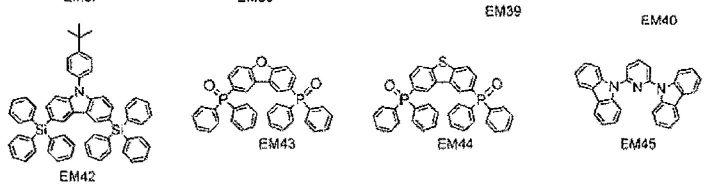

EM42 EM43 EM44 EM45

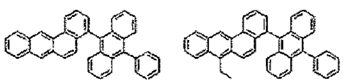

EM46 EM47

Any electron transport material capable of transporting electrons injected from the cathode to the light-emitting layer can be freely selected in consideration of, for example, the balance with the hole mobility of a hole transport material. Examples of materials capable of transporting electrons include oxadiazole derivatives, oxazole derivatives, pyrazine derivatives, triazole derivatives, triazine derivatives, quinoline derivatives, quinoxaline derivatives, phenanthroline

derivatives, organic aluminum complexes, and fused-ring compounds (e.g., fluorene derivatives, naphthalene derivatives, chrysene derivatives, and anthracene derivatives). These electron transport materials are also suitable for use for the hole blocking layer.

Non-limiting specific examples of compounds usable as electron transport materials are shown below.

[Chem. 166]

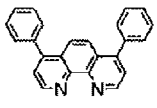
ET1
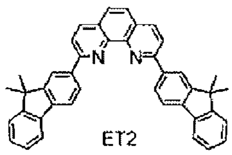
ET2
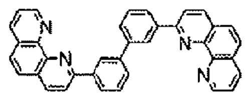
ET3
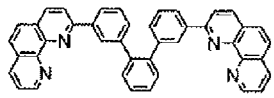
ET4
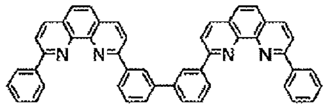
ET5
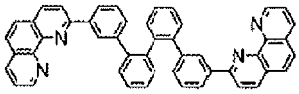
ET6
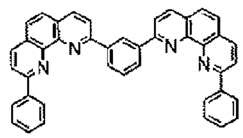
ET7
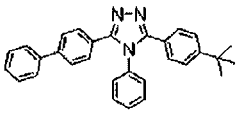
ET8
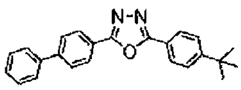
ET9
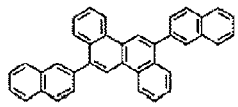
ET10
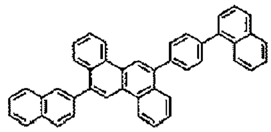
ET11
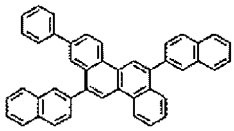
ET12
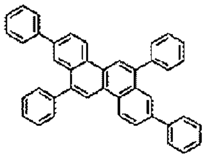
ET13
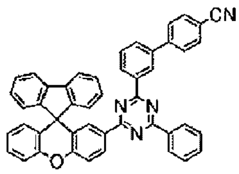
ET14
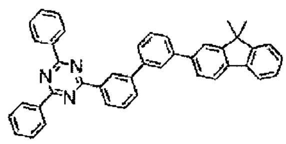
ET15
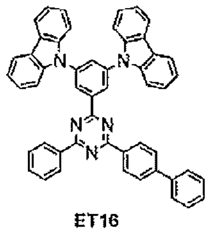
ET16
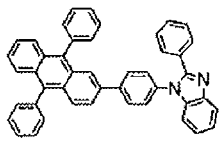
ET17
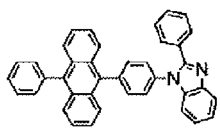
ET18
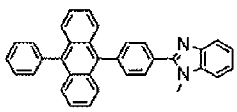
ET19
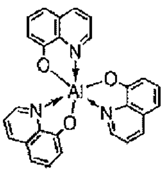
ET20
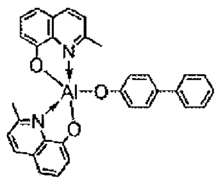
ET21
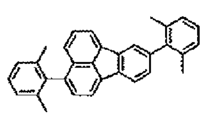
ET22
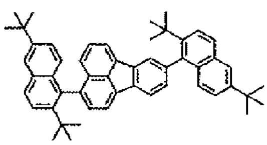
ET23
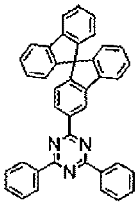
ET24
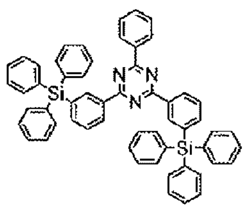
ET25
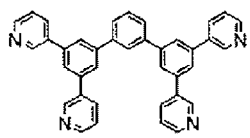
ET26

Any electron injection material capable of readily injecting electrons from the cathode can be freely selected in consideration of, for example, the balance with hole injectability. An n-type dopant and a reducing dopant are also

contained as organic compounds. Examples include alkali metal-containing compounds such as lithium fluoride, lithium complexes such as lithium quinolinol, benzimidazolidene derivatives, imidazolidene derivatives, fulvalene derivatives, and acridine derivatives.

These can also be used in combination with the electron transport materials above.

The organic compound according to the present invention can also be used in the form of an ink composition.

An ink composition according to the present embodiment contains at least one compound represented by general formula (1-1) or (1-2). Using the ink composition according to the present embodiment enables the layers formed of organic compounds constituting the organic light-emitting element, particularly, the light-emitting layer, to be formed by a coating method, so that a large-area element can be easily produced at a relatively low cost. Examples of solvents that dissolve the compound represented by general formula (1-1) or (1-2) include toluene, xylene, mesitylene, dioxane, methylnaphthalene, tetrahydrofuran, diglyme, 1,2-dichlorobenzene, and 1,2-dichloropropane. These solvents can be used alone or in combination of two or more. Of these, those having an appropriate evaporation rate, specifically, solvents having a boiling point of about 70°C to 200°C are preferably used because a thin film having a uniform thickness is easily provided. The ink composition according to the present embodiment may further contain a compound that serves as an additive. Examples of the compound that serves as an additive include the above-described known light-emitting layer hosts or light-emission assist materials, hole transport materials, light-emitting materials, and electron transport materials.

The concentration of the compound represented by general formula (1-1) or (1-2) in the ink composition according to the present embodiment is preferably 0.05 wt% or more and 50 wt% or less, more preferably 10 wt% or more and 30 wt% or less, relative to the whole composition.

The ink composition according to the present embodiment can be formed into a film by a spin coating method, a bar coating method, a slit coating method, an ink-jet method, a nozzle coating method, a casting method, a gravure printing method, or the like, whereby an organic layer of the organic light-emitting element described below can be formed.

(4) Configuration of Organic Light-Emitting Element

Hereinafter, constituent members constituting the organic light-emitting element according to the present embodiment will be described.

The organic light-emitting element is provided by forming an insulating layer, a first electrode, an organic compound layer, and a second electrode on a substrate. On the second electrode, a protective layer, a color filter, a microlens, etc. may be disposed. When the color filter is disposed, a planarization layer may be disposed between the color filter and the protective layer. The planarization layer may be formed of, for example, an acrylic resin. This also applies to the case where the planarization layer is disposed between the color filter and the microlens.

[Substrate]

Examples of the substrate include quartz, glass, silicon wafers, resins, and metals. The substrate may have switching elements, such as transistors, and wiring lines disposed thereon, and may have an insulating layer disposed thereon. The insulating layer may be made of any material as long as a contact hole can be formed therein so as to allow formation of a wiring line connecting to the first electrode and insulation from unconnected wiring lines can be provided. For example, resins such as polyimide, silicon oxide, silicon nitride, and the like can be used.

[Electrode]

The electrodes may be a pair of electrodes. The pair of electrodes are the first electrode and the second electrode. Specifically, the pair of electrodes may be an anode and a cathode. When an electric field is applied in a direction in which the organic light-emitting element emits light, one of the electrodes at a higher potential is the anode, and the other is the cathode. Stated another way, one of the electrodes that supplies holes to the light-emitting layer is the anode, and the other electrode that supplies electrons to the light-emitting layer is the cathode.

The constituent material of the anode preferably has as high a work function as possible. For example, elemental metals such as gold, platinum, silver, copper, nickel, palladium, cobalt, selenium, vanadium, and tungsten, mixtures containing these metals, alloys obtained by combining these metals, and metal oxides such as tin oxide, zinc oxide, indium oxide, indium tin oxide (ITO), and indium zinc oxide can be used. Conductive polymers such as polyaniline, polypyrrole, and polythiophene can also be used.

These electrode materials may be used alone or in combination of two or more. The anode may be composed of a single layer or a plurality of layers.

When the anode is used as a reflection electrode, for example, chromium, aluminum, silver, titanium, tungsten, molybdenum, an alloy thereof, or a stack thereof can be used. These materials can also be used to function as a reflective film that does not have a role of an electrode. When the anode is used as a transparent electrode, it may be, for example,

but not necessarily, a transparent conductive layer made of an oxide such as indium tin oxide (ITO) or indium zinc oxide. Photolithography can be used to form the electrode.

The constituent material of the cathode preferably has a low work function. Examples of such materials include alkali metals such as lithium, alkaline earth metals such as calcium, elemental metals such as aluminum, titanium, manganese, silver, lead, and chromium, and mixtures thereof. Alloys obtained by combining these elemental metals can also be used. For example, magnesium-silver, aluminum-lithium, aluminum-magnesium, silver-copper, and zinc-silver alloys can be used. Metal oxides such as indium tin oxide (ITO) can also be used. These electrode materials may be used alone or in combination of two or more. The cathode may be composed of a single layer or a plurality of layers. In particular, silver is preferably used, and a silver alloy is more preferred to reduce aggregation of silver. As long as aggregation of silver can be reduced, the content ratio in the alloy is not limited. For example, the ratio of silver to other metals may be, for example, 1:1 or 3:1.

The cathode is not particularly limited; a conductive layer formed of an oxide such as ITO may be used to provide a top-emission element, or a reflection electrode formed of aluminum (Al) or the like may be used to provide a bottom-emission element. The method of forming the cathode is not particularly limited, but the use of DC sputtering, AC sputtering, or the like is more preferred because good film coverage can be achieved and the resistance tends to decrease.

[Organic Compound Layer]

The organic compound layer may be formed of a single layer or a plurality of layers. When the organic compound layer includes a plurality of layers, the layers may be referred to as a hole injection layer, a hole transport layer, an electron blocking layer, a light-emitting layer, a hole blocking layer, an electron transport layer, and an electron injection layer depending on their functions. The organic compound layer is composed mainly of an organic compound and may contain an inorganic atom and an inorganic compound. For example, the organic compound layer may contain copper, lithium, magnesium, aluminum, iridium, platinum, molybdenum, zinc, or the like. The organic compound layer may be disposed between the first electrode and the second electrode and may be disposed in contact with the first electrode and the second electrode.

The organic compound layers (e.g., the hole injection layer, the hole transport layer, the electron blocking layer, the light-emitting layer, the hole blocking layer, the electron transport layer, and the electron injection layer) constituting the organic light-emitting element according to the present embodiment are formed by the following methods.

The organic compound layers constituting the organic light-emitting element according to the present embodiment can be formed using a dry process such as vacuum deposition, ionized deposition, sputtering, or plasma. Instead of the dry process, a wet process in which a solution in an appropriate solvent is used to form a layer by a known coating method (e.g., spin coating, dipping, a casting method, an LB method, or an ink jet method) can also be used.

When the layers are formed by, for example, vacuum deposition or solution coating, the layers are unlikely to undergo crystallization or the like and are highly stable over time. When a coating method is used for film formation, an appropriate binder resin can be used in combination to form a film.

Examples of the binder resin include polyvinylcarbazole resins, polycarbonate resins, polyester resins, ABS resins, acrylic resins, polyimide resins, phenol resins, epoxy resins, silicone resins, and urea resins, but are not limited thereto.

These binder resins may be used alone as a homopolymer or copolymer or may be used as a mixture of two or more. In addition, known additives such as plasticizers, antioxidants, and UV absorbers may be used in combination as required.

[Protective Layer]

A protective layer may be disposed on the cathode. For example, a glass member provided with a moisture absorbent can be bonded onto the cathode to reduce the entry of, for example, water into the organic compound layer, thereby reducing the occurrence of display failure. In another embodiment, a passivation film made of silicon nitride or the like may be disposed on the cathode to reduce the entry of, for example, water into the organic compound layer. For example, the protective layer may be formed in a manner that after the formation of the cathode, the resultant is conveyed to another chamber without breaking the vacuum, and a silicon nitride film having a thickness of 2 $\mu$m is formed by CVD. After the film formation by CVD, atomic layer deposition (ALD) may be performed to form a protective layer. The material of the film formed by ALD is not limited and may be, for example, silicon nitride, silicon oxide, or aluminum oxide. On the film formed by ALD, silicon nitride may further be formed by CVD. The film formed by ALD may have a smaller thickness than the film formed by CVD. Specifically, the thickness of the film formed by ALD may be 50% or less, or even 10% or less of the thickness of the film formed by CVD.

[Color Filter]

A color filter may be disposed on the protective layer. For example, a color filter may be formed on another substrate so as to correspond to the size of the organic light-emitting element and bonded to the substrate having the organic light-emitting element disposed thereon. Alternatively, a color filter may be patterned on the above-described protective layer by photolithography. The color filter may be formed of a polymer.

[Planarization Layer]

A planarization layer may be disposed between the color filter and the protective layer. The planarization layer is disposed for the purpose of reducing unevenness of the underlying layer. The planarization layer may be referred to as a material resin layer without limiting the purpose. The planarization layer may be formed of an organic compound. The organic compound may have a low molecular weight or a high molecular weight, and preferably has a high molecular weight.
The planarization layer may be disposed on opposite surfaces of the color filter, and the constituent materials thereof may be the same or different. Specific examples include polyvinylcarbazole resins, polycarbonate resins, polyester resins, ABS resins, acrylic resins, polyimide resins, phenol resins, epoxy resins, silicone resins, and urea resins.

[Microlens]

The organic light-emitting element according to the present embodiment may include, on its light-emitting side, an optical member such as a microlens. The microlens can be formed of an acrylic resin, an epoxy resin, or the like. The microlens may be used to increase the amount of light extracted from the organic light-emitting element and to control the direction of the extracted light. The microlens may have a hemispherical shape. In the case of a hemispherical shape, among tangents to the hemisphere, there is a tangent parallel to the insulating layer, and the point of contact between this tangent and the hemisphere is the vertex of the microlens. The vertex of the microlens can be determined in the same manner in any sectional view. That is, among tangents to the semicircle of the microlens in a sectional view, there is a tangent parallel to the insulating layer, and the point of contact between this tangent and the semicircle is the vertex of the microlens.
The midpoint of the microlens can also be defined. In a section of the microlens, a line segment from the start point of an arc shape to the start point of another arc shape is imagined, and the midpoint of the line segment can be referred to as the midpoint of the microlens. The section used to determine the vertex and the midpoint may be a section perpendicular to the insulating layer.

[Opposite Substrate]

An opposite substrate may be disposed on the planarization layer. The opposite substrate is disposed at a position opposite to the above-described substrate and thus is referred to as the opposite substrate. The constituent material of the opposite substrate may be the same as that of the above-described substrate. When the above-described substrate is a first substrate, the opposite substrate may be a second substrate.

[Pixel Circuit]

A light-emitting apparatus may include a pixel circuit connected to the light-emitting element. The pixel circuit may be an active matrix-type circuit which independently controls the light emission of a first light-emitting element and a second light-emitting element. The active matrix-type circuit may be voltage programmed or current programmed. A drive circuit includes the pixel circuit for each pixel. The pixel circuit may include a light-emitting element, a transistor that controls the emission luminance of the light-emitting element, a transistor that controls the timing of light emission, a capacitor that holds the gate voltage of the transistor that controls the emission luminance, and a transistor for providing connection to GND not through the light-emitting element.
The light-emitting apparatus has a display region and a peripheral region disposed around the display region. The display region includes the pixel circuit, and the peripheral region includes a display control circuit. The mobility of the transistor constituting the pixel circuit may be lower than the mobility of a transistor constituting the display control circuit.
The gradient of the current-voltage characteristics of the transistor constituting the pixel circuit may be smaller than the gradient of the current-voltage characteristics of the transistor constituting the display control circuit. The gradient of the current-voltage characteristics can be determined on the basis of what is called Vg-Ig characteristics.
The transistor constituting the pixel circuit is a transistor connected to a light-emitting element such as the first light-emitting element.

[Pixel]

The organic light-emitting apparatus includes a plurality of pixels. Each pixel includes subpixels that emit light of colors different from each other. The subpixels may respectively have, for example, R, G, and B emission colors.
In each pixel, a region also referred to as a pixel aperture emits light. This region is the same as the first region. The size of the pixel aperture may be 15 μm or less and 5 μm or more. More specifically, the size may be, for example, 11 μm, 9.5 μm, 7.4 μm, or 6.4 μm.
The distance between the subpixels may be 10 μm or less, specifically 8 μm, 7.4 μm, or 6.4 μm.
The pixels may be in a known arrangement when viewed in plan. For example, the arrangement may be the stripe arrangement, the delta arrangement, the PenTile arrangement, or the Bayer arrangement. The shape of the subpixels in plan view may be any known shape. Examples include quadrangles, such as rectangles and rhombuses, and hexagons. It is appreciated that shapes that are not exactly rectangles but are similar to rectangles are also regarded as rectangles. The shape of the subpixels and the pixel arrangement can be used in combination.

(5) Applications of Organic Light-Emitting Element According to Present Embodiment

The organic light-emitting element according to the present embodiment can be used as a constituent member of a display apparatus, an image-forming apparatus, or a lighting apparatus. Other applications include an exposure light source in an electrophotographic image-forming apparatus, a backlight in a liquid crystal display, and a light-emitting apparatus including a white light source with a color filter.
The display apparatus may be an image information processor that includes an image input unit to which image information from an area CCD, a linear CCD, a memory card, or the like is input, includes an information-processing unit configured to process the input information, and displays the input image on a display unit.
The display unit of an image pickup apparatus or an ink-jet printer may have a touch panel function. The touch panel function may be activated by any system, such as an infrared system, an electrostatic capacitive system, a resistive film system, or an electromagnetic induction system. The display apparatus may also be used as a display unit of a multifunctional printer.
Next, a display apparatus according to the present embodiment will be described with reference to the drawings.
Fig. 1A and Fig. 1B are schematic sectional views each showing an example of a display apparatus including an organic light-emitting element and a transistor connected to the organic light-emitting element. The transistor is an example of an active element. The transistor may be a thin film transistor (TFT).
Fig. 1A is an example of a pixel that is a component of the display apparatus according to the present embodiment. The pixel includes subpixels 10. The subpixels are divided into 10R, 10G, and 10B according to their light emission. The emission color may be distinguished on the basis of the wavelength of light emitted from a light-emitting layer, or light emitted from the subpixels may undergo selective transmission or color conversion through a color filter or the like. Each of the subpixels includes, on an interlayer insulating layer 1, a reflective electrode 2 that is a first electrode, an insulating layer 3 that covers the edge of the reflective electrode 2, an organic compound layer 4 that covers the first electrode and the insulating layer, a transparent electrode 5, a protective layer 6, and a color filter 7.
The interlayer insulating layer 1 may include a transistor and a capacitor element below or inside the interlayer insulating layer 1. The transistor and the first electrode may be electrically connected to each other through a contact hole (not illustrated) or the like.
The insulating layer 3 is also referred to as a bank or a pixel-separating film. The insulating layer 3 is disposed so as to cover the edge of the first electrode and surround the first electrode. A portion in which the insulating layer is not disposed is in contact with the organic compound layer 4 and serves as a light-emitting region.
The organic compound layer 4 includes a hole injection layer 41, a hole transport layer 42, a first light-emitting layer 43, a second light-emitting layer 44, and an electron transport layer 45.
The second electrode 5 may be a transparent electrode, a reflective electrode, or a semitransparent electrode.
The protective layer 6 reduces permeation of water into the organic compound layer. Although the protective layer is illustrated as a single layer, it may be constituted by a plurality of layers. The layers may be constituted by an inorganic compound layer and an organic compound layer.
The color filter 7 is divided into 7R, 7G, and 7B according to their color. The color filter may be formed on a planarization film (not illustrated). A resin protective layer (not illustrated) may be disposed on the color filter. The color filter may be formed on the protective layer 6. The color filter may be bonded after being formed on an opposite substrate such as a glass substrate.
In a display apparatus 100 in Fig. 1B, an organic light-emitting element 26 and a TFT 18 as an example of a transistor are illustrated. The display apparatus 100 includes a substrate 11 made of glass, silicon, or the like and an insulating layer 12 disposed thereon. An active element 18 such as a TFT is disposed on the insulating layer, and a gate electrode 13, a gate insulating film 14, and a semiconductor layer 15 of the active element are disposed. The active

element 18 also includes the semiconductor layer 15, a drain electrode 16, and a source electrode 17. An insulating film 19 is disposed over the active element 18. An anode 21 constituting the organic light-emitting element 26 and the source electrode 17 are connected to each other through a contact hole 20 extending through the insulating film.

The electrodes (the anode and the cathode) included in the organic light-emitting element 26 and the electrodes (the source electrode and the drain electrode) included in the TFT need not necessarily be electrically connected to each other in the manner illustrated in Fig. 1B. It is only required that either the anode or the cathode be electrically connected to either the source electrode or the drain electrode of the TFT. TFT stands for a thin film transistor.

Although the organic compound layer is illustrated as a single layer in the display apparatus 100 in Fig. 1B, the organic compound layer 22 may be constituted by a plurality of layers. A first protective layer 24 and a second protective layer 25 for reducing deterioration of the organic light-emitting element are disposed over the cathode 23.

Although a transistor is used as a switching element in the display apparatus 100 in Fig. 1B, another switching element may be used instead.

The transistor used in the display apparatus 100 in Fig. 1B may be not only a transistor obtained using a single-crystal silicon wafer but also a thin film transistor including a substrate and an active layer on an insulating surface of the substrate. The active layer may be made of, for example, single-crystal silicon, non-single-crystal silicon such as amorphous silicon or microcrystalline silicon, or a non-single-crystal oxide semiconductor such as indium zinc oxide or indium gallium zinc oxide. The thin film transistor is also referred to as a TFT element.

The transistor included in the display apparatus 100 in Fig. 1B may be formed in a substrate such as a Si substrate. The phrase "formed in a substrate" means producing a transistor by processing a substrate itself, such as a Si substrate. That is, having a transistor in a substrate can also mean that the substrate and the transistor are integrally formed.

The emission luminance of the organic light-emitting element according to the present embodiment is controlled by a TFT, which is an example of a switching element, and disposing a plurality of the organic light-emitting elements in a screen enables a display of an image with different emission luminances. The switching element according to the present embodiment is not limited to a TFT, and may be a transistor formed of low-temperature polysilicon or an active-matrix driver formed on a substrate such as a Si substrate. The phrase "on a substrate" may also be referred to as "in a substrate". Whether a transistor is provided in the substrate or a TFT is used is chosen depending on the size of the display unit. For example, when the display unit has a size of about 0.5 inches, the organic light-emitting element is preferably disposed on a Si substrate.

Fig. 2 is a schematic view showing an example of the display apparatus according to the present embodiment. A display apparatus 1000 may include an upper cover 1001, a lower cover 1009, and a touch panel 1003, a display panel 1005, a frame 1006, a circuit board 1007, and a battery 1008 disposed between the covers. The display panel 1005 may include the organic light-emitting element according to the present embodiment. Flexible print circuits (FPCs) 1002 and 1004 are connected to the touch panel 1003 and the display panel 1005, respectively. A transistor is printed on the circuit board 1007. The battery 1008 may be omitted if the display apparatus is not a mobile device. If the display apparatus is a mobile device, the battery 1008 may be disposed in another position.

The display apparatus according to the present embodiment may include red, green, and blue color filters. The red, green, and blue color filters may be disposed in the delta arrangement.

The display apparatus according to the present embodiment may be used as a display unit of a mobile terminal. In this case, the display apparatus may have both a display function and an operating function. Examples of the mobile terminal include cellular phones such as smart phones, tablets, and head-mounted displays.

The display apparatus according to the present embodiment may be used as a display unit of an image pickup apparatus that includes an image pickup element configured to receive light. The image pickup apparatus may include a display unit configured to display information acquired by the image pickup element. The display unit may be exposed to the outside of the image pickup apparatus or disposed in a viewfinder. The image pickup apparatus may be a digital camera or a digital camcorder.

Fig. 3A is a schematic view showing an example of an image pickup apparatus according to the present embodiment. An image pickup apparatus 1100 may include a viewfinder 1101, a rear display 1102, an operation unit 1103, and a housing 1104. The viewfinder 1101 and the rear display 1102 may include the organic light-emitting element according to the present embodiment. In this case, the viewfinder 1101 and the rear display 1102 may display not only an image to be captured but also environmental information, image capture instructions, etc. The environmental information may be, for example, the intensity of external light, the direction of external light, the moving speed of a subject, or the possibility that the subject is hidden by an object.

Since the timing appropriate for capturing an image is only a moment, the information is desirably displayed as quickly as possible. Thus, the display apparatus including the organic light-emitting element according to the present embodiment is preferably used. This is because the organic light-emitting element has a high response speed.

The image pickup apparatus 1100 may further include an optical unit (not illustrated). The optical unit may have a single lens or a plurality of lenses and focuses an image on the image pickup element accommodated in the housing 1104.

By adjusting the relative positions of the plurality of lenses, the focal point can be adjusted. This operation can also be performed automatically. The image pickup apparatus may be referred to as a photoelectric conversion apparatus. Instead of sequential imaging, the photoelectric conversion apparatus may involve, as an imaging method, detection of a difference from the previous image, extraction from continuously recorded images, or the like.

Fig. 3B is a schematic view showing an example of an electronic apparatus according to the present embodiment. An electronic apparatus 1200 includes a display unit 1201, an operation unit 1202, and a housing 1203. The housing 1203 may include a circuit, a printed board including the circuit, a battery, and a communication unit. The operation unit 1202 may be a button or a touch-sensitive response unit. The operation unit may be a biometric recognition unit that, for example, releases a lock upon recognition of fingerprints. An electronic apparatus including a communication unit can also be referred to as a communication apparatus. The electronic apparatus may further have a camera function by including lenses and an image pickup element. An image captured by the camera function is displayed on the display unit. Examples of the electronic apparatus include smartphones and notebook computers.

Fig. 4A and Fig. 4B show schematic views showing examples of the display apparatus according to the present embodiment. Fig. 4A is a display apparatus such as a television monitor or a PC monitor. A display apparatus 1300 includes a housing 1301 and a display unit 1302. The organic light-emitting element according to the present embodiment may be used in the display unit 1302.

The display apparatus 1300 may include a base 1303 that supports the housing 1301 and the display unit 1302. The base 1303 need not necessarily be in the form illustrated in Fig. 4A. The lower side of the housing 1301 may serve as a base.

The housing 1301 and the display unit 1302 may be curved. The radius of curvature may be 5000 mm or more and 6000 mm or less.

Fig. 4B is a schematic view showing another example of the display apparatus according to the present embodiment. A display apparatus 1310 in Fig. 4B is configured to be folded and is what is called a foldable display apparatus. The display apparatus 1310 includes a first display unit 1311, a second display unit 1312, a housing 1313, and a bending point 1314. The first display unit 1311 and the second display unit 1312 may include the organic light-emitting element according to the present embodiment. The first display unit 1311 and the second display unit 1312 may be a single seamless display apparatus. The first display unit 1311 and the second display unit 1312 can be divided by the bending point. The first display unit 1311 and the second display unit 1312 may display different images, or the first and second display units may together display a single image.

Fig. 5A is a schematic view showing an example of a lighting apparatus according to the present embodiment. A lighting apparatus 1400 may include a housing 1401, a light source 1402, and a circuit board 1403. The light source 1402 may include the organic light-emitting element according to the present embodiment. To improve the color rendering properties of the light source, the lighting apparatus 1400 may include an optical film 1404. To effectively diffuse light from the light source, the lighting apparatus 1400 may include a light diffusion unit 1405. The lighting apparatus 1400 including the light diffusion unit 1405 enables the light to reach a wide region. The optical film 1404 and the light diffusion unit 1405 may be disposed on the light-emitting side of the lighting apparatus. If necessary, a cover may be disposed at an outermost portion.

The lighting apparatus is, for example, an indoor lighting apparatus. The lighting apparatus may emit light of white, daylight white, or any other color from blue to red. The lighting apparatus according to the present embodiment may include a modulation circuit configured to modulate the light. The lighting apparatus according to the present embodiment may include a power supply circuit connected to the organic light-emitting element according to the present embodiment. The power supply circuit may be a circuit configured to convert AC voltage to DC voltage. White is a color with a color temperature of 4200 K, and daylight white is a color with a color temperature of 5000 K. The lighting apparatus according to the present embodiment may further include a color filter.

The lighting apparatus according to the present embodiment may also include a heat dissipation unit. The heat dissipation unit is configured to dissipate heat in the apparatus to the outside and may be, for example, a metal with high thermal conductivity or a ceramic.

Fig. 5B is a schematic view of an automobile that is an example of a moving object according to the present embodiment. The automobile includes a tail lamp that is an example of a lighting fixture. An automobile 1500 includes a tail lamp 1501 and a car body 1503, and the tail lamp may be configured to be turned on in response to, for example, brake operation. The car body 1503 can also be referred to as the body. The automobile 1500 may include a window 1502 attached to the car body 1503.

The tail lamp 1501 may include the organic light-emitting element according to the present embodiment. The tail lamp may include a protective member that protects the light source. The protective member may be formed of any material that has a certain degree of high strength and is transparent, but is preferably formed of a polycarbonate or the like. The polycarbonate may be mixed with a furandicarboxylic acid derivative, an acrylonitrile derivative, or the like.

The window 1502 may be a transparent display unless it is a window for checking the front and rear of the automobile. The transparent display may include the organic light-emitting element according to the present embodiment.

In this case, components of the organic light-emitting element according to the present invention, such as electrodes, are constituted by transparent members.

The moving object according to the present embodiment includes one or both of a driving force generation unit configured to generate a driving force mainly used for movement of the moving object and a rotary body mainly used for movement of the moving object. The driving force generation unit may be an engine, a motor, or the like. The rotary body may be a tire, a wheel, a screw for a ship, a propeller for an air vehicle, or the like. Specifically, the moving object may be a bicycle, an automobile, a train, a ship, an aircraft, a drone, or the like. The moving object may include a body and a lighting fixture disposed on the body. The lighting fixture may emit light for allowing the position of the body to be recognized.

Application examples of the display apparatuses according to the above-described embodiments will be described with reference to Fig. 6A and Fig. 6B. The display apparatuses can be applied to systems that can be worn as wearable devices such as smart glasses, head-mounted displays, and smart contact lenses. A display apparatus usable for a wearable device may include an image pickup apparatus that can photoelectrically convert visible light and a display apparatus that can emit visible light.

Fig. 6A and Fig. 6B are schematic views illustrating examples of eyeglasses (smart glasses) according to the present embodiment. Eyeglasses 1600 (smart glasses) will be described with reference to Fig. 6A. The eyeglasses 1600 include a display unit on the rear side of a lens 1601. The display unit may include the organic light-emitting element according to the present invention. Furthermore, an image pickup apparatus 1602, such as a CMOS sensor or a SPAD, may be disposed on the front side of the lens 1601.

The eyeglasses 1600 further include a controller 1603. The controller 1603 functions as a power source for supplying electricity to the image pickup apparatus 1602 and the display unit. The controller 1603 controls the operation of the image pickup apparatus 1602 and the display unit. The lens 1601 is provided with an optical system for collecting light of the image pickup apparatus 1602 and the display unit.

Eyeglasses 1610 (smart glasses) will be described with reference to Fig. 6B. The eyeglasses 1610 include a controller 1612, and the controller 1612 is provided with a display apparatus including the organic light-emitting element according to the present invention. The controller 1612 may further include an image pickup apparatus corresponding to the image pickup apparatus 1602. A lens 1611 is provided with an optical system for projecting light emitted from the controller 1612, and an image is projected onto the lens 1611. The controller 1612 functions as a power source for supplying electricity to the image pickup apparatus and the display apparatus and also controls the operation of the image pickup apparatus and the display apparatus. The controller may include a gaze detection unit configured to detect the gaze of a wearer. The gaze may be detected using infrared radiation. An infrared light emission unit emits infrared light to an eyeball of a user gazing at a displayed image. Among emitted infrared light, reflected light from the eyeball is detected by an image pickup unit including a light-receiving element, whereby a captured image of the eyeball is obtained. Due to the presence of a reduction unit configured to reduce light from the infrared light emission unit to the display unit in plan view, degradation of image quality is reduced.

From the captured image of the eyeball obtained by infrared imaging, the controller 1612 detects the gaze of the user toward the displayed image. Any known method can be used for the gaze detection using the captured image of the eyeball. For example, a gaze detection method based on a Purkinje image formed by the reflection of irradiation light on a cornea can be used.

More specifically, a gaze detection process based on a pupil-corneal reflection method is performed. Using the pupil-corneal reflection method, a gaze vector representing the direction (rotation angle) of the eyeball is produced on the basis of a pupil image and a Purkinje image included in the captured image of the eyeball, whereby the gaze of the user is detected.

The display apparatus according to the present embodiment may include an image pickup apparatus including a light-receiving element and may control a displayed image on the display apparatus on the basis of the gaze information of the user from the image pickup apparatus.

Specifically, the display apparatus determines, on the basis of the gaze information, a first viewing region at which the user gazes and a second viewing region other than the first viewing region. The first viewing region and the second viewing region may be determined by the controller of the display apparatus, or may be determined by an external controller and sent therefrom. In a display region of the display apparatus, the display resolution of the first viewing region may be controlled to be higher than the display resolution in the second viewing region. That is, the resolution in the second viewing region may be set to be lower than that in the first viewing region.

The display region includes a first viewing region and a second viewing region different from the first viewing region, and a region of high priority is determined from the first viewing region and the second viewing region on the basis of the gaze information. The first viewing region and the second viewing region may be determined by the controller of the display apparatus, or may be determined by an external controller and sent therefrom. The resolution in the region of high priority may be controlled to be higher than the resolution in the region other than the region of high priority. That is, the resolution in a region of relatively low priority may be set to be lower.

AI may be used to determine the first viewing region or the viewing region of high priority. AI may be a model

configured to use, as teaching data, an image of an eyeball and the actual gaze direction of the eyeball in the image and estimate, from the image of the eyeball, the angle of gaze and the distance to an object gazed. AI may be included in the display apparatus, the image pickup apparatus, or an external apparatus. When the external apparatus includes AI, it can be suitably used in smart glasses further including an image pickup apparatus configured to capture an external image. Smart glasses can display captured external information in real time.

Fig. 7A is a schematic view showing an example of an image-forming apparatus according to the present embodiment. An image-forming apparatus 40 is an electrophotographic image-forming apparatus and includes a photoreceptor 27, an exposure light source 28, a charging unit 30, a developing unit 31, a transfer unit 32, a conveyer roller 33, and a fixing unit 35. The exposure light source 28 emits light 29, and an electrostatic latent image is formed on the surface of the photoreceptor 27. The exposure light source 28 may include the organic light-emitting element according to the present embodiment. The developing unit 31 contains a toner and the like. The charging unit 30 charges the photoreceptor 27. The transfer unit 32 transfers a developed image onto a recording medium 34. The conveyer roller 33 conveys the recording medium 34. The recording medium 34 is, for example, paper. The fixing unit 35 fixes an image formed on the recording medium 34.

Fig. 7B and Fig. 7C each illustrate the exposure light source 28 and each schematically illustrate how a plurality of light-emitting portions 36 are arranged on a long substrate. An arrow 37 indicates a row direction in which organic light-emitting elements are aligned. The row direction is the same as the direction of the rotation axis of the photoreceptor 27. This direction can also be referred to as the major-axis direction of the photoreceptor 27. In Fig. 7B, the light-emitting portions 36 are arranged along the major-axis direction of the photoreceptor 27. In Fig. 7C, unlike Fig. 7B, the light-emitting portions 36 are alternately arranged in the row direction in a first row and in a second row. The first row and the second row are located at different positions in the column direction. In the first row, the plurality of light-emitting portions 36 are arranged at intervals. In the second row, the light-emitting portions 36 are arranged at positions corresponding to the spaces between the light-emitting portions 36 in the first row. That is, the plurality of light-emitting portions 36 are arranged at intervals also in the column direction. The arrangement in Fig. 7C can be referred to as, for example, a lattice arrangement, a staggered arrangement, or a checkered pattern.

As described above, the use of an apparatus including the organic light-emitting element according to the present embodiment enables a stable display with good image quality over a long period of time.

## EXAMPLES

The present invention will now be described with reference to Examples. It should be noted that the present invention is not limited thereto.

### [Example 1 (Synthesis of Compounds)]

#### (1) Synthesis of Exemplary Compound 1

Exemplary compound 1 was synthesized according to the following procedure.

[Chem. 167]

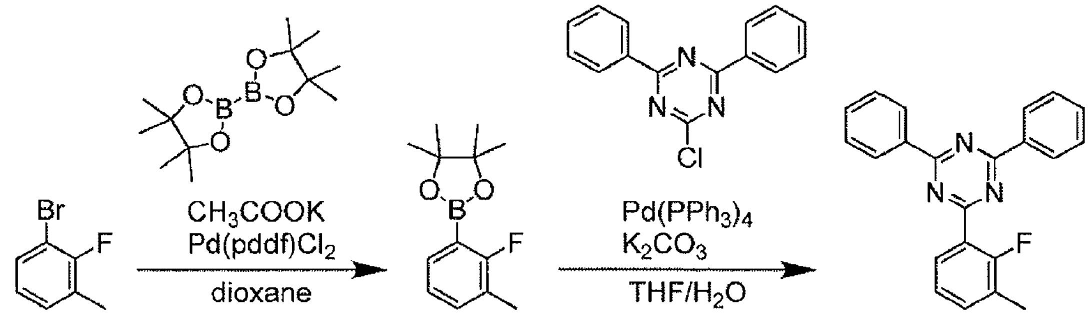


1

2

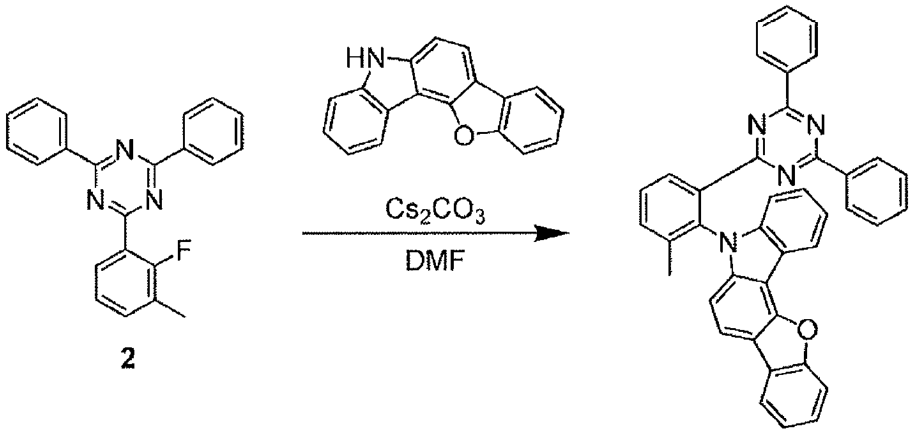


2

Exemplary compound 1

(1-1) Synthesis of Intermediate 1

3-Bromo-2-fluorotoluene (2.01 g), potassium acetate (3.28 g), Bis(pinacolato)diboron (4.03 g), [1,1'-Bis(diphenylphosphino)ferrocene]palladium(II) dichloride (Pd(dppf)Cl2, 0.50 g), and 1,4-dioxane (40 mL) were placed in a 100 mL recovery flask in a nitrogen atmosphere and stirred at 104°C for 11 hours. The reaction solution was concentrated with an evaporator, and toluene was added. Insoluble components were removed by filtration. The solution was concentrated to dryness, and the resulting crude product was separated by column chromatography ($SiO_2$, ethyl acetate/heptane = 1/10) to obtain an oily orange solid (2.05 g).

(1-2) Synthesis of Intermediate 2

Intermediate 1 (1.06 g), 2-chloro-4,6-diphenyl-1,3,5-triazine (0.80 g), potassium carbonate (1.27 g), tetrakis(triphenylphosphine)palladium(0) (23.3 mg), tetrahydrofuran (18 mL), and ion-exchanged water (6 mL) were placed in a 100 mL recovery flask in a nitrogen atmosphere and stirred at 70°C for 11 hours. After the reaction solution was returned to room temperature, ethyl acetate was added thereto. After extraction with ethyl acetate, the resultant was dried over sodium sulfate, and a solution was recovered by filtration. The solvent was removed from the recovered solution using an evaporator, and the resulting crude product was dissolved in ethyl acetate. The solution was added dropwise to methanol to yield a white solid. The white solid (0.65 g) was obtained by filtration.

(1-3) Synthesis of Exemplary Compound 1

Intermediate 2 (0.49 g), 5H-benzofuro[3,2-c]carbazole (0.42 g), cesium carbonate (1.13 g), and N,N-dimethylformamide (20 mL) were placed in a 300 mL recovery flask in a nitrogen atmosphere and stirred at 135°C for 11 hours. After the reaction solution was returned to room temperature, water and ethyl acetate were added thereto to perform extraction with ethyl acetate. The solvent was removed with an evaporator, and after the crude product was dissolved in ortho-dichlorobenzene, the solution was passed through a $SiO_2$ column. The resulting solution was concentrated by removing the solvent with an evaporator, and the concentrated solution was added dropwise to methanol to obtain a suspension. A yellow powder was recovered by filtration and subjected to sublimation purification (300°C, $5.0 \times 10^{-1}$ Pa) to obtain a

yellow solid (670 mg). The results of the $^1$H NMR measurement of the obtained yellow solid are shown below.

$^1$H NMR (500 MHz, $CDCl_3$): δ 8.43 (d, J = 7.5 Hz, 1H), 8.36 (dd, J = 4, 2.5 Hz, 1H), 7.94 (d, J = 9 Hz, 1H), 7.92 (d, J = 8 Hz, 4H), 7.89 (d, J = 9 Hz, 1H), 7.74-7.66 (m, 3H), 7.44-7.32 (m, 6H), 7.19-7.12 (m, 5H), 7.09 (d, J = 8 Hz, 1H), 2.10 (s, 3H).

(2) Synthesis of Exemplary Compound 2

Exemplary compound 2 was synthesized according to the following procedure.

[Chem. 168]

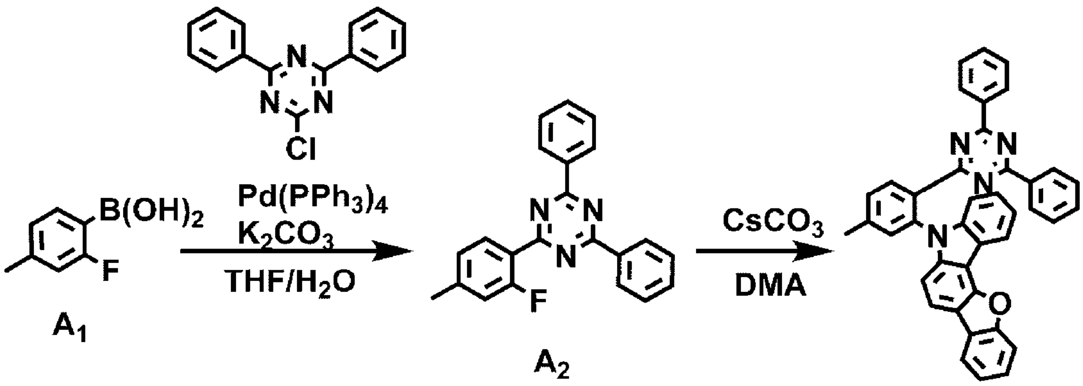


Exemplary compound 2

(2-1) Synthesis of Intermediate A2

To a solution of compound A1 (3.17 g, 20.6 mmol) and 2-choloro-4,6-diphenyl-1,3,5-triazine (5,00 g, 18.7 mmol) in THF (100 mL), potassium carbonate (6.45 g, 46.9 mmol), water (20 mL), and $Pd(PPh_3)_4$ (755 mg, 0.655 mmol) were added, and the mixture was heated under reflux for 20 hours. The resulting mixture was cooled to room temperature, and chloroform/water was added. The organic layer was washed with water and dried over anhydrous sodium sulfate. The solvent was distilled away under reduced pressure, and the resulting residue was purified by silica gul column chromatography (elution solvent; hexane/chloroform = 6/1 → 3/1) to obtain compound A2 of interest (6.25 g, 98%, white solid).
MS: 342, cald.: 342 ($M+H^+$)

(2-2) Synthesis of Exemplary Compound 2

Cesium carbonate (2.69 g, 8.78 mmol) was added to a solution of 5H-Benzofuro[3,2-c]carbazole (1.19 g, 4.61 mmol) in anhydrous DMA (45 mL), and the mixture was stirred for 80 minutes. Compound A2 (1.50 g, 4.39 mmol) was added, and the mixture was stirred at 150°C for 12 hours. The mixture was cooled to room temperature, filtered over celite, and washed with toluene. The filtrate was concentrated, and the resulting residue was purified by silica gul column chromatography (elution solvent; hexane/toluene = 5/1 → 3/1). The residue was recrystallized from chloroform/methanol to obtain exemplary compound 2 of interest (1.85 g, 73%, pale yellow solid).
MS: 579, cald.: 579 ($M+H^+$)

(3) Synthesis of Comparative Compound 1

Comparative compound 1 was synthesized according to the following procedure.

[Chem. 169]

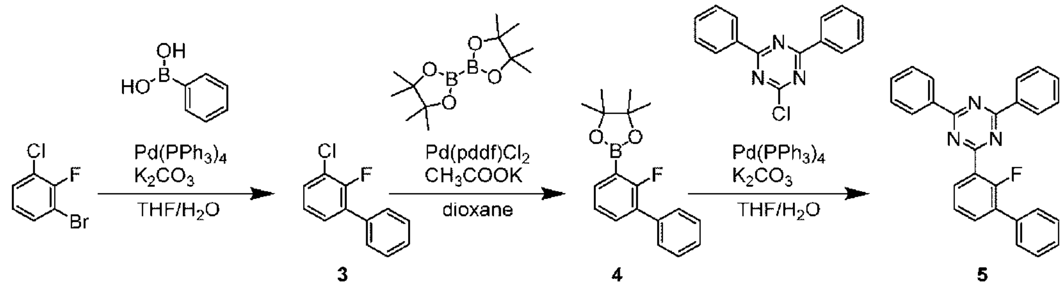


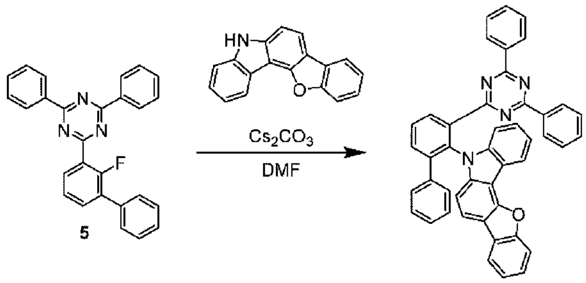


Comparative compound 1

(3-1) Synthesis of Intermediate 3

1-Bromo-3-chloro-2-fluorobenzene (2.5 g), phenylboronic acid (1.46 g), potassium carbonate (5.03 g), Tetrakis(triphenylphosphine)palladium(0) (85.0 mg), tetrahydrofuran (60 mL), and ion-exchanged water (20 mL) were placed in a 100 mL recovery flask in a nitrogen atmosphere and stirred at 70°C for 11 hours. After the reaction solution was returned to room temperature, ethyl acetate was added thereto. After extraction with ethyl acetate, the resultant was dried over sodium sulfate, and a solution was recovered by filtration. The solvent was removed from the recovered solution using an evaporator to obtain a crude product. The obtained crude product was separated by column chromatography ($SiO_2$, ethyl acetate/heptane = 1/20) to obtain a colorless liquid (2.02 g).

(3-2) Synthesis of Intermediate 4 and Intermediate 5

Intermediate 3 (1.01 g), potassium acetate (1.42 g), bis(pinacolato) diboron, [1,1'-Bis(diphenylphosphino) ferrocene]palladium(II) dichloride (Pd(dppf)$Cl_2$, 0.30 g), and 1,4-dioxane (20 mL) were placed in a 100 mL recovery flask in a nitrogen atmosphere and stirred at 104°C for 11 hours. The reaction solution was concentrated with an evaporator. Toluene was added, and the mixture was passed through a short column ($SiO_2$). The passed solution was concentrated to dryness, and 2-chloro-4,6-diphenyl-1,3,5-triazine (1.99 g), potassium carbonate (2.30 g), tetrakis(triphenylphosphine)palladium(0) (40 mg), tetrahydrofuran, and ion-exchanged water were added thereto in a nitrogen atmosphere and stirred at 70°C for 11 hours. After the reaction solution was returned to room temperature, ethyl acetate was added thereto. After extraction with ethyl acetate, the resultant was dried over sodium sulfate, and a solution was recovered by filtration. The solvent was removed from the recovered solution using an evaporator, and the resulting crude product was dissolved in ethyl acetate. The solution was added dropwise to heptane to yield a white solid. The white solid (0.74 g) was obtained by filtration.

(3-3) Synthesis of Comparative Compound 1

Intermediate 5 (0.41 g), 5H-benzofuro[3,2-c]carbazole (0.29 g), cesium carbonate (0.66 g), and N,N-dimethylformamide (20 mL) were placed in a 300 mL recovery flask in a nitrogen atmosphere and stirred at 135°C for 11 hours. After the reaction solution was returned to room temperature, water and ethyl acetate were added thereto to perform extraction with ethyl acetate. The solvent was removed with an evaporator, and after the crude product was dissolved in ortho-dichlorobenzene, the solution was passed through a $SiO_2$ column. The solvent was removed using an evaporator, and the concentrated solution was added dropwise to methanol to obtain a suspension. A yellow powder was recovered by filtration and subjected to sublimation purification (290°C, $5.0 \times 10^{-1}$ Pa) to obtain a yellow solid (150 mg). The results of the $^1$H NMR measurement of the obtained yellow solid are shown below.

$^1$H NMR (500 MHz, $CDCl_3$): δ 8.43 (dd, J = 5, 2.5 Hz, 1H), 8.25 (d, J = 7.5 Hz, 1H), 7.93-7.83 (m, 7H), 7.78 (d, J =

8.5 Hz, 1H), 7.62 (d, J = 8 Hz, 1H), 7.4-7.3 (m, 4H), 7.27 (t, J = 6.5 Hz, 1H), 7.21 (t, J = 7 Hz, 1H), 7.17-7.07 (m, 8H), 7.02-6.95 (m, 3H).

(4) Synthesis of Comparative Compound 2

Comparative compound 2 was synthesized according to the following procedure.

[Chem. 170]

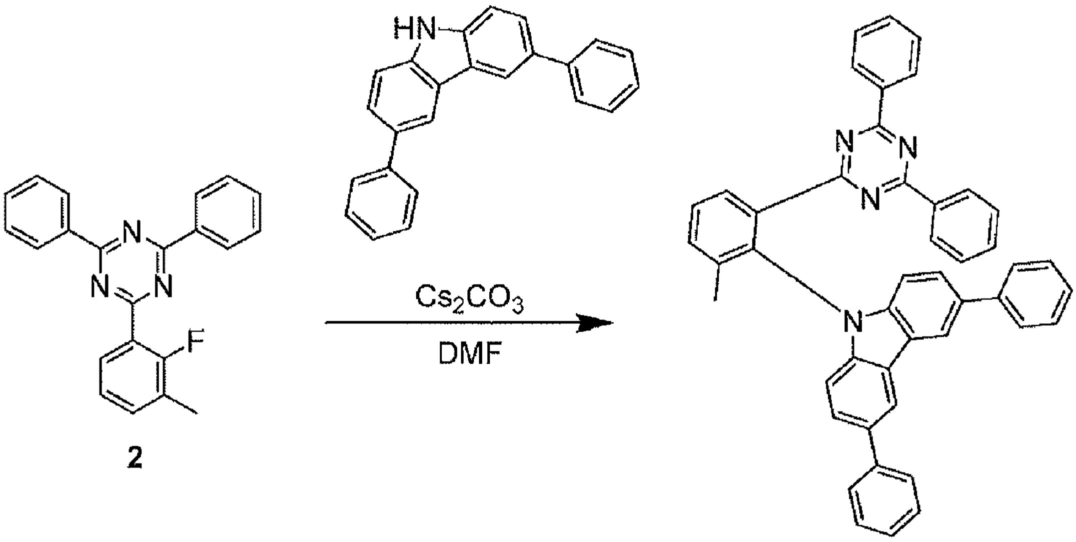


2

Comparative compound 2

(4-1) Synthesis of Comparative Compound 2

Intermediate 2 (0.42 g), 3,6-diphenyl-9H-carbazole (0.43 g), cesium carbonate (0.80 g), and N,N-dimethylformamide (20 mL) were placed in a 300 mL recovery flask in a nitrogen atmosphere and stirred at 135°C for 11 hours. After the reaction solution was returned to room temperature, water and ethyl acetate were added thereto to perform extraction with ethyl acetate. The solvent was removed with an evaporator, and after the crude product was dissolved in ortho-dichlorobenzene, the solution was passed through a $SiO_2$ column. The solvent was removed using an evaporator, and the concentrated solution was added dropwise to methanol to obtain a suspension. A yellow powder was recovered by filtration and subjected to sublimation purification (290°C, $5.0 \times 10^{-1}$ Pa) to obtain a yellow solid (400 mg). The results of the $^1$H NMR measurement of the obtained yellow solid are shown below.

$^1$H NMR (500 MHz, $CDCl_3$): δ 8.32 (dd, J = 4, 2.5 Hz, 1H), 8.28 (d, J = 2 Hz, 2H), 7.74-7.66 (m, 6H), 7.63 (d, J = 1.5 Hz, 1H), 7.61 (d, J = 1.5 Hz, 1H), 7.45 (t, J = 7.5 Hz, 4H), 7.40 (t, J = 8 Hz, 2H), 7.32 (t, J = 7.5 Hz, 2H), 7.26 (t, J = 7 Hz, 4H), 7.15 (d, J = 8.5 Hz, 2H), 2.14 (s, 3H).

(5) Synthesis of Comparative Compound 3

Comparative compound 3 was synthesized according to the following procedure.

[Chem. 171]

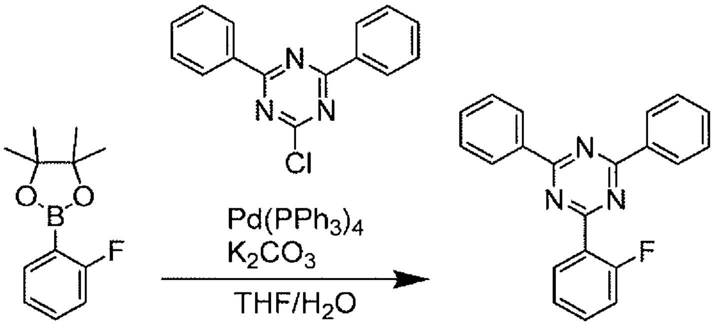


**6**

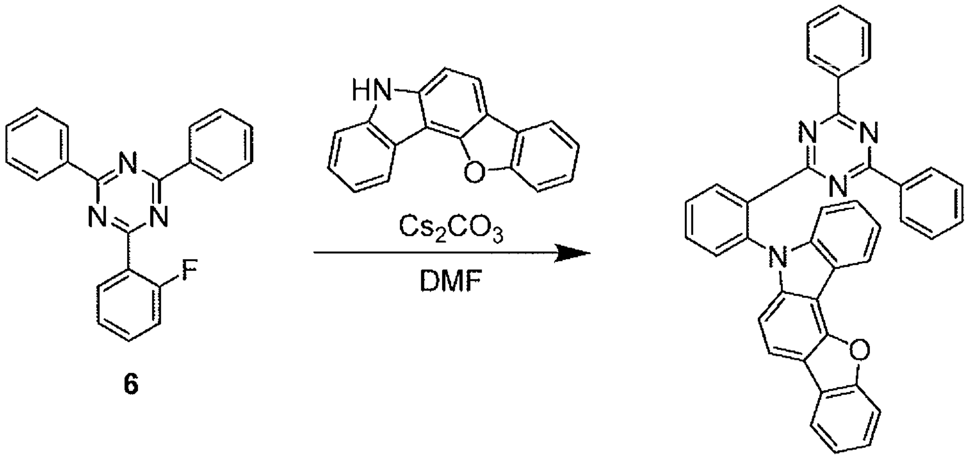


**6**

Comparative compound 3

(5-1) Synthesis of Intermediate 6

2-(2-Fluorophenyl)-4,4,5,5-tetramethyl-1,3,2-dioxaborolane (1.10 g), 2-chloro-4,6-diphenyl-1,3,5-triazine (0.93 g), potassium carbonate (1.70 g), tetrakis(triphenylphosphine)palladium(0) (25.0 mg), tetrahydrofuran, and ion-exchanged water were placed in a 100 mL recovery flask in a nitrogen atmosphere and stirred at 70°C for 11 hours. After the reaction solution was returned to room temperature, ethyl acetate was added thereto. After extraction with ethyl acetate, the resultant was dried over sodium sulfate, and a solution was recovered by filtration. The solvent was removed from the recovered solution using an evaporator, and the resulting crude product was dissolved in ethyl acetate. The solution was added dropwise to methanol to yield a white solid. The white solid (0.83 g) was recovered by filtration.

(5-2) Synthesis of Comparative Compound 3

Intermediate 6 (2.50 g), 5H-benzofuro[3,2-c]carbazole (2.23 g), cesium carbonate (5.04 g), and N,N-dimethylformamide (100 mL) were placed in a 300 mL recovery flask in a nitrogen atmosphere and stirred at 135°C for 11 hours. After the reaction solution was returned to room temperature, water and ethyl acetate were added thereto to perform extraction with ethyl acetate. The solvent was removed with an evaporator, and after the crude product was dissolved in ortho-dichlorobenzene, the solution was passed through a $SiO_2$ column. The solvent was removed using an evaporator, and the concentrated solution was added dropwise to methanol to obtain a suspension. A yellow powder was recovered by filtration and subjected to sublimation purification (260°C, $5.0 \times 10^{-1}$ Pa) to obtain a yellow solid (1.59 g). The results of the $^1H$ NMR measurement of the obtained yellow solid are shown below.

$^1H$ NMR (500 MHz, $CDCl_3$): δ 8.6 (d, J = 8 Hz, 1H), 8.44 (d, J = 8 Hz, 1H), 7.95 (d, J = 7.5 Hz, 4H), 7.92 (d, J = 6.5 Hz, 1H), 7.85 (d, J = 8 Hz, 2H), 7.83-7.74 (m, 2H), 7.69 (d, J = 8 Hz, 1H), 7.45-7.30 (m, 6H), 7.20-7.16 (m, 5H), 7.26 (d, J = 8 Hz, 1H).

(6) Synthesis of Comparative Compound 4

Comparative compound 4 was synthesized according to the following procedure.

[Chem. 172]

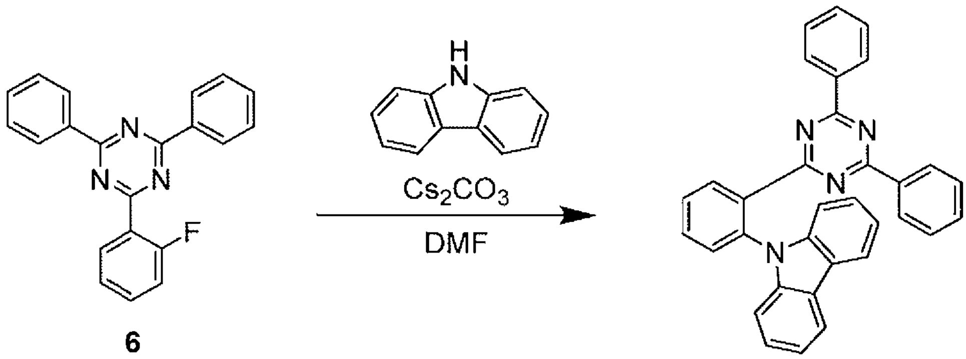


6

Comparative compound 4

(6-1) Synthesis of Comparative Compound 4

Intermediate 6 (0.50 g), 9H-carbazole (0.28 g), cesium carbonate (1.03 g), and N,N-dimethylformamide (20 mL) were placed in a 100 mL recovery flask in a nitrogen atmosphere and stirred at 135°C for 11 hours. After the reaction solution was returned to room temperature, water and ethyl acetate were added thereto to perform extraction with ethyl acetate. The solvent was removed with an evaporator, and after the crude product was dissolved in ortho-dichlorobenzene, the solution was passed through a $SiO_2$ column. The solvent was removed with an evaporator, and the concentrated solution was added dropwise to methanol to obtain a suspension. A yellow powder was recovered by filtration and subjected to sublimation purification (260°C, $5.0 \times 10^{-1}$ Pa) to obtain a yellow solid (130 mg). The results of the $^1$H NMR measurement of the obtained yellow solid are shown below.

$^1$H NMR (500 MHz, $CDCl_3$): $\delta$ 8.54 (d, J = 8 Hz, 1H), 7.96-7.84 (m, 6H), 7.81 (t, J = 7.5 Hz, 1H), 7.75 (t, J = 8 Hz, 1H), 7.70 (d, J = 7.5 Hz, 1H), 7.45 (t, J = 7.5 Hz, 2H), 7.30 (t, J = 7 Hz, 6H), 7.22-7.16 (m, 4H).

[Example 2 (Evaluation of Optical Properties Using Solution)]

Toluene solutions of exemplary compound 1 and comparative compounds 1 to 4 at $10^{-5}$ mol/L were prepared, and their PL spectra were measured. The peak top of each spectrum was determined as PL [nm]. The reverse intersystem crossing rates ($k_{RISC}$ [$10^6$ $s^{-1}$]) of exemplary compound 1 and comparative compounds 1 to 3 were calculated. The reverse intersystem crossing rates were calculated with reference to the previously reported paper (Angewandte Chemie, 2022, 134, e202205684). The results are shown in Table 2.

Comparative compound 2 and compound Ref-3 have in common that they have a methyl group at an ortho position with respect to a carbazole skeleton, and the carbazole skeleton is not a fused carbazole skeleton, and thus the $k_{RISC}$ value of comparative compound 2 can be estimated to be comparable to that of compound Ref-3.

[Table 2]

**Table 2**

| | PL [nm] | $k_{RISC}$ [$10^6$ $s^{-1}$] |
|---|---|---|
| Exemplary compound 1 | 479 | 2.48 |
| Exemplary compound 2 | 485 | 1.02 |
| Comparative compound1 | 491 | 0.94 |

(continued)

| | PL [nm] | $k_{RISC}$ [$10^6$ $s^{-1}$] |
|---|---|---|
| Comparative compound 2 | 480 | 0.77 |
| Comparative compound 3 | 481 | 0.67 |
| Comparative compound 4 | 472 | - |

Table 2 shows that exemplary compound 1 and exemplary compound 2, which are organic compounds according to the present invention, exhibited particularly large $K_{RISC}$ values compared with comparative compounds 1 to 3. This is probably because the reverse intersystem crossing of excitons was promoted due to the smaller $\Delta E_{ST}$ of the organic compound according to the present invention.

[Example 3 (Evaluation of Optical Properties Using Vapor-Deposited Film)]

Exemplary compound 1 and comparative compounds 1 to 4 were each deposited on a synthetic quartz substrate (20 mm × 20 mm × 0.7 mm thick) so as to have a thickness of 30 nm, and a fluorescence spectrum of each sample was measured at normal temperature (300 K). In the fluorescence spectrum where the vertical axis represents emission intensity and the horizontal axis represents wavelength, $\lambda_{S1}$ [nm] was determined from a shorter wavelength among wavelengths having an emission intensity 1/10 times the maximum emission intensity and converted into an energy value by the following conversion formula.

$$\text{Conversion formula: } E_{S1}\ [\text{eV}] = 1240/\lambda_{S1}$$

Next, a phosphorescence spectrum of each vapor-deposited film was measured at 77 K. In the fluorescence spectrum where the vertical axis represents emission intensity and the horizontal axis represents wavelength, $\lambda_{T1}$ [nm] was determined from a longer wavelength among wavelengths having an emission intensity 1/10 times the maximum emission intensity and converted into an energy value by the following conversion formula.

$$\text{Conversion formula: } E_{T1}\ [\text{eV}] = 1240/\lambda_{T1}$$

$\Delta E_{ST}$ (= $E_{S1}$ - $E_{T1}$) was determined from $E_{S1}$ and $E_{T1}$ obtained above. $E_{T1}$ and $\Delta E_{ST}$ are shown in Table 3.
Comparative compound 2 and compound Ref-3 have in common that they have a methyl group at an ortho position with respect to a carbazole skeleton, and the carbazole skeleton is not a fused carbazole skeleton, and thus the $\Delta E_{ST}$ value of comparative compound 2 can be estimated to be comparable to that of compound Ref-3.

[Table 3]

**Table 3**

| | $E_{T1}$ **[eV]** | $\Delta E_{ST}$ [eV] |
|---|---|---|
| Exemplary compound 1 | 2.82 | 0.023 |
| Exemplary compound 2 | 2.79 | 0.039 |
| Comparative compound1 | 2.76 | 0.049 |
| Comparative compound 2 | 2.75 | 0.077 |
| Comparative compound 3 | 2.73 | 0.055 |
| Comparative compound 4 | 2.79 | 0.090 |

Table 3 shows that exemplary compound 1 and exemplary compound 2, which are organic compounds according to the present invention, exhibited particularly small $\Delta E_{ST}$ values compared with comparative compounds 1 to 3. This is probably because the organic compound according to the present invention has a small overlap between the HOMO orbital distribution and the LUMO orbital distribution, resulting in a smaller $\Delta E_{ST}$.

It follows from the foregoing that the organic compound according to the present invention is an organic compound having a smaller $\Delta E_{ST}$. The organic compound according to an embodiment of the present invention is an organic compound having particularly high durability. Therefore, the organic light-emitting element including the organic compound according to the present invention is expected to have high element durability. The organic light-emitting element including the organic compound according to the present invention is expected to have high luminous efficiency.
The present invention may also have the following configurations.

(Configuration 1)

An organic compound represented by general formula (1-1) or (1-2).

[Chem. 173]

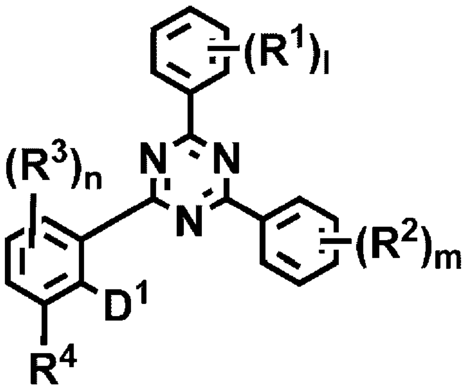

(1-1)

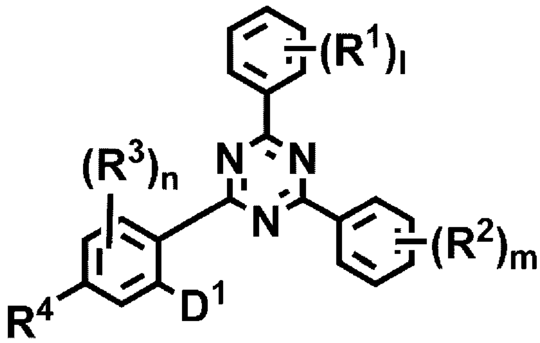

(1-2)

In general formulas (1-1) and (1-2), $R^1$ to $R^3$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. $R^4$ is a substituted or unsubstituted alkyl group or a substituted or unsubstituted alkoxy group. l and m are each an integer of 0 to 5, and n is an integer of 0 to 3. A plurality of $R^1$'s may be the same as or different from each other, a plurality of $R^2$'s may be the same as or different from each other, and a plurality of $R^3$'s may be the same as or different from each other. $D^1$ is represented by general formula (2).

[Chem. 174]

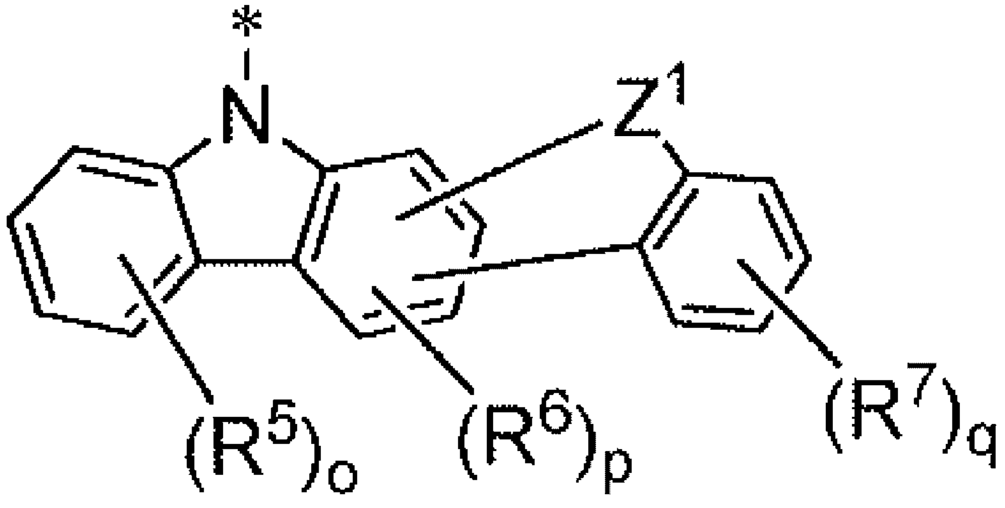

(2)

In general formula (2), $R^5$ to $R^7$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. o and q are each an integer of 0 to 4, and p is an integer of 0 to 2. A plurality of $R^5$'s may be the same as or different from each other, a plurality of $R^6$'s may be the same as or different from each other, and a plurality of $R^7$'s may be the same as or different from each other. $Z^1$ is an oxygen atom, a sulfur atom, a selenium atom, $NR^8$, or $CR^9R^{10}$. $R^8$ to $R^{10}$ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group. * represents a position of bonding to general formula (1-1) or (1-2).

(Configuration 2)

The organic compound according to configuration 1, wherein in general formula (2), $Z^1$ is an oxygen atom, a sulfur atom, or $NR^8$.

(Configuration 3)

The organic compound according to configuration 1 or 2, wherein in general formula (2), $Z^1$ is an oxygen atom.

(Configuration 4)

The organic compound according to any one of configurations 1 to 3, wherein in general formula (1-1) or (1-2), $R^4$ is an alkyl group having 1 to 7 carbon atoms.

(Configuration 5)

The organic compound according to any one of configurations 1 to 4, wherein in general formula (1-1) or (1-2), $R^4$ is a methyl group.

(Configuration 6)

The organic compound according to any one of configurations 1 to 5, wherein general formula (2) is general formula (2-1).

[Chem. 175]

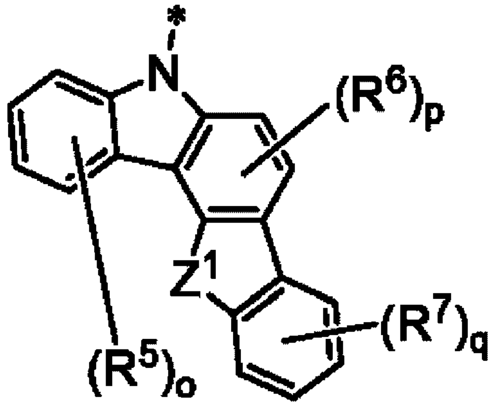

( 2 − 1 )

(Configuration 7)

The organic compound according to any one of configurations 1 to 6, wherein general formula (1-1) or (1-2) is general formula (3) or (4).

[Chem. 176]

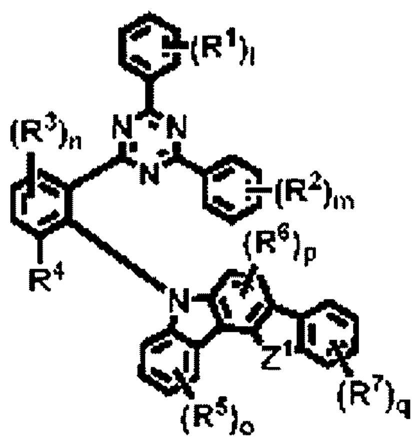

(3)

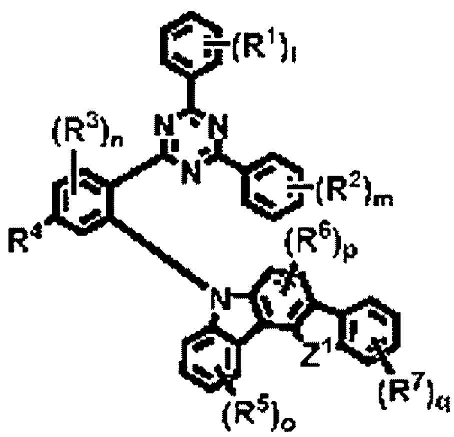

(4)

(Configuration 8)

The organic compound according to any one of configurations 1 to 7, wherein general formula (1-1) or (1-2) is general formula (5) or (6).

[Chem. 177]

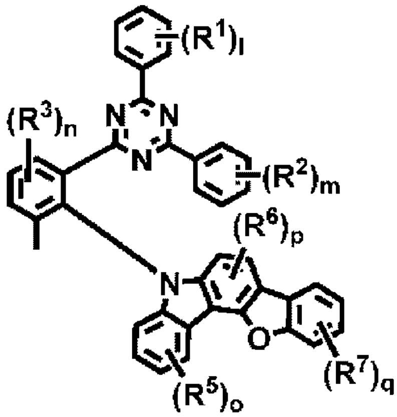

(5)

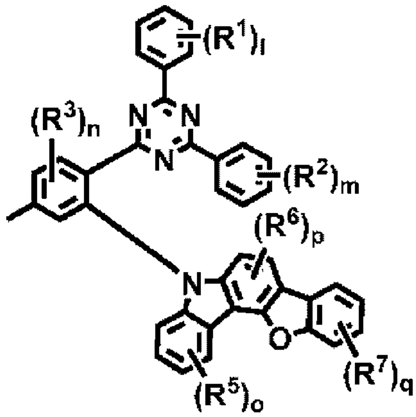

(6)

(Configuration 9)

An organic light-emitting element including: a first electrode and a second electrode; and an organic compound layer disposed between the first electrode and the second electrode,
wherein at least one layer of the organic compound layer contains the organic compound according to any one of configurations 1 to 8.

(Configuration 10)

The organic light-emitting element according to configuration 9, wherein the organic compound layer includes a light-emitting layer, and
the light-emitting layer contains the organic compound.

(Configuration 11)

The organic light-emitting element according to configuration 10, wherein the light-emitting layer further contains a first compound, and
a lowest excited singlet energy of the organic compound is lower than a lowest excited singlet energy of the first compound.

(Configuration 12)

The organic light-emitting element according to any one of configurations 10 to 11, wherein the light-emitting layer further contains a third compound, and
a lowest excited singlet energy of the third compound is lower than a lowest excited singlet energy of the organic compound.

(Configuration 13)

The organic light-emitting element according to configuration 12, wherein the third compound is a fluorescent material.

(Configuration 14)

The organic light-emitting element according to any one of configurations 10 to 13, wherein in the light-emitting layer, a concentration of the organic compound is 0.01 mass% or more and 99 mass% or less relative to the whole light-emitting layer.

(Configuration 15)

The organic light-emitting element according to any one of configurations 10 to 14, wherein in the light-emitting layer, a concentration of the organic compound is 10 mass% or more and 50 mass% or less relative to the whole light-emitting layer.

(Configuration 16)

A display apparatus including a plurality of pixels, wherein at least one of the plurality of pixels includes the organic light-emitting element according to any one of configurations 9 to 15 and a transistor connected to the organic light-emitting element.

(Configuration 17)

A photoelectric conversion apparatus including: an image pickup element configured to receive light; and a display unit configured to display an image captured by the image pickup element,
wherein the display unit includes the organic light-emitting element according to any one of configurations 9 to 15.

(Configuration 18)

An image display apparatus including: a display unit including the organic light-emitting element according to any one of configurations 9 to 15; and a housing provided with the display unit.

(Configuration 19)

An electronic apparatus including: a display unit including the organic light-emitting element according to any one of configurations 9 to 15; a housing provided with the display unit; and a communication unit provided in the housing and configured to communicate with an external unit.

(Configuration 20)

A wearable device including: a display unit including the organic light-emitting element according to any one of configurations 9 to 15; an optical system configured to collect light of the display unit; and a controller configured to control display of the display unit.

(Configuration 21)

A lighting apparatus including: a light source including the organic light-emitting element according to any one of configurations 9 to 15; and a housing provided with the light source.

(Configuration 22)

A moving object including: a lighting fixture including the organic light-emitting element according to any one of configurations 9 to 15; and a body provided with the lighting fixture.

(Configuration 23)

An image-forming apparatus including: a photoreceptor; and an exposure light source configured to expose the photoreceptor,
wherein the exposure light source includes the organic light-emitting element according to any one of configurations 9 to 15.

The present invention is not limited to the above embodiments, and various alterations and modifications can be made without departing from the spirit and scope of the present invention. Accordingly, the following claims are attached to make public the scope of the present invention.

This application claims priority to Japanese Patent Application No. 2023-112257 filed July 7, 2023 and Japanese Patent Application No. 2024-088438 filed May 30, 2024, which are hereby incorporated by reference herein in their entirety.

Reference Signs List

| | |
|---|---|
| 1 | interlayer insulating layer |
| 2 | reflective electrode |
| 3 | insulating layer |
| 4 | organic compound layer |
| 5 | transparent electrode |
| 6 | protective layer |
| 7 | color filter |
| 10 | subpixel |
| 11 | substrate |
| 12 | insulating layer |
| 13 | gate electrode |
| 14 | gate insulating film |
| 15 | semiconductor layer |
| 16 | drain electrode |
| 17 | source electrode |
| 18 | thin-film transistor |
| 19 | insulating film |
| 20 | contact hole |
| 21 | lower electrode |
| 22 | organic compound layer |
| 23 | upper electrode |
| 24 | first protective layer |
| 25 | second protective layer |
| 26 | organic light-emitting element |
| 27 | photoreceptor |
| 28 | exposure light source |
| 29 | light |
| 30 | charging unit |
| 31 | developing unit |
| 32 | transfer unit |
| 33 | conveying unit |
| 34 | recording medium |
| 35 | fixing unit |
| 36 | light-emitting portion |
| 37 | first direction parallel to major axis of photoreceptor |
| 40 | image-forming apparatus |
| 100 | display apparatus |
| 1000 | display apparatus |
| 1001 | upper cover |
| 1002 | flexible printed circuit |
| 1003 | touch panel |
| 1004 | flexible printed circuit |
| 1005 | display panel |
| 1006 | frame |
| 1007 | circuit board |
| 1008 | battery |
| 1009 | lower cover |
| 1100 | image pickup apparatus |
| 1101 | viewfinder |
| 1102 | rear display |
| 1103 | operation unit |
| 1104 | housing |
| 1200 | electronic apparatus |
| 1201 | display unit |

1202 operation unit
1203 housing
1300 display apparatus
1301 frame
1302 display unit
1303 base
1310 display apparatus
1311 first display unit
1312 second display unit
1313 housing
1314 bending point
1400 lighting apparatus
1401 housing
1402 light source
1403 circuit board
1404 optical film
1405 light diffusion unit
1500 automobile
1501 tail lamp
1502 window
1503 car body
1600 smart glasses
1601 lens
1602 image pickup apparatus
1603 controller
1610 smart glasses
1611 lens
1612 controller

## Claims

1. An organic compound represented by general formula (1-1) or (1-2):

[Chem. 1]

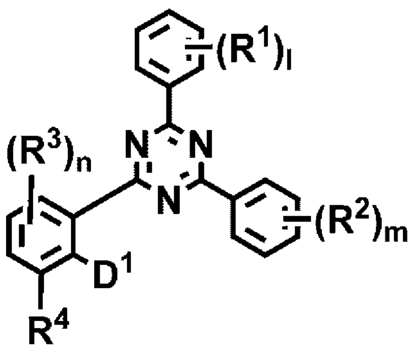

(1-1)

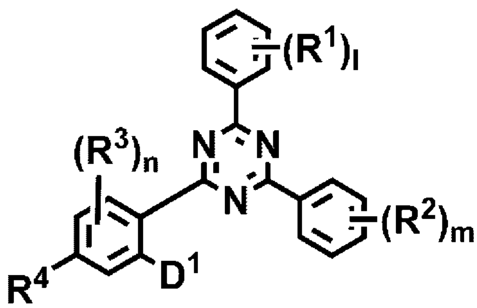

(1-2)

wherein in general formulas (1-1) and (1-2), $R^1$ to $R^3$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group; $R^4$ is a substituted or unsubstituted alkyl group or a substituted or unsubstituted alkoxy group; 1 and m are each an integer of 0 to 5, and n is an integer of 0 to 3; a plurality of $R^1$'s may be the same as or different from each other, a plurality of $R^2$'s may be the same as or different from each other, and a plurality of $R^3$'s may be the same as or different from each

other; and $D^1$ is represented by general formula (2):

[Chem. 2]

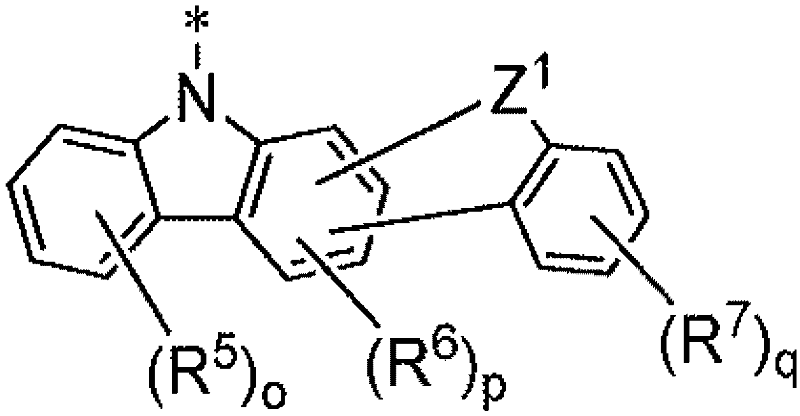

(2)

in general formula (2), $R^5$ to $R^7$ are each independently selected from the group consisting of a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group; o and q are each an integer of 0 to 4, and p is an integer of 0 to 2; a plurality of $R^5$'s may be the same as or different from each other, a plurality of $R^6$'s may be the same as or different from each other, and a plurality of $R^7$'s may be the same as or different from each other; $Z^1$ is an oxygen atom, a sulfur atom, a selenium atom, $NR^8$, or $CR^9R^{10}$, where $R^8$ to $R^{10}$ are each independently selected from the group consisting of a hydrogen atom, a deuterium atom, a halogen atom, a substituted or unsubstituted alkyl group, a substituted or unsubstituted aryl group, a substituted or unsubstituted heterocyclic group, a substituted or unsubstituted amino group, a substituted or unsubstituted alkoxy group, a substituted or unsubstituted aryloxy group, a substituted or unsubstituted heteroaryloxy group, a substituted or unsubstituted silyl group, and a cyano group; and * represents a position of bonding to general formula (1-1) or (1-2).

**2.** The organic compound according to claim 1, wherein in general formula (2), $Z^1$ is an oxygen atom, a sulfur atom, or $NR^8$.

**3.** The organic compound according to claim 1, wherein in general formula (2), $Z^1$ is an oxygen atom.

**4.** The organic compound according to claim 1, wherein in general formula (1-1) or (1-2), $R^4$ is an alkyl group having 1 to 7 carbon atoms.

**5.** The organic compound according to claim 1, wherein in general formula (1-1) or (1-2), $R^4$ is a methyl group.

**6.** The organic compound according to claim 1, wherein general formula (2) is general formula (2-1):

[Chem. 3]

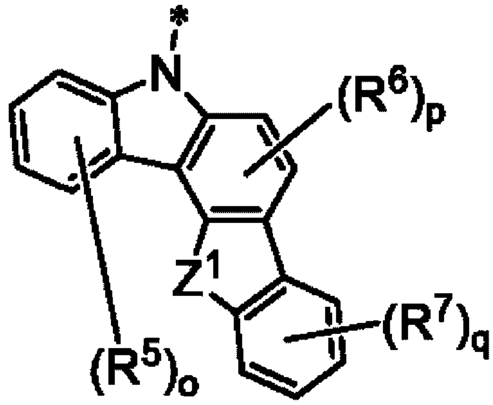

( 2 − 1 )

.

7. The organic compound according to claim 1, wherein general formula (1-1) or (1-2) is general formula (3) or (4):

[Chem. 4]

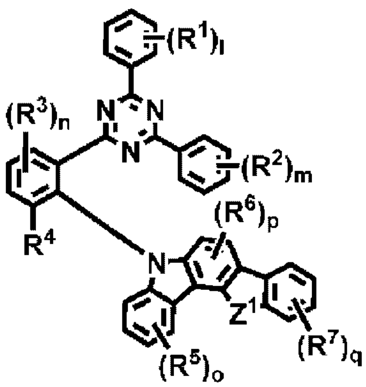

(3)

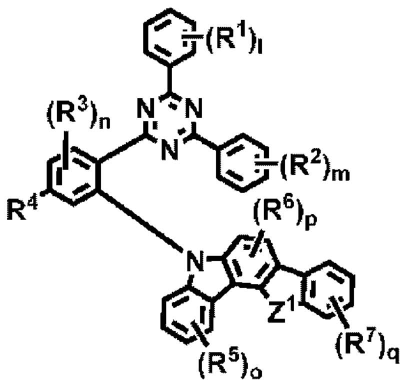

(4)

.

8. The organic compound according to claim 1, wherein general formula (1-1) or (1-2) is general formula (5) or (6):

[Chem. 5]

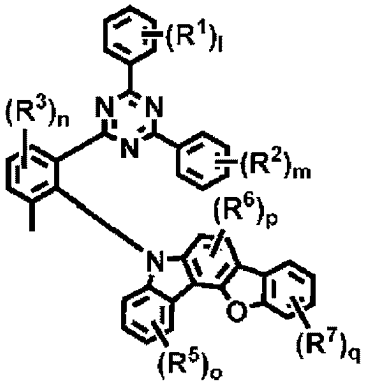

(5)

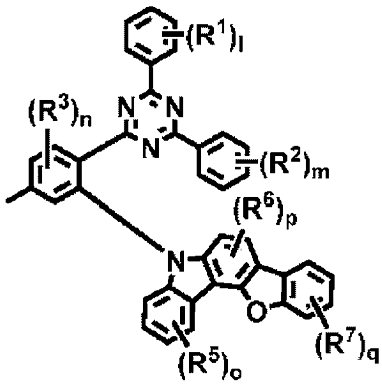

(6)

.

9. An organic light-emitting element comprising: a first electrode and a second electrode; and an organic compound layer disposed between the first electrode and the second electrode,
wherein at least one layer of the organic compound layer contains the organic compound according to claim 1.

10. The organic light-emitting element according to claim 9, wherein the organic compound layer includes a light-emitting layer, and
the light-emitting layer contains the organic compound.

11. The organic light-emitting element according to claim 10, wherein the light-emitting layer further contains a first compound, and
a lowest excited singlet energy of the organic compound is lower than a lowest excited singlet energy of the first compound.

12. The organic light-emitting element according to claim 10, wherein the light-emitting layer further contains a third compound, and
a lowest excited singlet energy of the third compound is lower than a lowest excited singlet energy of the organic compound.

13. The organic light-emitting element according to claim 12, wherein the third compound is a fluorescent material.

14. The organic light-emitting element according to claim 10, wherein in the light-emitting layer, a concentration of the organic compound is 0.01 mass% or more and 99 mass% or less relative to the whole light-emitting layer.

15. The organic light-emitting element according to claim 10, wherein in the light-emitting layer, a concentration of the organic compound is 10 mass% or more and 50 mass% or less relative to the whole light-emitting layer.

16. A display apparatus comprising a plurality of pixels, wherein at least one of the plurality of pixels includes the organic light-emitting element according to any one of claims 9 to 15 and a transistor connected to the organic light-emitting element.

17. A photoelectric conversion apparatus comprising: an image pickup element configured to receive light; and a display unit configured to display an image captured by the image pickup element,
wherein the display unit includes the organic light-emitting element according to any one of claims 9 to 15.

18. An image display apparatus comprising: a display unit including the organic light-emitting element according to any one of claims 9 to 15; and a housing provided with the display unit.

19. An electronic apparatus comprising: a display unit including the organic light-emitting element according to any one of claims 9 to 15; a housing provided with the display unit; and a communication unit provided in the housing and configured to communicate with an external unit.

**20.** A wearable device comprising: a display unit including the organic light-emitting element according to any one of claims 9 to 15; an optical system configured to collect light of the display unit; and a controller configured to control display of the display unit.

**21.** A lighting apparatus comprising: a light source including the organic light-emitting element according to any one of claims 9 to 15; and a housing provided with the light source.

**22.** A moving object comprising: a lighting fixture including the organic light-emitting element according to any one of claims 9 to 15; and a body provided with the lighting fixture.

**23.** An image-forming apparatus comprising: a photoreceptor; and an exposure light source configured to expose the photoreceptor,
wherein the exposure light source includes the organic light-emitting element according to any one of claims 9 to 15.

FIG. 1A

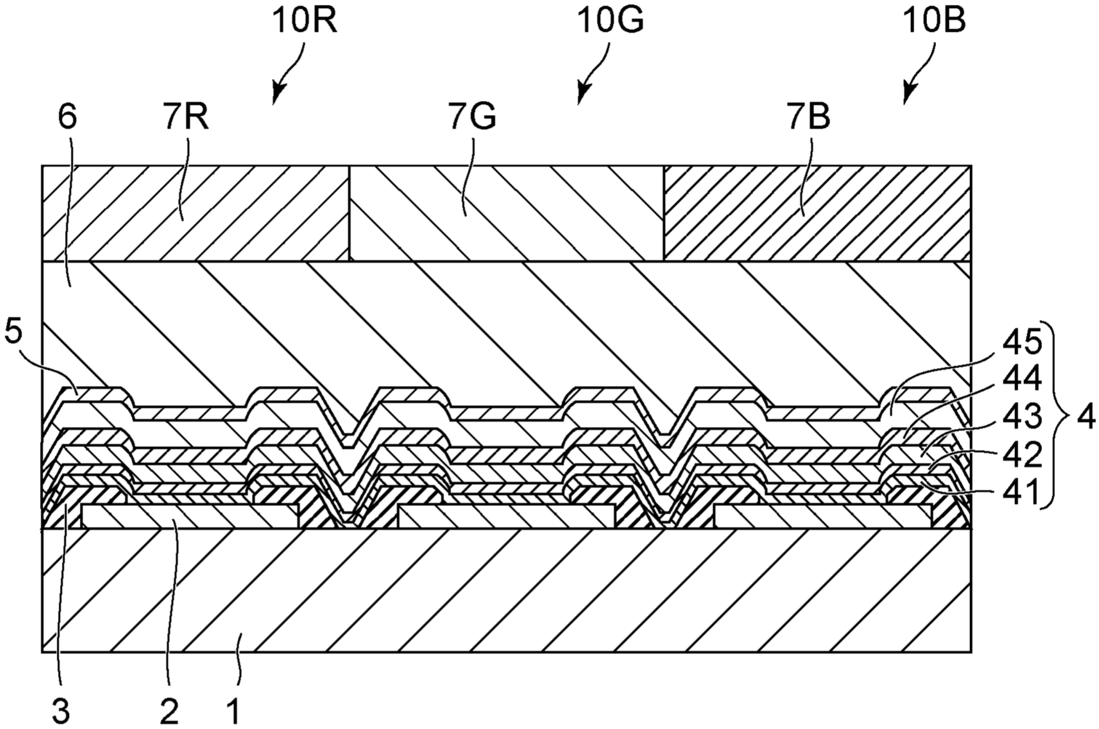
10R
10G
10B
6
7R
7G
7B
5
45
44
43
42
41
4
3
2
1

FIG. 1B

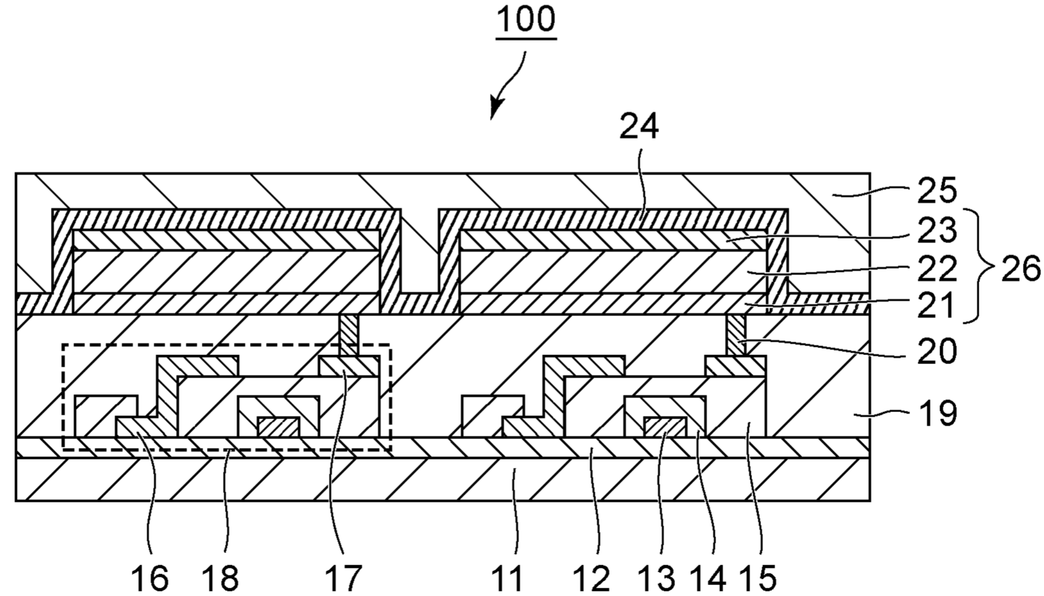
100
24
25
23
22
21
26
20
19
16
18
17
11
12
13
14
15

FIG. 2

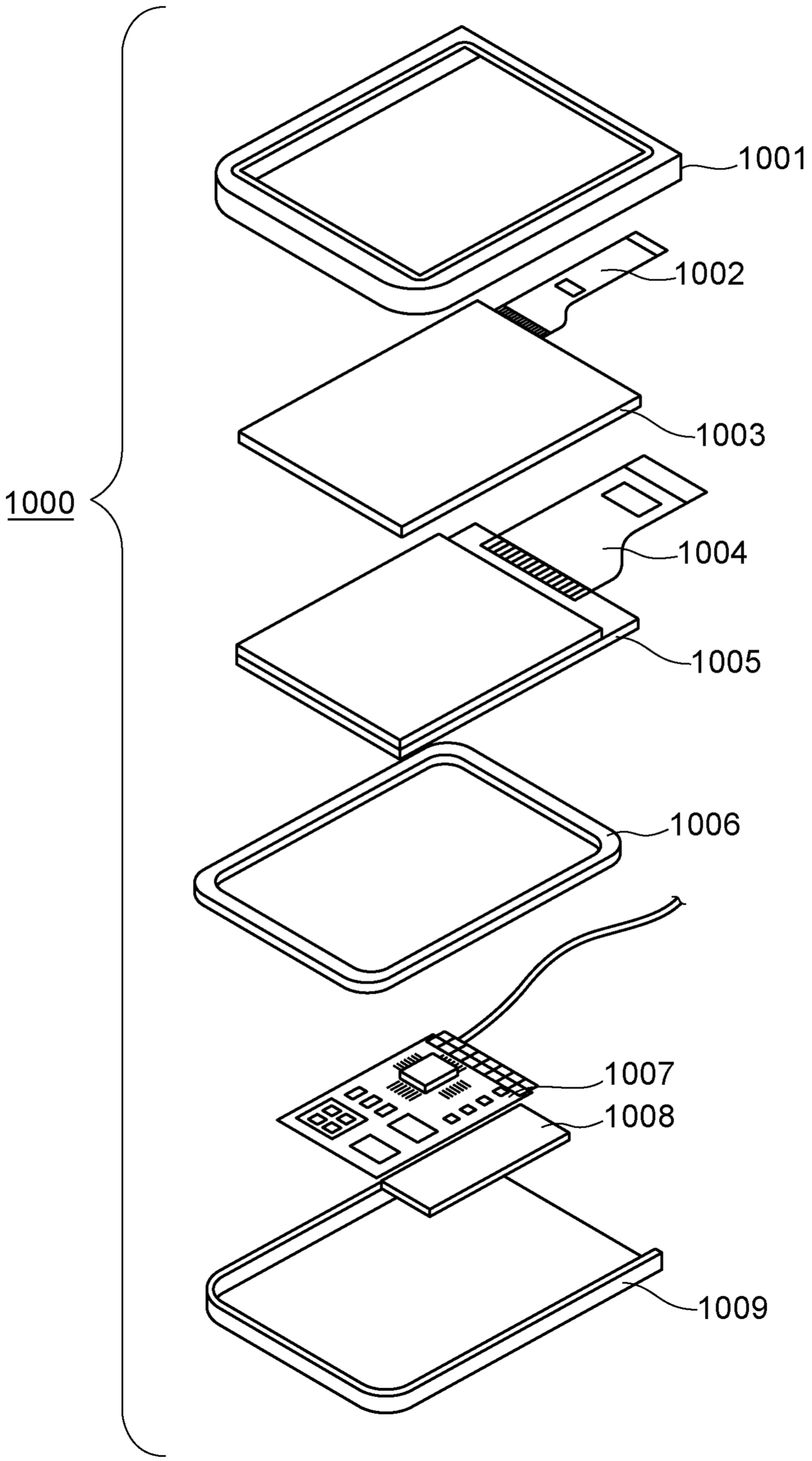

FIG. 3A

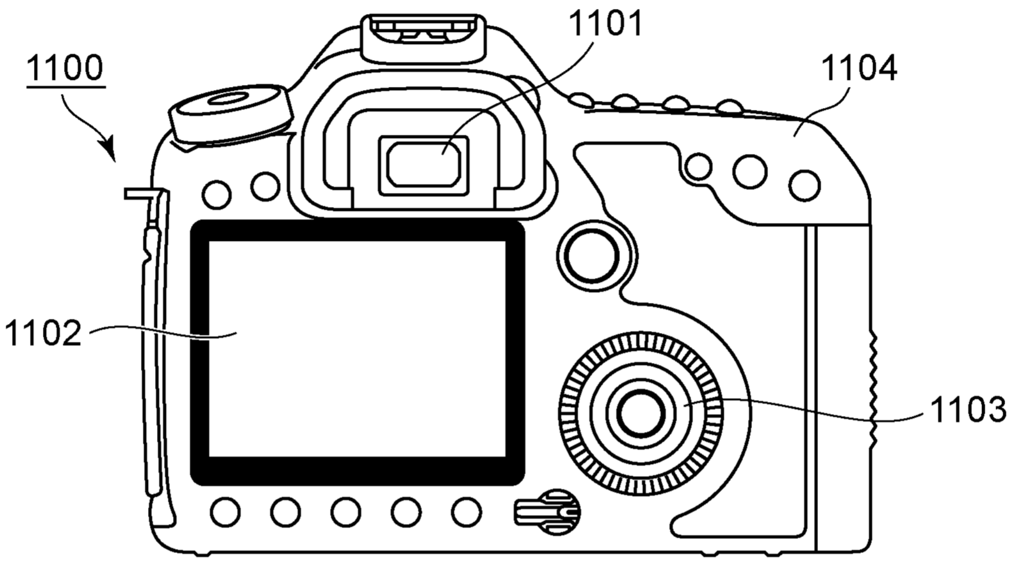


FIG. 3B

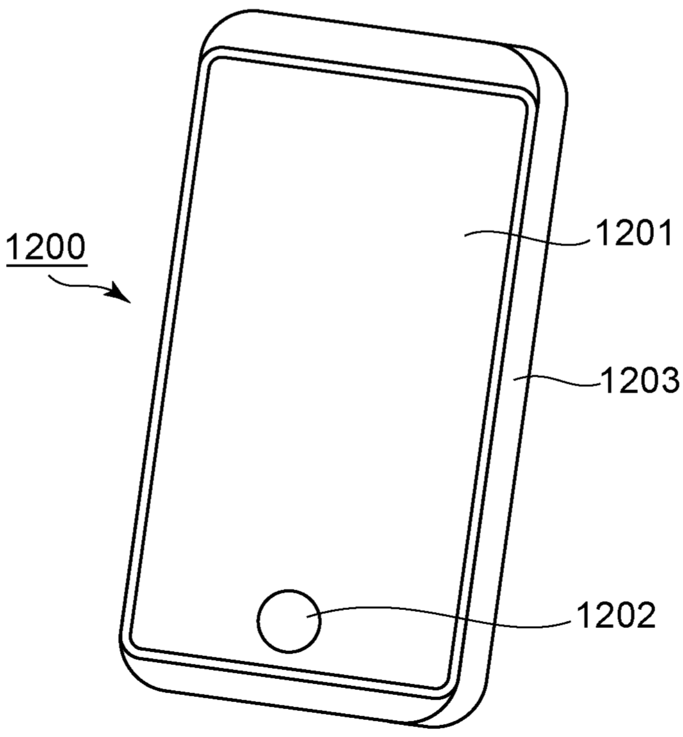

FIG. 4A

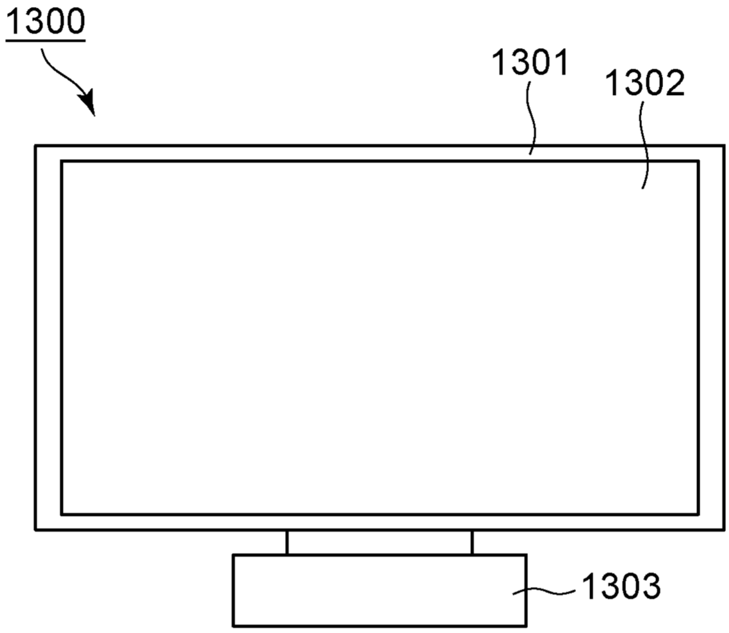


FIG. 4B

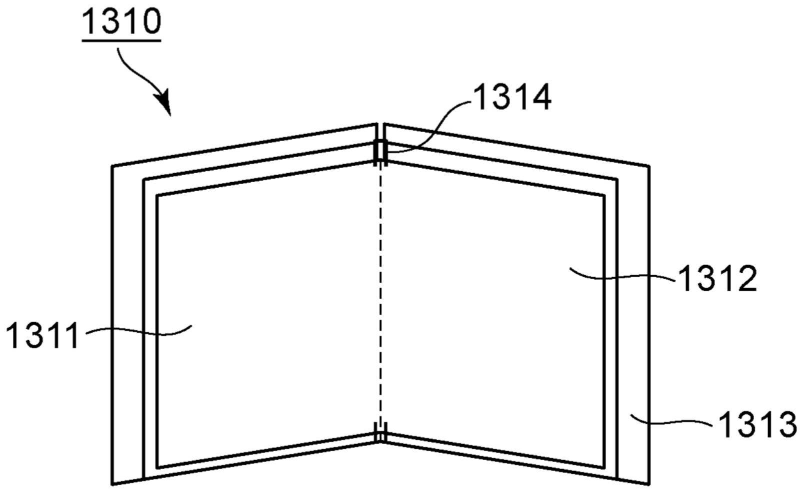

FIG. 5A

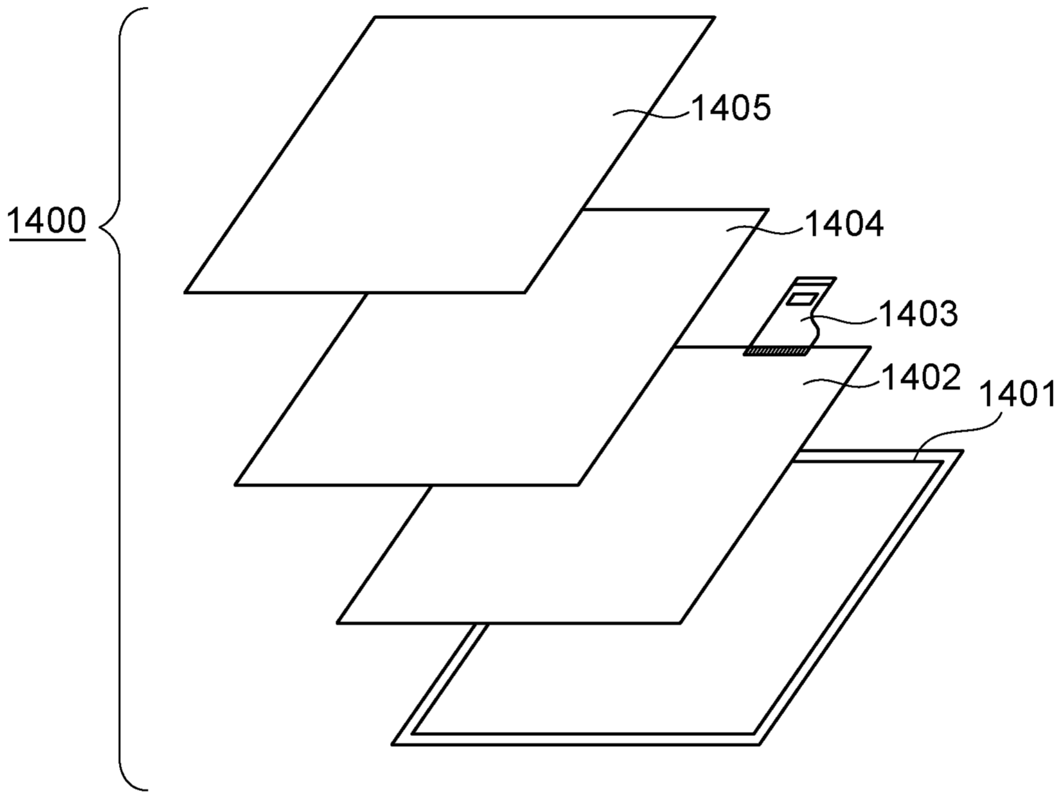


FIG. 5B

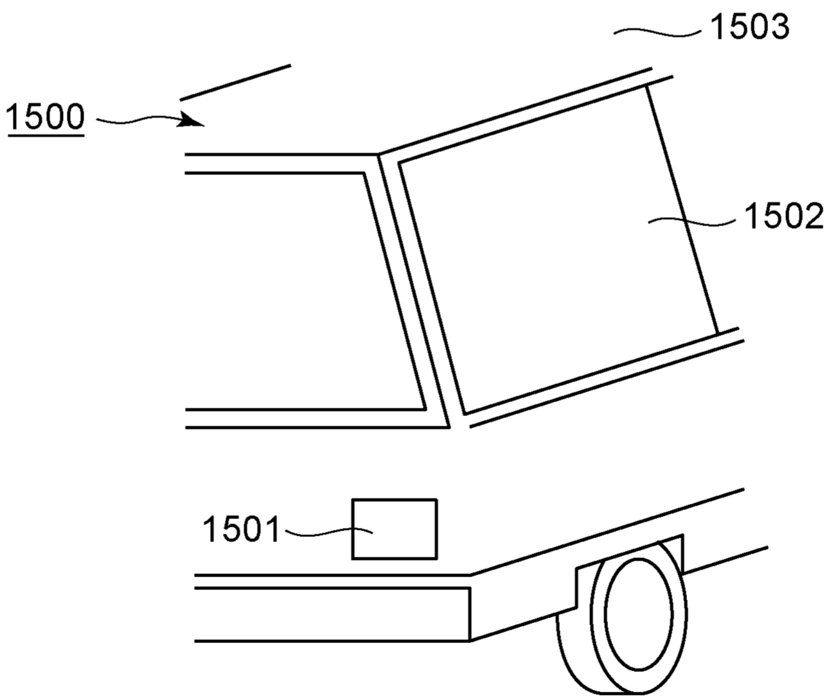

FIG. 6A

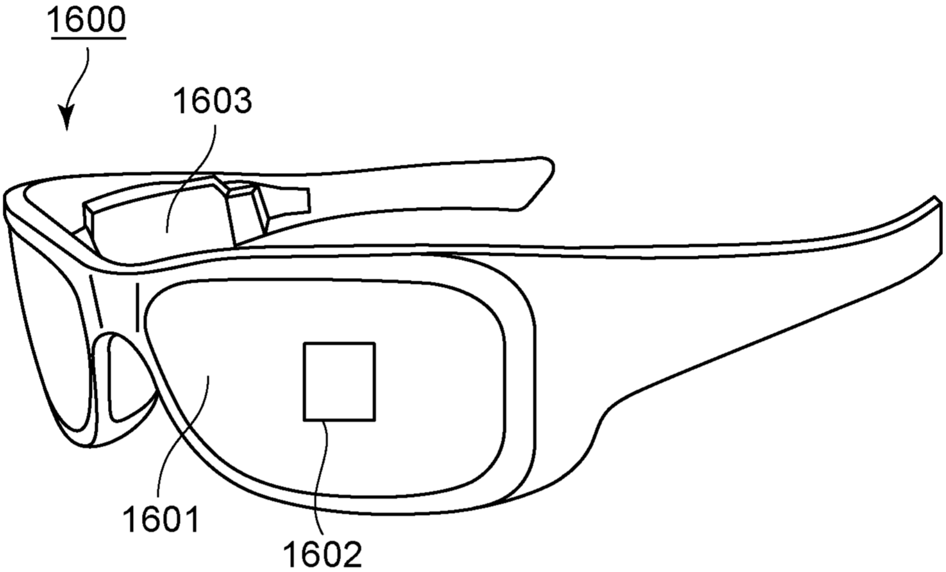


FIG. 6B

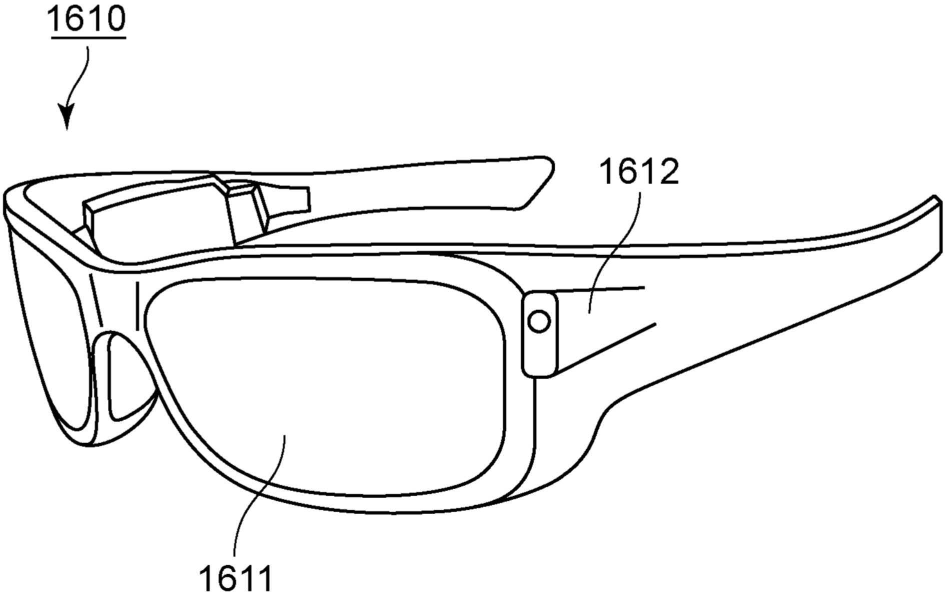

FIG. 7A

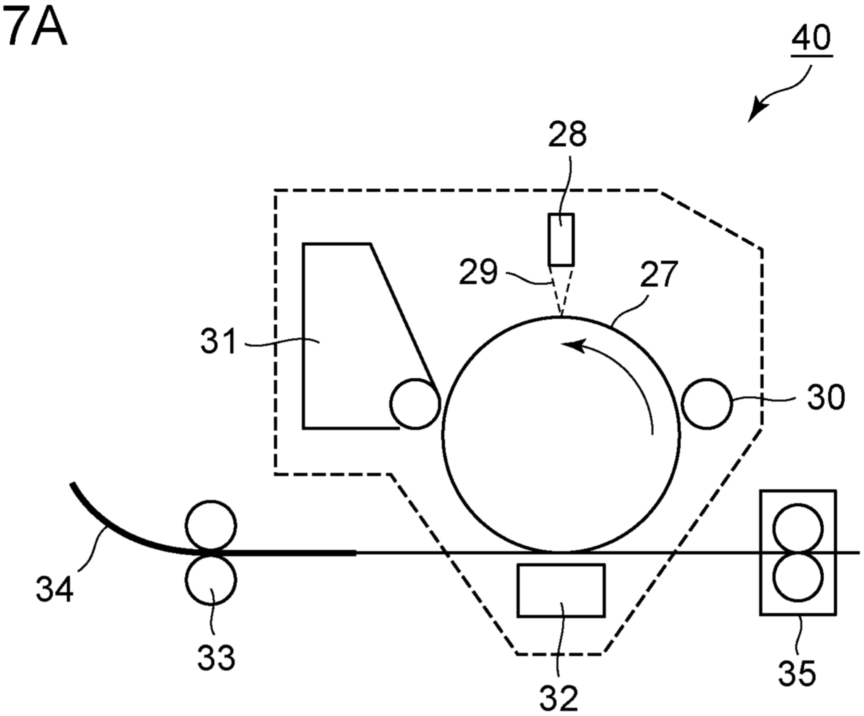


FIG. 7B

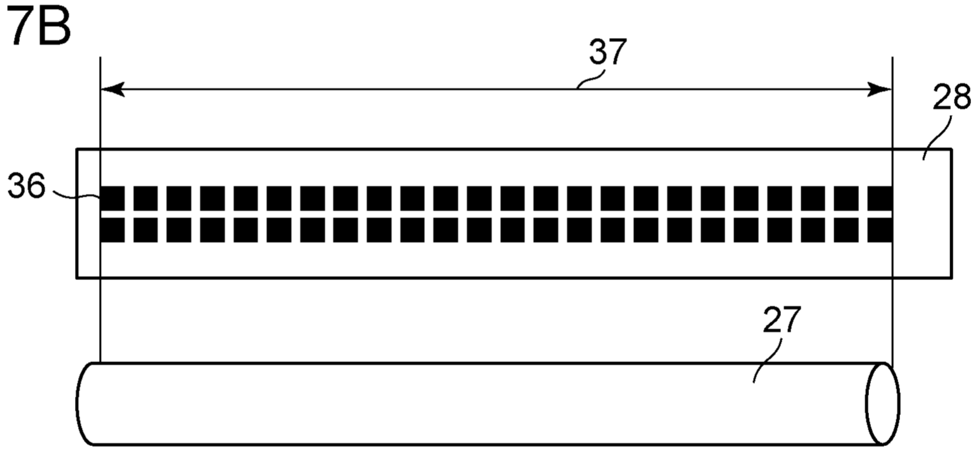


FIG. 7C

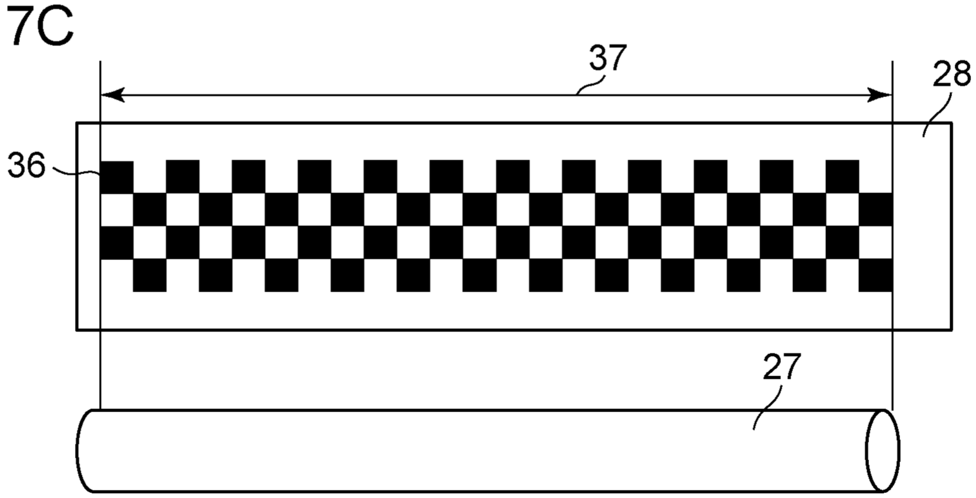

# FIG. 8

| | HOMO ORBITAL DISTRIBUTION | LUMO ORBITAL DISTRIBUTION |
|---|---|---|
| 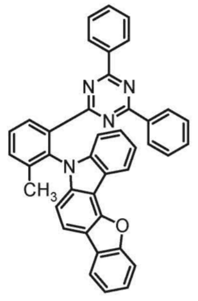 COMPOUND 1 | 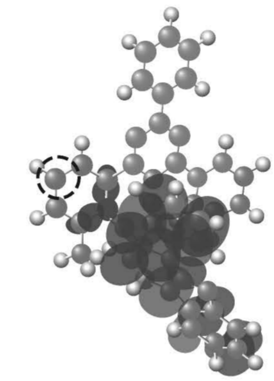 | 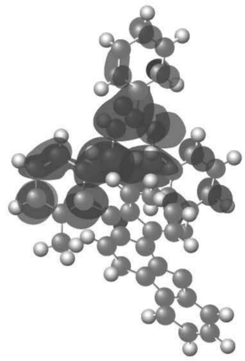 |
| 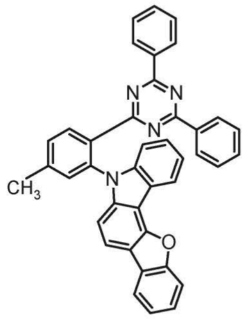 COMPOUND 5 | 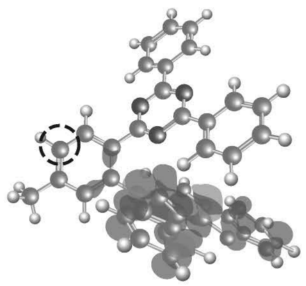 | 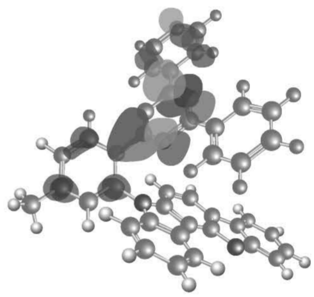 |
| 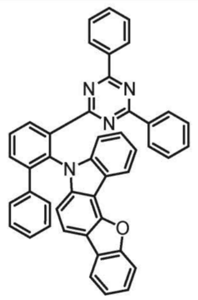 COMPOUND Ref-1 | 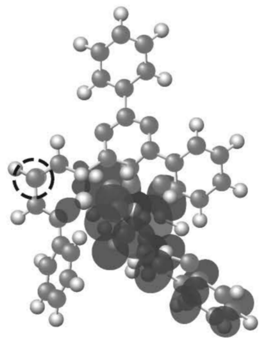 | 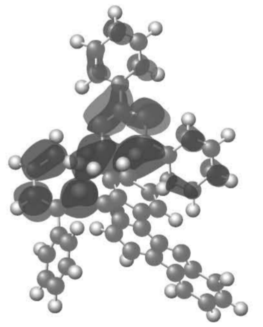 |
| 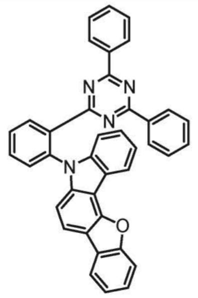 COMPOUND Ref-2 | 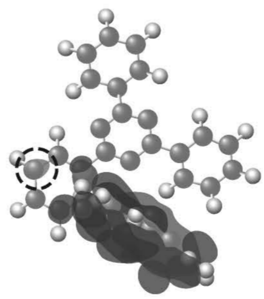 | 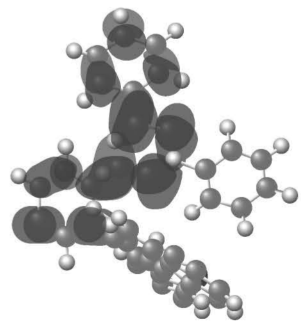 |

# FIG. 9

| | CARBON NUMBER | HOMO ORBITAL DISTRIBUTION | LUMO ORBITAL DISTRIBUTION |
|---|---|---|---|
| 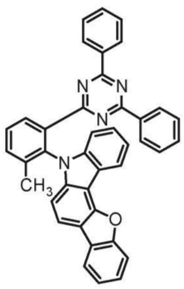 COMPOUND 1 | 1 | 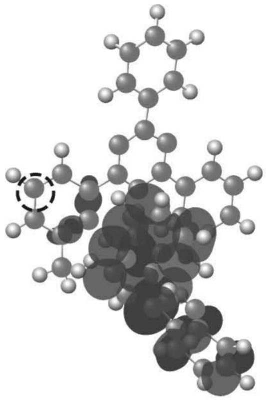 | 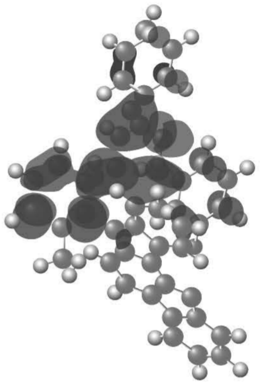 |
| 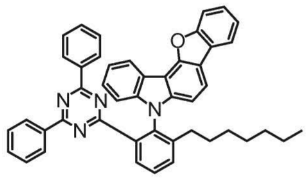 COMPOUND 2 | 7 | 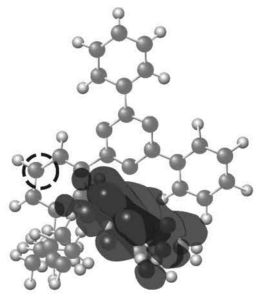 | 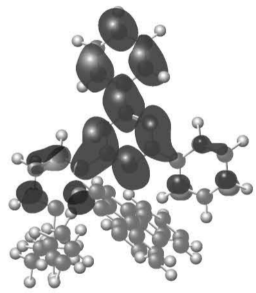 |
| 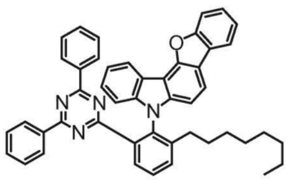 COMPOUND 3 | 8 | 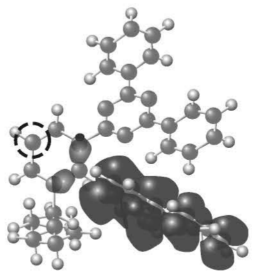 | 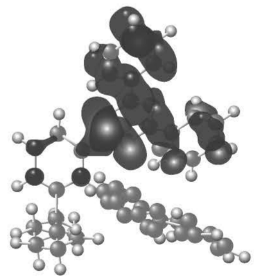 |
| 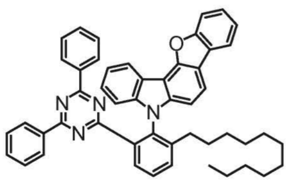 COMPOUND 4 | 11 | 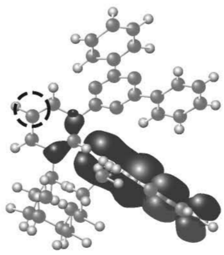 | 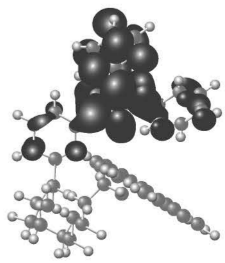 |

# INTERNATIONAL SEARCH REPORT

International application No.
**PCT/JP2024/022486**

**A. CLASSIFICATION OF SUBJECT MATTER**

***C07D 491/048***(2006.01)i; ***C07B 61/00***(2006.01)i; ***C09K 11/06***(2006.01)i; ***H10K 50/12***(2023.01)i; ***H10K 59/12***(2023.01)i; ***H10K 59/60***(2023.01)i; ***H10K 59/65***(2023.01)i; ***H10K 59/95***(2023.01)i; ***H10K 85/60***(2023.01)i; *H10K 101/30*(2023.01)n; *H10K 101/40*(2023.01)n

FI: C07D491/048 CSP; H10K50/12; H10K59/12; H10K59/60; H10K59/95; H10K85/60; C09K11/06 655; C09K11/06; H10K59/65; C07B61/00 300; H10K101:30; H10K101:40

According to International Patent Classification (IPC) or to both national classification and IPC

**B. FIELDS SEARCHED**

Minimum documentation searched (classification system followed by classification symbols)

C07D491/048; C07B61/00; C09K11/06; H10K50/12; H10K59/12; H10K59/60; H10K59/65; H10K59/95; H10K85/60; H10K101/30; H10K101/40

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | EP 3613744 A1 (CYNORA GMBH) 26 February 2020 (2020-02-26) abstract, claims, examples, pp. 39-43, 49-51 | 1, 2, 4, 9-23 |
| P, X | JP 2023-132286 A (KYULUX, INC.) 22 September 2023 (2023-09-22) abstract, claims, examples, p. 52 | 1-23 |
| A | JP 2019-85410 A (CYNORA GMBH) 06 June 2019 (2019-06-06) | 1-23 |
| A | JP 2022-19638 A (ROHM & HAAS ELECTRONIC MATERIALS KOREA LTD.) 27 January 2022 (2022-01-27) | 1-23 |
| A | US 2018/0123049 A1 (INDUSTRY-ACADEMIC COOPERATION FOUNDATION, DANKOOK UNIVERSITY) 03 May 2018 (2018-05-03) | 1-23 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 August 2024** | **27 August 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**
**Information on patent family members**

International application No.
**PCT/JP2024/022486**

| Patent document cited in search report | Publication date (day/month/year) | Patent family member(s) | Publication date (day/month/year) |
|---|---|---|---|
| EP 3613744 A1 | 26 February 2020 | KR 10-2020-0021892 A | |
| JP 2023-132286 A | 22 September 2023 | (Family: none) | |
| JP 2019-85410 A | 06 June 2019 | US 2019/0177303 A1 | |
| | | EP 3483156 A1 | |
| | | KR 10-2019-0052625 A | |
| | | CN 109942551 A | |
| | | TW 202017919 A | |
| JP 2022-19638 A | 27 January 2022 | US 2022/0029109 A1 | |
| | | DE 102021118107 A | |
| | | KR 10-2022-0010427 A | |
| | | CN 113943571 A | |
| US 2018/0123049 A1 | 03 May 2018 | WO 2016/159479 A1 | |
| | | EP 3275968 A1 | |
| | | KR 10-2016-0116297 A | |
| | | CN 107667102 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2022019638 A
- JP 2014092083 A
- JP 2016159479 A
- JP 2023112257 A
- JP 2024088438 A

### Non-patent literature cited in the description

- **M. J. FRISCH et al.** Gaussian 16, Revision C.01. Gaussian, Inc., 2019
- **M. J. FRISCH** ; **G. W. TRUCKS** ; **H. B. SCHLEGEL** ; **G. E. SCUSERIA** ; **M. A. ROBB** ; **J. R. CHEESEMAN** ; **G. SCALMANI** ; **V. BARONE** ; **B. MENNUCCI** ; **G. A. PETERSSON**. Gaussian 09, Revision C.01. Gaussian, Inc., 2010
- *Angewandte Chemie*, 2022, vol. 134, e202205684